# EUROPEAN PATENT SPECIFICATION

(11) **EP 3 443 088 B1**
(45) Date of publication and mention of the grant of the patent: **18.09.2024**
(21) Application number: 17722197.5
(22) Date of filing: 12.04.2017
(51) Int. Cl.: C12N 15/11, C12N 15/10

(54) **GRNA FUSION MOLECULES, GENE EDITING SYSTEMS, AND METHODS OF USE THEREOF**
GRNA-FUSIONSMOLEKÜLE, GENEDITIERUNGSSYSTEME UND VERFAHREN ZUR VERWENDUNG DAVON
MOLÉCULES ARNG DE FUSION, SYSTÈMES D'ÉDITION DE GÈNES ET LEURS PROCÉDÉS D'UTILISATION

(30) Priority: 13.04.2016 US 201662322099 P
(43) Date of publication of application: 20.02.2019
(60) Divisional application: 24193956.0
(73) Proprietor: Editas Medicine, Inc., Cambridge, MA 02141 (US)
(72) Inventor: COTTA-RAMUSINO, Cecilia, Cambridge, MA 02139 (US); SELLECK, William, Jr., Bolton, MA 01740 (US); BOTHMER, Anne, Helen, Cambridge, MA 02138 (US); TILLOTSON, Eric, L., Jamaica Plain, MA 02130 (US)
(74) Representative: Lock, Graham James
(86) International application number: PCT/US2017/027150
(87) International publication number: WO 2017/180711

(56) References cited:
- WO-A1-2014/100473
- WO-A1-2015/134812
- WO-A1-2016/028682
- WO-A1-2016/033246
- WO-A1-2017/040511
- WO-A2-2014/201015
- WO-A2-2015/048577
- WO-A2-2015/191693
- WO-A2-2016/049258
- US-A1- 2016 032 292
- HAO YIN ET AL: "Therapeutic genome editing by combined viral and non-viral delivery of CRISPR system components in vivo", NATURE BIOTECHNOLOGY, vol. 34, no. 3, 1 February 2016 (2016-02-01), US, pages 328 - 333, XP055382113, ISSN: 1087-0156, DOI: 10.1038/nbt.3471
- M. L. SCHWARTZ ET AL: "SapTrap, a Toolkit for High-Throughput CRISPR/Cas9 Gene Modification in Caenorhabditis elegans", GENETICS, vol. 202, no. 4, 29 January 2016 (2016-01-29), US, pages 1277 - 1288, XP055381031, ISSN: 0016-6731, DOI: 10.1534/genetics.115.184275
- MASAKI ENDO ET AL: "Biallelic Gene Targeting in Rice", PLANT PHYSIOLOGY, vol. 170, no. 2, 14 December 2015 (2015-12-14), Rockville, Md, USA, pages 667 - 677, XP055380940, ISSN: 0032-0889, DOI: 10.1104/pp.15.01663
- WILES MICHAEL V ET AL: "CRISPR-Cas9-mediated genome editing and guide RNA design", MAMMALIAN GENOME, SPRINGER NEW YORK LLC, US, vol. 26, no. 9, 20 May 2015 (2015-05-20), pages 501 - 510, XP035600403, ISSN: 0938-8990, [retrieved on 20150520], DOI: 10.1007/S00335-015-9565-Z
- MANUEL KAULICH ET AL: "Combining CRISPR/Cas9 and rAAV Templates for Efficient Gene Editing", NUCLEIC ACID THERAPEUTICS, vol. 25, no. 6, 5 November 2015 (2015-11-05), US, pages 287 - 296, XP055369081, ISSN: 2159-3337, DOI: 10.1089/nat.2015.0545
- ALEXANDRA E. BRINER ET AL: "Guide RNA Functional Modules Direct Cas9 Activity and Orthogonality", MOLECULAR CELL., vol. 56, no. 2, 16 October 2014 (2014-10-16), US, pages 333 - 339, XP055376599, ISSN: 1097-2765, DOI: 10.1016/j.molcel.2014.09.019
- HAL P. BOGERD ET AL: "Specific induction of endogenous viral restriction factors using CRISPR/Cas-derived transcriptional activators", PROCEEDINGS NATIONAL ACADEMY OF SCIENCES PNAS, vol. 112, no. 52, 14 December 2015 (2015-12-14), US, pages E7249 - E7256, XP055381170, ISSN: 0027-8424, DOI: 10.1073/pnas.1516305112

## Description

### FIELD OF THE INVENTION

The invention relates to gRNA fusion molecules and methods and components for increasing editing of a target nucleic acid sequence by gene correction using an exogenous homologous region, and applications thereof.

### BACKGROUND

The CRISPR (Clustered Regularly Interspaced Short Palindromic Repeats)/Cas (CRISPR-associated) system evolved in bacteria and archaea as an adaptive immune system to defend against viral attack. Upon exposure to a virus, short segments of viral DNA are integrated into the CRISPR locus. RNA is transcribed from a portion of the CRISPR locus that includes the viral sequence. That RNA, which contains a sequence complimentary to the viral genome, mediates targeting of a Cas9 protein to the sequence in the viral genome. The Cas9 protein cleaves and thereby silences the viral target.

Recently, the CRISPR/Cas system has attracted widespread interest as a tool for genome editing through the generation of site-specific double strand breaks (DSBs). Current CRISPR/Cas sytems that generate site-specific DSBs can be used to edit DNA in eukaryotic cells, *e.g.,* by producing deletions, insertions and/or changes in nucleotide sequence.

Without wishing to be bound by any theory, it is thought that the mechanism by which an individual DSB is repaired varies depending on whether or not the DNA ends created by the DSB undergo endo- or exonucleolytic processing (also referred to as "end resection" or "processing"). When no end resection takes place, a DSB is generally repaired by a pathway referred to as classical non-homologous end joining (C-NHEJ). C-NHEJ is considered an "error-prone" pathway inasmuch as it leads in some cases to the formation of small insertions and deletions, though it may also result in perfect repair of a DSB without sequence alterations.

In contrast, if end resection does take place, the ends of a DSB may include one or more overhangs (for example, 3' overhangs or 5' overhangs), which can interact with nearby homologous sequences. Again, the mechanism by which the DSB is repaired may vary depending on the extent of processing. When the ends of a DSB undergo relatively limited end resection, the DSB is generally processed by alternative non-homologous end joining (ALT-NHEJ), a class of pathways that includes blunt end-joining (blunt EJ), microhomology mediated end joining (MMEJ), and synthesis dependent micro homology mediated end joining (SD-MMEJ). However, when end resection is extensive, the resulting overhangs may undergo strand invasion of highly homologous sequences (which can be endogenous sequences, for instance from a sister chromatid, or heterologous sequences from an exogenous template), followed by repair of the DSB by a homology-dependent recombination (HDR) pathway.

WO 2015/191693 A2 discloses a fusion gRNA molecule comprising gRNA and a template nucleic acid in which the template nucleic acid is covalently or non-covalently bound to the gRNA.

WO 2016/049258 A2 discloses gRNAs capable of hybridzing to a target sequence in a cell wherein the gRNAs comprise hairpins at their 3' end.

While a cell could, in theory, repair DNA breaks via any of a number of DNA damage repair pathways, in certain circumstances it is useful or desirable to manipulate the local environment in which a DSB is formed in order to drive a particular mode of repair. For instance, the addition of an exogenous homologous DNA sequence (also referred to as a "donor template" or a "template nucleic acid") to a CRISPR/Cas system may tend to drive repair of DSBs through HDR-based gene correction. However, gene correction strategies that rely on exogenous donor templates are complicated by the potential for interactions between the donor template, the Cas9 and the guide RNA. At the same time, because the donor template is not a naturally occurring part of the CRISPR/Cas complex, it may only be present and accessible at a fraction of the DSBs formed by the CRISPR/Cas system, and the desired gene correction may only occur in a fraction of instances. Accordingly, there remains
a need to improve the efficiency of gene correction-mediated modification in order to broaden the applicability and efficiency of genome editing by CRISPR/Cas systems.

### SUMMARY

This disclosure provides systems, methods and compositions that facilitate gene correction by reconciling the need to localize the donor template at DSBs with the need to prevent interactions between the donor template and the guide RNA or the Cas9. In the various aspects of the disclosure, one or more gRNA fusion molecules comprising a gRNA molecule linked to a template nucleic acid sequence are utilized to increase the frequency and efficiency of DNA repair of DSBs using gene correction. The gRNA fusion molecules of the invention comprise gRNA molecules linked covalently to donor template nucleic acids. While not wishing to be bound by theory, it is believed that gRNA fusions that incorporate sequences that form hairpins, stem-loops or other semi-rigid structures between the 3' end of a TRACR domain of a gRNA and the 5' end of a template nucleic acid reduce, minimize, or even eliminate the potential of the template nucleic acid to interfere with Cas9 activity, when the gRNA fusion molecule is complexed with Cas9, while at the same time ensuring that the template nucleic acid is available to participate in HDR, thereby improving the efficiency of gene correction. In some cases, the efficiency of DNA repair via gene correction pathways may be enhanced (e.g., doubled) when the donor template is linked to the gRNA molecule, as compared to the un-linked molecule. Again, without wishing to be bound by any theory, it is also believed that by linking the donor template to the gRNA, the potential for degradation of the donor template (e.g., during trafficking into the nucleus) is reduced and nuclear localization of the template is improved.

The invention is set out in the appended set of claims.

Headings, including numeric and alphabetical headings and subheadings, are for organization and presentation and are not intended to be limiting.

Other features and advantages of the invention will be apparent from the detailed description, drawings, and from the claims.

### BRIEF DESCRIPTION OF THE DRAWINGS

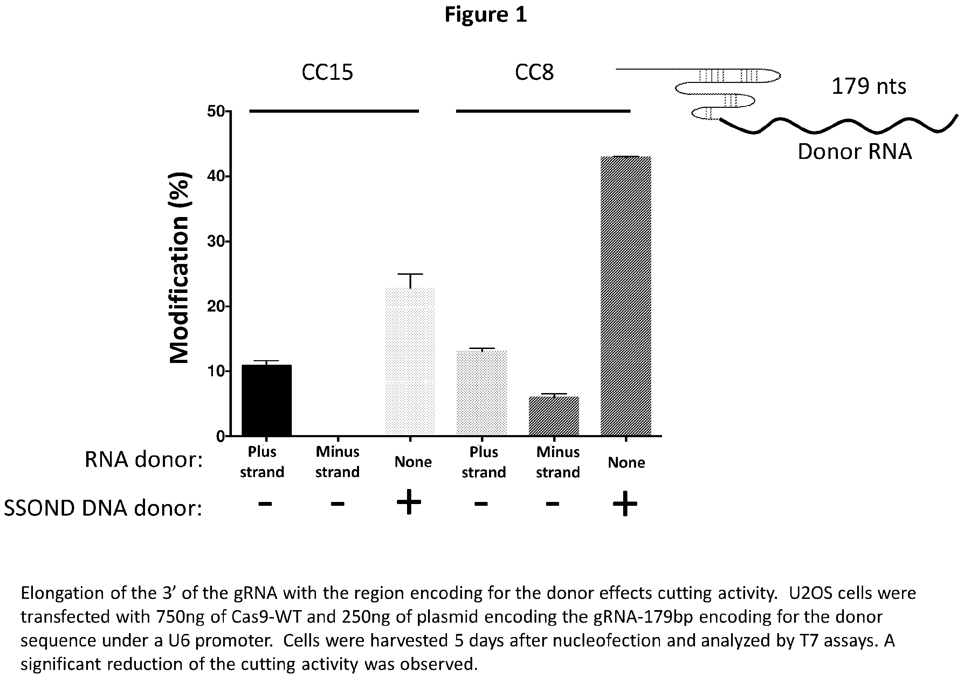

**Fig. 1** depicts the overall modification frequency at the *HBB* locus after WT (wild type) *S. pyogenes* Cas9-induced DNA lesions were induced in U2OS cells using either an elongated gRNA comprising gRNA-15 fused to a plus strand 179 nt donor template (CC15 - Plus strand), gRNA-15 fused to a minus strand 179 nt donor template (CC15 - Minus strand), both in the absence of single-stranded deoxynucleotide (ssODN) donor template, or gRNA-15 in the presence of a 179 nt ssODN donor template; or an elongated gRNA comprising gRNA-8 fused to a plus strand 179 nt donor template (CC8 - Plus strand), gRNA-8 fused to a minus strand 179 nt donor template (CC8 - Minus strand), both in the absence of single-stranded deoxynucleotide (ssODN) donor template, or gRNA-8 in the presence of a 179 nt ssODN donor template.
**Fig. 2** depicts the overall cutting efficiency at the *HBB* locus after WT Cas9-induced DNA lesions were induced in U2OS cells using gRNA-8 modified to incorporate MS2 hairpin sequences at distinct positions and with different sequences in the gRNA molecule.
**Fig. 3** depicts the overall modification frequency at the *HBB* locus after WT Cas9-induced DNA lesions were induced in U2OS cells using either (a) gRNA-8 in the presence of a minus strand 179 nt ssODN donor template, (b) gRNA-8 fused to a minus strand 129 nt donor template (GB47), (c) gRNA-8 fused to two MS2 hairpin sequences followed by a plus strand 179 nt donor template (GB55), (d) gRNA-8 fused to two MS2 hairpin sequences followed by a minus strand 179 nt donor template (GB56), or (d) gRNA-8 fused to two MS2 hairpin sequences followed by a minus strand 129 nt donor template (GB58).

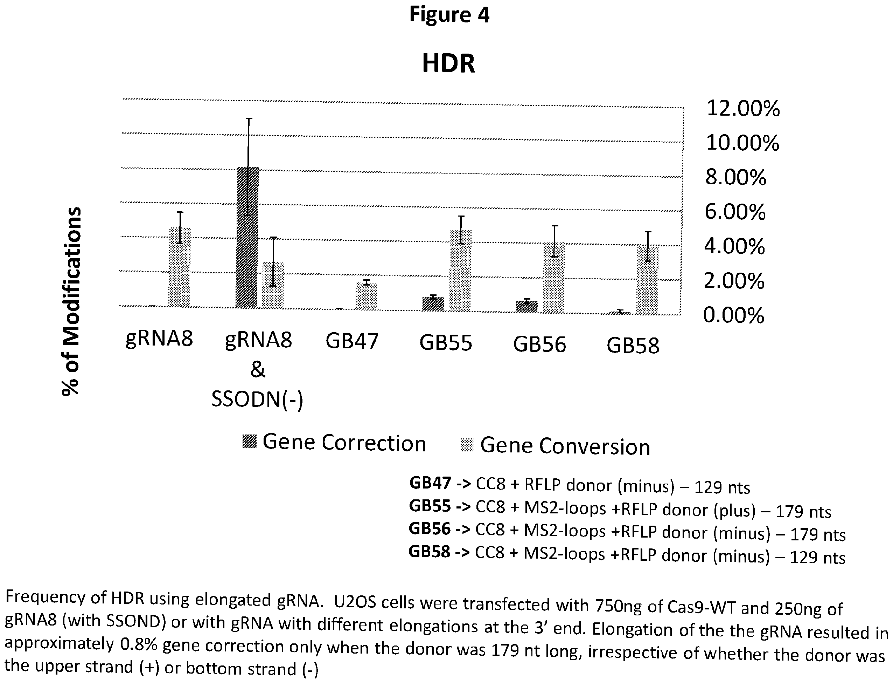

**Fig. 4** depicts the frequency of gene correction and gene conversion events at the *HBB* locus after WT Cas9-induced DNA lesions were induced in U2OS cells using either (a) gRNA-8 in the absence of ssODN donor template (gRNA8), (b) gRNA-8 in the presence of a minus strand 179 nt ssODN donor template (gRNA8 & SSODN(-)), (c) gRNA-8 fused to a minus strand 129 nt donor template (GB47), (d) gRNA-8 fused to two MS2 hairpin sequences followed by a plus strand 179 nt donor template (GB55), (e) gRNA-8 fused to two MS2 hairpin sequences followed by a minus strand 179 nt donor template (GB56), or (f) gRNA-8 fused to two MS2 hairpin sequences followed by a minus strand 129 nt donor template (GB58).
**Fig. 5A** depicts the analysis of ligation efficiency by denaturing polyacrylamide gel electrophoresis of a DNA splint ligation reaction using T4 DNA ligase to covalently linked a 179 nt ssDNA template to a 100mer gRNA.
**Fig. 5B** depicts a differential scanning fluorimetry shift assay after complexing WT SpCas9 with a 100mer gRNA covalently linked to a 179 nt ssDNA template at a 1:1 molar ratio. The melting curves for SpCas9 alone (Apo SpCas9), SpCas9 with non-covalently linked 100mer gRNA in the absence of ssDNA template (gRNA RNP), SpCas9 with non-covalently linked 100mer gRNA in the presence of ssDNA template (gRNA RNP + ssDNA), and SpCas9 with 100mer gRNA covalently linked to a 179 nt ssDNA template (elongated gRNA RNP), are shown.
**Fig. 6A** depicts the analysis of ligation efficiency by denaturing polyacrylamide gel electrophoresis of a RNA splint ligation reaction using T4 DNA ligase to covalently linked a 179 nt ssDNA template to a 100mer gRNA.
**Fig. 6B** depicts the analysis of ligation efficiency by denaturing polyacrylamide gel electrophoresis of a DNA splint ligation reaction using T4 DNA ligase to covalently linked a 179 nt ssDNA template to a 90mer hybrid gRNA.
**Fig. 7A** depicts the analysis of ligation efficiency by denaturing polyacrylamide gel electrophoresis of a DNA splint ligation reaction using T4 DNA ligase to covalently linked a 179 nt ssDNA template to a 202mer gRNA with two MS2 hairpin sequences.

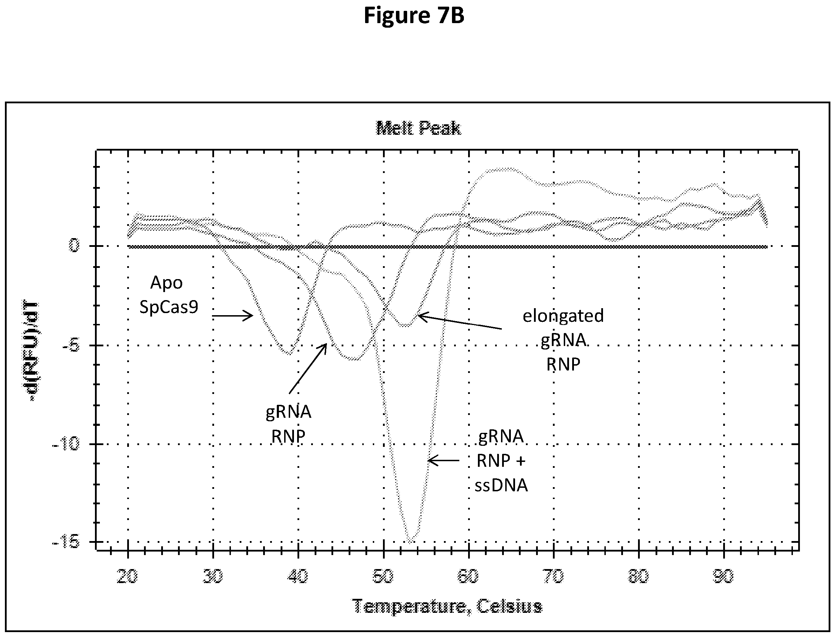

**Fig. 7B** depicts a differential scanning fluorimetry shift assay after complexing WT S. *pyogenes* (SpCas9) with a 202mer gRNA with two MS2 hairpin sequences covalently linked to a 179 nt ssDNA template at a 1:1 molar ratio. The melting curves for SpCas9 alone (Apo SpCas9), SpCas9 with non-covalently linked 202mer gRNA with two MS2 hairpin sequences in the absence of ssDNA template (gRNA RNP), SpCas9 with non-covalently linked 202mer gRNA with two MS2 hairpin sequences in the presence of ssDNA template (gRNA RNP + ssDNA), and SpCas9 with 202mer gRNA with two MS2 hairpin sequences covalently linked to a 179 nt ssDNA template (elongated gRNA RNP), are shown.
**Fig. 8** depicts the analysis of ligation efficiency by denaturing polyacrylamide gel electrophoresis of a DNA splint ligation reaction using T4 RNA ligase 2 to covalently linked a 179 nt ssDNA template to a 100mer gRNA.
**Fig. 9A** depicts the analysis of ligation efficiency by denaturing polyacrylamide gel electrophoresis of an adenylated ligation reaction using T4 RNA ligase 2, truncated K227Q to covalently linked a 179 nt ssDNA template to a 100mer gRNA.
**Fig. 9B** depicts the analysis of ligation efficiency by denaturing polyacrylamide gel electrophoresis of an adenylated ligation reaction using T4 RNA ligase 2, truncated K227Q to covalently linked a 179 nt ssDNA template to a 202mer gRNA with two MS2 hairpin sequences.

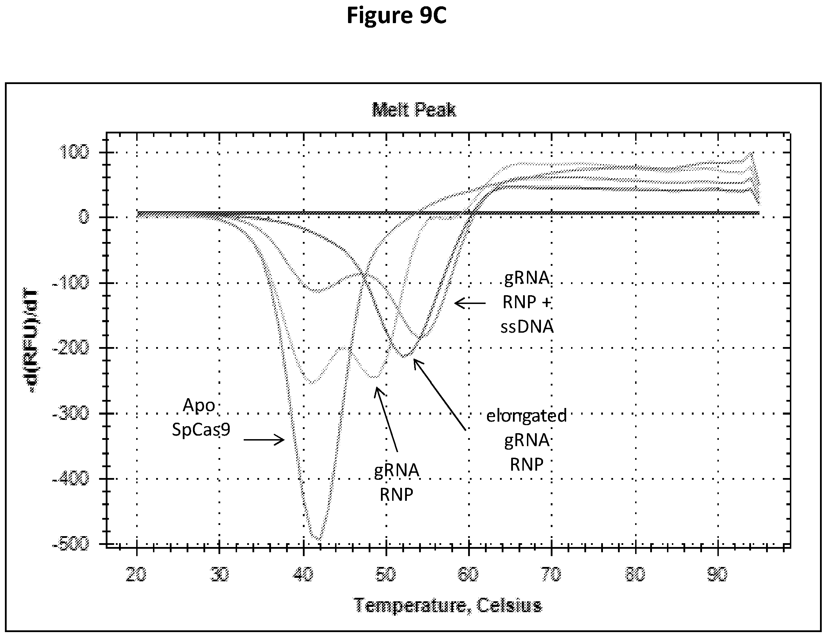

**Fig. 9C** depicts a differential scanning fluorimetry shift assay after complexing WT S. *pyogenes* (SpCas9) with a 202mer gRNA with two MS2 hairpin sequences covalently linked to a 179 nt ssDNA template at a 1:1 molar ratio. The melting curves for SpCas9 alone (Apo SpCas9), SpCas9 with non-covalently linked 202mer gRNA with two MS2 hairpin sequences in the absence of ssDNA template (gRNA RNP), SpCas9 with non-covalently linked 202mer gRNA with two MS2 hairpin sequences in the presence of ssDNA template (gRNA RNP + ssDNA), and SpCas9 with 202mer gRNA with two MS2 hairpin sequences covalently linked to a 179 nt ssDNA template (elongated gRNA RNP), are shown.
**Fig. 10A** depicts the analysis of hybridization efficiency of a 90 nt hybrid gRNA to a 179 nt ssDNA donor template via an annealed 40 nt DNA splint using non-denaturing polyacrylamide gel electrophoresis.
**Fig. 10B** depicts the analysis of the isolated and purified 90 nt hybrid gRNA hybridized to a 179 nt ssDNA donor template via an annealed 40 nt DNA splint following purification by electoelution from a non-denaturing polyacrylamide gel using the Elutrap^{®} electroelution system.
**Fig. 10C** depicts the analysis of the composition of isolated and purified 90 nt hybrid gRNA hybridized to a 179 nt ssDNA donor template via an annealed 40 nt DNA splint by denaturing polyacrylamide gel electrophoresis.

### DETAILED DESCRIPTION

In order that the invention is understood, certain terms are herein defined.

### Definitions

An "adaptor molecule" or "adaptor," as that term is used herein, refers to an entity which, by virtue of its specific affinity for a binding partner, mediates the association of a gRNA with a template nucleic acid. An adaptor molecule coupled to a gRNA can covalently or non-covalently associate with a template nucleic acid directly, or by specific covalent or non-covalent association with a second adaptor coupled to the template nucleic acid. Similarly, an adaptor molecule coupled to a template nucleic acid can covalently or non-covalently associate with a gRNA directly, or by specific covalent or non-covalent association with an adaptor coupled to the gRNA.

"Alt-HDR" or "alternative HDR," or alternative homology-directed repair, as used herein, refers to the process of repairing DNA damage using a homologous nucleic acid (*e.g.,* an endogenous homologous sequence, *e.g.,* a sister chromatid, or an exogenous nucleic acid, *e.g.,* a template nucleic acid). Alt-HDR is distinct from canonical HDR in that the process utilizes different pathways from canonical HDR, and can be inhibited by the canonical HDR mediators, RAD51 and BRCA2. Also, alt-HDR uses a single-stranded or nicked homologous nucleic acid for repair of the break.

"ALT-NHEJ" or "alternative NHEJ", or alternative non-homologous end joining, as used herein, is a type of alternative end joining repair process, and utilizes a different pathway than that of canonical NHEJ. In alternative NHEJ, a small degree of resection occurs at the break ends on both sides of the break to reveal single-stranded overhangs. Ligation or annealing of the overhangs results in the deletion of sequence. ALT-NHEJ is a category that includes microhomology-mediated end joining (MMEJ), blunt end joining (EJ), and synthesis-dependent microhomology-mediated end joining (SD-MMEJ). In MMEJ, microhomologies, or short spans of homologous sequences, *e.g.,* 5 nucleotides or more, on the single-strand are aligned to guide repair, and leads to the deletion of sequence between the microhomologies.

"Amino acids" as used herein encompasses the canonical amino acids as well as analogs thereof. "Canonical HDR," or canonical homology-directed repair, as used herein, refers to the process of repairing DNA damage using a homologous nucleic acid (*e.g.,* an endogenous homologous sequence, *e.g.,* a sister chromatid, or an exogenous nucleic acid, *e.g.,* a template nucleic acid). Canonical HDR typically acts when there has been significant resection at the double-strand break, forming at least one single stranded portion of DNA. In a normal cell, HDR typically involves a series of steps such as recognition of the break, stabilization of the break, resection, stabilization of single stranded DNA, formation of a DNA crossover intermediate, resolution of the crossover intermediate, and ligation. The process requires RAD51 and BRCA2, and the homologous nucleic acid is typically double-stranded.

"Canonical NHEJ", or canonical non-homologous end joining, as used herein, refers to the process of repairing double-strand breaks in which the break ends are directly ligated. This process does not require a homologous nucleic acid to guide the repair, and can result in deletion or insertion of one or more nucleotides. This process requires the Ku heterodimer (Ku70/Ku80), the catalytic subunit of DNA-PK (DN-PKcs), and/or DNA ligase XRCC4/LIG4. Unless indicated otherwise, the term "HDR" as used herein encompasses canonical HDR and alt-HDR.

A "Cas9 molecule," as used herein, refers to a Cas9 polypeptide or a nucleic acid encoding a Cas9 polypeptide. A "Cas9 polypeptide" is a polypeptide that can interact with a gRNA molecule and, in concert with the gRNA molecule, localize to a site comprising a target domain and, in certain embodiments, a PAM sequence. Cas9 molecules include both naturally occurring Cas9 molecules and Cas9 molecules and engineered, altered, or modified Cas9 molecules or Cas9 polypeptides that differ, *e.g.,* by at least one amino acid residue, from a reference sequence, *e.g.,* the most similar naturally occurring Cas9 molecule, including without limitation split Cas9s and/or inducible Cas9s. (The terms altered, engineered or modified, as used in this context, refer merely to a difference from a reference or naturally occurring sequence, and impose no specific process or origin limitations.) A Cas9 molecule may be a Cas9 polypeptide or a nucleic acid encoding a Cas9 polypeptide. A Cas9 molecule may be a nuclease (an enzyme that cleaves both strands of a double-stranded nucleic acid), a nickase (an enzyme that cleaves one strand of a double-stranded nucleic acid), or an enzymatically inactive (or dead) Cas9 molecule. A Cas9 molecule having nuclease or nickase activity is referred to as an "enzymatically active Cas9 molecule" (an "eaCas9" molecule). A Cas9 molecule lacking the ability to cleave target nucleic acid is referred to as an "enzymatically inactive Cas9 molecule" (an "eiCas9" molecule).

In certain embodiments, a Cas9 molecule meets one or both of the following criteria: it has at least 20, 30, 40, 50, 55, 60, 65, 70, 75, 80, 81, 82, 83, 84, 85, 86, 87, 88, 89, 90, 91, 92, 93, 94, 95, 96, 97, 98, 99, or 100% homology with, or it differs by no more than 1, 2, 3, 4, 5, 6, 7, 8, 9, 10, 11, 12, 13, 14, 15, 16, 17, 18, 19, 20, 25, 30, 35, 40, 35, 50, 55, 60, 65, 70, 75, 80, 85, 90, 95, 100, 150, 200, 250, 300, 350 or 400, amino acid residues from, the amino acid sequence of a reference sequences, *e*.*g*., naturally occurring Cas9 molecule.

In certain embodiments, each domain of the Cas9 molecule (*e.g.,* the domains named herein) will, independently have: at least 20, 30, 40, 50, 55, 60, 65, 70, 75, 80, 81, 82, 83, 84, 85, 86, 87, 88, 89, 90, 91, 92, 93, 94, 95, 96, 97, 98, 99, or 100% homology with such a domain described herein. In certain embodiments at least 1, 2, 3, 4, 5, of 6 domains will have, independently, at least 50, 60, 70, 80, 81, 82, 83, 84, 85, 86, 87, 88, 89, 90, 91, 92, 93, 94, 95, 96, 97, 98, 99, or 100% homology with a corresponding domain, while any remaining domains will be absent, or have less homology to their corresponding naturally occurring domains.

In certain embodiments, the Cas9 molecule is *a S. pyogenes* Cas9 variant. In certain embodiments, the Cas9 variant is the EQR variant. In certain embodiments, the Cas9 variant is the VRER variant. In certain embodiments, the eiCas9 molecule is *a S. pyogenes* Cas9 variant. In certain embodiments, the Cas9 variant is the EQR variant. In certain embodiments, the Cas9 variant is the VRER variant.

In certain embodiments, a Cas9 system comprises a Cas9 molecule, *e.g.,* a Cas9 molecule described herein, *e.g.,* the Cas9 EQR variant or the Cas9 VRER variant.

In certain embodiments, the Cas9 molecule is a *S*. *aureus* Cas9 variant. In certain embodiments, the Cas9 variant is the KKH (E782K/N968K/R1015H) variant (see, *e.g.,* Kleinstiver 2015. In certain embodiments, the Cas9 variant is the E782K/K929R/R1015H variant (see, *e*.*g*., Kleinstiver 2015). In certain embodiments, the Cas9 variant is the E782K/K929R/N968K/R1015H variant (see, *e*.*g*., Kleinstiver 2015). In certain embodiments the Cas9 variant comprises one or more mutations in one of the following residues: E782, K929, N968, R1015. In certain embodiments the Cas9 variant comprises one or more of the following mutations: E782K, K929R, N968K, R1015H and R1015Q (see, *e*.*g*., Kleinstiver 2015). In certain embodiments, a Cas9 system comprises a Cas9 molecule, *e.g.,* a Cas9 molecule described herein, *e.g.,* the Cas9 KKH variant.

As used herein, the term "Cas9 system" or "gene editing system" refers to a system capable of altering a target nucleic acid by one of many DNA repair pathways. In certain embodiments, the Cas9 system described herein promotes repair of a target nucleic acid via an HDR pathway. In some embodiments, a Cas9 system comprises a gRNA, *e.g.,* a gRNA fusion molecule as described herein, and a Cas9 molecule. In some embodiments, a Cas9 system further comprises a second gRNA. In some embodiments, the second gRNA is a second gRNA fusion molecule. In yet another embodiment, a Cas9 system comprises a gRNA, a Cas9 molecule, and a second gRNA. In some embodiments, a Cas9 system comprises a gRNA, two Cas9 molecules, and a second gRNA. In some embodiments, a Cas9 system comprises a first gRNA, a second gRNA, a first Cas9 molecule, and a second Cas9 molecule. In exemplary embodiments, a Cas9 system further comprises a template nucleic acid fused to one or more gRNA molecules.

As used herein, the term "cleavage event" refers to a break in a nucleic acid molecule. A cleavage event may be a single-strand cleavage event, or a double-strand cleavage event. A single-strand cleavage event may result in a 5' overhang or a 3' overhang. A double-stranded cleavage event may result in blunt ends, two 5' overhangs, or two 3' overhangs.

A disorder "caused by" a mutation, as used herein, refers to a disorder that is made more likely or severe by the presence of the mutation, compared to a subject that does not have the mutation. The mutation need not be the only cause of a disorder, *i.e.,* the disorder can still be caused by the mutation even if other causes, such as environmental factors or lifestyle factors, contribute causally to the disorder. In embodiments, the disorder is caused by the mutation if the mutation is a medically recognized risk factor for developing the disorder, and/or if a study has found that the mutation contributes causally to development of the disorder.

The term "covalent", as used herein, refers to a form of chemical bonding characterized by the sharing of one or more pairs of electrons between two components, producing a mutual attraction that holds the two components together. The sharing of the one or more pairs of electrons between two components may either be direct (*e.g.,* via reactive groups on the surface the two components, *e*.*g*., a gRNA and a template nucleic acid) or indirect (via a linker molecule).

"Derived from", as used herein, refers to the source or origin of a molecular entity, *e.g.,* a nucleic acid or protein. The source of a molecular entity may be naturally-occurring, recombinant, unpurified, or a purified molecular entity. For example, a polypeptide that is derived from a second polypeptide comprises an amino acid sequence that is identical or substantially similar, *e*.*g*., is more than 50% homologous to, the amino acid sequence of the second protein. The derived molecular entity, *e.g.,* a nucleic acid or protein, can comprise one or more modifications, *e*.*g*., one or more amino acid or nucleotide changes.

"Domain," as used herein, is used to describe a segment of, or a portion of a protein or nucleic acid. Unless otherwise indicated, a domain is not required to have any specific functional property.

As used herein, the terms "template nucleic acid," "exogenous homologous region," "donor nucleic acid," "exogenous template," or "donor template" refer to a nucleic acid sequence which is homologous to at least a portion of a target gene, and which can be used in conjunction with a Cas9 molecule and a gRNA molecule to modify, *e*.*g*., correct, a sequence of the target gene. In some embodiments, the template nucleic acid is a nucleic acid, *e.g.,* DNA or RNA. In one embodiment, the template nucleic acid is single-stranded. In another embodiment, the template nucleic acid is double-stranded. In some embodiments the template nucleic acid is circular nucleic acid. In other embodiments, the template nucleic acid is linear nucleic acid.

As used herein, the term "endogenous" gene, "endogenous" nucleic acid, or "endogenous" homologous region refers to a native gene, nucleic acid, or region of a gene, which is in its natural location in the genome, *e*.*g*., chromosome or plasmid, of a cell. In contrast, the term "exogenous" gene or "exogenous" nucleic acid refers to a gene, nucleic acid, or region of a gene which is not native within a cell, but which is introduced into the cell during the methods of the invention. An exogenous gene or exogenous nucleic acid may be homologous to, or identical to, an endogenous gene or an endogenous nucleic acid.

As used herein, the term "endogenous homologous region" refers to an endogenous template nucleic acid sequence which is homologous to at least a portion of a target gene, and which can be used in conjunction with a Cas9 molecule and a gRNA molecule to modify, *e.g.,* correct, a sequence of the target gene. In one embodiment, the endogenous homologous region is DNA. In another embodiment, the endogenous homologous region is double stranded DNA. In another embodiment, the endogenous homologous region is single stranded DNA. In one embodiment, the endogenous homologous region is at least 70%, 75%, 80%, 81%, 82%, 83%, 84%, 85%, 86%, 875, 885, 89%, 90%, 91%, 92%, 93%, 94%, 95%, 96%, 9%, 98%, or 99% homologous to at least a portion of the target gene.

As used herein, the term "enzymatically inactive Cas9" ("eiCas9") or eiCas9 polypeptide refers to Cas9 molecules having no, or no substantial, cleavage activity. For example, an eiCas9 molecule or eiCas9 polypeptide can lack cleavage activity or have substantially less, *e.g*., less than 20, 10, 5, 1 or 0.1 % of the cleavage activity of a reference Cas9 molecule or eiCas9 polypeptide, as measured by an assay described herein.

In one embodiment, a Cas9 molecule is an eiCas9 molecule comprising one or more differences in a RuvC domain and/or in an HNH domain as compared to a reference Cas9 molecule, and the eiCas9 molecule does not cleave a nucleic acid, or cleaves with significantly less efficiency than does wild type, *e.g.,* when compared with wild type in a cleavage assay, *e.g*., as described herein, cuts with less than 50, 25, 10, or 1% of a reference Cas9 molecule, as measured by an assay described herein. The reference Cas9 molecule can be a naturally occurring unmodified Cas9 molecule, *e.g.,* a naturally occurring Cas9 molecule such as a Cas9 molecule of *S*. *pyogenes, S. thermophilus, S. aureus, C. jejuni* or *N. meningitidis.* In one embodiment, the reference Cas9 molecule is the naturally occurring Cas9 molecule having the closest sequence identity or homology. In one embodiment, the eiCas9 molecule lacks substantial cleavage activity associated with a RuvC domain and cleavage activity associated with an HNH domain.

Whether or not a particular sequence, *e.g.,* a substitution, may affect one or more activity, such as targeting activity, cleavage activity, etc., can be evaluated or predicted, *e.g.,* by evaluating whether the mutation is conservative. In one embodiment, a "non-essential" amino acid residue, as used in the context of a Cas9 molecule, is a residue that can be altered from the wild-type sequence of a Cas9 molecule, *e.g.,* a naturally occurring Cas9 molecule, *e.g.,* an eaCas9 molecule, without abolishing or more preferably, without substantially altering a Cas9 activity (*e*.*g*., cleavage activity), whereas changing an "essential" amino acid residue results in a substantial loss of activity (*e*.*g*., cleavage activity).

Although an enzymatically inactive (eiCas9) Cas9 molecule itself can block transcription when recruited to early regions in the coding sequence, more robust repression can be achieved by fusing a transcriptional repression domain (for example KRAB, SID or ERD) to the Cas9 and recruiting it to the target knockdown position, *e*.*g*., within 1000 bp of sequence 3' of the start codon or within 500 bp of a promoter region 5' of the start codon of a gene. It is likely that targeting DNAseI hypersensitive sites (DHSs) of the promoter may yield more efficient gene repression or activation because these regions are more likely to be accessible to the Cas9 protein and are also more likely to harbor sites for endogenous transcription factors. Especially for gene repression, it is contemplated herein that blocking the binding site of an endogenous transcription factor would aid in downregulating gene expression. In one embodiment, one or more eiCas9 molecules may be used to block binding of one or more endogenous transcription factors. In another embodiment, an eiCas9 molecule can be fused to a chromatin modifying protein. Altering chromatin status can result in decreased expression of the target gene. One or more eiCas9 molecules fused to one or more chromatin modifying proteins may be used to alter chromatin status.

As used herein, "error-prone" repair refers to a DNA repair process that has a higher tendency to introduce mutations into the site being repaired. For instance, alt-NHEJ and SSA are error-prone pathways; C-NHEJ is also error prone because it sometimes leads to the creation of a small degree of alteration of the site (even though in some instances C-NHEJ results in error-free repair); and HR, alt-HR, and SSA in the case of a single-strand oligo donor are not error-prone. As used herein, the term "gRNA molecule" or "gRNA" refers to a guide RNA which is capable of targeting a Cas9 molecule to a target nucleic acid. In one embodiment, the term "gRNA molecule" refers to a guide ribonucleic acid. In another embodiment, the term "gRNA molecule" refers to a nucleic acid encoding a gRNA. In one embodiment, a gRNA molecule is non-naturally occurring. In one embodiment, a gRNA molecule is a synthetic gRNA molecule. In some embodiments, a gRNA molecule contains one or more hairpin sequences incorporated at the 3' end. In such embodiments, the one or more hairpin sequences are added to the 3'end of the core gRNA sequence. Exemplary embodiments of gRNA molecules containing one or more 3' hairpin sequences are shown in **Fig. 4****,** bars E-G. The structure of the "core" gRNA sequence is shown in **Fig. 4****,** bar A.

As used herein, the term "gRNA fusion molecule" or "gRNA fusion" refers to a gRNA molecule that is covalently linked to a template nucleic acid. The 3' end of the gRNA molecule is linked to the 5' end of the template nucleic acid.

"Governing gRNA molecule," as used herein, refers to a gRNA molecule that comprises a targeting domain that is complementary to a target domain on a nucleic acid that comprises a sequence that encodes a component of the CRISPR/Cas system that is introduced into a cell or subject. A governing gRNA does not target an endogenous cell or subject sequence. In an embodiment, a governing gRNA molecule comprises a targeting domain that is complementary with a target sequence on: (a) a nucleic acid that encodes a Cas9 molecule; (b) a nucleic acid that encodes a gRNA molecule which comprises a targeting domain that targets the *HBB* gene (a target gene gRNA); or on more than one nucleic acid that encodes a CRISPR/Cas component, *e*.*g*., both (a) and (b). In an embodiment, a nucleic acid molecule that encodes a CRISPR/Cas component, *e*.*g*., that encodes a Cas9 molecule or a target gene gRNA molecule, comprises more than one target domain that is complementary with a governing gRNA targeting domain. While not wishing to be bound by theory, it is believed that a governing gRNA molecule complexes with a Cas9 molecule and results in Cas9 mediated inactivation of the targeted nucleic acid, *e.g.,* by cleavage or by binding to the nucleic acid, and results in cessation or reduction of the production of a CRISPR/Cas system component. In an embodiment, the Cas9 molecule forms two complexes: a complex comprising a Cas9 molecule with a target gene gRNA molecule, which complex will alter the *HBB* gene; and a complex comprising a Cas9 molecule with a governing gRNA molecule, which complex will act to prevent further production of a CRISPR/Cas system component, *e.g.,* a Cas9 molecule or a target gene gRNA molecule. In an embodiment, a governing gRNA molecule/Cas9 molecule complex binds to or promotes cleavage of a control region sequence, *e.g.,* a promoter, operably linked to a sequence that encodes a Cas9 molecule, a sequence that encodes a transcribed region, an exon, or an intron, for the Cas9 molecule. In an embodiment, a governing gRNA molecule/Cas9 molecule complex binds to or promotes cleavage of a control region sequence, *e.g.,* a promoter, operably linked to a gRNA molecule, or a sequence that encodes the gRNA molecule. In an embodiment, the governing gRNA molecule, *e.g.,* a Cas9-targeting governing gRNA molecule, or a target gene gRNA-targeting governing gRNA molecule, limits the effect of the Cas9 molecule/target gene gRNA molecule complex-mediated gene targeting. In an embodiment, a governing gRNA places temporal, level of expression, or other limits, on activity of the Cas9 molecule/target gene gRNA molecule complex. In an embodiment, a governing gRNA reduces off-target or other unwanted activity. In an embodiment, a governing gRNA molecule inhibits, *e*.*g*., entirely or substantially entirely inhibits, the production of a component of the Cas9 system and thereby limits, or governs, its activity.

"HDR", or homology-directed repair, as used herein, refers to the process of repairing DNA damage using a homologous nucleic acid (*e.g.,* an endogenous nucleic acid, *e.g.,* a sister chromatid, or an exogenous nucleic acid, *e.g.,* a template nucleic acid). HDR typically occurs when there has been significant resection at a double-strand break, forming at least one single stranded portion of DNA. HDR is a category that includes, for example, single-strand annealing (SSA), homologous recombination (HR), single strand template repair (SST-R), and a third, not yet fully characterized alternative homologous recombination (alt-HR) DNA repair pathway. In some embodiments, HDR includes gene conversion and gene correction. In some embodiments, the term HDR does not encompass canonical NHEJ (C-NHEJ). In some embodiments, the term HDR does not encompass alternative non-homologous end joining (Alt-NHEJ) (*e*.*g*., blunt end-joining (blunt EJ), (micro homology mediated end joining (MMEJ), and synthesis dependent microhomology-mediated end joining (SD-MMEJ)).

The terms "homology" or "identity," as used interchangeably herein, refer to sequence identity between two amino acid sequences or two nucleic acid sequences, with identity being a more strict comparison. The phrases "percent identity or homology" and "% identity or homology" refer to the percentage of sequence identity found in a comparison of two or more amino acid sequences or nucleic acid sequences. Two or more sequences can be anywhere from 0-100% identical, or any value there between. Identity can be determined by comparing a position in each sequence that can be aligned for purposes of comparison to a reference sequence. When a position in the compared sequence is occupied by the same nucleotide base or amino acid, then the molecules are identical at that position. A degree of identity of amino acid sequences is a function of the number of identical amino acids at positions shared by the amino acid sequences. A degree of identity between nucleic acid sequences is a function of the number of identical or matching nucleotides at positions shared by the nucleic acid sequences. A degree of homology of amino acid sequences is a function of the number of amino acids at positions shared by the polypeptide sequences.

Calculations of homology or sequence identity between two sequences (the terms are used interchangeably herein) are performed as follows. The sequences are aligned for optimal comparison purposes (*e.g*., gaps can be introduced in one or both of a first and a second amino acid or nucleic acid sequence for optimal alignment and non-homologous sequences can be disregarded for comparison purposes). The optimal alignment is determined as the best score using the GAP program in the GCG software package with a Blossum 62 scoring matrix with a gap penalty of 12, a gap extend penalty of 4, and a frame shift gap penalty of 5. The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position. The percent identity between the two sequences is a function of the number of identical positions shared by the sequences.

"Gene conversion", as used herein, refers to the process of repairing DNA damage by homology directed recombination (HDR) using an endogenous nucleic acid, *e.g.,* a sister chromatid or a plasmid, as a template nucleic acid. Without being bound by theory, in some embodiments, BRCA1, BRCA2 and/or RAD51 are believed to be involved in gene conversion. In some embodiments, the endogenous nucleic acid is a nucleic acid sequence having homology, *e.g.*, significant homology, with a fragment of DNA proximal to the site of the DNA lesion or mutation. In some embodiments, the template is not an exogenous nucleic acid.

"Gene correction", as used herein, refers to the process of repairing DNA damage by homology directed recombination using an exogenous nucleic acid, *e.g.,* a donor template nucleic acid. In some embodiments, the exogenous nucleic acid is single-stranded. In some embodiments, the exogenous nucleic acid is double-stranded. In one embodiment, the donor template nucleic acid is a circular nucleic acid sequence. In another embodiment, the donor template nucleic acid is a linear nucleic acid sequence.

"Homologous recombination" or "HR" refers to a type of HDR DNA-repair which typically acts occurs when there has been significant resection at the double-strand break, forming at least one single stranded portion of DNA. In a normal cell, HR typically involves a series of steps such as recognition of the break, stabilization of the break, resection, stabilization of single stranded DNA, formation of a DNA crossover intermediate, resolution of the crossover intermediate, and ligation. The process requires RAD51 and BRCA2, and the homologous nucleic acid is typically double-stranded. In some embodiments, homologous recombination includes gene conversion and gene correction.

The term "linked" or "linkage" as used herein means an interaction between molecules or parts of molecules. Two molecules that are linked may be covalently linked or non-covalently linked.

The term "linker," "peptide linker" or "polypeptide linker" as used herein means a peptide or polypeptide comprising two or more amino acids residues joined by peptide bonds. Such peptide or polypeptide linkers are well known in the art. Linkers comprise naturally occurring an/or non-naturally occurring peptides or polypeptides.

"Modulator," as used herein, refers to an entity, *e.g.,* a compound, that can alter the activity (*e*.*g*., enzymatic activity, transcriptional activity, or translational activity), amount, distribution, or structure of a subject molecule or genetic sequence. In an embodiment, modulation comprises cleavage, *e*.*g*., breaking of a covalent or non-covalent bond, or the forming of a covalent or non-covalent bond, *e.g.,* the attachment of a moiety, to the subject molecule. In an embodiment, a modulator alters the, three dimensional, secondary, tertiary, or quaternary structure, of a subject molecule. A modulator can increase, decrease, initiate, or eliminate a subject activity.

As used herein, the term "mutation" refers to a change in the sequence of a nucleic acid as compared to a wild-type sequence of the nucleic acid, resulting a variant form of the nucleic acid. A mutation in a nucleic acid may be caused by the alteration of a single base pair in the nucleic acid, or the insertion, deletion, or rearrangement of larger sections of the nucleic acid. A mutation in a gene may result in variants of the protein encoded by the gene which are associated with genetic disorders.

The term "non-covalent bond" refers to a variety of interactions between molecules or parts of molecules that are not covalent in nature, which provide force to hold the molecules or parts of molecules together usually in a specific orientation or conformation. Such non-covalent interactions include *inter alia* ionic bonds, hydrophobic interactions, hydrogen bonds, Van-der-Waals forces, and dipole-dipole bonds.

"Non-homologous end joining" or "NHEJ," as used herein, refers to ligation mediated repair and/or non-template mediated repair including canonical NHEJ (cNHEJ), alternative NHEJ (altNHEJ), microhomology-mediated end joining (MMEJ), single-strand annealing (SSA), and synthesis-dependent microhomology-mediated end joining (SD-MMEJ). Unless indicate otherwise, "NHEJ" as used herein encompasses canonical NHEJ, alt-NHEJ, MMEJ, SSA and SD-MMEJ.

"Polypeptide," as used herein, refers to a polymer of amino acids.

The term "protein", as used herein, is intended to refer to a biomolecule comprised of amino acids arranged in the form of a polypeptide. A protein may be a full-length protein, or a fragment thereof.

As used herein, the term "processing," with respect to overhangs, refers to either the endonucleolytic processing or the exonucleolytic processing of a break in a nucleic acid molecule. In one embodiment, processing of a 5' overhang in a nucleic acid molecule may result in a 3' overhang. In another embodiment, processing of a 3' overhang in a nucleic acid molecule may result in a 5' overhang.

A "reference molecule," as used herein, refers to a molecule to which a modified or candidate molecule is compared. For example, a reference Cas9 molecule refers to a Cas9 molecule to which a modified or candidate Cas9 molecule is compared. The modified or candidate molecule may me compared to the reference molecule on the basis of sequence (*e*.*g*., the modified or candidate may have X% sequence identity or homology with the reference molecule) or activity (*e*.*g.*, the modified or candidate molecule may have X% of the activity of the reference molecule). For example, where the reference molecule is a Cas9 molecule, a modified or candidate may be characterized as having no more than 10% of the nuclease activity of the reference Cas9 molecule. Examples of reference Cas9 molecules include naturally occurring unmodified Cas9 molecules, *e.g.,* a naturally occurring Cas9 molecule from *S. pyogenes, S. aureus, S. thermophilus* or *N. meningitidis.* In certain embodiments, the reference Cas9 molecule is the naturally occurring Cas9 molecule having the closest sequence identity or homology with the modified or candidate Cas9 molecule to which it is being compared. In certain embodiments, the reference Cas9 molecule is a parental molecule having a naturally occurring or known sequence on which a mutation has been made to arrive at the modified or candidate Cas9 molecule.

"Replacement," or "replaced," as used herein with reference to a modification of a molecule does not require a process limitation but merely indicates that the replacement entity is present.

"Resection", as used herein, refers to exonuclease-mediated digestion of one strand of a double-stranded DNA molecule, which results in a single-stranded overhang. Resection may occur, *e.g.,* on one or both sides of a double-stranded break. Resection can be measured by, for instance, extracting genomic DNA, digesting it with an enzyme that selectively degrades dsDNA, and performing quantitative PCR using primers spanning the DSB site, *e.g.,* as described herein.

"SSA" or "Single-strand Annealing", as used herein, refers to the process where RAD52 as opposed to RAD51 in the HR pathways, binds to the single stranded portion of DNA and promotes annealing of the two single stranded DNA segments at repetitive regions. Once RAD52 binds XFP/ERCC1 removes DNA flaps to make the DNA more suitable for ligation.

"SCD target point position," as used herein, refers to a target position in the *HBB* gene, typically a single nucleotide, which, if mutated, can result in a protein having a mutant amino acid and give rise to SCD. In an embodiment, the SCD target position is the target position at which a change can give rise to an E6 mutant protein, *e.g.,* a protein having an E6V substitution.

"Subject," as used herein, may mean either a human or non-human animal. The term includes, but is not limited to, mammals (*e.g.,* humans, other primates, pigs, rodents (*e.g.,* mice and rats or hamsters), rabbits, guinea pigs, cows, horses, cats, dogs, sheep, and goats). In an embodiment, the subject is a human. In another embodiment, the subject is poultry. In another embodiment, the subject is piscine. In certain embodiments, the subject is a human, and in certain of these embodiments the human is an infant, child, young adult, or adult.

As used herein, the terms "target nucleic acid" or "target gene" refer to a nucleic acid which is being targeted for alteration, *e*.*g*., by gene correction, by a Cas9 system described herein. In certain embodiments, a target nucleic acid comprises one gene. In certain embodiments, a target nucleic acid may comprise one or more genes, *e*.*g*., two genes, three genes, four genes, or five genes. In one embodiment, a target nucleic acid may comprise a promoter region, or control region, of a gene. In one embodiment, a target nucleic acid may comprise an intron of a gene. In another embodiment, a target nucleic acid may comprise an exon of a gene. In one embodiment, a target nucleic acid may comprise a coding region of gene. In one embodiment, a target nucleic acid may comprise a non-coding region of a gene.

"Target position" as used herein, refers to a site on a target nucleic acid that is modified by a Cas9 molecule-dependent process. For example, the target position can be modified by a Cas9 molecule-mediated cleavage of the target nucleic acid and template nucleic acid directed modification, *e*.*g*., correction, of the target position. In an embodiment, a target position can be a site between two nucleotides, *e*.*g*., adjacent nucleotides, on the target nucleic acid into which one or more nucleotides is added based on homology with a template nucleic acid. The target position may comprise one or more nucleotides that are altered, *e*.*g*., corrected, based on homology with a template nucleic acid. In another embodiment, the target position may comprise one or more nucleotides that are deleted based on homology with a template nucleic acid. In an embodiment, the target position is within a "target sequence" (*e*.*g*., the sequence to which the gRNA binds). In an embodiment, a target position is upstream or downstream of a target sequence (*e*.*g*., the sequence to which the gRNA binds).

"Target region," "target domain," or "target sequence," as used herein, is a nucleic acid sequence that comprises a target position and at least one nucleotide position outside the target position. In certain embodiments, the target position is flanked by sequences of the target position region, *i.e.,* the target position is disposed in the target position region such that there are target position region sequences both 5' and 3' to the target position. In certain embodiments, the target position region provides sufficient sequences on each side (*i.e.,* 5*'* and 3') of the target position to allow gene correction of the target position, wherein the gene correction uses the template nucleic acid of the gRNA fusion molecule for repair.

A "template nucleic acid," as the term is used herein, refers to a nucleic acid sequence which can be used in conjunction with a Cas9 molecule and a gRNA molecule to alter the structure of a target position. According to the invention, the template nucleic acid is covalently linked to the gRNA. In an embodiment, the target nucleic acid is modified to have the some or all of the sequence of the template nucleic acid, typically at or near cleavage site(s). In an embodiment, the template nucleic acid is single stranded. In an alternate embodiment, the template nucleic acid is double stranded. In an embodiment, the template nucleic acid is DNA, *e.g.,* double stranded DNA. In an alternate embodiment, the template nucleic acid is single stranded DNA. In an embodiment, the template nucleic acid is RNA, *e.g.,* double stranded RNA or single stranded RNA. In an embodiment, the template nucleic acid is encoded on the same vector backbone, *e.g*., AAV genome, plasmid DNA, as the Cas9 and gRNA. In an embodiment, the template nucleic acid is excised from a vector backbone *in vivo, e.g.,* it is flanked by gRNA recognition sequences. In one embodiment, the template DNA is in an ILDV. In one embodiment, the template nucleic acid is an exogenous nucleic acid sequence. In another embodiment, the template nucleic acid sequence is an endogenous nucleic acid sequence, *e*.*g*., an endogenous homologous region. In one embodiment, the template nucleic acid is not an endogenous sequence. In one embodiment, the template nucleic acid is a single stranded oligonucleotide corresponding to a plus strand of a nucleic acid sequence. In another embodiment, the template nucleic acid is a single stranded oligonucleotide corresponding to a minus strand of a nucleic acid sequence.

"Treat," "treating" and "treatment," as used herein, mean the treatment of a disease in a mammal, *e.g.,* in a human, including (a) inhibiting the disease, *i.e.,* arresting or preventing its development or progression; (b) relieving the disease, *i.e.,* causing regression of the disease state; and (c) relieving one or more symptoms of the disease; and (d) curing the disease.

"Prevent," "preventing" and "prevention," as used herein, means the prevention of a disease in a mammal, *e*.*g*., in a human, including (a) avoiding or precluding the disease; (b) affecting the predisposition toward the disease (c) preventing or delaying the onset of at least one symptom of the disease.

A "variant Cas9 molecule," as used herein refers to a Cas9 molecule with at least one modification, *e.g*., a mutation or chemical modification to at least one amino acid residue of the wild-type Cas9 molecule.

"Wild type", as used herein, refers to a gene or polypeptide which has the characteristics, *e*.*g*., the nucleotide or amino acid sequence, of a gene or polypeptide from a naturally-occurring source. The term "wild type" typically includes the most frequent observation of a particular gene or polypeptide in a population of organisms found in nature.

"X" as used herein in the context of an amino acid sequence, refers to any amino acid (*e.g.,* any of the twenty natural amino acids) unless otherwise specified.

### 1. Guide RNA (gRNA) Fusion Molecules

The present invention is based, at least in part, on the discovery that Cas9-mediated gene editing using an exogenous template nucleic acid can proceed with increased efficiency when the gRNA and the template nucleic acid are held in close proximity by covalently linking the gRNA to the template nucleic acid. Without wishing to be bound by theory, it is believed that by contacting a cell, or population of cells, with a gRNA linked to a template nucleic acid, as disclosed herein, the proximity of the Cas9/gRNA complex and the template nucleic acid used by the cell to repair a Cas9-mediated cleavage event is increased, allowing the particular DNA repair pathways, *e*.*g*., HDR, *e.g.,* gene correction, to proceed with enhanced efficiency. Moreover, such methods also decrease the likelihood that the template nucleic acid would bind to and interfere with Cas9 cutting of the DNA.

Accordingly, in exemplary embodiments, the present invention provides compositions for gene editing, which comprise a gRNA molecule covalently linked to a template nucleic acid. In addition, the gRNA and the template nucleic acid may be linked directly, or they may be linked through an adaptor molecule, *e.g.,* an adaptor protein, nucleic acid *(e.g.,* a splint oligonucleotide), or small molecule.

gRNA molecules promote the specific targeting of a gRNA/Cas9 complex to a target nucleic acid for gene editing. gRNA molecules can be selected to contain specific features suitable for particular gene editing applications. Exemplary gRNA molecules are described herein. Any of the gRNA molecules disclosed herein are suitable for generating gRNA fusions in which the gRNA is covalently linked to a template nucleic acid, *e.g.,* an exogenous template nucleic acid. The disclosure of specific gRNA formats provided herein is exemplary, and is not intended to be limiting, as it will be apparent to a skilled artisan that a variety of gRNA molecules known in the art are suitable for linkage to a template nucleic acid, as described herein.

A gRNA molecule of the invention contains one or more hairpin loops at or near the 3' end. A hairpin loop, or stem-loop, structure occurs when two regions of the same strand of a nucleic acid molecule have complementarity, and base-pair to form a double helix "stem" that ends in an unpaired loop. Surprisingly, the addition of hairpin loops at or near the 3'end of a gRNA provide a semi-rigid secondary structure that can serve as a point of attachment for coupling molecules to the gRNA. Accordingly, in the gRNA fusion molecule of the invention, a template nucleic acid is linked to the 3'end of a gRNA molecule that contains one or more 3' hairpin loops. Without wishing to be bound by theory, the secondary structure provided by the hairpin loops can minimize the potential of the template nucleic acid to interfere with Cas9 activity, when the gRNA fusion molecule is complexed with Cas9.

The composition of the hairpin loops can be altered to adjust the rigidity of the hairpin structure and the orientation of the 3' end of the gRNA.

In one embodiment, the gRNA molecule contains one hairpin loop at the 3'end. In another embodiment, the gRNA molecule contains 1-10 hairpin loops at the 3'end, *e.g.,* 1 hairpin loop, 2 hairpin loops, 3 hairpin loops, 4 hairpin loops, 5 hairpin loops, 6 hairpin loops, 7 hairpin loops, 8 hairpin loops, 9 hairpin loops, or 10 hairpin loops. In another embodiment, the gRNA molecule contains 1-5 hairpin loops. In another embodiment, the gRNA molecule contains 1-4 hairpin loops. In another embodiment, the gRNA molecule contains 1-3 hairpin loops.

The hairpin sequence can be altered to contain larger or smaller regions of complementarity, resulting in a larger or smaller "stem" region. In one embodiment, the length of the stem region is about 1-50 nucleotides. In another embodiment, the length of the stem region is about 1-40 nucleotides. In another embodiment, the length of the stem region is about 1-30 nucleotides. In another embodiment, the length of the stem region is about 1-20 nucleotides. In another embodiment, the length of the stem region is about 1-10 nucleotides. In another embodiment, the length of the stem region is about 1-5 nucleotides. In another embodiment, the length of the stem region is at least 5 nucleotides, *e*.*g*., at least 5, 6, 7, 8, 9, 10, 11, 12, 13, 14, 15, 16, 17, 18, 19, or at least 20 nucleotides. In exemplary embodiments, the stem region is 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 nucleotides.

Similarly, the length of the unpaired sequence between the stem region can be altered to form hairpins with larger or smaller loops. In one embodiment, the loop region contains 1-20 nucleotides, *e*.*g*., 1, 2, 3, 4, 5, 6, 7, 8, 9, 10, 11, 12, 13, 14, 15, 16, 17, 18, 19, or 20 nucleotides. In another embodiment, the loop region is about 1-15 nucleotides. In another embodiment, the loop region is about 1-10 nucleotides. In another embodiment, the loop region is about 1-5 nucleotides. In another embodiment, the loop region is about 5-10 nucleotides. In another embodiment, the loop region is about 5-15 nucleotides. In another embodiment, the loop region is about 1-10 nucleotides. In another embodiment, the loop region is about 5-20 nucleotides. In other embodiments, the loop region is more than 20 nucleotides, *e*.*g*., 20-25 nucleotides, etc.

In an exemplary embodiment, one or more of the hairpin loops comprise an MS2 binding site sequence, or a portion thereof sufficient for formation of a stem-loop structure. MS2 binding site sequences are stem-loop structures that serve as a binding site for the phage capsid protein MS2. In one embodiment, the gRNA contains one or more hairpin loops comprising all or a portion of the 19-nucleotide MS2 binding site sequence described by Bertrand 1998 
(GTTTTAGAGCTAGAAATAGCAAGTTAAAATAAGGCTAGTCCGTTATCAACTTGA
AAAAGTGGCACCGAGTCGGTGCTACGGTACTTATTGCCAAGAAAGCACGAGCAT
CAGCCGTGCCTCCAGGTCGAATCTTCAAACGACGACGATCACGCGTCGCTCCAGT
ATTCCAGGGTTCATCTTTTTTT; SEQ ID NO:206). In another embodiment, the gRNA
 contains one or more hairpin loops comprising all or a portion of the MS2 binding site sequence described by Konermann 2015 
(GTTTTAGAGCTAGGCCAACATGAGGATCACCCATGTCTGCAGGGCCTAGCAAGT
TAAAATAAGGCTAGTCCGTTATCAACTTGGCCAACATGAGGATCACCCATGTCTG
CAGGGCCAAGTGGCACCGAGTCGGTGCTTTTTTT; SEQ ID NO:207).
 embodiment, the gRNA contains one or more hairpin loops containing all or a portion of SEQ ID NO:206 (Bertrand 1998), and one or more hairpin loops containing all or a portion of SEQ ID NO:207 (Konermann 2015).

In embodiments where the gRNA contains more than one 3' hairpins, the hairpin sequences can be separated by intervening single-stranded RNA nucleotides. The length of the intervening nucleotides can be varied to further adjust the secondary structure of the hairpin region at the 3'end of the gRNA. In exemplary embodiments, 3' hairpins are separated by 0-100 nucleotides, and ranges therein, *e.g.,* 0, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, 55, 60, 65, 70, 75, 80, 90, 95, or 100 nucleotides. In some embodiments, 3' hairpins are separated by 1-50 nucleotides. In other embodiments, 3' hairpins are separated by 1-25 nucleotides. In other embodiments, 3' hairpins are separated by 1-20 nucleotides. In other embodiments, 3' hairpins are separated by 1-15 nucleotides. In other embodiments, 3' hairpins are separated by 1-10 nucleotides. In other embodiments, 3' hairpins are separated by 1-5 nucleotides.

According to the invention, the gRNA molecule contains one or more 3' hairpin loops at or near the 3' end of the gRNA. For example, the hairpin loops can be positioned within 0-20 nucleotides of the 3' end of the gRNA, *e*.*g*., within 0, 1, 2, 3, 4, 5, 6, 7, 8, 9, 10, 11, 12, 13, 14, 15, 16, 17, 18, 19, or 20 nucleotides of the 3' end.

Template nucleic acids, also referred to as donor templates, serve as the template sequence for alteration of a target nucleic acid at a specified position. It is believed that alteration of the target sequence can occur by homology-dependent repair (HDR) with the template nucleic acid. A template nucleic acid may comprise double-stranded DNA, single-stranded DNA, or single-stranded RNA. Any of these embodiments are suitable for generating the gRNA fusion molecules of the present invention, in which a gRNA is covalently linked to a donor template nucleic acid. Additional features of template nucleic acids suitable for use with the present invention are described herein, and in WO2015/048577.

In some embodiments of the invention, the template nucleic acid linked to the gRNA is about 100-200 nucleotides in length. For example, the template nucleic acid can be about 100 nucleotides, about 110 nucleotides, about 120 nucleotides, about 130 nucleotides, about 140 nucleotides, about 150 nucleotides, about 160 nucleotides, about 170 nucleotides, about 180 nucleotides, about 190 nucleotides, or about 200 nucleotides. In other embodiments, the template nucleic acid can be about 200-300 nucleotides. In an exemplary embodiment, the template nucleic acid is about 150-200 nucleotides. In another embodiment, the template nucleic acid is about 160-190 nucleotides. In another embodiment, the template nucleic acid is about 170-180 nucleotides. In an exemplary embodiment, the template nucleic acid is 179 nucleotides.

The following examples illustrate several embodiments of gRNA fusion molecules. These examples are illustrative, and are not intended to be limiting.

### (A) gRNA Molecules Linked to Template Nucleic Acid Using Ligases

In one embodiment, the invention provides compositions comprising a gRNA molecule covalently linked to a template nucleic acid, wherein the gRNA molecule is linked to the template nucleic acid, *e.g.,* using a ligase. In exemplary embodiments, the 3' end of the gRNA molecule is ligated to the 5' end of the template nucleic acid.

Any suitable method known in the art for ligation of nucleic acid molecules can be used to ligate the gRNA molecule to the template nucleic acid. For example, ligation can be performed using a ligase, such as a DNA ligase or an RNA ligase, which catalyze the formation of a phosphodiester bond between the 3'end of the gRNA and the 5'end of the template nucleic acid. Exemplary ligases that may be used to ligate the gRNA and the template nucleic acid include T4 DNA ligase, T4 RNA ligase, 5'App DNA/RNA ligase, and SplintR ligase.

In some embodiments, a splint oligonucleotide is used to bring the 3'end of the gRNA and the 5' end of the template nucleic acid into proximity for ligation. The splint oligonucleotide has a short region of complementarity to the 3' end of the gRNA, and a short region of complementarity to the 5' end of the template nucleic acid, such that the splint oligonucleotide hybridizes to both the 3' end of the gRNA and the 5' end of the template nucleic acid. In some embodiments, the splint oligonucleotide has complementarity to 5, 6, 7, 8, 9, 10, 11, 12, 13, 14, 15, 16, 17, 18, 19, 20, 25, 30, or more bp of the 3' end of the gRNA (e.g., the tracr sequence of the gRNA). In one embodiment, the splint oligonucleotide has complementarity to 10 bp of the 3' end of the gRNA. In another embodiment, the splint oligonucleotide has complementarity to 20 bp of the 3' end of the gRNA. In yet another embodiment, the splint oligonucleotide has complementarity to 30 bp of the 3' end of the gRNA. In some embodiments, the splint oligonucleotide has complementarity to 5, 6, 7, 8, 9, 10, 11, 12, 13, 14, 15, 16, 17, 18, 19, 20, 25, 30, or more bp of the 5' end of the template nucleic acid. In one embodiment, the splint oligonucleotide has complementarity to 10 bp of the 5' end of the template nucleic acid. In another embodiment, the splint oligonucleotide has complementarity to 20 bp of the 5' end of the template nucleic acid. In yet another embodiment, the splint oligonucleotide has complementarity to 30 bp of the 5' end of the template nucleic acid. The splint oligonucleotide can comprise RNA or DNA. In some embodiments, the splint oligonucleotide is contains RNA and DNA, for example, an RNA portion that is complementary to the 3' end of the gRNA and a DNA portion that is complementary to the 5' end of the template nucleic acid, or a DNA portion that is complementary to the 3' end of the gRNA and an RNA portion that is complementary to the 5' end of the template nucleic acid.

T4 DNA ligase catalyzes the formation of a phosphodiester bond between 5' and 3' ends in duplex DNA or RNA, *e.g.,* to repair nicks (*i.e.,* a single strand break or a single strand cleavage event). Accordingly, in embodiments where a DNA, RNA, or DNA/RNA splint oligonucleotide is used to recruit the gRNA and the template nucleic acid, T4 DNA ligase can be used to ligate the gRNA to the template nucleic acid.

T4 RNA ligase 2 can also ligate nicks in double stranded RNA or DNA, and can similarly be used to ligate the gRNA to the template nucleic acid in embodiments where a DNA, RNA, or DNA/RNA splint oligonucleotide is used to recruit the gRNA and the template nucleic acid.

5'App ligase *(e.g.,* T4 RNA ligase K227Q, 5'App DNA/RNA ligase) contains a point mutation at a catalytic lysine, which renders the ligase unable to adenylate the 5' phosphate of RNA or single-stranded DNA. 5'App ligase thus requires adenylation of the 5'end of the oligonucleotide to be ligated. Accordingly, in some embodiments, 5'App ligase can be used to ligate the 3' end of the gRNA to an adenylated template nucleic acid, *i.e.,* 5' adenylated RNA template nucleic acid or 5'adenylated single stranded DNA template nucleic acid. In some embodiments, 5'App ligase is used in the absence of a splint oligonucleotide.

SplintR ligase, also known as PBCV-1 DNA ligase, can be used to ligate the 3'end of RNA to the 5'end of single-stranded DNA in the presence of a splint oligonucleotide. Accordingly, in some embodiments, SplintR ligase is used to ligate the 3' end of the gRNA to the 5' end of a single-stranded DNA template nucleic acid, in the presence of a splint oligonucleotide.

According to the invention, the 3' end of the gRNA ligated to the donor template nucleic acid contains one or more hairpin loops. There is some evidence that cells may react poorly to the presence of a DNA/RNA hybrid molecule, in which a region of DNA is hybridized to a region of RNA. Accordingly, in some embodiments, a splint oligonucleotide can be selected to minimize formation of a DNA/RNA hybrid. For example, if the template nucleic acid is single stranded RNA, an RNA splint oligonucleotide can be selected, which will form an RNA/RNA duplex with the 3' end of the gRNA and with the 5' end of the template nucleic acid. If the template nucleic acid is single stranded DNA, a splint oligonucleotide can be selected which has an RNA region and a DNA region, such that the splint oligonucleotide will form an RNA/RNA duplex with the 3'end of the gRNA, and a DNA/DNA duplex with the 5' end of the template nucleic acid. In some embodiments, a synthetic gRNA molecule may be used which contains a region of DNA at the 3' end. If the template nucleic acid is single stranded DNA, and the gRNA molecule contains a region of DNA at the 3'end, a DNA splint oligonucleotide can be selected, which will form a DNA/DNA duplex with the 3' end of the gRNA and with the 5' end of the template nucleic acid.

### (B) gRNA Molecules Linked to Template Nucleic Acid using Adaptor Molecules

In some embodiments described herein, a gRNA molecule is directly linked to a donor template nucleic acid, *e*.*g*., by ligation. In other embodiments, a gRNA molecule can be indirectly linked to a template nucleic acid by way of one or more adaptor molecules. Adaptor molecules mediate the covalent or non-covalent linkage of a gRNA molecule to a template nucleic acid. Adaptor molecules can be proteins, nucleic acids or small molecules. However, "adaptor molecule," as the term is used herein, does not encompass aptamers.

In other embodiments, the gRNA molecule is coupled to a first adaptor, and the template nucleic acid is coupled to a second adaptor which interacts covalently with the first adaptor. Accordingly, adaptor molecules (*e*.*g*., a protein, nucleic acid, or small molecule) can be coupled to the gRNA and to the template nucleic acid, such that interaction between the two adaptors links the gRNA and the template nucleic acid. This embodiment allows a broad range of molecular interactions to be used as the means to link the gRNA and the template nucleic acid. For example, the first adaptor can comprises a protein, and the second adaptor can comprise a protein. In another embodiment, the first adaptor can comprise a protein, and the second adaptor can comprise a nucleic acid (*e*.*g*., ssDNA, dsDNA, RNA). In another embodiment, the first adaptor can comprise a nucleic acid (*e*.*g*., ssDNA, dsDNA, RNA), and the second adaptor can comprise a protein. In another embodiment, the first adaptor can comprise a protein, and the second adaptor can comprise a small molecule. In another embodiment, the first adaptor can comprise a small molecule, and the second adaptor can comprise a protein. In one embodiment, the first adaptor can comprise a small molecule, and the second adaptor can comprise a small molecule. In another embodiment, the first adaptor can comprise a small molecule, and the second adaptor can comprise a nucleic acid (*e*.*g*., ssDNA, dsDNA, RNA). In another embodiment, the first adaptor can comprise a nucleic acid (*e*.*g*., ssDNA, dsDNA, RNA), and the second adaptor can comprise a small molecule. In one embodiment, the first adaptor can comprise a nucleic acid (*e*.*g*., ssDNA, dsDNA, RNA), and the second adaptor can comprise a nucleic acid (*e*.*g*., ssDNA, dsDNA, RNA).

For covalent interaction between the gRNA and the template nucleic acid, the two adaptors can be covalently linked using methods known in the art, for example, by crosslinking, fusion, ligation, etc.

In some embodiments, adaptor molecules can be covalently bound to the gRNA and/or the template nucleic acid, *e.g*., using a linker. In some embodiments, an adaptor molecule and the gRNA or template nucleic acid are encoded in tandem by a single nucleic acid, and are expressed as a single RNA construct. In other embodiments, the adaptor molecule and the gRNA or template nucleic acid are produced separately, and are then joined covalently. In some embodiments, an adaptor molecule is derived from a wild-type protein. For example, the adaptor molecule may be a fragment of a wild-type protein, a mutagenized wild-type protein, a mutagenized wild-type protein fragment, or a synthetic protein that has been modeled after the three dimensional structure of a naturally-occurring protein. In other embodiments, the adaptor molecule may be mutagenized to increase its affinity for the other adaptor, or mutagenized to decrease its affinity for the other adaptor.

In embodiments, an adaptor is coupled to the gRNA and/or the template nucleic acid through a linker. In one embodiment, the linker is sufficiently long to allow the gRNA to interact with a Cas9 molecule and to bind to a target nucleic acid without steric interference from the template nucleic acid. In one embodiment, the linker comprises a polypeptide. In one embodiment, the linker is a peptide linker at least 3, but no longer than 60 amino acids in length. In one embodiment, the linker peptide is 3-20 amino acids in length. In another embodiment, the linker peptide is 5-10 amino acids in length. In exemplary embodiments, the linker is 3, 4, 5, 6, 7, 8, 9, 10, 11, 12, 13, 14, 15, 16, 17, 18, 19, 20, 21, 22, 23, 24, 25, 26, 27, 28, 29, or 30 amino acids in length. In one embodiment, the linker comprises serine, glycine, or glycine and serine. In another embodiment, the linker is a nucleic acid linker that is at least 3, but no longer than 200 nucleotides in length. In one embodiment, the linker is 5-50 nucleotides in length. In another embodiment, the linker is 5-20 nucleotides in length. In exemplary embodiments, the linker is 3, 4, 5, 6, 7, 8, 9, 10, 11, 12, 13, 14, 15, 16, 17, 18, 19, 20, 21, 22, 23, 24, 25, 26, 27, 28, 29, or 30 nucleotides in length. In one embodiment, the linker is at least 10, 50, 100, 200, 500, 1000, 2000, 5000, or 10000 Angstroms in length. In one embodiment, the linker is no more than 10, 50, 100, 200, 500, 1000, 2000, 5000, or 10000 Angstroms in length.

### (C) gRNA Elongation

In some embodiments, the invention provides a gRNA molecule that is covalently linked to an RNA template nucleic acid by a phosphodiester bond. In such embodiments, the gRNA molecule and the template nucleic acid can comprise different regions of a continuous RNA molecule. In exemplary embodiments, the gRNA is positioned at the 5' end of the RNA molecule, and the template nucleic acid is positioned at the 3' end of the RNA molecule. In one embodiment, the RNA molecule comprises a continuous nucleic acid sequence containing, from 5' to 3', (i) a gRNA sequence, and (ii) a template nucleic acid sequence.

According to the invention, the RNA molecule contains one or more hairpin sequences, as described herein, positioned at or near the 3'end of the gRNA portion of the RNA molecule. The one or more hairpins can provide a semi-rigid secondary structure between the gRNA and the template nucleic acid portions of the RNA molecule.

In some embodiments, the RNA molecule further comprises a linker. For example, the gRNA and the template nucleic acid portions of the RNA molecule can be separated by an RNA linker. The linker can be, for example, at least 3, but no longer than 200 nucleotides in length. In one embodiment, the linker is 5-50 nucleotides in length. In another embodiment, the linker is 5-20 nucleotides in length. In exemplary embodiments, the linker is 3, 4, 5, 6, 7, 8, 9, 10, 11, 12, 13, 14, 15, 16, 17, 18, 19, 20, 21, 22, 23, 24, 25, 26, 27, 28, 29, or 30 nucleotides in length. In one embodiment, the linker is at least 10, 50, 100, 200, 500, 1000, 2000, 5000, or 10000 Angstroms in length. In one embodiment, the linker is no more than 10, 50, 100, 200, 500, 1000, 2000, 5000, or 10000 Angstroms in length. In one embodiment, the RNA molecule comprises a continuous nucleic acid sequence containing, from 5' to 3', (i) a gRNA sequence, (ii) an RNA linker, and (iii) a template nucleic acid sequence.

An RNA molecule comprising a gRNA region and a template nucleic acid region can optionally further comprise a tracr nucleic acid sequence, or a combination thereof. In some embodiments, the RNA molecule comprises (i) a gRNA sequence, (ii) a tracr sequence, and (iii) a template nucleic acid sequence, arranged in any order from 5'-3' on the RNA molecule. An RNA linker, as described above, can optionally be inserted between any one or more of elements (i)-(iii). In an exemplary instance, the RNA molecule comprises a continuous nucleic acid sequence containing from 5' to 3' (i) a gRNA sequence, (ii) a tracr sequence, and (iii) a template nucleic acid sequence. In this embodiment, an RNA linker, as described above, can optionally be inserted between any one or more of elements (i)-(iii).

In any of the foregoing instances in which the gRNA and the template nucleic acid sequence are part of a continuous RNA molecule, the elements of the continuous RNA molecule (*e.g.,* the gRNA and the template nucleic acid; the gRNA, the linker, and the template nucleic acid; the gRNA, the tracr sequence, and the template nucleic acid, etc.) can be transcribed in tandem. Accordingly, in some instances, an RNA molecule comprising a gRNA and a template nucleic acid are provided, wherein the RNA molecule is transcribed as single transcription unit from a nucleic acid sequence encoding the gRNA and the template nucleic acid. In some instances, RNA molecule further comprises a linker sequence, a tracr sequence, or combinations thereof.

In another aspect, the invention provides a nucleic acid molecule that encodes an RNA fusion molecule as defined in claim 1, wherein the gRNA molecule and the template nucleic acid are expressed in tandem. In one embodiment, the nucleic acid molecule is an isolated nucleic acid molecule. The nucleic acid molecule can be DNA. In one embodiment, the nucleic acid molecule is plasmid DNA. The nucleic acid can encode any of the RNA fusion molecules described herein which comprise a gRNA fused to an RNA template nucleic acid. For example, the nucleic acid molecule can encode a gRNA region comprising one or more hairpin sequences at or near the 3' end. The nucleic acid molecule can encode a gRNA region fused directly to a template nucleic acid. In other embodiments, the nucleic acid molecule encodes a gRNA region linked to a template nucleic acid by a linker, e.g., a nucleic acid linker. In other embodiments, the nucleic acid molecule further encodes a tracr sequence, optionally separated from the gRNA and/or the tracr sequence by a linker.

In a further aspect, the invention provides a vector comprising a nucleic acid molecule that encodes an RNA fusion molecule as defined in claim 1. The vector can be an expression vector. For example, the vector can be a plasmid expression vector. In other embodiments, the vector can be a viral expression vector, e.g., an adenoviral expression vector or a lentiviral expression vector. The vector can further comprise a promoter that drives expression of the RNA fusion molecule. In some embodiments, the promoter is an RNA polymerase III promoter. In other embodiments, the promoter is an RNA polymerase II promoter. In exemplary embodiments, the promoter is a T7 promoter or a U6 promoter.

In another aspect, the invention comprises a cell comprising a nucleic acid molecule which encodes an RNA fusion molecule as defined in claim 1. In one embodiment, the cell comprises a vector, e.g., an expression vector, such as a plasmid vector or a viral vector, which encodes an RNA fusion molecule comprising a gRNA and a template nucleic acid. In one embodiment, the cell is a prokaryotic cell. In one embodiment, the cell is a bacterial cell. In one embodiment, the cell is a eukaryotic cell. In one embodiment, the cell is a plant cell. In one embodiment, the cell is a yeast cell. In one embodiment, the cell is an insect cell. In one embodiment, the cell is an avian cell. In one embodiment, the cell is a mammalian cell. In one embodiment, the cell is a mouse cell, a rat cell, or a hamster cell. In one embodiment, the cell is a human cell.

### II. Compositions Comprising gRNA Fusion Molecules

Compositions may be provided comprising the gRNA fusion molecules described herein, in which a gRNA molecule is covalently or non-covalently linked to a template nucleic acid.

In one instance, the composition comprises a gRNA fusion molecule and a pharmaceutically acceptable carrier or excipient. The pharmaceutically acceptable carrier or excipient is suitable for delivery of nucleic acid, e.g., RNA, to a cell. In one embodiment, the cell is a cell *in vitro* or *ex vivo.* In another embodiment, the cell is *in vivo.*

In another instance, the composition comprises at least one Cas9 molecule. For example, the Cas9 molecule can be a wild-type Cas9, a nickase Cas9, a dead Cas9, a split Cas9, or an inducible Cas9, or combinations thereof. In some instances, the Cas9 molecule is a split Cas9 molecule or an inducible Cas9 molecule, as described in more detail in WO15/089427 and WO14/018423. In one instance, the Cas9 molecule is an enzymatically active Cas9 (eaCas9). In another instance, the Cas9 molecule is an enzymatically inactive Cas9. The Cas9 molecule can comprise N-terminal RuvC-like domain cleavage activity, but has no HNH-like domain cleavage activity. In other instances, the Cas9 molecule contains an amino acid mutation at an amino acid position corresponding to amino acid position N863 of *Streptococcus pyogenes* Cas9. In other instances, the Cas9 molecule contains an amino acid mutation at an amino acid position corresponding to amino acid position D10 of *Streptococcus pyogenes* Cas9. Other exemplary Cas9 molecules that can be provided in a composition with a gRNA fusion molecule are discussed herein.

The at least one Cas9 molecule can be a Cas9 polypeptide. In this embodiment, it is possible to provide the gRNA fusion molecule and the Cas9 polypeptide associated in a pre-formed ribonucleoprotein complex. Accordingly, a composition comprising a gRNA fusion molecule and a Cas9 polypeptide may be provided, wherein the gRNA fusion molecule and the Cas9 polypeptide are associated in a pre-formed ribonucleoprotein complex.

Alternatively, the at least one Cas9 molecule can be a nucleic acid encoding a Cas9 polypeptide. The nucleic acid encoding the Cas9 molecule can be provided, for example, in a vector, e.g., an expression vector. For example, the composition can comprise an expression vector, such as a plasmid vector or a viral vector, which encodes a Cas9 polypeptide.

In one aspect, the composition comprises a nucleic acid encoding a gRNA fusion molecule, in which, for example, the gRNA and the template nucleic acid are transcribed in tandem. In instances, the composition can further comprise a Cas9 molecule. The Cas9 molecule can be a Cas9 protein, or a nucleic acid encoding a Cas9 protein. In instances in which the composition comprises a nucleic acid encoding a gRNA fusion molecule, and a nucleic acid encoding a Cas9 protein, the nucleic acid molecules may be provided on the same vector, or on separate vectors.

Kits comprising the foregoing compositions, and instructions for use thereof in gene silencing, are also provided.

### III. Guide RNA (gRNA) Molecules

A gRNA molecule, as that term is used herein, refers to a nucleic acid that promotes the specific targeting or homing of a gRNA molecule/Cas9 molecule complex to a target nucleic acid. gRNA molecules can be unimolecular (having a single RNA molecule) *(e.g.,* chimeric or modular (comprising more than one, and typically two, separate RNA molecules). The gRNA molecules provided herein comprise a targeting domain comprising, consisting of, or consisting essentially of a nucleic acid sequence fully or partially complementary to a target domain. In certain embodiments, the gRNA molecule further comprises one or more additional domains, including for example a first complementarity domain, a linking domain, a second complementarity domain, a proximal domain, a tail domain, and a 5' extension domain. Each of these domains is discussed in detail below. Additional details on gRNAs are provided in Section I entitled "gRNA molecules" of PCT Application WO 2015/048577. In certain embodiments, one or more of the domains in the gRNA molecule comprises an amino acid sequence identical to or sharing sequence homology with a naturally occurring sequence, *e.g.,* from *S. pyogenes, S. aureus, or S. thermophilus.*

In certain embodiments, a unimolecular, or chimeric, gRNA comprises, preferably from 5' to 3':
a targeting domain complementary to a target domain in a gene;
a first complementarity domain;
a linking domain;
a second complementarity domain (which is complementary to the first complementarity domain);
a proximal domain; and
optionally, a tail domain.

In certain embodiments, a modular gRNA comprises:
a first strand comprising, preferably from 5' to 3':
   a targeting domain; and
   a first complementarity domain; and
a second strand, comprising, preferably from 5' to 3':
   optionally, a 5' extension domain;
   a second complementarity domain;
   a proximal domain; and
   optionally, a tail domain.

Each of these domains are described in more detail, below.

### Targeting Domain

The targeting domain (sometimes referred to alternatively as the guide sequence or complementarity region) comprises, consists of, or consists essentially of a nucleic acid sequence that is complementary or partially complementary to a target nucleic acid sequence, *e.g.,* a target nucleic acid sequence in a *HBB* target gene. The nucleic acid sequence in a target gene, *e.g., HBB,* to which all or a portion of the targeting domain is complementary or partially complementary is referred to herein as the target domain. In certain embodiments, the target domain comprises a target position within the target gene, *e.g., HBB.* In other embodiments, a target position lies outside (*i.e.,* upstream or downstream of) the target domain. In certain embodiments, the target domain is located entirely within a target gene, *e.g.,* in a coding region, an intron, or an exon. In other embodiments, all or part of the target domain is located outside of a target gene, *e.g.,* in a control region or in a non-coding region. Methods for selecting targeting domains are known in the art (see, *e.g.,* Fu 2014; Sternberg 2014).

The strand of the target nucleic acid comprising the target domain is referred to herein as the "complementary strand" because it is complementary to the targeting domain sequence. Since the targeting domain is part of a gRNA molecule, it comprises the base uracil (U) rather than thymine (T); conversely, any DNA molecule encoding the gRNA molecule will comprise thymine rather than uracil. In a targeting domain/target domain pair, the uracil bases in the targeting domain will pair with the adenine bases in the target domain. In certain embodiments, the degree of complementarity between the targeting domain and target domain is sufficient to allow targeting of a Cas9 molecule to the target nucleic acid.

In certain embodiments, the targeting domain comprises a core domain and an optional secondary domain. In certain of these embodiments, the core domain is located 3' to the secondary domain, and in certain of these embodiments the core domain is located at or near the 3' end of the targeting domain. In certain of these embodiments, the core domain consists of or consists essentially of about 8 to about 13 nucleotides at the 3' end of the targeting domain. In certain embodiments, only the core domain is complementary or partially complementary to the corresponding portion of the target domain, and in certain of these embodiments the core domain is fully complementary to the corresponding portion of the target domain. In other embodiments, the secondary domain is also complementary or partially complementary to a portion of the target domain. In certain embodiments, the core domain is complementary or partially complementary to a core domain target in the target domain, while the secondary domain is complementary or partially complementary to a secondary domain target in the target domain. In certain embodiments, the core domain and secondary domain have the same degree of complementarity with their respective corresponding portions of the target domain. In other embodiments, the degree of complementarity between the core domain and its target and the degree of complementarity between the secondary domain and its target may differ. In certain of these embodiments, the core domain may have a higher degree of complementarity for its target than the secondary domain, whereas in other embodiments the secondary domain may have a higher degree of complementarity than the core domain.

In certain embodiments, the targeting domain and/or the core domain within the targeting domain is 3 to 100, 5 to 100, 10 to 100, or 20 to 100 nucleotides in length, and in certain of these embodiments the targeting domain or core domain is 3 to 15, 3 to 20, 5 to 20, 10 to 20, 15 to 20, 5 to 50, 10 to 50, or 20 to 50 nucleotides in length. In certain embodiments, the targeting domain and/or the core domain within the targeting domain is 6, 7, 8, 9, 10, 11, 12, 13, 14, 15, 16, 17, 18, 19, 20, 21, 22, 23, 24, 25, or 26 nucleotides in length. In certain embodiments, the targeting domain and/or the core domain within the targeting domain is 6 +/-2, 7+/-2, 8+/-2, 9+/-2, 10+/-2, 10+/-4, 10 +/-5, 11+/-2, 12+/-2, 13+/- 2, 14+/-2, 15+/-2, or 16+-2, 20+/-5, 30+/-5, 40+/-5, 50+/-5, 60+/-5, 70+/-5, 80+/-5, 90+/-5, or 100+/-5 nucleotides in length.

In certain embodiments wherein the targeting domain includes a core domain, the core domain is 3 to 20 nucleotides in length, and in certain of these embodiments the core domain 5 to 15 or 8 to 13 nucleotides in length. In certain embodiments wherein the targeting domain includes a secondary domain, the secondary domain is 0, 1, 2, 3, 4, 5, 6, 7, 8, 9, 10, 11, 12, 13, 14 or 15 nucleotides in length. In certain embodiments wherein the targeting domain comprises a core domain that is 8 to 13 nucleotides in length, the targeting domain is 26, 25, 24, 23, 22, 21, 20, 19, 18, 17, or 16 nucleotides in length, and the secondary domain is 13 to 18, 12 to 17, 11 to 16, 10 to 15, 9 to 14, 8 to 13, 7 to 12, 6 to 11, 5 to 10, 4 to 9, or 3 to 8 nucleotides in length, respectively.

In certain embodiments, the targeting domain is fully complementary to the target domain. Likewise, where the targeting domain comprises a core domain and/or a secondary domain, in certain embodiments one or both of the core domain and the secondary domain are fully complementary to the corresponding portions of the target domain. In other embodiments, the targeting domain is partially complementary to the target domain, and in certain of these embodiments where the targeting domain comprises a core domain and/or a secondary domain, one or both of the core domain and the secondary domain are partially complementary to the corresponding portions of the target domain. In certain of these embodiments, the nucleic acid sequence of the targeting domain, or the core domain or targeting domain within the targeting domain, is at least 80%, 85%, 90%, or 95% complementary to the target domain or to the corresponding portion of the target domain. In certain embodiments, the targeting domain and/or the core or secondary domains within the targeting domain include one or more nucleotides that are not complementary with the target domain or a portion thereof, and in certain of these embodiments the targeting domain and/or the core or secondary domains within the targeting domain include 1, 2, 3, 4, 5, 6, 7, or 8 nucleotides that are not complementary with the target domain. In certain embodiments, the core domain includes 1, 2, 3, 4, or 5 nucleotides that are not complementary with the corresponding portion of the target domain. In certain embodiments wherein the targeting domain includes one or more nucleotides that are not complementary with the target domain, one or more of said non-complementary nucleotides are located within five nucleotides of the 5' or 3' end of the targeting domain. In certain of these embodiments, the targeting domain includes 1, 2, 3, 4, or 5 nucleotides within five nucleotides of its 5' end, 3' end, or both its 5' and 3' ends that are not complementary to the target domain. In certain embodiments wherein the targeting domain includes two or more nucleotides that are not complementary to the target domain, two or more of said non-complementary nucleotides are adjacent to one another, and in certain of these embodiments the two or more consecutive non-complementary nucleotides are located within five nucleotides of the 5' or 3' end of the targeting domain. In other embodiments, the two or more consecutive non-complementary nucleotides are both located more than five nucleotides from the 5' and 3' ends of the targeting domain.

In an embodiment, the gRNA molecule, *e.g.,* a gRNA molecule comprising a targeting domain, which is complementary with a target gene of interest, is a modular gRNA molecule. In another embodiment, the gRNA molecule is a unimolecular or chimeric gRNA molecule.

In certain embodiments, the targeting domain comprises 16 nucleotides. In certain embodiments, the targeting domain comprises 17 nucleotides. In certain embodiments, the targeting domain comprises 18 nucleotides. In certain embodiments, the targeting domain comprises 19 nucleotides. In certain embodiments, the targeting domain comprises 20 nucleotides. In certain embodiments, the targeting domain comprises 21 nucleotides. In certain embodiments, the targeting domain comprises 22 nucleotides. In certain embodiments, the targeting domain comprises 23 nucleotides. In certain embodiments, the targeting domain comprises 24 nucleotides. In certain embodiments, the targeting domain comprises 25 nucleotides. In certain embodiments, the targeting domain comprises 26 nucleotides.

In certain embodiments, the targeting domain which is complementary with the *HBB* gene is 16 nucleotides or more in length. In certain embodiments, the targeting domain is 16 nucleotides in length. In certain embodiments, the targeting domain is 17 nucleotides in length. In another embodiment, the targeting domain is 18 nucleotides in length. In still another embodiment, the targeting domain is 19 nucleotides in length. In still another embodiment, the targeting domain is 20 nucleotides in length. In still another embodiment, the targeting domain is 21 nucleotides in length. In still another embodiment, the targeting domain is 22 nucleotides in length. In still another embodiment, the targeting domain is 23 nucleotides in length. In still another embodiment, the targeting domain is 24 nucleotides in length. In still another embodiment, the targeting domain is 25 nucleotides in length. In still another embodiment, the targeting domain is 26 nucleotides in length.

In an embodiment, a nucleic acid encodes a modular gRNA molecule, *e.g.,* one or more nucleic acids encode a modular gRNA molecule. In another embodiment, a nucleic acid encodes a chimeric gRNA molecule. The nucleic acid may encode a gRNA molecule, *e.g.,* the first gRNA molecule, comprising a targeting domain comprising 16 nucleotides or more in length. In one embodiment, the nucleic acid encodes a gRNA molecule, *e.g.,* the first gRNA molecule, comprising a targeting domain that is 16 nucleotides in length. In another embodiment, the nucleic acid encodes a gRNA molecule, *e.g.,* the first gRNA molecule, comprising a targeting domain that is 17 nucleotides in length. In still another embodiment, the nucleic acid encodes a gRNA molecule, *e.g.,* the first gRNA molecule, comprising a targeting domain that is 18 nucleotides in length. In still another embodiment, the nucleic acid encodes a gRNA molecule, *e.g.,* the first gRNA molecule, comprising a targeting domain that is 19 nucleotides in length. In still another embodiment, the nucleic acid encodes a gRNA molecule, *e.g.,* the first gRNA molecule, comprising a targeting domain that is 20 nucleotides in length. In still another embodiment, the nucleic acid encodes a gRNA molecule, *e.g.,* the first gRNA molecule, comprising a targeting domain that is 21 nucleotides in length. In still another embodiment, the nucleic acid encodes a gRNA molecule, *e.g.,* the first gRNA molecule, comprising a targeting domain that is 22 nucleotides in length. In still another embodiment, the nucleic acid encodes a gRNA molecule, *e.g.,* the first gRNA molecule, comprising a targeting domain that is 23 nucleotides in length. In still another embodiment, the nucleic acid encodes a gRNA molecule, *e.g.,* the first gRNA molecule, comprising a targeting domain that is 24 nucleotides in length. In still another embodiment, the nucleic acid encodes a gRNA molecule, *e.g.,* the first gRNA molecule, comprising a targeting domain that is 25 nucleotides in length. In still another embodiment, the nucleic acid encodes a gRNA molecule, *e.g.,* the first gRNA molecule, comprising a targeting domain that is 26 nucleotides in length.

In certain embodiments, the targeting domain, core domain, and/or secondary domain do not comprise any modifications. In other embodiments, the targeting domain, core domain, and/or secondary domain, or one or more nucleotides therein, have a modification, including but not limited to the modifications set forth below. In certain embodiments, one or more nucleotides of the targeting domain, core domain, and/or secondary domain may comprise a 2' modification (*e.g.,* a modification at the 2' position on ribose), *e.g.,* a 2-acetylation, *e.g.,* a 2' methylation. In certain embodiments, the backbone of the targeting domain can be modified with a phosphorothioate. In certain embodiments, modifications to one or more nucleotides of the targeting domain, core domain, and/or secondary domain render the targeting domain and/or the gRNA comprising the targeting domain less susceptible to degradation or more bio-compatible, *e.g*., less immunogenic. In certain embodiments, the targeting domain and/or the core or secondary domains include 1, 2, 3, 4, 5, 6, 7, or 8 or more modifications, and in certain of these embodiments the targeting domain and/or core or secondary domains include 1, 2, 3, or 4 modifications within five nucleotides of their respective 5' ends and/or 1, 2, 3, or 4 modifications within five nucleotides of their respective 3' ends. In certain embodiments, the targeting domain and/or the core or secondary domains comprise modifications at two or more consecutive nucleotides.

In certain embodiments wherein the targeting domain includes core and secondary domains, the core and secondary domains contain the same number of modifications. In certain of these embodiments, both domains are free of modifications. In other embodiments, the core domain includes more modifications than the secondary domain, or vice versa.

In certain embodiments, modifications to one or more nucleotides in the targeting domain, including in the core or secondary domains, are selected to not interfere with targeting efficacy, which can be evaluated by testing a candidate modification using a system as set forth below. gRNAs having a candidate targeting domain having a selected length, sequence, degree of complementarity, or degree of modification can be evaluated using a system as set forth below. The candidate targeting domain can be placed, either alone or with one or more other candidate changes in a gRNA molecule/Cas9 molecule system known to be functional with a selected target, and evaluated.

In certain embodiments, all of the modified nucleotides are complementary to and capable of hybridizing to corresponding nucleotides present in the target domain. In another embodiment, 1, 2, 3, 4, 5, 6, 7 or 8 or more modified nucleotides are not complementary to or capable of hybridizing to corresponding nucleotides present in the target domain.

### First and second complementarity domains

The first and second complementarity (sometimes referred to alternatively as the crRNA-derived hairpin sequence and tracrRNA-derived hairpin sequences, respectively) domains are fully or partially complementary to one another. In certain embodiments, the degree of complementarity is sufficient for the two domains to form a duplexed region under at least some physiological conditions. In certain embodiments, the degree of complementarity between the first and second complementarity domains, together with other properties of the gRNA, is sufficient to allow targeting of a Cas9 molecule to a target nucleic acid.

In certain embodiments the first and/or second complementarity domain includes one or more nucleotides that lack complementarity with the corresponding complementarity domain. In certain embodiments, the first and/or second complementarity domain includes 1, 2, 3, 4, 5, or 6 nucleotides that do not complement with the corresponding complementarity domain. For example, the second complementarity domain may contain 1, 2, 3, 4, 5, or 6 nucleotides that do not pair with corresponding nucleotides in the first complementarity domain. In certain embodiments, the nucleotides on the first or second complementarity domain that do not complement with the corresponding complementarity domain loop out from the duplex formed between the first and second complementarity domains. In certain of these embodiments, the unpaired loop-out is located on the second complementarity domain, and in certain of these embodiments the unpaired region begins 1, 2, 3, 4, 5, or 6 nucleotides from the 5' end of the second complementarity domain.

In certain embodiments, the first complementarity domain is 5 to 30, 5 to 25, 7 to 25, 5 to 24, 5 to 23, 7 to 22, 5 to 22, 5 to 21, 5 to 20, 7 to 18, 7 to 15, 9 to 16, or 10 to 14 nucleotides in length, and in certain of these embodiments the first complementarity domain is 5, 6, 7, 8, 9, 10, 11, 12, 13, 14, 15, 16, 17, 18, 19, 20, 21, 22, 23, 24, or 25 nucleotides in length. In certain embodiments, the second complementarity domain is 5 to 27, 7 to 27, 7 to 25, 5 to 24, 5 to 23, 5 to 22, 5 to 21, 7 to 20, 5 to 20, 7 to 18, 7 to 17, 9 to 16, or 10 to 14 nucleotides in length, and in certain of these embodiments the second complementarity domain is 5, 6, 7, 8, 9, 10, 11, 12, 13, 14, 15, 16, 17, 18, 19, 20, 21, 22, 23, 24, 25, or 26 nucleotides in length. In certain embodiments, the first and second complementarity domains are each independently 6 +/-2, 7+/-2, 8+/-2, 9+/-2, 10+/-2, 11+/-2, 12+/-2, 13+/-2, 14+/-2, 15+/-2, 16+/-2, 17+/-2, 18+/-2, 19+/-2, or 20+/-2, 21+/-2, 22+/-2, 23+/-2, or 24+/-2 nucleotides in length. In certain embodiments, the second complementarity domain is longer than the first complementarity domain, *e.g.,* 2, 3, 4, 5, or 6 nucleotides longer.

In certain embodiments, the first and/or second complementarity domains each independently comprise three subdomains, which, in the 5' to 3' direction are: a 5' subdomain, a central subdomain, and a 3' subdomain. In certain embodiments, the 5' subdomain and 3' subdomain of the first complementarity domain are fully or partially complementary to the 3' subdomain and 5' subdomain, respectively, of the second complementarity domain.

In certain embodiments, the 5' subdomain of the first complementarity domain is 4 to 9 nucleotides in length, and in certain of these embodiments the 5' domain is 4, 5, 6, 7, 8, or 9 nucleotides in length. In certain embodiments, the 5' subdomain of the second complementarity domain is 3 to 25, 4 to 22, 4 to 18, or 4 to 10 nucleotides in length, and in certain of these embodiments the 5' domain is 3, 4, 5, 6, 7, 8, 9, 10, 11, 12, 13, 14, 15, 16, 17, 18, 19, 20, 21, 22, 23, 24, or 25 nucleotides in length. In certain embodiments, the central subdomain of the first complementarity domain is 1, 2, or 3 nucleotides in length. In certain embodiments, the central subdomain of the second complementarity domain is 1, 2, 3, 4, or 5 nucleotides in length. In certain embodiments, the 3' subdomain of the first complementarity domain is 3 to 25, 4 to 22, 4 to 18, or 4 to 10 nucleotides in length, and in certain of these embodiments the 3' subdomain is 3, 4, 5, 6, 7, 8, 9, 10, 11, 12, 13, 14, 15, 16, 17, 18, 19, 20, 21, 22, 23, 24, or 25 nucleotides in length. In certain embodiments, the 3' subdomain of the second complementarity domain is 4 to 9, *e.g.,* 4, 5, 6, 7, 8 or 9 nucleotides in length.

The first and/or second complementarity domains can share homology with, or be derived from, naturally occurring or reference first and/or second complementarity domain. In certain of these embodiments, the first and/or second complementarity domains have at least 50%, 60%, 70%, 80%, 85%, 90%, or 95% homology with, or differ by no more than 1, 2, 3, 4, 5, or 6 nucleotides from, the naturally occurring or reference first and/or second complementarity domain. In certain of these embodiments, the first and/or second complementarity domains may have at least 50%, 60%, 70%, 80%, 85%, 90%, or 95% homology with homology with a first and/or second complementarity domain from S. *pyogenes* or S. *aureus.*

In certain embodiments, the first and/or second complementarity domains do not comprise any modifications. In other embodiments, the first and/or second complementarity domains or one or more nucleotides therein have a modification, including but not limited to a modification set forth below. In certain embodiments, one or more nucleotides of the first and/or second complementarity domain may comprise a 2' modification (e.g., a modification at the 2' position on ribose), *e.g.,* a 2-acetylation, *e.g.,* a 2' methylation. In certain embodiments, the backbone of the targeting domain can be modified with a phosphorothioate. In certain embodiments, modifications to one or more nucleotides of the first and/or second complementarity domain render the first and/or second complementarity domain and/or the gRNA comprising the first and/or second complementarity less susceptible to degradation or more bio-compatible, e.g., less immunogenic. In certain embodiments, the first and/or second complementarity domains each independently include 1, 2, 3, 4, 5, 6, 7, or 8 or more modifications, and in certain of these embodiments the first and/or second complementarity domains each independently include 1, 2, 3, or 4 modifications within five nucleotides of their respective 5' ends, 3' ends, or both their 5' and 3' ends. In other embodiments, the first and/or second complementarity domains each independently contain no modifications within five nucleotides of their respective 5' ends, 3' ends, or both their 5' and 3' ends. In certain embodiments, one or both of the first and second complementarity domains comprise modifications at two or more consecutive nucleotides.

In certain embodiments, modifications to one or more nucleotides in the first and/or second complementarity domains are selected to not interfere with targeting efficacy, which can be evaluated by testing a candidate modification in a system as set forth below. gRNAs having a candidate first or second complementarity domain having a selected length, sequence, degree of complementarity, or degree of modification can be evaluated in a system as set forth below. The candidate complementarity domain can be placed, either alone or with one or more other candidate changes in a gRNA molecule/Cas9 molecule system known to be functional with a selected target, and evaluated.

In certain embodiments, the duplexed region formed by the first and second complementarity domains is, for example, 6, 7, 8, 9, 10, 11, 12, 13, 14, 15, 16, 17, 18, 19, 20, 21, or 22 bp in length, excluding any looped out or unpaired nucleotides.

In certain embodiments, the first and second complementarity domains, when duplexed, comprise 11 paired nucleotides (see, for *e.g.,* gRNA of SEQ ID NO:5). In certain embodiments, the first and second complementarity domains, when duplexed, comprise 15 paired nucleotides (see, *e.g.,* gRNA of SEQ ID NO:27). In certain embodiments, the first and second complementarity domains, when duplexed, comprise 16 paired nucleotides (see, *e.g.,* gRNA of SEQ ID NO:28). In certain embodiments, the first and second complementarity domains, when duplexed, comprise 21 paired nucleotides (see, *e.g.,* gRNA of SEQ ID NO:29).

In certain embodiments, one or more nucleotides are exchanged between the first and second complementarity domains to remove poly-U tracts. For example, nucleotides 23 and 48 or nucleotides 26 and 45 of the gRNA of SEQ ID NO:5 may be exchanged to generate the gRNA of SEQ ID NOs:30 or 31, respectively. Similarly, nucleotides 23 and 39 of the gRNA of SEQ ID NO:29 may be exchanged with nucleotides 50 and 68 to generate the gRNA of SEQ ID NO:32.

### Linking domain

The linking domain is disposed between and serves to link the first and second complementarity domains in a unimolecular or chimeric gRNA. In certain embodiments, part of the linking domain is from a crRNA-derived region, and another part is from a tracrRNA-derived region.

In certain embodiments, the linking domain links the first and second complementarity domains covalently. In certain of these embodiments, the linking domain consists of or comprises a covalent bond. In other embodiments, the linking domain links the first and second complementarity domains non-covalently. In certain embodiments, the linking domain is ten or fewer nucleotides in length, *e.g.,* 1, 2, 3, 4, 5, 6, 7, 8, 9, or 10 nucleotides. In other embodiments, the linking domain is greater than 10 nucleotides in length, *e.g.,* 11, 12, 13, 14, 15, 16, 17, 18, 19, 20, 21, 22, 23, 24, or 25 or more nucleotides. In certain embodiments, the linking domain is 2 to 50, 2 to 40, 2 to 30, 2 to 20, 2 to 10, 2 to 5, 10 to 100, 10 to 90, 10 to 80, 10 to 70, 10 to 60, 10 to 50, 10 to 40, 10 to 30, 10 to 20, 10 to 15, 20 to 100, 20 to 90, 20 to 80, 20 to 70, 20 to 60, 20 to 50, 20 to 40, 20 to 30, or 20 to 25 nucleotides in length. In certain embodiments, the linking domain is 10 +/-5, 20+/-5, 20+/- 10, 30+/-5, 30+/-10, 40+/-5, 40+/-10, 50+/-5, 50+/-10, 60+/-5, 60+/-10, 70+/-5, 70+/-10, 80+/-5, 80+/-10, 90+/-5, 90+/-10, 100+/-5, or 100+/-10 nucleotides in length.

In certain embodiments, the linking domain shares homology with, or is derived from, a naturally occurring sequence, *e.g.,* the sequence of a tracrRNA that is 5' to the second complementarity domain. In certain embodiments, the linking domain has at least 50%, 60%, 70%, 80%, 90%, or 95% homology with or differs by no more than 1, 2, 3, 4, 5, or 6 nucleotides from a linking domain disclosed herein.

In certain embodiments, the linking domain does not comprise any modifications. In other embodiments, the linking domain or one or more nucleotides therein have a modification, including but not limited to the modifications set forth below. In certain embodiments, one or more nucleotides of the linking domain may comprise a 2' modification (*e.g.,* a modification at the 2' position on ribose), *e.g.,* a 2-acetylation, *e.g.,* a 2' methylation. In certain embodiments, the backbone of the linking domain can be modified with a phosphorothioate. In certain embodiments, modifications to one or more nucleotides of the linking domain render the linking domain and/or the gRNA comprising the linking domain less susceptible to degradation or more bio-compatible, e.g., less immunogenic. In certain embodiments, the linking domain includes 1, 2, 3, 4, 5, 6, 7, or 8 or more modifications, and in certain of these embodiments the linking domain includes 1, 2, 3, or 4 modifications within five nucleotides of its 5' and/or 3' end. In certain embodiments, the linking domain comprises modifications at two or more consecutive nucleotides.

In certain embodiments, modifications to one or more nucleotides in the linking domain are selected to not interfere with targeting efficacy, which can be evaluated by testing a candidate modification in a system as set forth below. gRNAs having a candidate linking domain having a selected length, sequence, degree of complementarity, or degree of modification can be evaluated in a system as set forth below. The candidate linking domain can be placed, either alone or with one or more other candidate changes in a gRNA molecule/Cas9 molecule system known to be functional with a selected target, and evaluated.

In certain embodiments, the linking domain comprises a duplexed region, typically adjacent to or within 1, 2, or 3 nucleotides of the 3' end of the first complementarity domain and/or the 5' end of the second complementarity domain. In certain of these embodiments, the duplexed region of the linking region is 10+/-5, 15+/-5, 20+/-5, 20+/-10, or 30+/-5 bp in length. In certain embodiments, the duplexed region of the linking domain is 1, 2, 3, 4, 5, 6, 7, 8, 9, 10, 11, 12, 13, 14, or 15 bp in length. In certain embodiments, the sequences forming the duplexed region of the linking domain are fully complementarity. In other embodiments, one or both of the sequences forming the duplexed region contain one or more nucleotides (*e.g.,* 1, 2, 3, 4, 5, 6, 7, or 8 nucleotides) that are not complementary with the other duplex sequence.

### 5' extension domain

In certain embodiments, a modular gRNA as disclosed herein comprises a 5' extension domain, *i.e.,* one or more additional nucleotides 5' to the second complementarity domain. In certain embodiments, the 5' extension domain is 2 to 10 or more, 2 to 9, 2 to 8, 2 to 7, 2 to 6, 2 to 5, or 2 to 4 nucleotides in length, and in certain of these embodiments the 5' extension domain is 2, 3, 4, 5, 6, 7, 8, 9, or 10 or more nucleotides in length.

In certain embodiments, the 5' extension domain nucleotides do not comprise modifications, e.g., modifications of the type provided below. However, in certain embodiments, the 5' extension domain comprises one or more modifications, e.g., modifications that it render it less susceptible to degradation or more bio-compatible, e.g., less immunogenic. By way of example, the backbone of the 5' extension domain can be modified with a phosphorothioate, or other modification(s) as set forth below. In certain embodiments, a nucleotide of the 5' extension domain can comprise a 2' modification (e.g., a modification at the 2' position on ribose), *e.g.,* a 2-acetylation, *e.g.,* a 2' methylation, or other modification(s) as set forth below.

In certain embodiments, the 5' extension domain can comprise as many as 1, 2, 3, 4, 5, 6, 7, or 8 modifications. In certain embodiments, the 5' extension domain comprises as many as 1, 2, 3, or 4 modifications within 5 nucleotides of its 5' end, *e.g.,* in a modular gRNA molecule. In certain embodiments, the 5' extension domain comprises as many as 1, 2, 3, or 4 modifications within 5 nucleotides of its 3' end, *e.g.,* in a modular gRNA molecule.

In certain embodiments, the 5' extension domain comprises modifications at two consecutive nucleotides, *e.g*., two consecutive nucleotides that are within 5 nucleotides of the 5' end of the 5' extension domain, within 5 nucleotides of the 3' end of the 5' extension domain, or more than 5 nucleotides away from one or both ends of the 5' extension domain. In certain embodiments, no two consecutive nucleotides are modified within 5 nucleotides of the 5' end of the 5' extension domain, within 5 nucleotides of the 3' end of the 5' extension domain, or within a region that is more than 5 nucleotides away from one or both ends of the 5' extension domain. In certain embodiments, no nucleotide is modified within 5 nucleotides of the 5' end of the 5' extension domain, within 5 nucleotides of the 3' end of the 5' extension domain, or within a region that is more than 5 nucleotides away from one or both ends of the 5' extension domain.

Modifications in the 5' extension domain can be selected so as to not interfere with gRNA molecule efficacy, which can be evaluated by testing a candidate modification in a system as set forth below. gRNAs having a candidate 5' extension domain having a selected length, sequence, degree of complementarity, or degree of modification, can be evaluated in a system as set forth below. The candidate 5' extension domain can be placed, either alone, or with one or more other candidate changes in a gRNA molecule/Cas9 molecule system known to be functional with a selected target and evaluated.

In certain embodiments, the 5' extension domain has at least 60, 70, 80, 85, 90, or 95% homology with, or differs by no more than 1, 2, 3, 4, 5, or 6 nucleotides from, a reference 5' extension domain, *e.g.,* a naturally occurring, *e.g.,* an S. *pyogenes, S. aureus,* or *S. thermophilus,* 5' extension domain, or a 5' extension domain described herein.

### Proximal domain

In certain embodiments, the proximal domain is 5 to 20 or more nucleotides in length, e.g., 5, 6, 7, 8, 9, 10, 11, 12, 13, 14, 15, 16, 17, 18, 19, 20, 21, 22, 23, 24, 25, or 26 nucleotides in length. In certain of these embodiments, the proximal domain is 6 +/-2, 7+/-2, 8+/-2, 9+/-2, 10+/-2, 11+/-2, 12+/-2, 13+/-2, 14+/-2, 14+/-2, 16+/-2, 17+/-2, 18+/-2, 19+/-2, or 20+/-2 nucleotides in length. In certain embodiments, the proximal domain is 5 to 20, 7, to 18, 9 to 16, or 10 to 14 nucleotides in length.

In certain embodiments, the proximal domain can share homology with or be derived from a naturally occurring proximal domain. In certain of these embodiments, the proximal domain has at least 50%, 60%, 70%, 80%, 85%, 90%, or 95% homology with or differs by no more than 1, 2, 3, 4, 5, or 6 nucleotides from a proximal domain disclosed herein, *e.g.,* an S. *pyogenes, S. aureus,* or *S. thermophilus* proximal domain.

In certain embodiments, the proximal domain does not comprise any modifications. In other embodiments, the proximal domain or one or more nucleotides therein have a modification, including but not limited to the modifications set forth in herein. In certain embodiments, one or more nucleotides of the proximal domain may comprise a 2' modification (*e.g.,* a modification at the 2' position on ribose), *e.g.,* a 2-acetylation, *e.g.,* a 2' methylation. In certain embodiments, the backbone of the proximal domain can be modified with a phosphorothioate. In certain embodiments, modifications to one or more nucleotides of the proximal domain render the proximal domain and/or the gRNA comprising the proximal domain less susceptible to degradation or more bio-compatible, *e*.*g*., less immunogenic. In certain embodiments, the proximal domain includes 1, 2, 3, 4, 5, 6, 7, or 8 or more modifications, and in certain of these embodiments the proximal domain includes 1, 2, 3, or 4 modifications within five nucleotides of its 5' and/or 3' end. In certain embodiments, the proximal domain comprises modifications at two or more consecutive nucleotides.

In certain embodiments, modifications to one or more nucleotides in the proximal domain are selected to not interfere with targeting efficacy, which can be evaluated by testing a candidate modification in a system as set forth below. gRNAs having a candidate proximal domain having a selected length, sequence, degree of complementarity, or degree of modification can be evaluated in a system as set forth below. The candidate proximal domain can be placed, either alone or with one or more other candidate changes in a gRNA molecule/Cas9 molecule system known to be functional with a selected target, and evaluated.

### Tail domain

A broad spectrum of tail domains are suitable for use in the gRNA molecules disclosed herein.

In certain embodiments, the tail domain is absent. In other embodiments, the tail domain is 1 to 100 or more nucleotides in length, *e.g.,* 1, 2, 3, 4, 5, 6, 7, 8, 9, 10, 20, 30, 40, 50, 60, 70, 80, 90, or 100 nucleotides in length. In certain embodiments, the tail domain is 1 to 5, 1 to 10, 1 to 15, 1 to 20, 1 to 50, 10 to 100, 20 to 100, 10 to 90, 20 to 90, 10 to 80, 20 to 80, 10 to 70, 20 to 70, 10 to 60, 20 to 60, 10 to 50, 20 to 50, 10 to 40, 20 to 40, 10 to 30, 20 to 30, 20 to 25, 10 to 20, or 10 to 15 nucleotides in length. In certain embodiments, the tail domain is 5 +/-5, 10 +/-5, 20+/-10, 20+/-5, 25+/-10, 30+/-10, 30+/-5, 40+/-10, 40+/-5, 50+/- 10, 50+/-5, 60+/-10, 60+/-5, 70+/-10, 70+/-5, 80+/-10, 80+/-5, 90+/-10, 90+/-5, 100+/-10, or 100+/-5 nucleotides in length,

In certain embodiments, the tail domain can share homology with or be derived from a naturally occurring tail domain or the 5' end of a naturally occurring tail domain. In certain of these embodiments, the proximal domain has at least 50%, 60%, 70%, 80%, 85%, 90%, or 95% homology with or differs by no more than 1, 2, 3, 4, 5, or 6 nucleotides from a naturally occurring tail domain disclosed herein, *e.g.,* an *S*. *pyogenes, S. aureus,* or *S*. *thermophilus* tail domain.

In certain embodiments, the tail domain includes sequences that are complementary to each other and which, under at least some physiological conditions, form a duplexed region. In certain of these embodiments, the tail domain comprises a tail duplex domain which can form a tail duplexed region. In certain embodiments, the tail duplexed region is 3, 4, 5, 6, 7, 8, 9, 10, 11, or 12 bp in length. In certain embodiments, the tail domain comprises a single stranded domain 3' to the tail duplex domain that does not form a duplex. In certain of these embodiments, the single stranded domain is 3 to 10 nucleotides in length, *e.g.,* 3, 4, 5, 6, 7, 8, 9, 10, or 4 to 6 nucleotides in length.

In certain embodiments, the tail domain does not comprise any modifications. In other embodiments, the tail domain or one or more nucleotides therein have a modification, including but not limited to the modifications set forth herein. In certain embodiments, one or more nucleotides of the tail domain may comprise a 2' modification (e.g., a modification at the 2' position on ribose), *e.g.,* a 2-acetylation, *e.g.,* a 2' methylation. In certain embodiments, the backbone of the tail domain can be modified with a phosphorothioate. In certain embodiments, modifications to one or more nucleotides of the tail domain render the tail domain and/or the gRNA comprising the tail domain less susceptible to degradation or more bio-compatible, *e.g*., less immunogenic. In certain embodiments, the tail domain includes 1, 2, 3, 4, 5, 6, 7, or 8 or more modifications, and in certain of these embodiments the tail domain includes 1, 2, 3, or 4 modifications within five nucleotides of its 5' and/or 3' end. In certain embodiments, the tail domain comprises modifications at two or more consecutive nucleotides.

In certain embodiments, modifications to one or more nucleotides in the tail domain are selected to not interfere with targeting efficacy, which can be evaluated by testing a candidate modification as set forth below. gRNAs having a candidate tail domain having a selected length, sequence, degree of complementarity, or degree of modification can be evaluated using a system as set forth below. The candidate tail domain can be placed, either alone or with one or more other candidate changes in a gRNA molecule/Cas9 molecule system known to be functional with a selected target, and evaluated.

In certain embodiments, the tail domain includes nucleotides at the 3' end that are related to the method of *in vitro* or *in vivo* transcription. When a T7 promoter is used for *in vitro* transcription of the gRNA, these nucleotides may be any nucleotides present before the 3' end of the DNA template. When a U6 promoter is used for *in vivo* transcription, these nucleotides may be the sequence UUUUUU. When an H1 promoter is used for transcription, these nucleotides may be the sequence UUUU. When alternate pol-III promoters are used, these nucleotides may be various numbers of uracil bases depending on, *e.g.,* the termination signal of the pol-III promoter, or they may include alternate bases.

In certain embodiments, the proximal and tail domain taken together comprise, consist of, or consist essentially of the sequence set forth in SEQ ID NOs: 33, 34, 35, 36, or 38.

### Exemplary unimolecular/chimeric gRNAs

In certain embodiments, a gRNA as disclosed herein has the structure: 5' [targeting domain]-[first complementarity domain]-[linking domain]-[second complementarity domain]-[proximal domain]-[tail domain]-3', wherein:
the targeting domain comprises a core domain and optionally a secondary domain, and is 10 to 50 nucleotides in length;
the first complementarity domain is 5 to 25 nucleotides in length and, in certain embodiments has at least 50, 60, 70, 80, 85, 90, or 95% homology with a reference first complementarity domain disclosed herein;
the linking domain is 1 to 5 nucleotides in length;
the second complementarity domain is 5 to 27 nucleotides in length and, in certain embodiments has at least 50, 60, 70, 80, 85, 90, or 95% homology with a reference second complementarity domain disclosed herein;
the proximal domain is 5 to 20 nucleotides in length and, in certain embodiments has at least 50, 60, 70, 80, 85, 90, or 95% homology with a reference proximal domain disclosed herein; and
the tail domain is absent or a nucleotide sequence is 1 to 50 nucleotides in length and, in certain embodiments has at least 50, 60, 70, 80, 85, 90, or 95% homology with a reference tail domain disclosed herein.

In certain embodiments, a unimolecular gRNA as disclosed herein comprises, preferably from 5' to 3':
a targeting domain, e.g., comprising 10-50 nucleotides;
a first complementarity domain, e.g., comprising 15, 16, 17, 18, 19, 20, 21, 22, 23, 24, 25, or 26 nucleotides;
a linking domain;
a second complementarity domain;
a proximal domain; and
a tail domain,
wherein,
(a) the proximal and tail domain, when taken together, comprise at least 15, 18, 20, 25, 30, 31, 35, 40, 45, 49, 50, or 53 nucleotides;
(b) there are at least 15, 18, 20, 25, 30, 31, 35, 40, 45, 49, 50, or 53 nucleotides 3' to the last nucleotide of the second complementarity domain; or
(c) there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In certain embodiments, the sequence from (a), (b), and/or (c) has at least 50%, 60%, 70%, 75%, 80%, 85%, 90%, 95%, or 99% homology with the corresponding sequence of a naturally occurring gRNA, or with a gRNA described herein.

In certain embodiments, the proximal and tail domain, when taken together, comprise at least 15, 18, 20, 25, 30, 31, 35, 40, 45, 49, 50, or 53 nucleotides.

In certain embodiments, there are at least 15, 18, 20, 25, 30, 31, 35, 40, 45, 49, 50, or 53 nucleotides 3' to the last nucleotide of the second complementarity domain.

In certain embodiments, there are at least 16, 19, 21, 26, 31, 32, 36, 41, 46, 50, 51, or 54 nucleotides 3' to the last nucleotide of the second complementarity domain that are complementary to the corresponding nucleotides of the first complementarity domain.

In certain embodiments, the targeting domain consists of, consists essentially of, or comprises 16, 17, 18, 19, 20, 21, 22, 23, 24, 25, or 26 nucleotides (e.g., 16, 17, 18, 19, 20, 21, 22, 23, 24, 25, or 26 consecutive nucleotides) complementary or partially complementary to the target domain or a portion thereof, *e.g.,* the targeting domain is 16, 17, 18, 19, 20, 21, 22, 23, 24, 25, or 26 nucleotides in length. In certain of these embodiments, the targeting domain is complementary to the target domain over the entire length of the targeting domain, the entire length of the target domain, or both.

In certain embodiments, a unimolecular or chimeric gRNA molecule disclosed herein (comprising a targeting domain, a first complementary domain, a linking domain, a second complementary domain, a proximal domain and, optionally, a tail domain) comprises the amino acid sequence set forth in SEQ ID NO:45, wherein the targeting domain is listed as 20 N's (residues 1-20) but may range in length from 16 to 26 nucleotides, and wherein the final six residues (residues 97-102) represent a termination signal for the U6 promoter buy may be absent or fewer in number. In certain embodiments, the unimolecular, or chimeric, gRNA molecule is a S. *pyogenes* gRNA molecule.

In certain embodiments, a unimolecular or chimeric gRNA molecule disclosed herein (comprising a targeting domain, a first complementary domain, a linking domain, a second complementary domain, a proximal domain and, optionally, a tail domain) comprises the amino acid sequence set forth in SEQ ID NO:40, wherein the targeting domain is listed as 20 Ns (residues 1-20) but may range in length from 16 to 26 nucleotides, and wherein the final six residues (residues 97-102) represent a termination signal for the U6 promoter but may be absent or fewer in number. In certain embodiments, the unimolecular or chimeric gRNA molecule is an S. *aureus* gRNA molecule.

### Exemplary modular gRNAs

In certain embodiments, a modular gRNA disclosed herein comprises:
a first strand comprising, preferably from 5' to 3';
   a targeting domain, e.g., comprising 15, 16, 17, 18, 19, 20, 21, 22, 23, 24, 25, or 26 nucleotides;
   a first complementarity domain; and
a second strand, comprising, preferably from 5' to 3':
   optionally a 5' extension domain;
   a second complementarity domain;
   a proximal domain; and
   a tail domain,
wherein:
(a) the proximal and tail domain, when taken together, comprise at least 15, 18, 20, 25, 30, 31, 35, 40, 45, 49, 50, or 53 nucleotides;
(b) there are at least 15, 18, 20, 25, 30, 31, 35, 40, 45, 49, 50, or 53 nucleotides 3' to the last nucleotide of the second complementarity domain; or
(c) there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In certain embodiments, the sequence from (a), (b), or (c), has at least 60, 75, 80, 85, 90, 95, or 99% homology with the corresponding sequence of a naturally occurring gRNA, or with a gRNA described herein.

In certain embodiments, the proximal and tail domain, when taken together, comprise at least 15, 18, 20, 25, 30, 31, 35, 40, 45, 49, 50, or 53 nucleotides.

In certain embodiments, there are at least 15, 18, 20, 25, 30, 31, 35, 40, 45, 49, 50, or 53 nucleotides 3' to the last nucleotide of the second complementarity domain.

In certain embodiments, there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In certain embodiments, the targeting domain comprises, has, or consists of, 16, 17, 18, 19, 20, 21, 22, 23, 24, 25, or 26 nucleotides (e.g., 16, 17, 18, 19, 20, 21, 22, 23, 24, 25, or 26 consecutive nucleotides) having complementarity with the target domain, e.g., the targeting domain is 16, 17, 18, 19, 20, 21, 22, 23, 24, 25, or 26 nucleotides in length.

In certain embodiments, the targeting domain consists of, consists essentially of, or comprises 16, 17, 18, 19, 20, 21, 22, 23, 24, 25, or 26 nucleotides (e.g., 16, 17, 18, 19, 20, 21, 22, 23, 24, 25, or 26 consecutive nucleotides) complementary to the target domain or a portion thereof. In certain of these embodiments, the targeting domain is complementary to the target domain over the entire length of the targeting domain, the entire length of the target domain, or both.

In certain embodiments, the targeting domain comprises, has, or consists of, 16 nucleotides (e.g., 16 consecutive nucleotides) having complementarity with the target domain, *e.g.,* the targeting domain is 16 nucleotides in length; and the proximal and tail domain, when taken together, comprise at least 15, 18, 20, 25, 30, 31, 35, 40, 45, 49, 50, or 53 nucleotides.

In certain embodiments, the targeting domain comprises, has, or consists of, 16 nucleotides (e.g., 16 consecutive nucleotides) having complementarity with the target domain, *e.g.,* the targeting domain is 16 nucleotides in length; and there are at least 15, 18, 20, 25, 30, 31, 35, 40, 45, 49, 50, or 53 nucleotides 3' to the last nucleotide of the second complementarity domain.

In certain embodiments, the targeting domain comprises, has, or consists of, 16 nucleotides (e.g., 16 consecutive nucleotides) having complementarity with the target domain, *e.g.,* the targeting domain is 16 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In certain embodiments, the targeting domain has, or consists of, 17 nucleotides (*e.g*., 17 consecutive nucleotides) having complementarity with the target domain, *e.g.,* the targeting domain is 17 nucleotides in length; and the proximal and tail domain, when taken together, comprise at least 15, 18, 20, 25, 30, 31, 35, 40, 45, 49, 50, or 53 nucleotides.

In certain embodiments, the targeting domain has, or consists of, 17 nucleotides (*e.g*., 17 consecutive nucleotides) having complementarity with the target domain, *e.g.,* the targeting domain is 17 nucleotides in length; and there are at least 15, 18, 20, 25, 30, 31, 35, 40, 45, 49, 50, or 53 nucleotides 3' to the last nucleotide of the second complementarity domain.

In certain embodiments, the targeting domain has, or consists of, 17 nucleotides (*e.g*., 17 consecutive nucleotides) having complementarity with the target domain, *e.g.,* the targeting domain is 17 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In certain embodiments, the targeting domain has, or consists of, 18 nucleotides (*e.g*., 18 consecutive nucleotides) having complementarity with the target domain, *e.g.,* the targeting domain is 18 nucleotides in length; and the proximal and tail domain, when taken together, comprise at least 15, 18, 20, 25, 30, 31, 35, 40, 45, 49, 50, or 53 nucleotides.

In certain embodiments, the targeting domain has, or consists of, 18 nucleotides (*e.g.*, 18 consecutive nucleotides) having complementarity with the target domain, *e.g.,* the targeting domain is 18 nucleotides in length; and there are at least 15, 18, 20, 25, 30, 31, 35, 40, 45, 49, 50, or 53 nucleotides 3' to the last nucleotide of the second complementarity domain.

In certain embodiments, the targeting domain has, or consists of, 18 nucleotides (*e.g*., 18 consecutive nucleotides) having complementarity with the target domain, *e.g.,* the targeting domain is 18 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In certain embodiments, the targeting domain comprises, has, or consists of, 19 nucleotides (*e.g.*, 19 consecutive nucleotides) having complementarity with the target domain, *e.g.,* the targeting domain is 19 nucleotides in length; and the proximal and tail domain, when taken together, comprise at least 15, 18, 20, 25, 30, 31, 35, 40, 45, 49, 50, or 53 nucleotides.

In certain embodiments, the targeting domain comprises, has, or consists of, 19 nucleotides (*e.g.*, 19 consecutive nucleotides) having complementarity with the target domain, *e.g.,* the targeting domain is 19 nucleotides in length; and there are at least 15, 18, 20, 25, 30, 31, 35, 40, 45, 49, 50, or 53 nucleotides 3' to the last nucleotide of the second complementarity domain.

In certain embodiments, the targeting domain comprises, has, or consists of, 19 nucleotides (*e.g*., 19 consecutive nucleotides) having complementarity with the target domain, *e.g*., the targeting domain is 19 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In certain embodiments, the targeting domain comprises, has, or consists of, 20 nucleotides (*e.g*., 20 consecutive nucleotides) having complementarity with the target domain, *e.g.,* the targeting domain is 20 nucleotides in length; and the proximal and tail domain, when taken together, comprise at least 15, 18, 20, 25, 30, 31, 35, 40, 45, 49, 50, or 53 nucleotides.

In certain embodiments, the targeting domain comprises, has, or consists of, 20 nucleotides (*e.g*., 20 consecutive nucleotides) having complementarity with the target domain, *e.g.,* the targeting domain is 20 nucleotides in length; and there are at least 15, 18, 20, 25, 30, 31, 35, 40, 45, 49, 50, or 53 nucleotides 3' to the last nucleotide of the second complementarity domain.

In certain embodiments, the targeting domain comprises, has, or consists of, 20 nucleotides (*e.g*., 20 consecutive nucleotides) having complementarity with the target domain, *e.g.,* the targeting domain is 20 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In certain embodiments, the targeting domain comprises, has, or consists of, 21 nucleotides (*e.g*., 21 consecutive nucleotides) having complementarity with the target domain, *e.g.,* the targeting domain is 21 nucleotides in length; and the proximal and tail domain, when taken together, comprise at least 15, 18, 20, 25, 30, 31, 35, 40, 45, 49, 50, or 53 nucleotides.

In certain embodiments, the targeting domain comprises, has, or consists of, 21 nucleotides (*e.g*., 21 consecutive nucleotides) having complementarity with the target domain, *e.g.,* the targeting domain is 21 nucleotides in length; and there are at least 15, 18, 20, 25, 30, 31, 35, 40, 45, 49, 50, or 53 nucleotides 3' to the last nucleotide of the second complementarity domain.

In certain embodiments, the targeting domain comprises, has, or consists of, 21 nucleotides (*e.g*., 21 consecutive nucleotides) having complementarity with the target domain, *e.g.,* the targeting domain is 21 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In certain embodiments, the targeting domain comprises, has, or consists of, 22 nucleotides (*e.g*., 22 consecutive nucleotides) having complementarity with the target domain, *e.g.,* the targeting domain is 22 nucleotides in length; and the proximal and tail domain, when taken together, comprise at least 15, 18, 20, 25, 30, 31, 35, 40, 45, 49, 50, or 53 nucleotides.

In certain embodiments, the targeting domain comprises, has, or consists of, 22 nucleotides (*e.g*., 22 consecutive nucleotides) having complementarity with the target domain, *e.g.,* the targeting domain is 22 nucleotides in length; and there are at least 15, 18, 20, 25, 30, 31, 35, 40, 45, 49, 50, or 53 nucleotides 3' to the last nucleotide of the second complementarity domain.

In certain embodiments, the targeting domain comprises, has, or consists of, 22 nucleotides (*e.g.,* 22 consecutive nucleotides) having complementarity with the target domain, *e.g.,* the targeting domain is 22 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In certain embodiments, the targeting domain comprises, has, or consists of, 23 nucleotides (*e.g.,* 23 consecutive nucleotides) having complementarity with the target domain, *e.g.,* the targeting domain is 23 nucleotides in length; and the proximal and tail domain, when taken together, comprise at least 15, 18, 20, 25, 30, 31, 35, 40, 45, 49, 50, or 53 nucleotides.

In certain embodiments, the targeting domain comprises, has, or consists of, 23 nucleotides (*e.g.,* 23 consecutive nucleotides) having complementarity with the target domain, *e.g.,* the targeting domain is 23 nucleotides in length; and there are at least 15, 18, 20, 25, 30, 31, 35, 40, 45, 49, 50, or 53 nucleotides 3' to the last nucleotide of the second complementarity domain.

In certain embodiments, the targeting domain comprises, has, or consists of, 23 nucleotides (*e.g.,* 23 consecutive nucleotides) having complementarity with the target domain, *e.g.,* the targeting domain is 23 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In certain embodiments, the targeting domain comprises, has, or consists of, 24 nucleotides (*e.g.,* 24 consecutive nucleotides) having complementarity with the target domain, *e.g.,* the targeting domain is 24 nucleotides in length; and the proximal and tail domain, when taken together, comprise at least 15, 18, 20, 25, 30, 31, 35, 40, 45, 49, 50, or 53 nucleotides.

In certain embodiments, the targeting domain comprises, has, or consists of, 24 nucleotides (*e.g.,* 24 consecutive nucleotides) having complementarity with the target domain, *e.g.,* the targeting domain is 24 nucleotides in length; and there are at least 15, 18, 20, 25, 30, 31, 35, 40, 45, 49, 50, or 53 nucleotides 3' to the last nucleotide of the second complementarity domain.

In certain embodiments, the targeting domain comprises, has, or consists of, 24 nucleotides (*e.g.,* 24 consecutive nucleotides) having complementarity with the target domain, *e.g.,* the targeting domain is 24 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In certain embodiments, the targeting domain comprises, has, or consists of, 25 nucleotides (*e.g.,* 25 consecutive nucleotides) having complementarity with the target domain, *e.g.,* the targeting domain is 25 nucleotides in length; and the proximal and tail domain, when taken together, comprise at least 15, 18, 20, 25, 30, 31, 35, 40, 45, 49, 50, or 53 nucleotides.

In certain embodiments, the targeting domain comprises, has, or consists of, 25 nucleotides (*e.g.,* 25 consecutive nucleotides) having complementarity with the target domain, *e.g.,* the targeting domain is 25 nucleotides in length; and there are at least 15, 18, 20, 25, 30, 31, 35, 40, 45, 49, 50, or 53 nucleotides 3' to the last nucleotide of the second complementarity domain.

In certain embodiments, the targeting domain comprises, has, or consists of, 25 nucleotides (*e.g.,* 25 consecutive nucleotides) having complementarity with the target domain, *e.g.,* the targeting domain is 25 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In certain embodiments, the targeting domain comprises, has, or consists of, 26 nucleotides (*e.g.,* 26 consecutive nucleotides) having complementarity with the target domain, *e.g.,* the targeting domain is 26 nucleotides in length; and the proximal and tail domain, when taken together, comprise at least 15, 18, 20, 25, 30, 31, 35, 40, 45, 49, 50, or 53 nucleotides.

In certain embodiments, the targeting domain comprises, has, or consists of, 26 nucleotides (*e.g.,* 26 consecutive nucleotides) having complementarity with the target domain, *e.g.,* the targeting domain is 26 nucleotides in length; and there are at least 15, 18, 20, 25, 30, 31, 35, 40, 45, 49, 50, or 53 nucleotides 3' to the last nucleotide of the second complementarity domain.

In certain embodiments, the targeting domain comprises, has, or consists of, 26 nucleotides (*e.g.,* 26 consecutive nucleotides) having complementarity with the target domain, *e.g.,* the targeting domain is 26 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

### gRNA delivery

In certain embodiments of the methods provided herein, the methods comprise delivery of one or more (*e.g.,* two, three, or four) gRNA molecules as described herein. In certain instances, the gRNA molecules are delivered by intravenous injection, intramuscular injection, subcutaneous injection, or inhalation.

### IV. Methods for Designing gRNA Molecules

Methods for selecting, designing, and validating targeting domains for use in the gRNAs described herein are provided. Exemplary targeting domains for incorporation into gRNAs are also provided herein.

Methods for selection and validation of target sequences as well as off-target analyses have been described (see, *e.g.,* Mali 2013; Hsu 2013; Fu 2014; Heigwer 2014; Bae 2014; and Xiao 2014). For example, a software tool can be used to optimize the choice of potential targeting domains corresponding to a user's target sequence, *e.g.,* to minimize total off-target activity across the genome. Off-target activity may be other than cleavage. For each possible targeting domain choice using *S. pyogenes* Cas9, the tool can identify all off-target sequences (preceding either NAG or NGG PAMs) across the genome that contain up to certain number (*e.g.,* 1, 2, 3, 4, 5, 6, 7, 8, 9, or 10) of mismatched base-pairs. The cleavage efficiency at each off-target sequence can be predicted, *e.g.,* using an experimentally-derived weighting scheme. Each possible targeting domain is then ranked according to its total predicted off-target cleavage; the top-ranked targeting domains represent those that are likely to have the greatest on-target cleavage and the least off-target cleavage. Other functions, *e.g.,* automated reagent design for CRISPR construction, primer design for the on-target Surveyor assay, and primer design for high-throughput detection and quantification of off-target cleavage via next-gen sequencing, can also be included in the tool. Candidate targeting domains and gRNAs comprising those targeting domains can be functionally evaluated by using methods known in the art and/or as set forth herein.

As a non-limiting example, targeting domains for use in gRNAs for use with *S. pyogenes,* and *S. aureus* Cas9s were identified using a DNA sequence searching algorithm. 17-mer and 20-mer targeting domains were designed for *S. pyogenes* targets, while 18-mer, 19-mer, 20-mer, 21-mer, 22-mer, 23-mer, and 24-mer targeting domains were designed for *S. aureus* targets. gRNA design was carried out using a custom gRNA design software based on the public tool cas-offinder (Bae 2014). This software scores guides after calculating their genome-wide off-target propensity. Typically matches ranging from perfect matches to 7 mismatches are considered for guides ranging in length from 17 to 24. Once the off-target sites are computationally-determined, an aggregate score is calculated for each guide and summarized in a tabular output using a web-interface. In addition to identifying potential target sites adjacent to PAM sequences, the software also identifies all PAM adjacent sequences that differ by 1, 2, 3 or more than 3 nucleotides from the selected target sites. Genomic DNA sequences for a *HBB* gene was obtained from the UCSC Genome browser and sequences were screened for repeat elements using the publically available RepeatMasker program. RepeatMasker searches input DNA sequences for repeated elements and regions of low complexity. The output is a detailed annotation of the repeats present in a given query sequence.

Following identification, targeting domains were ranked into tiers based on their distance to the target site, their orthogonality and presence of a 5' G (based on identification of close matches in the human genome containing a relevant PAM *e.g.,* NGG PAM for *S. pyogenes,* NNGRRT or NNGRRV PAM for *S. aureus.* Orthogonality refers to the number of sequences in the human genome that contain a minimum number of mismatches to the target sequence. A "high level of orthogonality" or "good orthogonality" may, for example, refer to 20-mer targeting domains that have no identical sequences in the human genome besides the intended target, nor any sequences that contain one or two mismatches in the target sequence. Targeting domains with good orthogonality are selected to minimize off-target DNA cleavage.

Targeting domains were identified for both single-gRNA nuclease cleavage and for a dual-gRNA paired "nickase" strategy. Criteria for selecting targeting domains and the determination of which targeting domains can be incorporated into a gRNA and used for the dual-gRNA paired "nickase" strategy is based on two considerations:
1. gRNA pairs should be oriented on the DNA such that PAMs are facing out and cutting with the D10A Cas9 nickase will result in 5' overhangs.
2. An assumption that cleaving with dual nickase pairs will result in deletion of the entire intervening sequence at a reasonable frequency. However, cleaving with dual nickase pairs can also result in indel mutations at the site of only one of the gRNA molecules. Candidate pair members can be tested for how efficiently they remove the entire sequence versus causing indel mutations at the target site of one gRNA molecule.

### Other gRNA Design Strategy

In certain embodiments, two or more (*e.g.,* three or four) gRNA molecules are used with one Cas9 molecule. In another embodiment, when two or more (*e.g.,* three or four) gRNAs are used with two or more Cas9 molecules, at least one Cas9 molecule is from a different species than the other Cas9 molecule(s). For example, when two gRNA molecules are used with two Cas9 molecules, one Cas9 molecule can be from one species and the other Cas9 molecule can be from a different species. Both Cas9 species are used to generate a single or double-strand break, as desired.

In certain embodiments, dual targeting is used to create two nicks on opposite DNA strands by using Cas9 nickases (*e.g.,* a *S. pyogenes* Cas9 nickase) with two targeting domains that are complementary to opposite DNA strands, *e.g.,* a gRNA molecule comprising any minus strand targeting domain may be paired any gRNA molecule comprising a plus strand targeting domain provided that the two gRNAs are oriented on the DNA such that PAMs face outward and the distance between the 5' ends of the gRNAs is 0-50bp. When selecting gRNA molecules for use in a nickase pair, one gRNA molecule targets a domain in the complementary strand and the second gRNA molecule targets a domain in the non-complementary strand, *e.g.,* a gRNA comprising any minus strand targeting domain may be paired any gRNA molecule comprising a plus strand targeting domain targeting the same target position. In certain embodiments, two 20-mer gRNAs are used to target two Cas9 nucleases (*e.g.,* two *S. pyogenes* Cas9 nucleases) or two Cas9 nickases (*e.g.,* two *S. pyogenes* Cas9 nickases),are used. In certain embodiments, two 17-mer gRNAs are used to target two Cas9 nucleases or two Cas9 nickases, are used. Any of the targeting domains described herein can be used with a Cas9 molecule that generates a single-strand break (*i.e.,* a S. *pyogenes* or *S. aureus* Cas9 nickase) or with a Cas9 molecule that generates a double-strand break (*i.e., S. pyogenes* or *S. aureus* Cas9 nuclease).

gRNA molecules, as described herein, may comprise from 5' to 3': a targeting domain (comprising a "core domain", and optionally a "secondary domain"); a first complementarity domain; a linking domain; a second complementarity domain; a proximal domain; and a tail domain. In an embodiment, the proximal domain and tail domain are taken together as a single domain.

In an embodiment, a gRNA molecule comprises a linking domain of no more than 25 nucleotides in length; a proximal and tail domain, that taken together, are at least 20 nucleotides in length; and a targeting domain equal to or greater than 16, 17, 18, 19, 20, 21, 22, 23, 24, 25 or 26 nucleotides in length.

In another embodiment, a gRNA molecule comprises a linking domain of no more than 25 nucleotides in length; a proximal and tail domain, that taken together, are at least 25 nucleotides in length; and a targeting domain equal to or greater than 16, 17, 18, 19, 20, 21, 22, 23, 24, 25 or 26 nucleotides in length.

In another embodiment, a gRNA molecule comprises a linking domain of no more than 25 nucleotides in length; a proximal and tail domain, that taken together, are at least 30 nucleotides in length; and a targeting domain equal to or greater than 16, 17, 18, 19, 20, 21, 22, 23, 24, 25 or 26 nucleotides in length.

In another embodiment, a gRNA molecule comprises a linking domain of no more than 25 nucleotides in length; a proximal and tail domain, that taken together, are at least 40 nucleotides in length; and a targeting domain equal to or greater than 16, 17, 18, 19, 20, 21, 22, 23, 24, 25 or 26 nucleotides in length.

When two gRNAs are designed for use with two Cas9 molecules, the two Cas9 molecules may be from different species. Both Cas9 species may be used to generate a single or double strand break, as desired.

It is contemplated herein that any upstream gRNA described herein may be paired with any downstream gRNA described herein. When an upstream gRNA designed for use with one species of Cas9 molecule is paired with a downstream gRNA designed for use from a different species of Cas9 molecule, both Cas9 species are used to generate a single or double-strand break, as desired.

### V. Template Nucleic Acids

A "template nucleic acid," as that term is used herein, refers to a nucleic acid sequence which can be used in conjunction with a Cas9 molecule and a gRNA molecule and services as a guide for altering the structure of a target position. In one embodiment, the target nucleic acid is modified to have the some or all of the sequence of the template nucleic acid, typically at or near cleavage site(s). In one embodiment, the template nucleic acid is single stranded. In an alternate embodiment, the template nucleic acid is double stranded. In one embodiment, the template nucleic acid is DNA, *e.g.,* double stranded DNA. In an alternate embodiment, the template nucleic acid is single stranded DNA. In one embodiment, the template nucleic acid is encoded on the same vector backbone, *e.g.*, AAV genome, plasmid DNA, as the Cas9 and gRNA. In one embodiment, the template nucleic acid is excised from a vector backbone *in vivo, e.g.,* it is flanked by gRNA recognition sequences. In one embodiment, the template nucleic acid comprises endogenous genomic sequence. In one embodiment, the template nucleic acid is an RNA.

In one embodiment, the template nucleic acid alters the structure of the target position by participating in a homology directed repair event, *e.g.,* a gene correction event. In one embodiment, the template nucleic acid alters the sequence of the target position. In one embodiment, the template nucleic acid results in the incorporation of a modified, or non-naturally occurring base into the target nucleic acid.

Typically, the template sequence undergoes a breakage mediated or catalyzed recombination with the target sequence. In one embodiment, the template nucleic acid includes sequence that corresponds to a site on the target sequence that is cleaved by an eaCas9 mediated cleavage event. In one embodiment, the template nucleic acid includes sequence that corresponds to both, a first site on the target sequence that is cleaved in a first Cas9 mediated event, and a second site on the target sequence that is cleaved in a second Cas9 mediated event.

In one embodiment, the template nucleic acid can include sequence which results in an alteration in the coding sequence of a translated sequence, *e.g.,* one which results in the substitution of one amino acid for another in a protein product, *e.g.,* transforming a mutant allele into a wild type allele, transforming a wild type allele into a mutant allele, and/or introducing a stop codon, insertion of an amino acid residue, deletion of an amino acid residue, or a nonsense mutation.

In other embodiments, the template nucleic acid can include sequence which results in an alteration in a non-coding sequence, *e.g.,* an alteration in an exon or in a 5' or 3' non-translated or non-transcribed region. Such alterations include an alteration in a control element, *e.g.,* a promoter, enhancer, and an alteration in a cis-acting or trans-acting control element.

A template nucleic acid having homology with a target position in a gene, *e.g.,* a gene described herein, can be used to alter the structure of a target sequence. The template sequence can be used to alter an unwanted structure, *e.g.,* an unwanted or mutant nucleotide.

A template nucleic acid typically comprises the following components:
[5' homology arm]-[replacement sequence]-[3' homology arm].

The homology arms provide for recombination into the chromosome, thus replacing the undesired element, *e.g.,* a mutation or signature, with a replacement sequence. In one embodiment, the homology arms flank the most distal cleavage sites.

In one embodiment, the 3' end of the 5' homology arm is the position next to the 5' end of the replacement sequence. In one embodiment, the 5' homology arm can extend at least 10, 20, 30, 40, 50, 100, 200, 300, 400, 500, 600, 700, 800, 900, 1000, 1500, 2000, 3000, 4000, or 5000 nucleotides 5' from the 5' end of the replacement sequence.

In one embodiment, the 5' end of the 3' homology arm is the position next to the 3' end of the replacement sequence. In one embodiment, the 3' homology arm can extend at least 10, 20, 30, 40, 50, 100, 200, 300, 400, 500, 600, 700, 800, 900, 1000, 1500, 2000, 3000, 4000, or 5000 nucleotides 3' from the 3' end of the replacement sequence.

In one embodiment, to correct a mutation, the homology arms, *e.g.,* the 5' and 3' homology arms, may each comprise about 1000 base pairs (bp) of sequence flanking the most distal gRNAs (*e.g.,* 1000bp of sequence on either side of the mutation).

It is contemplated herein that one or both homology arms may be shortened to avoid including certain sequence repeat elements, *e.g.,* Alu repeats or LINE elements. For example, a 5' homology arm may be shortened to avoid a sequence repeat element. In other embodiments, a 3' homology arm may be shortened to avoid a sequence repeat element. In some embodiments, both the 5' and the 3' homology arms may be shortened to avoid including certain sequence repeat elements.

It is contemplated herein that template nucleic acids for correcting a mutation may be designed for use as a single-stranded oligonucleotide, *e.g.,* a single-stranded oligodeoxynucleotide (ssODN). When using a ssODN, 5' and 3' homology arms may range up to about 200 base pairs (bp) in length, *e.g.,* at least 25, 50, 75, 100, 125, 150, 175, or 200 bp in length. Longer homology arms are also contemplated for ssODNs as improvements in oligonucleotide synthesis continue to be made. In some embodiments, a longer homology arm is made by a method other than chemical synthesis, *e.g.,* by denaturing a long double stranded nucleic acid and purifying one of the strands, *e.g.,* by affinity for a strand-specific sequence anchored to a solid substrate.

While not wishing to be bound by theory, in some embodiments HDR proceeds more efficiently when the template nucleic acid has extended homology 5' to a nick (*i.e.,* in the 5' direction of the nicked strand). Accordingly, in some embodiments, the template nucleic acid has a longer homology arm and a shorter homology arm, wherein the longer homology arm can anneal 5' of the nick. In some embodiments, the arm that can anneal 5' to the nick is at least 25, 50, 75, 100, 125, 150, 175, or 200, 300, 400, 500, 600, 700, 800, 900, 1000, 1500, 2000, 3000, 4000, or 5000 nucleotides from the nick or the 5' or 3' end of the replacement sequence. In some embodiments, the arm that can anneal 5' to the nick is at least 10%, 20%, 30%, 40%, or 50% longer than the arm that can anneal 3' to the nick. In some embodiments, the arm that can anneal 5' to the nick is at least 2x, 3x, 4x, or 5x longer than the arm that can anneal 3' to the nick. Depending on whether a ssDNA template can anneal to the intact strand or the nicked strand, the homology arm that anneals 5' to the nick may be at the 5' end of the ssDNA template or the 3' end of the ssDNA template, respectively.

Similarly, in some embodiments, the template nucleic acid has a 5' homology arm, a replacement sequence, and a 3' homology arm, such that the template nucleic acid has extended homology to the 5' of the nick. For example, the 5' homology arm and 3' homology arm may be substantially the same length, but the replacement sequence may extend farther 5' of the nick than 3' of the nick. In some embodiments, the replacement sequence extends at least 10%, 20%, 30%, 40%, 50%, 2x, 3x, 4x, or 5x further to the 5' end of the nick than the 3' end of the nick.

While not wishing to be bound by theory, in some embodiments HDR proceeds more efficiently when the template nucleic acid is centered on the nick. Accordingly, in some embodiments, the template nucleic acid has two homology arms that are essentially the same size. For instance, the first homology arm of a template nucleic acid may have a length that is within 10%, 9%, 8%, 7%, 6%, 5%, 4%, 3%, 2%, or 1% of the second homology arm of the template nucleic acid.

Similarly, in some embodiments, the template nucleic acid has a 5' homology arm, a replacement sequence, and a 3' homology arm, such that the template nucleic acid extends substantially the same distance on either side of the nick. For example, the homology arms may have different lengths, but the replacement sequence may be selected to compensate for this. For example, the replacement sequence may extend further 5' from the nick than it does 3' of the nick, but the homology arm 5' of the nick is shorter than the homology arm 3' of the nick, to compensate. The converse is also possible, *e.g.,* that the replacement sequence may extend further 3' from the nick than it does 5' of the nick, but the homology arm 3' of the nick is shorter than the homology arm 5' of the nick, to compensate.

### Exemplary arrangements of linear nucleic acid template systems

In one embodiment, the template nucleic acid is double stranded. In one embodiment, the template nucleic acid is single stranded. In one embodiment, the nucleic acid template system comprises a single stranded portion and a double stranded portion. In one embodiment, the template nucleic acid comprises about 50 to 100, *e.g.,* 55 to 95, 60 to 90, 65 to 85, or 70 to 80, base pairs, homology on either side of the nick and/or replacement sequence. In one embodiment, the template nucleic acid comprises about 50, 55, 60, 65, 70, 75, 80, 85, 90, 95, or 100 base pairs homology 5' of the nick or replacement sequence, 3' of the nick or replacement sequence, or both 5' and 3' of the nick or replacement sequences.

In one embodiment, the template nucleic acid comprises about 150 to 200, *e.g.,* 155 to 195, 160 to 190, 165 to 185, or 170 to 180, base pairs homology 3' of the nick and/or replacement sequence. In one embodiment, the template nucleic acid comprises about 150, 155, 160, 165, 170, 175, 180, 185, 190, 195, or 200 base pairs homology 3' of the nick or replacement sequence. In one embodiment, the template nucleic acid comprises less than about 100, 90, 80, 70, 60, 50, 40, 30, 20, 15, or 10 base pairs homology 5' of the nick or replacement sequence.

In one embodiment, the template nucleic acid comprises about 150 to 200, *e.g.,* 155 to 195, 160 to 190, 165 to 185, or 170 to 180, base pairs homology 5' of the nick and/or replacement sequence. In one embodiment, the template nucleic acid comprises about 150, 155, 160, 165, 170, 175, 180, 185, 190, 195, or 200 base pairs homology 5' of the nick or replacement sequence. In one embodiment, the template nucleic acid comprises less than about 100, 90, 80, 70, 60, 50, 40, 30, 20, 15, or 10 base pairs homology 3' of the nick or replacement sequence.

### Exemplary Template Nucleic Acids

In one embodiment, the template nucleic acid is a single stranded nucleic acid. In another embodiment, the template nucleic acid is a double stranded nucleic acid. In some embodiments, the template nucleic acid comprises a nucleotide sequence, *e.g.,* of one or more nucleotides, that will be added to or will serve as a template for a change in the target nucleic acid. In other embodiments, the template nucleic acid comprises a nucleotide sequence that may be used to modify the target position. In other embodiments, the template nucleic acid comprises a nucleotide sequence, *e.g.,* of one or more nucleotides, that corresponds to wild type sequence of the target nucleic acid, *e.g.,* of the target position.

The template nucleic acid may comprise a replacement sequence. A replacement sequence, as the term is used herein, refers to a sequence which will serve as the template for making the desired change, or correction, in the target nucleic acid. The replacement sequence may be homologous, but not identical to, the target nucleic acid. In some embodiments, the template nucleic acid comprises a 5' homology arm. In other embodiments, the template nucleic acid comprises a 3' homology arm.

In embodiments, the template nucleic acid is linear double stranded DNA. The length may be, *e.g.,* about 150-200 base pairs, *e.g.,* about 150, 160, 170, 180, 190, or 200 base pairs. The length may be, *e.g.,* at least 150, 160, 170, 180, 190, or 200 base pairs. In some embodiments, the length is no greater than 150, 160, 170, 180, 190, or 200 base pairs. In some embodiments, a double stranded template nucleic acid has a length of about 160 base pairs, *e.g.,* about 155-165, 150-170, 140-180, 130-190, 120-200, 110-210, 100-220, 90-230, or 80-240 base pairs.

The template nucleic acid can be linear single stranded DNA. In embodiments, the template nucleic acid is (i) linear single stranded DNA that can anneal to the nicked strand of the target nucleic acid, (ii) linear single stranded DNA that can anneal to the intact strand of the target nucleic acid, (iii) linear single stranded DNA that can anneal to the transcribed strand of the target nucleic acid, (iv) linear single stranded DNA that can anneal to the non-transcribed strand of the target nucleic acid, or more than one of the preceding. The length may be, *e.g.,* about 150-200 nucleotides, *e.g.,* about 150, 160, 170, 180, 190, or 200 nucleotides. The length may be, *e.g.,* at least 150, 160, 170, 180, 190, or 200 nucleotides. In some embodiments, the length is no greater than 150, 160, 170, 180, 190, or 200 nucleotides. In some embodiments, a single stranded template nucleic acid has a length of about 160 nucleotides, *e.g.,* about 155-165, 150-170, 140-180, 130-190, 120-200, 110-210, 100-220, 90-230, or 80-240 nucleotides.

In some embodiments, the template nucleic acid is circular double stranded DNA, *e.g.,* a plasmid. In some embodiments, the template nucleic acid comprises about 500 to 1000 base pairs of homology on either side of the replacement sequence and/or the nick. In some embodiments, the template nucleic acid comprises about 300, 400, 500, 600, 700, 800, 900, 1000, 1500, or 2000 base pairs of homology 5' of the nick or replacement sequence, 3' of the nick or replacement sequence, or both 5' and 3' of the nick or replacement sequence. In some embodiments, the template nucleic acid comprises at least 300, 400, 500, 600, 700, 800, 900, 1000, 1500, or 2000 base pairs of homology 5' of the nick or replacement sequence, 3' of the nick or replacement sequence, or both 5' and 3' of the nick or replacement sequence. In some embodiments, the template nucleic acid comprises no more than 300, 400, 500, 600, 700, 800, 900, 1000, 1500, or 2000 base pairs of homology 5' of the nick or replacement sequence, 3' of the nick or replacement sequence, or both 5' and 3' of the nick or replacement sequence.

In some embodiments, the template nucleic acid is an adenovirus vector, *e.g.,* an AAV vector, *e.g.,* a ssDNA molecule of a length and sequence that allows it to be packaged in an AAV capsid. The vector may be, *e.g.,* less than 5 kb and may contain an ITR sequence that promotes packaging into the capsid. The vector may be integration-deficient. In some embodiments, the template nucleic acid comprises about 150 to 1000 nucleotides of homology on either side of the replacement sequence and/or the nick. In some embodiments, the template nucleic acid comprises about 100, 150, 200, 300, 400, 500, 600, 700, 800, 900, 1000, 1500, or 2000 nucleotides 5' of the nick or replacement sequence, 3' of the nick or replacement sequence, or both 5' and 3' of the nick or replacement sequence. In some embodiments, the template nucleic acid comprises at least 100, 150, 200, 300, 400, 500, 600, 700, 800, 900, 1000, 1500, or 2000 nucleotides 5' of the nick or replacement sequence, 3' of the nick or replacement sequence, or both 5' and 3' of the nick or replacement sequence. In some embodiments, the template nucleic acid comprises at most 100, 150, 200, 300, 400, 500, 600, 700, 800, 900, 1000, 1500, or 2000 nucleotides 5' of the nick or replacement sequence, 3' of the nick or replacement sequence, or both 5' and 3' of the nick or replacement sequence.

In some embodiments, the template nucleic acid is a lentiviral vector, *e.g.,* an IDLV (integration deficiency lentivirus). In some embodiments, the template nucleic acid comprises about 500 to 1000 base pairs of homology on either side of the replacement sequence and/or the nick. In some embodiments, the template nucleic acid comprises about 300, 400, 500, 600, 700, 800, 900, 1000, 1500, or 2000 base pairs of homology 5' of the nick or replacement sequence, 3' of the nick or replacement sequence, or both 5' and 3' of the nick or replacement sequence. In some embodiments, the template nucleic acid comprises at least 300, 400, 500, 600, 700, 800, 900, 1000, 1500, or 2000 base pairs of homology 5' of the nick or replacement sequence, 3' of the nick or replacement sequence, or both 5' and 3' of the nick or replacement sequence. In some embodiments, the template nucleic acid comprises no more than 300, 400, 500, 600, 700, 800, 900, 1000, 1500, or 2000 base pairs of homology 5' of the nick or replacement sequence, 3' of the nick or replacement sequence, or both 5' and 3' of the nick or replacement sequence.

In one embodiment, the template nucleic acid comprises one or more mutations, *e.g.,* silent mutations, that prevent Cas9 from recognizing and cleaving the template nucleic acid. The template nucleic acid may comprise, *e.g.,* at least 1, 2, 3, 4, 5, 10, 20, or 30 silent mutations relative to the corresponding sequence in the genome of the cell to be altered. In embodiments, the template nucleic acid comprises at most 2, 3, 4, 5, 10, 20, 30, or 50 silent mutations relative to the corresponding sequence in the genome of the cell to be altered. In one embodiment, the cDNA comprises one or more mutations, *e.g.,* silent mutations that prevent Cas9 from recognizing and cleaving the template nucleic acid. The template nucleic acid may comprise, *e.g.,* at least 1, 2, 3, 4, 5, 10, 20, or 30 silent mutations relative to the corresponding sequence in the genome of the cell to be altered. In embodiments, the template nucleic acid comprises at most 2, 3, 4, 5, 10, 20, 30, or 50 silent mutations relative to the corresponding sequence in the genome of the cell to be altered.

In one embodiment, the template nucleic acid alters the structure of the target position by participating in a homology directed repair event. In one embodiment, the template nucleic acid alters the sequence of the target position. In one embodiment, the template nucleic acid results in the incorporation of a modified, or non-naturally occurring base into the target nucleic acid.

Typically, the template sequence undergoes a breakage mediated or catalyzed recombination with the target sequence. In one embodiment, the template nucleic acid includes sequence that corresponds to a site on the target sequence that is cleaved by an eaCas9 mediated cleavage event. In one embodiment, the template nucleic acid includes sequence that corresponds to both, a first site on the target sequence that is cleaved in a first Cas9 mediated event, and a second site on the target sequence that is cleaved in a second Cas9 mediated event.

In one embodiment, the template nucleic acid can include sequence which results in an alteration in the coding sequence of a translated sequence, *e.g.,* one which results in the substitution of one amino acid for another in a protein product, *e.g.,* transforming a mutant allele into a wild type allele, transforming a wild type allele into a mutant allele, and/or introducing a stop codon, insertion of an amino acid residue, deletion of an amino acid residue, or a nonsense mutation.

In other embodiments, the template nucleic acid can include sequence which results in an alteration in a non-coding sequence, *e.g.,* an alteration in an exon or in a 5' or 3' non-translated or non-transcribed region. Such alterations include an alteration in a control element, *e.g.,* a promoter, enhancer, and an alteration in a cis-acting or trans-acting control element.

A template nucleic acid having homology with a target position can be used to alter the structure of a target sequence. The template sequence can be used to alter an unwanted structure, *e.g.,* an unwanted or mutant nucleotide.

**Table A** below provides exemplary template nucleic acids. In one embodiment, the template nucleic acid includes the 5' homology arm and the 3' homology arm of a row from **Table A**. In another embodiment, a 5' homology arm from the first column can be combined with a 3' homology arm from **Table A**. In each embodiment, a combination of the 5' and 3' homology arms include a replacement sequence.

**Table A**

| Length of the 5' homology arm (the number of nucleotides) | Replacement Sequence: G, A, C or T, as described herein | Length of the 3' homology arm (the number of nucleotides) |
|---|---|---|
| 10 or more | | 10 or more |
| 20 or more | | 20 or more |
| 50 or more | | 50 or more |
| 100 or more | | 100 or more |
| 150 or more | | 150 or more |
| 200 or more | | 200 or more |
| 250 or more | | 250 or more |
| 300 or more | | 300 or more |
| 350 or more | | 350 or more |
| 400 or more | | 400 or more |
| 450 or more | | 450 or more |
| 500 or more | | 500 or more |
| 550 or more | | 550 or more |
| 600 or more | | 600 or more |
| 650 or more | | 650 or more |
| 700 or more | | 700 or more |
| 750 or more | | 750 or more |
| 800 or more | | 800 or more |
| 850 or more | | 850 or more |
| 900 or more | | 900 or more |
| 1000 or more | | 1000 or more |
| 1100 or more | | 1100 or more |
| 1200 or more | | 1200 or more |
| 1300 or more | | 1300 or more |
| 1400 or more | | 1400 or more |
| 1500 or more | | 1500 or more |
| 1600 or more | | 1600 or more |
| 1700 or more | | 1700 or more |
| 1800 or more | | 1800 or more |
| 1900 or more | | 1900 or more |
| 1200 or more | | 1200 or more |
| At least 50 but not long enough to include a repeated element. | | At least 50 but not long enough to include a repeated element. |
| At least 100 but not long enough to include a repeated element. | | At least 100 but not long enough to include a repeated element. |
| At least 150 but not long enough to include a repeated element. | | At least 150 but not long enough to include a repeated element. |
| 5 to 100 nucleotides | | 5 to 100 nucleotides |
| 10 to 150 nucleotides | | 10 to 150 nucleotides |
| 20 to 150 nucleotides | | 20 to 150 nucleotides |
| Template Construct | | |

### VI. Cas9 Molecules

Cas9 molecules of a variety of species can be used in the methods and compositions described herein. While *S. pyogenes* and *S. aureus* Cas9 molecules are the subject of much of the disclosure herein, Cas9 molecules of, derived from, or based on the Cas9 proteins of other species listed herein can be used as well. These include, for example, Cas9 molecules *from Acidovorax avenae, Actinobacillus pleuropneumoniae, Actinobacillus succinogenes, Actinobacillus suis, Actinomyces sp., cycliphilus denitrificans, Aminomonas paucivorans, Bacillus cereus, Bacillus smithii, Bacillus thuringiensis, Bacteroides sp., Blastopirellula marina, Bradyrhizobium sp., Brevibacillus laterosporus, Campylobacter coli, Campylobacter jejuni, Campylobacter lari, Candidatus Puniceispirillum, Clostridium cellulolyticum, Clostridium perfringens, Corynebacterium accolens, Corynebacterium diphtheria, Corynebacterium matruchotii, Dinoroseobacter shibae, Eubacterium dolichum, gamma proteobacterium, Gluconacetobacter diazotrophicus, Haemophilus parainfluenzae, Haemophilus sputorum, Helicobacter canadensis, Helicobacter cinaedi, Helicobacter mustelae, Ilyobacter polytropus, Kingella kingae, Lactobacillus crispatus, Listeria ivanovii, Listeria monocytogenes, Listeriaceae bacterium, Methylocystis sp., Methylosinus trichosporium, Mobiluncus mulieris, Neisseria bacilliformis, Neisseria cinerea, Neisseria flavescens, Neisseria lactamica, Neisseria meningitidis, Neisseria sp., Neisseria wadsworthii, Nitrosomonas sp., Parvibaculum lavamentivorans, Pasteurella multocida, Phascolarctobacterium succinatutens, Ralstonia syzygii, Rhodopseudomonas palustris, Rhodovulum sp., Simonsiella muelleri, Sphingomonas sp., Sporolactobacillus vineae, Staphylococcus lugdunensis, Streptococcus sp., Subdoligranulum sp., Tistrella mobilis, Treponema sp.,* or *Verminephrobacter eiseniae.* The amino acid sequences of exemplary Cas9 orthologs are set forth in the sequence listing.

### Cas9 Domains

Crystal structures have been determined for two different naturally occurring bacterial Cas9 molecules (Jinek *et al.* 2014) and for *S. pyogenes* Cas9 with a guide RNA (*e.g.,* a synthetic fusion of crRNA and tracrRNA) (Nishimasu *et al.* 2014; and Anders 2014).

A naturally-occurring Cas9 molecule comprises two lobes: a recognition (REC) lobe and a nuclease (NUC) lobe; each of which further comprise domains described herein. The domain nomenclature and the numbering of the amino acid residues encompassed by each domain used throughout this disclosure is as described previously in (Nishimasu 2014). The numbering of the amino acid residues is with reference to Cas9 from *S. pyogenes.*

The REC lobe comprises the arginine-rich bridge helix (BH), the REC1 domain, and the REC2 domain. The REC lobe does not share structural similarity with other known proteins, indicating that it is a Cas9-specific functional domain. The BH domain is a long α helix and arginine rich region and comprises amino acids 60-93 of the sequence of S. *pyogenes* Cas9. The REC1 domain is important for recognition of the repeat:anti-repeat duplex, *e.g.,* of a gRNA or a tracrRNA, and is therefore critical for Cas9 activity by recognizing the target sequence. The REC1 domain comprises two REC1 motifs at amino acids 94 to 179 and 308 to 717 of the sequence of *S. pyogenes* Cas9. These two REC1 domains, though separated by the REC2 domain in the linear primary structure, assemble in the tertiary structure to form the REC1 domain. The REC2 domain, or parts thereof, may also play a role in the recognition of the repeat:anti-repeat duplex. The REC2 domain comprises amino acids 180-307 of the sequence of *S. pyogenes* Cas9.

The NUC lobe comprises the RuvC domain, the HNH domain, and the PAM-interacting (PI) domain. The RuvC domain shares structural similarity to retroviral integrase superfamily members and cleaves a single strand, *e.g.,* the non-complementary strand of the target nucleic acid molecule. The RuvC domain is assembled from the three split RuvC motifs (RuvC I, RuvCII, and RuvCIII, which are often commonly referred to in the art as RuvCI domain, or N-terminal RuvC domain, RuvCII domain, and RuvCIII domain) at amino acids 1-59, 718-769, and 909-1098, respectively, of the sequence of *S. pyogenes* Cas9. Similar to the REC1 domain, the three RuvC motifs are linearly separated by other domains in the primary structure, however in the tertiary structure, the three RuvC motifs assemble and form the RuvC domain. The HNH domain shares structural similarity with HNH endonucleases, and cleaves a single strand, *e.g.,* the complementary strand of the target nucleic acid molecule. The HNH domain lies between the RuvC II-III motifs and comprises amino acids 775-908 of the sequence of *S. pyogenes* Cas9. The PI domain interacts with the PAM of the target nucleic acid molecule, and comprises amino acids 1099-1368 of the sequence of *S*. *pyogenes* Cas9.

### RuvC-like domain and an HNH-like domain

In certain embodiments, a Cas9 molecule or Cas9 polypeptide comprises an HNH-like domain and a RuvC-like domain and in certain of these embodiments cleavage activity is dependent on the RuvC-like domain and the HNH-like domain. A Cas9 molecule or Cas9 polypeptide can comprise one or more of a RuvC-like domain and an HNH-like domain. In certain embodiments, a Cas9 molecule or Cas9 polypeptide comprises a RuvC-like domain, *e.g.,* a RuvC-like domain described below, and/or an HNH-like domain, *e.g.,* an HNH-like domain described below.

### RuvC-like domains

In certain embodiments, a RuvC-like domain cleaves, a single strand, *e.g.,* the non-complementary strand of the target nucleic acid molecule. The Cas9 molecule or Cas9 polypeptide can include more than one RuvC-like domain (*e.g*., one, two, three or more RuvC-like domains). In certain embodiments, a RuvC-like domain is at least 5, 6, 7, 8 amino acids in length but not more than 20, 19, 18, 17, 16 or 15 amino acids in length. In certain embodiments, the Cas9 molecule or Cas9 polypeptide comprises an N-terminal RuvC-like domain of about 10 to 20 amino acids, *e.g.,* about 15 amino acids in length.

### N-terminal RuvC-like domains

Some naturally occurring Cas9 molecules comprise more than one RuvC-like domain with cleavage being dependent on the N-terminal RuvC-like domain. Accordingly, a Cas9 molecule or Cas9 polypeptide can comprise an N-terminal RuvC-like domain. Exemplary N-terminal RuvC-like domains are described below.

In certain embodiments, a Cas9 molecule or Cas9 polypeptide comprises an N-terminal RuvC-like domain comprising an amino acid sequence of Formula I: D-X₁-G-X₂-X₃-X₄-X₅-G-X₆-X₇-X₈-X₉ (SEQ ID NO: 8),
wherein,
X₁ is selected from I, V, M, L and T (*e.g.,* selected from I, V, and L);
X₂ is selected from T, I, V, S, N, Y, E and L (*e.g.,* selected from T, V, and I);
X₃ is selected from N, S, G, A, D, T, R, M and F (*e.g.,* A or N);
X₄ is selected from S, Y, N and F (*e.g.,* S);
X₅ is selected from V, I, L, C, T and F (*e.g.,* selected from V, I and L);
X₆ is selected from W, F, V, Y, S and L (*e.g.,* W);
X₇ is selected from A, S, C, V and G (*e.g.,* selected from A and S);
X₈ is selected from V, I, L, A, M and H (*e.g.,* selected from V, I, M and L); and
X₉ is selected from any amino acid or is absent (*e.g.,* selected from T, V, I, L, Δ, F, S, A, Y, M and R, or, *e.g.,* selected from T, V, I, L and Δ).

In certain embodiments, the N-terminal RuvC-like domain differs from a sequence of SEQ ID NO:8, by as many as 1 but no more than 2, 3, 4, or 5 residues.

In certain embodiments, the N-terminal RuvC-like domain is cleavage competent.

In other embodiments, the N-terminal RuvC-like domain is cleavage incompetent.

In certain embodiments, a Cas9 molecule or Cas9 polypeptide comprises an N-terminal RuvC-like domain comprising an amino acid sequence of Formula II:
D-X₁-G-X₂-X₃-S-X₅-G-X₆-X₇-X₈-X₉, (SEQ ID NO: 9),
wherein
X₁ is selected from I, V, M, L and T (*e.g.,* selected from I, V, and L);
X₂ is selected from T, I, V, S, N, Y, E and L (*e.g.,* selected from T, V, and I);
X₃ is selected from N, S, G, A, D, T, R, M and F (*e.g.,* A or N);
X₅ is selected from V, I, L, C, T and F (*e.g.,* selected from V, I and L);
X₆ is selected from W, F, V, Y, S and L (*e.g.,* W);
X₇ is selected from A, S, C, V and G (*e.g.,* selected from A and S);
X₈ is selected from V, I, L, A, M and H (*e.g.,* selected from V, I, M and L); and
X₉ is selected from any amino acid or is absent (*e.g.,* selected from T, V, I, L, Δ, F, S, A, Y, M and R or selected from *e.g.,* T, V, I, L and Δ).

In certain embodiments, the N-terminal RuvC-like domain differs from a sequence of SEQ ID NO:9 by as many as 1 but not more than 2, 3, 4, or 5 residues.

In certain embodiments, the N-terminal RuvC-like domain comprises an amino acid sequence of Formula III:
D-I-G-X₂-X₃-S-V-G-W-A-X₈-X₉ (SEQ ID NO: 10),
wherein
X₂ is selected from T, I, V, S, N, Y, E and L (*e.g.,* selected from T, V, and I);
X₃ is selected from N, S, G, A, D, T, R, M and F (*e.g.,* A or N);
X₈ is selected from V, I, L, A, M and H (*e.g.,* selected from V, I, M and L); and
X₉ is selected from any amino acid or is absent (*e.g.,* selected from T, V, I, L, Δ, F, S, A, Y, M and R or selected from *e.g.,* T, V, I, L and Δ).
In certain embodiments, the N-terminal RuvC-like domain differs from a sequence of SEQ ID NO:10 by as many as 1 but not more than, 2, 3, 4, or 5 residues.

In certain embodiments, the N-terminal RuvC-like domain comprises an amino acid sequence of Formula IV:
D-I-G-T-N-S-V-G-W-A-V-X (SEQ ID NO: 11),
wherein
X is a non-polar alkyl amino acid or a hydroxyl amino acid, *e.g.,* X is selected from V, I, L and T.

In certain embodiments, the N-terminal RuvC-like domain differs from a sequence of SEQ ID NO:11 by as many as 1 but not more than, 2, 3, 4, or 5 residues.

In certain embodiments, the N-terminal RuvC-like domain differs from a sequence of an N-terminal RuvC like domain disclosed herein, *e.g.,* in any one of SEQ ID Nos: 54-103, as many as 1 but no more than 2, 3, 4, or 5 residues. In certain embodiments, 1, 2, 3 or all of the highly conserved residues of SEQ ID Nos: 54-103 are present.

In certain embodiment, the N-terminal RuvC-like domain differs from a sequence of an N-terminal RuvC-like domain disclosed herein, *e.g.,* in any one of SEQ ID Nos: 104-177, as many as 1 but no more than 2, 3, 4, or 5 residues. In certain embodiments, 1, 2, or all of the highly conserved residues identified of SEQ ID Nos: 104-177 are present.

### Additional RuvC-like domains

In addition to the N-terminal RuvC-like domain, the Cas9 molecule or Cas9 polypeptide can comprise one or more additional RuvC-like domains. In certain embodiments, the Cas9 molecule or Cas9 polypeptide can comprise two additional RuvC-like domains. Preferably, the additional RuvC-like domain is at least 5 amino acids in length and, *e.g.,* less than 15 amino acids in length, *e.g.,* 5 to 10 amino acids in length, *e.g.,* 8 amino acids in length.

An additional RuvC-like domain can comprise an amino acid sequence of Formula V: I-X₁-X₂-E-X₃-A-R-E (SEQ ID NO:12), wherein
X₁ is V or H;
X₂ is I, L or V (*e.g.,* I or V); and
X₃ is M or T.

In certain embodiments, the additional RuvC-like domain comprises an amino acid sequence of FormulaVI:
I-V-X₂-E-M-A-R-E (SEQ ID NO:13), wherein
X₂ is I, L or V (*e.g.,* I or V).

An additional RuvC-like domain can comprise an amino acid sequence of Formula VII:
H-H-A-X₁-D-A-X₂-X₃ (SEQ ID NO: 14), wherein
X₁ is H or L;
X₂ is R or V; and
X₃ is E or V.

In certain embodiments, the additional RuvC-like domain comprises the amino acid sequence: H-H-A-H-D-A-Y-L (SEQ ID NO:15).

In certain embodiments, the additional RuvC-like domain differs from a sequence of SEQ ID NOs: 12-15 by as many as 1 but not more than 2, 3, 4, or 5 residues.

In certain embodiment, the sequence flanking the N-terminal RuvC-like domain has the amino acid sequence of Formula VIII:
K-X₁'-Y-X₂'-X₃'-X₄'-Z-T-D-X₉'-Y, (SEQ ID NO: 16).
wherein
X₁' is selected from K and P;
X₂' is selected from V, L, I, and F (*e.g.,* V, I and L);
X₃' is selected from G, A and S (*e.g.,* G);
X₄' is selected from L, I, V and F (*e.g.,* L);
X₉' is selected from D, E, N and Q; and
Z is an N-terminal RuvC-like domain, *e.g.,* as described above, *e.g.,* having 5 to 20 amino acids.

### HNH-like domains

In certain embodiments, an HNH-like domain cleaves a single stranded complementary domain, *e.g.,* a complementary strand of a double stranded nucleic acid molecule. In certain embodiments, an HNH-like domain is at least 15, 20, or 25 amino acids in length but not more than 40, 35, or 30 amino acids in length, *e.g.,* 20 to 35 amino acids in length, *e.g.,* 25 to 30 amino acids in length. Exemplary HNH-like domains are described below.

In certain embodiments, a Cas9 molecule or Cas9 polypeptide comprises an HNH-like domain having an amino acid sequence of Formula IX:
X₁-X₂-X₃-H-X₄-X₅-P-X₆-X₇-X₈-X₉-X₁₀-X₁₁-X₁₂-X₁₃-X₁₄-X₁₅-N-X₁₆-X₁₇-X₁₈-X₁₉-X₂₀-X₂₁-X₂₂-X₂₃-N (SEQ ID NO: 17), wherein
X₁ is selected from D, E, Q and N (*e.g.,* D and E);
X₂ is selected from L, I, R, Q, V, M and K;
X₃ is selected from D and E;
X₄ is selected from I, V, T, A and L (*e.g.,* A, I and V);
X₅ is selected from V, Y, I, L, F and W (*e.g.,* V, I and L);
X₆ is selected from Q, H, R, K, Y, I, L, F and W;
X₇ is selected from S, A, D, T and K (*e.g.,* S and A);
X₈ is selected from F, L, V, K, Y, M, I, R, A, E, D and Q (*e.g.,* F);
X₉ is selected from L, R, T, I, V, S, C, Y, K, F and G;
X₁₀ is selected from K, Q, Y, T, F, L, W, M, A, E, G, and S;
X₁₁ is selected from D, S, N, R, L and T (*e.g.,* D);
X₁₂ is selected from D, N and S;
X₁₃ is selected from S, A, T, G and R (*e.g.,* S);
X₁₄ is selected from I, L, F, S, R, Y, Q, W, D, K and H (*e.g.,* I, L and F);
X₁₅ is selected from D, S, I, N, E, A, H, F, L, Q, M, G, Y and V;
X₁₆ is selected from K, L, R, M, T and F (*e.g.,* L, R and K);
X₁₇ is selected from V, L, I, A and T;
X₁₈ is selected from L, I, V and A (*e.g.,* L and I);
X₁₉ is selected from T, V, C, E, S and A (*e.g.,* T and V);
X₂₀ is selected from R, F, T, W, E, L, N, C, K, V, S, Q, I, Y, H and A;
X₂₁ is selected from S, P, R, K, N, A, H, Q, G and L;
X₂₂ is selected from D, G, T, N, S, K, A, I, E, L, Q, R and Y; and
X₂₃ is selected from K, V, A, E, Y, I, C, L, S, T, G, K, M, D and F.

In certain embodiments, a HNH-like domain differs from a sequence of SEQ ID NO: 17 by at least one but not more than, 2, 3, 4, or 5 residues.

In certain embodiments, the HNH-like domain is cleavage competent.

In other embodiments, the HNH-like domain is cleavage incompetent.

In certain embodiments, a Cas9 molecule or Cas9 polypeptide comprises an HNH-like domain comprising an amino acid sequence of Formula X:
X₁-X₂-X₃-H-X₄-X₅-P-X₆-S-X₈-X₉-X₁₀-D-D-S-X₁₄-X₁₅-N-K-V-L-X₁₉-X₂₀-X₂₁-X₂₂-X₂₃-N (SEQ ID NO: 18),
wherein
X₁ is selected from D and E;
X₂ is selected from L, I, R, Q, V, M and K;
X₃ is selected from D and E;
X₄ is selected from I, V, T, A and L (*e.g.,* A, I and V);
X₅ is selected from V, Y, I, L, F and W (*e.g.,* V, I and L);
X₆ is selected from Q, H, R, K, Y, I, L, F and W;
X₈ is selected from F, L, V, K, Y, M, I, R, A, E, D and Q (*e.g.,* F);
X₉ is selected from L, R, T, I, V, S, C, Y, K, F and G;
X₁₀ is selected from K, Q, Y, T, F, L, W, M, A, E, G, and S;
X₁₄ is selected from I, L, F, S, R, Y, Q, W, D, K and H (*e.g.,* I, L and F);
X₁₅ is selected from D, S, I, N, E, A, H, F, L, Q, M, G, Y and V;
X₁₉ is selected from T, V, C, E, S and A (*e.g.,* T and V);
X₂₀ is selected from R, F, T, W, E, L, N, C, K, V, S, Q, I, Y, H and A;
X₂₁ is selected from S, P, R, K, N, A, H, Q, G and L;
X₂₂ is selected from D, G, T, N, S, K, A, I, E, L, Q, R and Y; and
X₂₃ is selected from K, V, A, E, Y, I, C, L, S, T, G, K, M, D and F.

In certain embodiments, the HNH-like domain differs from a sequence of SEQ ID NO: 18 by 1, 2, 3, 4, or 5 residues.

In certain embodiments, a Cas9 molecule or Cas9 polypeptide comprises an HNH-like domain comprising an amino acid sequence of Formula XI:
X₁-V-X₃-H-I-V-P-X₆-S-X_{S}-X₉-X₁₀-D-D-S-X₁₄-X₁₅-N-K-V-L-T-X₂₀-X₂₁-X₂₂-X₂₃-N (SEQ ID NO: 19),
wherein
X₁ is selected from D and E;
X₃ is selected from D and E;
X₆ is selected from Q, H, R, K, Y, I, L and W;
X₈ is selected from F, L, V, K, Y, M, I, R, A, E, D and Q (*e.g.,* F);
X₉ is selected from L, R, T, I, V, S, C, Y, K, F and G;
X₁₀ is selected from K, Q, Y, T, F, L, W, M, A, E, G, and S;
X₁₄ is selected from I, L, F, S, R, Y, Q, W, D, K and H (*e.g.,* I, L and F);
X₁₅ is selected from D, S, I, N, E, A, H, F, L, Q, M, G, Y and V;
X₂₀ is selected from R, F, T, W, E, L, N, C, K, V, S, Q, I, Y, H and A;
X₂₁ is selected from S, P, R, K, N, A, H, Q, G and L;
X₂₂ is selected from D, G, T, N, S, K, A, I, E, L, Q, R and Y; and
X₂₃ is selected from K, V, A, E, Y, I, C, L, S, T, G, K, M, D and F.

In certain embodiments, the HNH-like domain differs from a sequence of SEQ ID NO: 19 by 1, 2, 3, 4, or 5 residues.

In certain embodiments, a Cas9 molecule or Cas9 polypeptide comprises an HNH-like domain having an amino acid sequence of Formula XII:
D-X₂-D-H-I-X₅-P-Q-X₇-F-X₉-X₁₀-D-X₁₂-S-I-D-N-X₁₆-V-L-X₁₉-X₂₀-S-X₂₂-X₂₃-N (SEQ ID NO:20),
wherein
X₂ is selected from I and V;
X₅ is selected from I and V;
X₇ is selected from A and S;
X₉ is selected from I and L;
X₁₀ is selected from K and T;
X₁₂ is selected from D and N;
X₁₆ is selected from R, K and L;
X₁₉ is selected from T and V;
X₂₀ is selected from S and R;
X₂₂ is selected from K, D and A; and
X₂₃ is selected from E, K, G and N (*e.g.,* the Cas9 molecule or Cas9 polypeptide can comprise an HNH-like domain as described herein).

In certain embodiments, the HNH-like domain differs from a sequence of SEQ ID NO: 20 by as many as 1 but not more than 2, 3, 4, or 5 residues.

In certain embodiments, a Cas9 molecule or Cas9 polypeptide comprises the amino acid sequence of formula XIII:
L-Y-Y-L-Q-N-G-X₁'-D-M-Y-X₂'-X₃'-X₄'-X₅'-L-D-I-X₆'-X₇'-L-S-X₈'-Y-Z-N-R-X₉'-K-X₁₀'-D-X₁₁'-V-P (SEQ ID NO: 21),
wherein
X₁' is selected from K and R;
X₂' is selected from V and T;
X₃' is selected from G and D;
X₄' is selected from E, Q and D;
X₅' is selected from E and D;
X₆' is selected from D, N and H;
X₇' is selected from Y, R and N;
X₈' is selected from Q, D and N;
X₉' is selected from G and E;
X₁₀' is selected from S and G;
X₁₁' is selected from D and N; and
Z is an HNH-like domain, *e.g.*, as described above.

In certain embodiment, a Cas9 molecule or Cas9 polypeptide comprises an amino acid sequence that differs from a sequence of SEQ ID NO:21 by as many as 1 but not more than 2, 3, 4, or 5 residues.

In certain embodiments, the HNH-like domain differs from a sequence of an HNH-like domain disclosed herein by as many as 1 but not more than 2, 3, 4, or 5 residues. In certain embodiments, 1 or both of the highly conserved residues are present.

In certain embodiments, the HNH -like domain differs from a sequence of an HNH-like domain disclosed herein by as many as 1 but not more than 2, 3, 4, or 5 residues. In certain embodiments, 1, 2, all 3 of the highly conserved residues are present.

### Inducible Cas9 Molecules and Gene Editing Systems

In some embodiments, the Cas9 fusion molecule comprises an inducible Cas9 molecule, as described in more detail in WO15/089427 and WO14/018423. Inducible Cas9 molecules are summarized briefly, below.

In one aspect, disclosed herein is a non-naturally occurring or engineered gene editing system, comprising a Cas9 molecule, which may comprise at least one switch, wherein the activity of said gene editing system is controlled by contact with at least one inducer energy source as to the switch. In an embodiment, the control as to the at least one switch or the activity of the gene editing system may be activated, enhanced, terminated or repressed. The contact with the at least one inducer energy source may result in a first effect and a second effect. The first effect may be one or more of nuclear import, nuclear export, recruitment of a secondary component (such as an effector molecule), conformational change (of protein, DNA or RNA), cleavage, release of cargo (such as a caged molecule or a co-factor), association or dissociation. The second effect may be one or more of activation, enhancement, termination or repression of the control as to the at least one switch or the activity of the gene editing system. In one embodiment, the first effect and the second effect may occur in a cascade.

In one embodiment, the Cas9 molecule may further comprise at least one nuclear localization signal (NLS), nuclear export signal (NES), functional domain, flexible linker, mutation, deletion, alteration or truncation. The one or more of the NLS, the NES or the functional domain may be conditionally activated or inactivated. In another embodiment, the mutation may be one or more of a mutation in a transcription factor homology region, a mutation in a DNA binding domain (such as mutating basic residues of a basic helix loop helix), a mutation in an endogenous NLS or a mutation in an endogenous NES. The disclosure comprehends that the inducer energy source may be heat, ultrasound, electromagnetic energy or chemical. The inducer energy source may be an antibiotic, a small molecule, a hormone, a hormone derivative, a steroid or a steroid derivative. The inducer energy source maybe abscisic acid (ABA), doxycycline (DOX), cumate, rapamycin, 4-hydroxytamoxifen (40HT), estrogen or ecdysone. The disclosure also provides that the at least one switch may be selected from the group consisting of antibiotic based inducible systems, electromagnetic energy based inducible systems, small molecule based inducible systems, nuclear receptor based inducible systems and hormone based inducible systems. In a more preferred embodiment, the at least one switch may be selected from the group consisting of tetracycline (Tet)/DOX inducible systems, light inducible systems, ABA inducible systems, cumate repressor/operator systems, 40HT/estrogen inducible systems, ecdysone-based inducible systems and FKBP12/FRAP (FKBP12-rapamycin complex) inducible systems.

The at least one functional domain may be selected from the group consisting of: transposase domain, integrase domain, recombinase domain, resolvase domain, invertase domain, protease domain, DNA methyltransferase domain, DNA hydroxylmethylase domain, DNA demethylase domain, histone acetylase domain, histone deacetylases domain, nuclease domain, repressor domain, activator domain, nuclear-localization signal domains, transcription-regulatory protein (or transcription complex recruiting) domain, cellular uptake activity associated domain, nucleic acid binding domain, antibody presentation domain, histone modifying enzymes, recruiter of histone modifying enzymes; inhibitor of histone modifying enzymes, histone methyltransferase, histone demethylase, histone kinase, histone phosphatase, histone ribosylase, histone deribosylase, histone ubiquitinase, histone deubiquitinase, histone biotinase or histone tail protease.

Specifically, the disclosure provides for systems or methods as described herein, wherein the gene editing system may comprise a vector system comprising: a) a first regulatory element operably linked to a gene editing system guide RNA that targets a locus of interest, b) a second regulatory inducible element operably linked to a Cas9 fusion protein, wherein components (a) and (b) may be located on same or different vectors of the system, wherein the guide RNA targets DNA of the locus of interest, wherein the Cas9 fusion protein and the guide RNA do not naturally occur together. The Cas9 fusion protein may comprise an inducible Cas9 enzyme. The disclosure also provides for the vector being a AAV or a lentivirus.

### Split Cas9 Molecules and Gene Editing Systems

In some embodiments, the Cas9 fusion molecule comprises a split Cas9 molecule, as described in more detail in WO15/089427 and WO14/018423. Split Cas9 molecules are summarized briefly, below.

In an aspect, disclosed herein is a non-naturally occurring or engineered inducible CRISPR enzyme, *e.g.*, Cas9 enzyme, comprising: a first CRISPR enzyme fusion construct attached to a first half of an inducible dimer and a second CRISPR enzyme fusion construct attached to a second half of the inducible dimer, wherein the first CRISPR enzyme fusion construct is operably linked to one or more nuclear localization signals, wherein the second CRISPR enzyme fusion construct is operably linked to one or more nuclear export signals, wherein contact with an inducer energy source brings the first and second halves of the inducible dimer together, wherein bringing the first and second halves of the inducible dimer together allows the first and second CRISPR enzyme fusion constructs to constitute a functional gene editing system.

In another aspect, in the inducible gene editing system, the inducible dimer is or comprises or consists essentially of or consists of an inducible heterodimer. In an aspect, in inducible gene editing system, the first half or a first portion or a first fragment of the inducible heterodimer is or comprises or consists of or consists essentially of an FKBP, optionally FKBP 12. In an aspect, in the inducible gene editing system, the second half or a second portion or a second fragment of the inducible heterodimer is or comprises or consists of or consists essentially of FRB. In one aspect, in the inducible gene editing system, the arrangement of the first CRISPR enzyme fusion construct is or comprises or consists of or consists essentially of N' terminal Cas9 part- FRB - NES. In another aspect, in the inducible gene editing system, the arrangement of the first CRISPR enzyme fusion construct is or comprises or consists of or consists essentially of NES-N' terminal Cas9 part- FRB - NES. In one aspect in the inducible gene editing system, the arrangement of the second CRISPR enzyme fusion construct is or comprises or consists essentially of or consists of C terminal Cas9 part-FKBP-NLS. In another aspect, in the inducible gene editing system, the arrangement of the second CRISPR enzyme fusion construct is or comprises or consists of or consists essentially of NLS-C terminal Cas9 part-FKBP-NLS. In an aspect, in inducible gene editing system there can be a linker that separates the Cas9 part from the half or portion or fragment of the inducible dimer. In an aspect, in the inducible gene editing system, the inducer energy source is or comprises or consists essentially of or consists of rapamycin. In an aspect, in inducible gene editing system, the inducible dimer is an inducible homodimer. In an aspect, in inducible gene editing system, the CRISPR enzyme is Cas9, *e.g.,* SpCas9 or SaCas9. In an aspect in an gene editing system, the Cas9 is split into two parts at any one of the following split points, according or with reference to SpCas9: a split position between 202A/203S; a split position between 255F/256D; a split position between 310E/3 111; a split position between 534R/535 ; a split position between 572E/573C; a split position between 713S/714G; a split position between 1003L/104E; a split position between 1 G54G/1 Q55E; a split position between 11 14N/1115S; a split position between 1152K/1153S; a split position between 1245K/1246G; or a split between 1098 and 1099. In an aspect, in the inducible gene editing system, one or more functional domains are associated with one or both parts of the Cas9 enzyme, *e.g.,* the functional domains optionally including a transcriptional activator, a transcriptional or a nuclease such as a fok I nuclease. In an aspect, in the inducible gene editing system, the functional gene editing system binds to the target sequence and the enzyme is a deadCas9, optionally having a diminished nuclease activity of at least 97%, or 100% (or no more than 3% and advantageously 0%) nuclease activity) as compared with the CRISPR enzyme not having the at least one mutation. In an aspect, in the inducible gene editing system, the deadCas9 (CRISPR enzyme) comprises two or more mutations wherein two or more of DIG, E762, H840, N854, N863, or D986 according to SpCas9 protein or any corresponding ortholog or N580 according to SaCas9 protein are mutated, or the CRISPR enzyme comprises at least one mutation, *e.g.,* wherein at least H840 is mutated. The disclosure further provides, a polynucleotide encoding the inducible gene editing system as herein discussed.

Also disclosed herein is a vector for delivery of the first CRISPR enzyme fusion construct, attached to a first half or portion or fragment of an inducible dimer and operably linked to one or more nuclear localization signals, according as herein discussed. In an aspect, disclosed herein is a vector for delivery of the second CRISPR enzyme fusion construct, attached to a second half or portion or fragment of an inducible dimer and operably linked to one or more nuclear export signals.

### Cas9 Activities

In certain embodiments, the Cas9 molecule or Cas9 polypeptide is capable of cleaving a target nucleic acid molecule. Typically wild-type Cas9 molecules cleave both strands of a target nucleic acid molecule. Cas9 molecules and Cas9 polypeptides can be engineered to alter nuclease cleavage (or other properties), *e.g.,* to provide a Cas9 molecule or Cas9 polypeptide which is a nickase, or which lacks the ability to cleave target nucleic acid. A Cas9 molecule or Cas9 polypeptide that is capable of cleaving a target nucleic acid molecule is referred to herein as an eaCas9 (an enzymatically active Cas9) molecule or eaCas9 polypeptide.

In certain embodiments, an eaCas9 molecule or eaCas9 polypeptide comprises one or more of the following enzymatic activities:
a nickase activity, *i.e.,* the ability to cleave a single strand, *e.g.,* the non-complementary strand or the complementary strand, of a nucleic acid molecule;
a double stranded nuclease activity, *i.e.,* the ability to cleave both strands of a double stranded nucleic acid and create a double stranded break, which in an embodiment is the presence of two nickase activities;
an endonuclease activity;
an exonuclease activity; and
a helicase activity, *i.e.,* the ability to unwind the helical structure of a double stranded nucleic acid.

In certain embodiments, an enzymatically active Cas9 or eaCas9 molecule or eaCas9 polypeptide cleaves both DNA strands and results in a double stranded break. In certain embodiments, an eaCas9 molecule or eaCas9 polypeptide cleaves only one strand, *e.g.,* the strand to which the gRNA hybridizes to, or the strand complementary to the strand the gRNA hybridizes with. In an embodiment, an eaCas9 molecule or eaCas9 polypeptide comprises cleavage activity associated with an HNH domain. In an embodiment, an eaCas9 molecule or eaCas9 polypeptide comprises cleavage activity associated with a RuvC domain. In an embodiment, an eaCas9 molecule or eaCas9 polypeptide comprises cleavage activity associated with an HNH domain and cleavage activity associated with a RuvC domain. In an embodiment, an eaCas9 molecule or eaCas9 polypeptide comprises an active, or cleavage competent, HNH domain and an inactive, or cleavage incompetent, RuvC domain. In an embodiment, an eaCas9 molecule or eaCas9 polypeptide comprises an inactive, or cleavage incompetent, HNH domain and an active, or cleavage competent, RuvC domain.

Some Cas9 molecules or Cas9 polypeptides have the ability to interact with a gRNA molecule, and in conjunction with the gRNA molecule localize to a core target domain, but are incapable of cleaving the target nucleic acid, or incapable of cleaving at efficient rates. Cas9 molecules having no, or no substantial, cleavage activity are referred to herein as an eiCas9 molecule or eiCas9 polypeptide. For example, an eiCas9 molecule or eiCas9 polypeptide can lack cleavage activity or have substantially less, *e.g.,* less than 20, 10, 5, 1 or 0.1 % of the cleavage activity of a reference Cas9 molecule or eiCas9 polypeptide, as measured by an assay described herein.

### Enzymatically Inactive Cas9

Cas9 molecules having no, or no substantial, cleavage activity are referred to herein as an enzymatically inactive ("eiCas9") molecule or eiCas9 polypeptide. For example, an eiCas9 molecule or eiCas9 polypeptide can lack cleavage activity or have substantially less, *e.g.,* less than 20, 10, 5, 1 or 0.1 % of the cleavage activity of a reference Cas9 molecule or eiCas9 polypeptide, as measured by an assay described herein.

In one embodiment, a Cas9 molecule is an eiCas9 molecule comprising one or more differences in a RuvC domain and/or in an HNH domain as compared to a reference Cas9 molecule, and the eiCas9 molecule does not cleave a nucleic acid, or cleaves with significantly less efficiency than does wild type, *e.g.,* when compared with wild type in a cleavage assay, *e.g.,* as described herein, cuts with less than 50, 25, 10, or 1% of a reference Cas9 molecule, as measured by an assay described herein. The reference Cas9 molecule can be a naturally occurring unmodified Cas9 molecule, *e.g.,* a naturally occurring Cas9 molecule such as a Cas9 molecule of *S. pyogenes, S. thermophilus, S. aureus, C. jejuni* or *N. meningitidis.* In one embodiment, the reference Cas9 molecule is the naturally occurring Cas9 molecule having the closest sequence identity or homology. In one embodiment, the eiCas9 molecule lacks substantial cleavage activity associated with a RuvC domain and cleavage activity associated with an HNH domain.

Whether or not a particular sequence, *e.g.,* a substitution, may affect one or more activity, such as targeting activity, cleavage activity, etc., can be evaluated or predicted, *e.g.,* by evaluating whether the mutation is conservative. In one embodiment, a "non-essential" amino acid residue, as used in the context of a Cas9 molecule, is a residue that can be altered from the wild-type sequence of a Cas9 molecule, *e.g.,* a naturally occurring Cas9 molecule, *e.g.,* an eaCas9 molecule, without abolishing or more preferably, without substantially altering a Cas9 activity (*e.g.*, cleavage activity), whereas changing an "essential" amino acid residue results in a substantial loss of activity (*e.g*., cleavage activity).

Although an enzymatically inactive (eiCas9) Cas9 molecule itself can block transcription when recruited to early regions in the coding sequence, more robust repression can be achieved by fusing a transcriptional repression domain (for example KRAB, SID or ERD) to the Cas9 and recruiting it to the target knockdown position, *e.g.,* within 1000bp of sequence 3' of the start codon or within 500 bp of a promoter region 5' of the start codon of a gene. It is likely that targeting DNAseI hypersensitive sites (DHSs) of the promoter may yield more efficient gene repression or activation because these regions are more likely to be accessible to the Cas9 protein and are also more likely to harbor sites for endogenous transcription factors. Especially for gene repression, it is contemplated herein that blocking the binding site of an endogenous transcription factor would aid in downregulating gene expression. In one embodiment, one or more eiCas9 molecules may be used to block binding of one or more endogenous transcription factors. In another embodiment, an eiCas9 molecule can be fused to a chromatin modifying protein. Altering chromatin status can result in decreased expression of the target gene. One or more eiCas9 molecules fused to one or more chromatin modifying proteins may be used to alter chromatin status.

### Targeting and PAMs

A Cas9 molecule or Cas9 polypeptide that can interact with a gRNA molecule and, in concert with the gRNA molecule, localizes to a site which comprises a target domain, and in certain embodiments, a PAM sequence.

In certain embodiments, the ability of an eaCas9 molecule or eaCas9 polypeptide to interact with and cleave a target nucleic acid is PAM sequence dependent. A PAM sequence is a sequence in the target nucleic acid. In an embodiment, cleavage of the target nucleic acid occurs upstream from the PAM sequence. eaCas9 molecules from different bacterial species can recognize different sequence motifs (*e.g.*, PAM sequences). In an embodiment, an eaCas9 molecule of S. *pyogenes* recognizes the sequence motif NGG and directs cleavage of a target nucleic acid sequence 1 to 10, *e.g.,* 3 to 5, bp upstream from that sequence (see, *e.g.,* Mali 2013). In an embodiment, an eaCas9 molecule of *S. thermophilus* recognizes the sequence motif NGGNG and/or NNAGAAW (W = A or T) and directs cleavage of a target nucleic acid sequence 1 to 10, *e.g.,* 3 to 5, bp upstream from these sequences (see, *e.g.,* Horvath 2010; Deveau 2008). In an embodiment, an eaCas9 molecule of *S. mutans* recognizes the sequence motif NGG and/or NAAR (R = A or G) and directs cleavage of a target nucleic acid sequence 1 to 10, *e.g.,* 3 to 5, bp upstream from this sequence (see, *e.g.,* Deveau 2008). In an embodiment, an eaCas9 molecule of *S. aureus* recognizes the sequence motif NNGRR (R = A or G) and directs cleavage of a target nucleic acid sequence 1 to 10, *e.g.,* 3 to 5, bp upstream from that sequence. In an embodiment, an eaCas9 molecule of *S. aureus* recognizes the sequence motif NNGRRN (R = A or G) and directs cleavage of a target nucleic acid sequence 1 to 10, *e.g.,* 3 to 5, bp upstream from that sequence. In an embodiment, an eaCas9 molecule of *S. aureus* recognizes the sequence motif NNGRRT (R = A or G) and directs cleavage of a target nucleic acid sequence 1 to 10, *e.g.,* 3 to 5, base pairs upstream from that sequence. In an embodiment, an eaCas9 molecule of *S. aureus* recognizes the sequence motif NNGRRV (R = A or G) and directs cleavage of a target nucleic acid sequence 1 to 10, *e.g.,* 3 to 5, bp upstream from that sequence. The ability of a Cas9 molecule to recognize a PAM sequence can be determined, *e.g*., using a transformation assay as described in Jinek 2012. In the aforementioned embodiments, N can be any nucleotide residue, *e.g.,* any of A, G, C, or T.

As is discussed herein, Cas9 molecules can be engineered to alter the PAM specificity of the Cas9 molecule.

Exemplary naturally occurring Cas9 molecules have been described previously (see, *e.g.,* Chylinski 2013). Such Cas9 molecules include Cas9 molecules of a cluster 1 bacterial family, cluster 2 bacterial family, cluster 3 bacterial family, cluster 4 bacterial family, cluster 5 bacterial family, cluster 6 bacterial family, a cluster 7 bacterial family, a cluster 8 bacterial family, a cluster 9 bacterial family, a cluster 10 bacterial family, a cluster 11 bacterial family, a cluster 12 bacterial family, a cluster 13 bacterial family, a cluster 14 bacterial family, a cluster 15 bacterial family, a cluster 16 bacterial family, a cluster 17 bacterial family, a cluster 18 bacterial family, a cluster 19 bacterial family, a cluster 20 bacterial family, a cluster 21 bacterial family, a cluster 22 bacterial family, a cluster 23 bacterial family, a cluster 24 bacterial family, a cluster 25 bacterial family, a cluster 26 bacterial family, a cluster 27 bacterial family, a cluster 28 bacterial family, a cluster 29 bacterial family, a cluster 30 bacterial family, a cluster 31 bacterial family, a cluster 32 bacterial family, a cluster 33 bacterial family, a cluster 34 bacterial family, a cluster 35 bacterial family, a cluster 36 bacterial family, a cluster 37 bacterial family, a cluster 38 bacterial family, a cluster 39 bacterial family, a cluster 40 bacterial family, a cluster 41 bacterial family, a cluster 42 bacterial family, a cluster 43 bacterial family, a cluster 44 bacterial family, a cluster 45 bacterial family, a cluster 46 bacterial family, a cluster 47 bacterial family, a cluster 48 bacterial family, a cluster 49 bacterial family, a cluster 50 bacterial family, a cluster 51 bacterial family, a cluster 52 bacterial family, a cluster 53 bacterial family, a cluster 54 bacterial family, a cluster 55 bacterial family, a cluster 56 bacterial family, a cluster 57 bacterial family, a cluster 58 bacterial family, a cluster 59 bacterial family, a cluster 60 bacterial family, a cluster 61 bacterial family, a cluster 62 bacterial family, a cluster 63 bacterial family, a cluster 64 bacterial family, a cluster 65 bacterial family, a cluster 66 bacterial family, a cluster 67 bacterial family, a cluster 68 bacterial family, a cluster 69 bacterial family, a cluster 70 bacterial family, a cluster 71 bacterial family, a cluster 72 bacterial family, a cluster 73 bacterial family, a cluster 74 bacterial family, a cluster 75 bacterial family, a cluster 76 bacterial family, a cluster 77 bacterial family, or a cluster 78 bacterial family.

Exemplary naturally occurring Cas9 molecules include a Cas9 molecule of a cluster 1 bacterial family. Examples include a Cas9 molecule of: *S. aureus, S. pyogenes* (*e.g.,* strain SF370, MGAS10270, MGAS10750, MGAS2096, MGAS315, MGAS5005, MGAS6180, MGAS9429, NZ131 and SSI-1), S. *thermophilus* (*e.g.,* strain LMD-9), *S. pseudoporcinus* (*e.g.,* strain SPIN 20026), S. *mutans* (*e.g.,* strain UA159, NN2025), S. *macacae* (*e.g.,* strain NCTC11558), S. *gallolyticus* (*e.g.,* strain UCN34, ATCC BAA-2069), S. *equines* (*e.g.,* strain ATCC 9812, MGCS 124), S. *dysdalactiae* (*e.g.,* strain GGS 124), S. *bovis* (*e.g.,* strain ATCC 700338), S. *anginosus* (*e.g.,* strain F0211), S. *agalactiae* (*e.g.,* strain NEM316, A909), *Listeria monocytogenes* (*e.g.,* strain F6854), *Listeria innocua* (*L. innocua, e.g.,* strain Clip11262), *Enterococcus italicus* (*e.g.,* strain DSM 15952), or *Enterococcus faecium* (*e.g.,* strain 1,231,408).

In certain embodiments, a Cas9 molecule or Cas9 polypeptide comprises an amino acid sequence: having 60%, 65%, 70%, 75%, 80%, 85%, 90%, 95%, 96%, 97%, 98% or 99% homology with; differs at no more than, 2, 5, 10, 15, 20, 30, or 40% of the amino acid residues when compared with; differs by at least 1, 2, 5, 10 or 20 amino acids, but by no more than 100, 80, 70, 60, 50, 40 or 30 amino acids from; or is identical to any Cas9 molecule sequence described herein, or to a naturally occurring Cas9 molecule sequence, *e.g.,* a Cas9 molecule from a species listed herein (*e.g.,* SEQ ID NO: 1-4 or described in Chylinski 2013 or Hou 2013). In an embodiment, the Cas9 molecule or Cas9 polypeptide comprises one or more of the following activities: a nickase activity; a double stranded cleavage activity (*e.g.*, an endonuclease and/or exonuclease activity); a helicase activity; or the ability, together with a gRNA molecule, to localize to a target nucleic acid.

A comparison of the sequence of a number of Cas9 molecules indicate that certain regions are conserved. These are identified below as: region 1 (residues 1 to 180, or in the case of region 1, residues 120 to 180); region 2 (residues 360 to 480); region 3 (residues 660 to 720); region 4 (residues 817 to 900); and region 5 (residues 900 to 960).

In an embodiment, a Cas9 molecule or Cas9 polypeptide comprises regions 1-5, together with sufficient additional Cas9 molecule sequence to provide a biologically active molecule, *e.g.,* a Cas9 molecule having at least one activity described herein. In an embodiment, each of regions 1-5, independently, have 50%, 60%, 70%, 80%, 85%, 90%, 95%, 96%, 97%, 98% or 99% homology with the corresponding residues of a Cas9 molecule or Cas9 polypeptide described herein, *e.g.,* a sequence from SEQ ID Nos: 1-4.

In an embodiment, a Cas9 molecule or Cas9 polypeptide comprises an amino acid sequence referred to as region 1:
having 50%, 60%, 70%, 80%, 85%, 90%, 95%, 96%, 97%, 98% or 99% homology with amino acids 1-180 of the amino acid sequence of Cas9 of *S. pyogenes;*
differs by at least 1, 2, 5, 10 or 20 amino acids but by no more than 90, 80, 70, 60, 50, 40 or 30 amino acids from amino acids 1-180 of the amino acid sequence of Cas9 of *S. pyogenes, S. thermophilus, S. mutans,* or *Listeria innocua;* or
is identical to amino acids 1-180 of the amino acid sequence of Cas9 of *S. pyogenes, S. thermophilus, S. mutans,* or *L. innocua.*

In an embodiment, a Cas9 molecule or Cas9 polypeptide comprises an amino acid sequence referred to as region 1':
having 55%, 60%, 65%, 70%, 75%, 80%, 85%, 90%, 95%, 96%, 97%, 98% or 99% homology with amino acids 120-180 of the amino acid sequence of Cas9 of *S. pyogenes, S. thermophilus, S. mutans* or *L. innocua;*
differs by at least 1, 2, or 5 amino acids but by no more than 35, 30, 25, 20 or 10 amino acids from amino acids 120-180 of the amino acid sequence of Cas9 of *S. pyogenes, S. thermophilus, S. mutans,* or *L. innocua* ; or
is identical to amino acids 120-180 of the amino acid sequence of Cas9 of *S. pyogenes, S. thermophilus, S. mutans,* or *L. innocua.*

In an embodiment, a Cas9 molecule or Cas9 polypeptide comprises an amino acid sequence referred to as region 2:
having 50%, 55%, 60%, 65%, 70%, 75%, 80%, 85%, 90%, 95%, 96%, 97%, 98% or 99% homology with amino acids 360-480 of the amino acid sequence of Cas9 of *S. pyogenes, S. thermophilus, S. mutans,* or *L. innocua;*
differs by at least 1, 2, or 5 amino acids but by no more than 35, 30, 25, 20 or 10 amino acids from amino acids 360-480 of the amino acid sequence of Cas9 of *S. pyogenes, S. thermophilus, S. mutans,* or *L. innocua;* or
is identical to amino acids 360-480 of the amino acid sequence of Cas9 of *S. pyogenes, S. thermophilus, S. mutans,* or *L. innocua.*

In certain embodiments, a Cas9 molecule or Cas9 polypeptide comprises an amino acid sequence referred to as region 3:
having 55%, 60%, 65%, 70%, 75%, 80%, 85%, 90%, 95%, 96%, 97%, 98%, or 99% homology with amino acids 660-720 of the amino acid sequence of Cas9 of *S. pyogenes, S. thermophilus, S. mutans* or *L. innocua;*
differs by at least 1, 2, or 5 amino acids but by no more than 35, 30, 25, 20 or 10 amino acids from amino acids 660-720 of the amino acid sequence of Cas9 of S. *pyogenes, S. thermophilus, S. mutans* or *L. innocua;* or
is identical to amino acids 660-720 of the amino acid sequence of Cas9 of *S. pyogenes, S. thermophilus, S. mutans* or *L. innocua.*

In an embodiment, a Cas9 molecule or Cas9 polypeptide comprises an amino acid sequence referred to as region 4:
having 50%, 55%, 60%, 65%, 70%, 75%, 80%, 85%, 90%, 95%, 96%, 97%, 98%, or 99% homology with amino acids 817-900 of the amino acid sequence of Cas9 of *S. pyogenes, S. thermophilus, S. mutans,* or *L. innocua;*
differs by at least 1, 2, or 5 amino acids but by no more than 35, 30, 25, 20 or 10 amino acids from amino acids 817-900 of the amino acid sequence of Cas9 of *S. pyogenes, S. thermophilus, S. mutans,* or *L. innocua;* or
is identical to amino acids 817-900 of the amino acid sequence of Cas9 of *S. pyogenes, S. thermophilus, S. mutans,* or *L. innocua.*

In an embodiment, a Cas9 molecule or Cas9 polypeptide comprises an amino acid sequence referred to as region 5:
having 50%, 55%, 60%, 65%, 70%, 75%, 80%, 85%, 90%, 95%, 96%, 97%, 98%, or 99% homology with amino acids 900-960 of the amino acid sequence of Cas9 of *S. pyogenes, S. thermophilus, S. mutans,* or *L. innocua;*
differs by at least 1, 2, or 5 amino acids but by no more than 35, 30, 25, 20 or 10 amino acids from amino acids 900-960 of the amino acid sequence of Cas9 of *S. pyogenes, S. thermophilus, S. mutans,* or *L. innocua;* or
is identical to amino acids 900-960 of the amino acid sequence of Cas9 of *S. pyogenes, S. thermophilus, S. mutans,* or *L. innocua.*

### Engineered Or Altered Cas9 Molecules And Cas9 Polypeptides

Cas9 molecules and Cas9 polypeptides described herein can possess any of a number of properties, including: nickase activity, nuclease activity (*e.g.*, endonuclease and/or exonuclease activity); helicase activity; the ability to associate functionally with a gRNA molecule; and the ability to target (or localize to) a site on a nucleic acid (*e.g.,* PAM recognition and specificity). In certain embodiments, a Cas9 molecule or Cas9 polypeptide can include all or a subset of these properties. In a typical embodiment, a Cas9 molecule or Cas9 polypeptide has the ability to interact with a gRNA molecule and, in concert with the gRNA molecule, localize to a site in a nucleic acid. Other activities, *e.g.*, PAM specificity, cleavage activity, or helicase activity can vary more widely in Cas9 molecules and Cas9 polypeptides.

Cas9 molecules include engineered Cas9 molecules and engineered Cas9 polypeptides (engineered, as used in this context, means merely that the Cas9 molecule or Cas9 polypeptide differs from a reference sequences, and implies no process or origin limitation). An engineered Cas9 molecule or Cas9 polypeptide can comprise altered enzymatic properties, *e.g*., altered nuclease activity, (as compared with a naturally occurring or other reference Cas9 molecule) or altered helicase activity. As discussed herein, an engineered Cas9 molecule or Cas9 polypeptide can have nickase activity (as opposed to double-strand nuclease activity). In an embodiment an engineered Cas9 molecule or Cas9 polypeptide can have an alteration that alters its size, *e.g.,* a deletion of amino acid sequence that reduces its size, *e.g*., without significant effect on one or more, or any Cas9 activity. In an embodiment, an engineered Cas9 molecule or Cas9 polypeptide can comprise an alteration that affects PAM recognition. For example, an engineered Cas9 molecule can be altered to recognize a PAM sequence other than that recognized by the endogenous wild-type PI domain. In an embodiment a Cas9 molecule or Cas9 polypeptide can differ in sequence from a naturally occurring Cas9 molecule but not have significant alteration in one or more Cas9 activities.

Cas9 molecules or Cas9 polypeptides with desired properties can be made in a number of ways, *e.g.,* by alteration of a parental, *e.g.,* naturally occurring, Cas9 molecules or Cas9 polypeptides, to provide an altered Cas9 molecule or Cas9 polypeptide having a desired property. For example, one or more mutations or differences relative to a parental Cas9 molecule, *e.g.,* a naturally occurring or engineered Cas9 molecule, can be introduced. Such mutations and differences comprise: substitutions (*e.g.*, conservative substitutions or substitutions of non-essential amino acids); insertions; or deletions. In an embodiment, a Cas9 molecule or Cas9 polypeptide can comprises one or more mutations or differences, *e.g.*, at least 1, 2, 3, 4, 5, 10, 15, 20, 30, 40 or 50 mutations but less than 200, 100, or 80 mutations relative to a reference, *e.g.,* a parental, Cas9 molecule.

In certain embodiments, a mutation or mutations do not have a substantial effect on a Cas9 activity, *e.g.,* a Cas9 activity described herein. In other embodiments, a mutation or mutations have a substantial effect on a Cas9 activity, *e.g.,* a Cas9 activity described herein.

### Non-Cleaving and Modified-Cleavage Cas9 Molecules and Cas9 Polypeptides

In an embodiment, a Cas9 molecule or Cas9 polypeptide comprises a cleavage property that differs from naturally occurring Cas9 molecules, *e.g*., that differs from the naturally occurring Cas9 molecule having the closest homology. For example, a Cas9 molecule or Cas9 polypeptide can differ from naturally occurring Cas9 molecules, *e.g.,* a Cas9 molecule of *S. pyogenes,* as follows: its ability to modulate, *e.g.,* decreased or increased, cleavage of a double stranded nucleic acid (endonuclease and/or exonuclease activity), *e.g*., as compared to a naturally occurring Cas9 molecule (*e.g.,* a Cas9 molecule of *S. pyogenes);* its ability to modulate, *e.g.*, decreased or increased, cleavage of a single-strand of a nucleic acid, *e.g.,* a non-complementary strand of a nucleic acid molecule or a complementary strand of a nucleic acid molecule (nickase activity), *e.g*., as compared to a naturally occurring Cas9 molecule (*e.g.,* a Cas9 molecule of *S. pyogenes*); or the ability to cleave a nucleic acid molecule, *e.g.,* a double stranded or single stranded nucleic acid molecule, can be eliminated.

In certain embodiments, an eaCas9 molecule or eaCas9 polypeptide comprises one or more of the following activities: cleavage activity associated with an N-terminal RuvC-like domain; cleavage activity associated with an HNH-like domain; cleavage activity associated with an HNH-like domain and cleavage activity associated with an N-terminal RuvC-like domain.

In certain embodiments, an eaCas9 molecule or eaCas9 polypeptide comprises an active, or cleavage competent, HNH-like domain (*e.g.*, an HNH-like domain described herein) and an inactive, or cleavage incompetent, N-terminal RuvC-like domain. An exemplary inactive, or cleavage incompetent N-terminal RuvC-like domain can have a mutation of an aspartic acid in an N-terminal RuvC-like domain, *e.g*., an aspartic acid at position 10 of SEQ ID NO:2, *e.g.,* can be substituted with an alanine. In an embodiment, the eaCas9 molecule or eaCas9 polypeptide differs from wild-type in the N-terminal RuvC-like domain and does not cleave the target nucleic acid, or cleaves with significantly less efficiency, *e.g.*, less than 20, 10, 5, 1 or .1 % of the cleavage activity of a reference Cas9 molecule, *e.g.,* as measured by an assay described herein. The reference Cas9 molecule can by a naturally occurring unmodified Cas9 molecule, *e.g.,* a naturally occurring Cas9 molecule such as a Cas9 molecule of *S. pyogenes, S. aureus,* or *S. thermophilus.* In an embodiment, the reference Cas9 molecule is the naturally occurring Cas9 molecule having the closest sequence identity or homology.

In certain embodiments, an eaCas9 molecule or eaCas9 polypeptide comprises an inactive, or cleavage incompetent, HNH domain and an active, or cleavage competent, N-terminal RuvC-like domain (*e.g.,* a RuvC-like domain described herein). Exemplary inactive, or cleavage incompetent HNH-like domains can have a mutation at one or more of: a histidine in an HNH-like domain, for example, at position 856 of the *S. pyogenes* Cas9 sequence (SEQ ID NO:2), *e.g.,* can be substituted with an alanine; and one or more asparagines in an HNH-like domain, for example, at position 870 and/or 879 of the *S. pyogenes* Cas9 sequence (SEQ ID NO:2) *e.g.,* can be substituted with an alanine. In an embodiment, the eaCas9 differs from wild-type in the HNH-like domain and does not cleave the target nucleic acid, or cleaves with significantly less efficiency, *e.g.,* less than 20, 10, 5, 1 or 0.1% of the cleavage activity of a reference Cas9 molecule, *e.g.,* as measured by an assay described herein. The reference Cas9 molecule can by a naturally occurring unmodified Cas9 molecule, *e.g.,* a naturally occurring Cas9 molecule such as a Cas9 molecule of *S. pyogenes, S. aureus,* or *S. thermophilus.* In an embodiment, the reference Cas9 molecule is the naturally occurring Cas9 molecule having the closest sequence identity or homology.

In certain embodiments, exemplary Cas9 activities comprise one or more of PAM specificity, cleavage activity, and helicase activity. A mutation(s) can be present, *e.g.,* in: one or more RuvC domains, *e.g.,* an N-terminal RuvC domain; an HNH domain; a region outside the RuvC domains and the HNH domain. In an embodiment, a mutation(s) is present in a RuvC domain. In an embodiment, a mutation(s) is present in an HNH domain. In an embodiment, mutations are present in both a RuvC domain and an HNH domain.

Exemplary mutations that may be made in the RuvC domain or HNH domain with reference to the *S. pyogenes* Cas9 sequence include: D10A, E762A, H840A, N854A, N863A and/or D986A. Exemplary mutations that may be made in the RuvC domain with reference to the *S. aureus* Cas9 sequence include N580A.

In an embodiment, a Cas9 molecule is an eiCas9 molecule comprising one or more differences in a RuvC domain and/or in an HNH domain as compared to a reference Cas9 molecule, and the eiCas9 molecule does not cleave a nucleic acid, or cleaves with significantly less efficiency than does wild type, *e.g.,* when compared with wild type in a cleavage assay, *e.g.,* as described herein, cuts with less than 50, 25, 10, or 1% of a reference Cas9 molecule, as measured by an assay described herein.

Whether or not a particular sequence, *e.g.,* a substitution, may affect one or more activity, such as targeting activity, cleavage activity, etc., can be evaluated or predicted, *e.g.,* by evaluating whether the mutation is conservative. In an embodiment, a "non-essential" amino acid residue, as used in the context of a Cas9 molecule, is a residue that can be altered from the wild-type sequence of a Cas9 molecule, *e.g.,* a naturally occurring Cas9 molecule, *e.g.,* an eaCas9 molecule, without abolishing or more preferably, without substantially altering a Cas9 activity (*e.g.*, cleavage activity), whereas changing an "essential" amino acid residue results in a substantial loss of activity (*e.g*., cleavage activity).

In an embodiment, a Cas9 molecule comprises a cleavage property that differs from naturally occurring Cas9 molecules, *e.g.,* that differs from the naturally occurring Cas9 molecule having the closest homology. For example, a Cas9 molecule can differ from naturally occurring Cas9 molecules, *e.g.,* a Cas9 molecule of *S aureus* or *S. pyogenes* as follows: its ability to modulate, *e.g.,* decreased or increased, cleavage of a double stranded break (endonuclease and/or exonuclease activity), *e.g.,* as compared to a naturally occurring Cas9 molecule (*e.g.,* a Cas9 molecule of *S aureus* or *S. pyogenes);* its ability to modulate, *e.g.,* decreased or increased, cleavage of a single-strand of a nucleic acid, *e.g.,* a non-complimentary strand of a nucleic acid molecule or a complementary strand of a nucleic acid molecule (nickase activity), *e.g.,* as compared to a naturally occurring Cas9 molecule (*e.g.,* a Cas9 molecule of *S aureus* or *S. pyogenes*); or the ability to cleave a nucleic acid molecule, *e.g.,* a double stranded or single stranded nucleic acid molecule, can be eliminated. In certain embodiments, the nickase is S. *aureus* Cas9-derived nickase comprising the sequence of SEQ ID NO: 10 (D10A) or SEQ ID NO: 11 (N580A) (Friedland 2015).

In certain embodiments, the altered Cas9 molecule is an eaCas9 molecule comprising one or more of the following activities: cleavage activity associated with a RuvC domain; cleavage activity associated with an HNH domain; cleavage activity associated with an HNH domain and cleavage activity associated with a RuvC domain.

In an embodiment, the altered Cas9 molecule is an eiCas9 molecule which does not cleave a nucleic acid molecule (either double stranded or single stranded nucleic acid molecules) or cleaves a nucleic acid molecule with significantly less efficiency, *e.g.,* less than 20, 10, 5, 1 or 0.1% of the cleavage activity of a reference Cas9 molecule, *e.g.,* as measured by an assay described herein. The reference Cas9 molecule can be a naturally occurring unmodified Cas9 molecule, *e.g.,* a naturally occurring Cas9 molecule such as a Cas9 molecule of *S. pyogenes, S. thermophilus, S. aureus, C. jejuni* or *N. meningitidis.* In an embodiment, the reference Cas9 molecule is the naturally occurring Cas9 molecule having the closest sequence identity or homology. In an embodiment, the eiCas9 molecule lacks substantial cleavage activity associated with a RuvC domain and cleavage activity associated with an HNH domain.

In certain embodiments, the altered Cas9 molecule or Cas9 polypeptide, *e.g.,* an eaCas9 molecule or eaCas9 polypeptide, can be a fusion, *e.g.,* of two of more different Cas9 molecules, *e.g.,* of two or more naturally occurring Cas9 molecules of different species. For example, a fragment of a naturally occurring Cas9 molecule of one species can be fused to a fragment of a Cas9 molecule of a second species. As an example, a fragment of a Cas9 molecule of *S. pyogenes* comprising an N-terminal RuvC-like domain can be fused to a fragment of Cas9 molecule of a species other than *S. pyogenes* (*e.g., S. thermophilus*) comprising an HNH-like domain.

### Cas9 with Altered or No PAM Recognition

Naturally-occurring Cas9 molecules can recognize specific PAM sequences, for example the PAM recognition sequences described above for, *e.g., S. pyogenes, S. thermophilus, S. mutans,* and *S. aureus.*

In certain embodiments, a Cas9 molecule or Cas9 polypeptide has the same PAM specificities as a naturally occurring Cas9 molecule. In other embodiments, a Cas9 molecule or Cas9 polypeptide has a PAM specificity not associated with a naturally occurring Cas9 molecule, or a PAM specificity not associated with the naturally occurring Cas9 molecule to which it has the closest sequence homology. For example, a naturally occurring Cas9 molecule can be altered, *e.g.,* to alter PAM recognition, *e.g.,* to alter the PAM sequence that the Cas9 molecule or Cas9 polypeptide recognizes in order to decrease off-target sites and/or improve specificity; or eliminate a PAM recognition requirement. In certain embodiments, a Cas9 molecule or Cas9 polypeptide can be altered, *e.g.,* to increase length of PAM recognition sequence and/or improve Cas9 specificity to high level of identity (*e.g.*, 98%, 99% or 100% match between gRNA and a PAM sequence), *e.g.,* to decrease off-target sites and/or increase specificity. In certain embodiments, the length of the PAM recognition sequence is at least 4, 5, 6, 7, 8, 9, 10 or 15 amino acids in length. In an embodiment, the Cas9 specificity requires at least 90%, 95%, 96%, 97%, 98%, 99% or more homology between the gRNA and the PAM sequence. Cas9 molecules or Cas9 polypeptides that recognize different PAM sequences and/or have reduced off-target activity can be generated using directed evolution. Exemplary methods and systems that can be used for directed evolution of Cas9 molecules are described (see, *e.g*., Esvelt 2011). Candidate Cas9 molecules can be evaluated, *e.g.,* by methods described below.

### Size-Optimized Cas9 Molecules

Engineered Cas9 molecules and engineered Cas9 polypeptides described herein include a Cas9 molecule or Cas9 polypeptide comprising a deletion that reduces the size of the molecule while still retaining desired Cas9 properties, *e.g.*, essentially native conformation, Cas9 nuclease activity, and/or target nucleic acid molecule recognition. Provided herein are Cas9 molecules or Cas9 polypeptides comprising one or more deletions and optionally one or more linkers, wherein a linker is disposed between the amino acid residues that flank the deletion. Methods for identifying suitable deletions in a reference Cas9 molecule, methods for generating Cas9 molecules with a deletion and a linker, and methods for using such Cas9 molecules will be apparent to one of ordinary skill in the art upon review of this document.

A Cas9 molecule, *e.g.,* a *S. aureus* or *S. pyogenes* Cas9 molecule, having a deletion is smaller, *e.g*., has reduced number of amino acids, than the corresponding naturally-occurring Cas9 molecule. The smaller size of the Cas9 molecules allows increased flexibility for delivery methods, and thereby increases utility for genome-editing. A Cas9 molecule can comprise one or more deletions that do not substantially affect or decrease the activity of the resultant Cas9 molecules described herein. Activities that are retained in the Cas9 molecules comprising a deletion as described herein include one or more of the following:
a nickase activity, *i.e.,* the ability to cleave a single strand, *e.g.,* the non-complementary strand or the complementary strand, of a nucleic acid molecule; a double stranded nuclease activity, *i.e.,* the ability to cleave both strands of a double stranded nucleic acid and create a double stranded break, which in an embodiment is the presence of two nickase activities; an endonuclease activity; an exonuclease activity; a helicase activity, *i.e.,* the ability to unwind the helical structure of a double stranded nucleic acid; and recognition activity of a nucleic acid molecule, *e.g.,* a target nucleic acid or a gRNA molecule.

Activity of the Cas9 molecules described herein can be assessed using the activity assays described herein or in the art.

### Identifying regions suitable for deletion

Suitable regions of Cas9 molecules for deletion can be identified by a variety of methods. Naturally-occurring orthologous Cas9 molecules from various bacterial species can be modeled onto the crystal structure of *S*. *pyogenes* Cas9 (Nishimasu 2014) to examine the level of conservation across the selected Cas9 orthologs with respect to the three-dimensional conformation of the protein. Less conserved or unconserved regions that are spatially located distant from regions involved in Cas9 activity, *e.g*., interface with the target nucleic acid molecule and/or gRNA, represent regions or domains are candidates for deletion without substantially affecting or decreasing Cas9 activity.

### Nucleic Acids Encoding Cas9 Molecules

Nucleic acids encoding the Cas9 molecules or Cas9 polypeptides, *e.g.,* an eaCas9 molecule or eaCas9 polypeptides are provided herein. Exemplary nucleic acids encoding Cas9 molecules or Cas9 polypeptides have been described previously (see, *e.g.,* Cong 2013; Wang 2013; Mali 2013; Jinek 2012).

In an embodiment, a nucleic acid encoding a Cas9 molecule or Cas9 polypeptide can be a synthetic nucleic acid sequence. For example, the synthetic nucleic acid molecule can be chemically modified, *e.g.,* as described herein. In an embodiment, the Cas9 mRNA has one or more (*e.g.,* all of the following properties: it is capped, polyadenylated, substituted with 5-methylcytidine and/or pseudouridine.

In addition, or alternatively, the synthetic nucleic acid sequence can be codon optimized, *e.g.,* at least one non-common codon or less-common codon has been replaced by a common codon. For example, the synthetic nucleic acid can direct the synthesis of an optimized messenger mRNA, *e.g.,* optimized for expression in a mammalian expression system, *e.g.,* described herein.

In addition, or alternatively, a nucleic acid encoding a Cas9 molecule or Cas9 polypeptide may comprise a nuclear localization sequence (NLS). Nuclear localization sequences are known in the art.

An exemplary codon optimized nucleic acid sequence encoding a Cas9 molecule of *S. pyogenes* is set forth in SEQ ID NO: 22. The corresponding amino acid sequence of an *S. pyogenes* Cas9 molecule is set forth in SEQ ID NO: 23.

Exemplary codon optimized nucleic acid sequence encoding a Cas9 molecule of *S. aureus* is set forth in SEQ ID NO: 26 and 39.

If any of the above Cas9 sequences are fused with a peptide or polypeptide at the C-terminus, it is understood that the stop codon will be removed.

### Other Cas Molecules and Cas Polypeptides

Various types of Cas molecules or Cas polypeptides can be used to practice the inventions disclosed herein. In some embodiments, Cas molecules of Type II Cas systems are used. In other embodiments, Cas molecules of other Cas systems are used. For example, Type I or Type III Cas molecules may be used. Exemplary Cas molecules (and Cas systems) have been described previously (see, *e.g.,* Haft 2005; Makarova 2011). Exemplary Cas molecules (and Cas systems) are also shown in **Table 4.**

| **Table 4: Cas Systems** | | | | | |
|---|---|---|---|---|---|
| **Gene name^{‡}** | **System type or subtype** | **Name from Haft 2005^{§}** | **Structure of encoded protein (PDB accessions)^{¶}** | **Families (and superfamily) of encoded protein^{#**}** | **Representatives** |
| *cas1* | • Type I | *cas1* | 3GOD, 3LFX and 2YZS | COG1518 | SERP2463, SPy1047 and *ygbT* |
| | • Type II | | | | |
| | • Type III | | | | |
| *cas2* | • Type I | *cas2* | 2IVY, 2I8E and 3EXC | COG1343 and COG3512 | SERP2462, SPy1048, SPyl723 (N-terminal domain) and *ygbF* |
| | • Type II | | | | |
| | • Type III | | | | |
| *cas3'* | • Type I^{‡‡} | *cas3* | NA | COG1203 | APE1232 and *ygcB* |
| *cas 3"* | • Subtype I-A | NA | NA | COG2254 | APE1231 and BH0336 |
| | • Subtype I-B | | | | |
| *cas4* | • Subtype I-A | *cas4* and *csa1* | NA | COG1468 | APE1239 and BH0340 |
| | • Subtype I-B | | | | |
| | • Subtype I-C | | | | |
| | • Subtype I-D | | | | |
| | • Subtype II-B | | | | |
| *cas5* | • Subtype I-A | *cas5a, cas5d, cas5e, cas5h, cas5p, cas5t* and *cmx5* | 3KG4 | COG1688 (RAMP) | APE1234, BH0337, *devS* and *ygcI* |
| | • Subtype I-B | | | | |
| | • Subtype I-C | | | | |
| | • Subtype I-E | | | | |
| *cas6* | • Subtype I-A | *cas6* and *cmx6* | 3I4H | COG1583 and COG5551 (RAMP) | PF1131 and slr7014 |
| | • Subtype I-B | | | | |
| | • Subtype I-D | | | | |
| | • Subtype III-A• Subtype III-B | | | | |
| *cas6e* | • Subtype I-E | *cse3* | 1WJ9 | (RAMP) | *ygcH* |
| *cas6f* | • Subtype I-F | *csy4* | 2XLJ | (RAMP) | y1727 |
| *cas7* | • Subtype I-A | *csa2, csd2, cse4, csh2, csp1* and *cst2* | NA | COG1857 and COG3649 (RAMP) | *devR* and *ygcJ* |
| | • Subtype I-B | | | | |
| | • Subtype I-C | | | | |
| | • Subtype I-E | | | | |
| *cas8a1* | • Subtype I-A^{‡‡} | *cmx1, cst1, csx8, csx13* and CXXC-CXXC | NA | BH0338-like | LA3191^{§§} and PG2018^{§§} |
| *cas8a2* | • Subtype I-A^{‡‡} | *csa4* and *csx9* | NA | PH0918 | AF0070, AF1873, MJ0385, PF0637, PH0918 and SSO1401 |
| *cas8b* | • Subtype I-B^{‡‡} | *csh1* and TM1802 | NA | BH0338-like | MTH1090 and TM1802 |
| *cas8c* | • Subtype I-C^{‡‡} | *csd1* and *csp2* | NA | BH0338-like | BH0338 |
| *cas9* | • Type II^{‡‡} | *csn1* and *csx12* | NA | COG3513 | FTN_0757 and SPy1046 |
| *cas10* | • Type III^{‡‡} | *cmr2, csm1* and *csx11* | NA | COG1353 | MTH326, Rv2823c^{§§} and TM1794^{§§} |
| *cas10d* | • Subtype I-D^{‡‡} | *csc3* | NA | COG1353 | slr7011 |
| *csy1* | • Subtype IF^{‡‡} | *csy1* | NA | y1724-like | y1724 |
| *csy2* | • Subtype I-F | *csy2* | NA | (RAMP) | y1725 |
| *csy3* | • Subtype I-F | *csy3* | NA | (RAMP) | y1726 |
| *cse1* | • Subtype I-E^{‡‡} | *cse1* | NA | YgcL-like | *ygcL* |
| *cse2* | • Subtype I-E | *cse2* | 2ZCA | YgcK-like | *ygcK* |
| *csc1* | • Subtype I-D | *csc1* | NA | alr1563-like (RAMP) | alr1563 |
| *csc2* | • Subtype I-D | *csc1* and *csc2* | NA | COG1337 (RAMP) | slr7012 |
| *csa5* | • Subtype I-A | *csa5* | NA | AF1870 | AF1870, MJ0380, PF0643 and SSO1398 |
| *csn2* | • Subtype II-A | *csn2* | NA | SPy1049-like | SPy1049 |
| *csm2* | • Subtype III-A^{‡‡} | *csm2* | NA | COG1421 | MTH1081 and SERP2460 |
| *csm3* | • Subtype III-A | *csc2* and *csm3* | NA | COG1337 (RAMP) | MTH1080 and SERP2459 |
| *csm4* | • Subtype III-A | *csm4* | NA | COG1567 (RAMP) | MTH1079 and SERP2458 |
| *csm5* | • Subtype III-A | *csm5* | NA | COG1332 (RAMP) | MTH1078 and SERP2457 |
| *csm6* | • Subtype III-A | APE2256 and *csm6* | 2WTE | COG1517 | APE2256 and SSO1445 |
| *cmr1* | • Subtype III-B | *cmr1* | NA | COG1367 (RAMP) | PF1130 |
| *cmr3* | • Subtype III-B | *cmr3* | NA | COG1769 (RAMP) | PF1128 |
| *cmr4* | • Subtype III-B | *cmr4* | NA | COG1336 (RAMP) | PF1126 |
| *cmr5* | • Subtype III-B^{‡‡} | *cmr5* | 2ZOP and 2OEB | COG3337 | MTH324 and PF1125 |
| *cmr6* | • Subtype III-B | *cmr6* | NA | COG1604 (RAMP) | PF1124 |
| *csb1* | • Subtype I-U | GSU0053 | NA | (RAMP) | Balac_1306 and GSU0053 |
| *csb2* | • Subtype I-U^{§§} | NA | NA | (RAMP) | Balac_1305 and GSU0054 |
| *csb3* | • Subtype I-U | NA | NA | (RAMP) | Balac_1303^{§§} |
| *csx17* | • Subtype I-U | NA | NA | NA | Btus_2683 |
| *csx14* | • Subtype I-U | NA | NA | NA | GSU0052 |
| *csx10* | • Subtype I-U | *csx10* | NA | (RAMP) | Caur_2274 |
| *csx16* | • Subtype III-U | VVA1548 | NA | NA | VVA1548 |
| *csaX* | • Subtype III-U | *csaX* | NA | NA | SSO1438 |
| *csx3* | • Subtype III-U | *csx3* | NA | NA | AF1864 |
| *csx1* | • Subtype III-U | *csa3, csx1, csx2,* DXTHG, NE0113 and TIGR02710 | 1XMX and 2171 | COG1517 and COG4006 | MJ1666, NE0113, PF1127 and TM1812 |
| *csx15* | • Unknown | NA | NA | TTE2665 | TTE2665 |
| *csf1* | • Type U | *csf1* | NA | NA | AFE_1038 |
| *csf2* | • Type U | *csf2* | NA | (RAMP) | AFE_1039 |
| *csf3* | • Type U | *csf3* | NA | (RAMP) | AFE_1040 |
| *csf4* | • Type U | *csf4* | NA | NA | AFE-_1037 |

### VII. Functional Analysis of Candidate Molecules

Candidate Cas9 molecules, candidate gRNA molecules, *e.g.,* candidate gRNA fusion molecules, and/or candidate Cas9 molecule/gRNA fusion molecule complexes, can be evaluated by art-known methods or as described herein. For example, exemplary methods for evaluating the endonuclease activity of Cas9 molecule have been described previously (Jinek 2012).

### Binding and Cleavage Assay: Testing the endonuclease activity of Cas9 molecule

The ability of a Cas9 molecule/gRNA fusion molecule complex to bind to and cleave a target nucleic acid can be evaluated in a plasmid cleavage assay. In this assay, a synthetic or *in vitro*-transcribed gRNA fusion molecule is pre-annealed prior to the reaction by heating to 95°C and slowly cooling down to room temperature. Native or restriction digest-linearized plasmid DNA (300 ng (~8 nM)) is incubated for 60 min at 37°C with purified Cas9 protein molecule (50-500 nM) and gRNA (50-500 nM, 1:1) in a Cas9 plasmid cleavage buffer (20 mM HEPES pH 7.5, 150 mM KCl, 0.5 mM DTT, 0.1 mM EDTA) with or without 10 mM MgCl₂. The reactions are stopped with 5X DNA loading buffer (30% glycerol, 1.2% SDS, 250 mM EDTA), resolved by a 0.8 or 1% agarose gel electrophoresis and visualized by ethidium bromide staining. The resulting cleavage products indicate whether the Cas9 molecule cleaves both DNA strands, or only one of the two strands. For example, linear DNA products indicate the cleavage of both DNA strands. Nicked open circular products indicate that only one of the two strands is cleaved.

Alternatively, the ability of a Cas9 molecule/gRNA fusion molecule complex to bind to and cleave a target nucleic acid can be evaluated in an oligonucleotide DNA cleavage assay. In this assay, DNA oligonucleotides (10 pmol) are radiolabeled by incubating with 5 units T4 polynucleotide kinase and ~3-6 pmol (~20-40 mCi) [γ-32P]-ATP in 1X T4 polynucleotide kinase reaction buffer at 37°C for 30 min, in a 50 µL reaction. After heat inactivation (65°C for 20 min), reactions are purified through a column to remove unincorporated label. Duplex substrates (100 nM) are generated by annealing labeled oligonucleotides with equimolar amounts of unlabeled complementary oligonucleotide at 95°C for 3 min, followed by slow cooling to room temperature. For cleavage assays, gRNA fusion molecules are annealed by heating to 95°C for 30 s, followed by slow cooling to room temperature. Cas9 (500 nM final concentration) is pre-incubated with the annealed gRNA fusion molecules (500 nM) in cleavage assay buffer (20 mM HEPES pH 7.5, 100 mM KCl, 5 mM MgCl2, 1 mM DTT, 5% glycerol) in a total volume of 9 µL. Reactions are initiated by the addition of 1 µl target DNA (10 nM) and incubated for 1 h at 37°C. Reactions are quenched by the addition of 20 µL of loading dye (5 mM EDTA, 0.025% SDS, 5% glycerol in formamide) and heated to 95°C for 5 min. Cleavage products are resolved on 12% denaturing polyacrylamide gels containing 7 M urea and visualized by phosphorimaging. The resulting cleavage products indicate that whether the complementary strand, the non-complementary strand, or both, are cleaved.

One or both of these assays can be used to evaluate the suitability of a candidate gRNA fusion molecule or candidate Cas9 molecule.

### Binding Assay: Testing the binding of Cas9 molecule to target DNA

Exemplary methods for evaluating the binding of Cas9 molecule to target DNA have been described previously (Jinek 2012).

For example, in an electrophoretic mobility shift assay, target DNA duplexes are formed by mixing of each strand (10 nmol) in deionized water, heating to 95°C for 3 min and slow cooling to room temperature. All DNAs are purified on 8% native gels containing 1X TBE. DNA bands are visualized by UV shadowing, excised, and eluted by soaking gel pieces in DEPC-treated H₂O. Eluted DNA is ethanol precipitated and dissolved in DEPC-treated H₂O. DNA samples are 5' end labeled with [γ-32P]-ATP using T4 polynucleotide kinase for 30 min at 37°C. Polynucleotide kinase is heat denatured at 65°C for 20 min, and unincorporated radiolabel is removed using a column. Binding assays are performed in buffer containing 20 mM HEPES pH 7.5, 100 mM KCl, 5 mM MgCl₂, 1 mM DTT and 10% glycerol in a total volume of 10 µL. Cas9 protein molecule is programmed with equimolar amounts of pre-annealed gRNA fusion molecule and titrated from 100 pM to 1 µM. Radiolabeled DNA is added to a final concentration of 20 pM. Samples are incubated for 1 h at 37°C and resolved at 4°C on an 8% native polyacrylamide gel containing 1X TBE and 5 mM MgCl₂. Gels are dried and DNA visualized by phosphorimaging.

### Differential Scanning Flourimetry (DSF)

The thermostability of Cas9 molecule-gRNA fusion ribonucleoprotein (RNP) complexes can be measured via DSF. This technique measures the thermostability of a protein, which can increase under favorable conditions such as the addition of a binding RNA molecule, *e.g.,* a gRNA fusion molecule.

The assay is performed using two different protocols, one to test the best stoichiometric ratio of gRNA:Cas9 protein and another to determine the best solution conditions for RNP formation.

To determine the best solution to form RNP complexes, a 2 µM solution of Cas9 in water + 10x SYPRO Orange^{®} (Life Technologies cat#S-6650) and dispensed into a 384 well plate. An equimolar amount of a gRNA fusion molecule diluted in solutions with varied pH and salt is then added. After incubating at room temperature for 10 min. and brief centrifugation to remove any bubbles,a Bio-Rad CFX384^{™} Real-Time System C1000 Touch^{™} Thermal Cycler with the Bio-Rad CFX Manager software is used to run a gradient from 20°C to 90°C with a 1°C increase in temperature every 10 seconds.

The second assay consists of mixing various concentrations of a gRNA fusion molecule with 2 µM Cas 9 in optimal buffer from the assay above and incubating at RT for 10 min in a 384 well plate. An equal volume of optimal buffer + 10x SYPRO Orange^{®} (Life Technologies cat#S-6650) is added and the plate sealed with Microseal^{®} B adhesive (MSB-1001). Following brief centrifugation to remove any bubbles, a Bio-Rad CFX384^{™} Real-Time System C1000 Touch^{™} Thermal Cycler with the Bio-Rad CFX Manager software is used to run a gradient from 20°C to 90°C with a 1°C increase in temperature every 10 seconds.

### Resection Assay: Testing a Cas9 to promote resection

The ability of a Cas9 to promote resection can be evaluated by measuring the levels of single stranded DNA at specific double strand break sites in human cells using quantitative methods (as described in Zhou 2014) . In this assay, a cell line is delivered, *e.g.,* by transfection, a candidate Cas9 or a candidate Cas9 fusion protein. The cells are cultured for a sufficient amount of time to allow nuclease activity and resection to occur. Genomic DNA is carefully extracted using a method in which cells are embedded in low-gelling point agar that protects the DNA from shearing and damage during extraction. The genomic DNA is digested with a restriction enzyme that selectively cuts double-stranded DNA. Primers for quantitative PCR that span up to 5 kb of the double strand break site are designed. The results from the PCR reaction show the levels of single strand DNA detected at each of the primer positions. Thus, the length and the level of resection promoted by the candidate Cas9 or Cas9 fusion protein can be determined from this assay.

Other qualitative assays for identifying the occurrence of resection include the detection of proteins or protein complexes that bind to single-stranded DNA after resection has occurred, e.g., RPA foci, Rad51 foci, or BrDU detection by immunofluorescence. Antibodies for RPA protein and Rad51 are known in the art.

### VIII. Genome Editing Approaches

Mutations in a target gene may be corrected using one of the approaches discussed herein. A mutation in a target gene can be corrected by homology directed repair (HDR) using an exogenously provided template nucleic acid fused to a gRNA molecule described herein, referred to herein as "gene correction".

### VIII.1 HDR Repair and Template Nucleic Acids

In certain embodiments of the methods provided herein, HDR-mediated sequence alteration is used to alter and/or correct (*e.g.,* repair or edit) the sequence of one or more nucleotides in a genome. While not wishing to be bound by theory, it is believed that HDR-mediated alteration of a target sequence within a target gene occurs by HDR with an exogenously provided donor template or template nucleic acid in a process referred to herein as gene correction. For example, the donor template or template nucleic acid provides for alteration of the target sequence. It is believed that fusion of the template nucleic acid to a gRNA molecule brings the template nucleic acid into close proximity with the gRNA/Cas9 complex, thereby enabling gene correction directed by the template nucleic acid to proceed with greater efficiency. It is contemplated that a double stranded donor can be used as a template nucleic acid for homologous recombination. It is further contemplated that a single stranded donor template can be used as a template for alteration of the target sequence by alternate methods of HDR (*e.g.*, single-strand annealing) between the target sequence and the donor template. Donor template-effected alteration of a target sequence depends on cleavage by a Cas9 molecule. Cleavage by Cas9 can comprise a double-strand break or two single-strand breaks.

In an embodiment, the target position or target position regions has at least 50%, 60%, 70%, 80%, 81%, 82%, 83%, 84%, 85%, 86%, 87%, 88%, 89%, 90%, 91%, 92%, 93%, 94%, 95%, 96%, 97%, 98%, or 99% homology with an endogenous homologous sequence.

In an embodiment, the target position region, except for the target position, differs by 1, 2, 3, 4, 5, 10, 25, 50, 100 or fewer, nucleotides with an endogenous homologous sequence.

In an embodiment, the target position region has at least 50%, 60%, 70%, 80%, 90%, 92%, 94%, 96%, 98%, or 99% homology with an endogenous homologous sequence over at least 10, 20, 30, 40, 50, 100, 200, 300, 400, 500, 750, 1,000, 2500, 5000, or 10000 nucleotides.

In an embodiment, the target position region, except for the target position, differs by 1, 2, 3, 4, 5, 10, 25, 50, 100 or fewer, nucleotides with an endogenous homologous sequence over at least 10, 20, 30, 40, 50, 100, 200, 300, 400, 500, 750, 1,000, 2500, 5000, or 10000 nucleotides.

In an embodiment, the endogenous homologous sequence comprises a domain, *e.g.,* a catalytic domain, a domain that binds a target, a structural domain, found in the gene that comprises the target position.

In certain embodiments of the methods provided herein, HDR-mediated alteration is used to alter a single nucleotide in a target sequence. These embodiments may utilize either one double-strand break or two single-strand breaks. In certain embodiments, a single nucleotide alteration is incorporated using (1) one double-strand break, (2) two single-strand breaks, (3) two double-strand breaks with a break occurring on each side of the target position, (4) one double-strand break and two single-strand breaks with the double-strand break and two single-strand breaks occurring on each side of the target position (5) four single-strand breaks with a pair of single stranded breaks occurring on each side of the target position, or (6) one single-strand break.

In certain embodiments wherein a single-stranded template nucleic acid is used, the target position can be altered by alternative HDR.

Donor template-effected alteration of a target position depends on cleavage by a Cas9 molecule. Cleavage by Cas9 can comprise a nick, a double-strand break, or two single-strand breaks, *e.g*., one on each strand of the target nucleic acid. After introduction of the breaks on the target nucleic acid, resection occurs at the break ends resulting in single stranded overhanging DNA regions.

In canonical HDR, a double-stranded donor template is introduced, comprising homologous sequence to the target nucleic acid that will either be directly incorporated into the target nucleic acid or used as a template to change the sequence of the target nucleic acid. After resection at the break, repair can progress by different pathways, *e.g*., by the double Holliday junction model (or double-strand break repair, DSBR, pathway) or the synthesis-dependent strand annealing (SDSA) pathway. In the double Holliday junction model, strand invasion by the two single stranded overhangs of the target nucleic acid to the homologous sequences in the donor template occurs, resulting in the formation of an intermediate with two Holliday junctions. The junctions migrate as new DNA is synthesized from the ends of the invading strand to fill the gap resulting from the resection. The end of the newly synthesized DNA is ligated to the resected end, and the junctions are resolved, resulting in the alteration of the target nucleic acid, *e.g.*, incorporation of the altered sequence of the donor template at the corresponding target position. Crossover with the donor template may occur upon resolution of the junctions. In the SDSA pathway, only one single stranded overhang invades the donor template and new DNA is synthesized from the end of the invading strand to fill the gap resulting from resection. The newly synthesized DNA then anneals to the remaining single stranded overhang, new DNA is synthesized to fill in the gap, and the strands are ligated to produce the altered DNA duplex.

In alternative HDR, a single-strand donor template, *e.g*., template nucleic acid, is introduced. A nick, single-strand break, or double-strand break at the target nucleic acid, for altering a desired target position, is mediated by a Cas9 molecule, *e.g.*, described herein, and resection at the break occurs to reveal single stranded overhangs. Incorporation of the sequence of the template nucleic acid to correct or alter the target position of the target nucleic acid typically occurs by the SDSA pathway, as described above.

Additional details on template nucleic acids are provided in Section IV entitled "Template nucleic acids" in International Application PCT/US2014/057905, now published as WO 2015/048577.

In certain embodiments, double-strand cleavage is effected by a Cas9 molecule having cleavage activity associated with an HNH-like domain and cleavage activity associated with a RuvC-like domain, *e.g.,* an N-terminal RuvC-like domain, *e.g.,* a wild type Cas9. Such embodiments require only a single gRNA molecule.

In certain embodiments, one single-strand break, or nick, is effected by a Cas9 molecule having nickase activity, *e.g.,* a Cas9 nickase as described herein. A nicked target nucleic acid can be a substrate for alt-HDR.

In other embodiments, two single-strand breaks, or nicks, are effected by a Cas9 molecule having nickase activity, *e.g*., cleavage activity associated with an HNH-like domain or cleavage activity associated with an N-terminal RuvC-like domain. Such embodiments usually require two gRNAs, one for placement of each single-strand break. One or both of the gRNAs can be gRNA fusion molecules, linked to the template nucleic acid. In an embodiment, the Cas9 molecule having nickase activity cleaves the strand to which the gRNA hybridizes, but not the strand that is complementary to the strand to which the gRNA hybridizes. In an embodiment, the Cas9 molecule having nickase activity does not cleave the strand to which the gRNA hybridizes, but rather cleaves the strand that is complementary to the strand to which the gRNA hybridizes.

In certain embodiments, the nickase has HNH activity, *e.g.,* a Cas9 molecule having the RuvC activity inactivated, *e.g.,* a Cas9 molecule having a mutation at D10, *e.g.,* the D10A mutation. D10A inactivates RuvC; therefore, the Cas9 nickase has (only) HNH activity and will cut on the strand to which the gRNA hybridizes (*e.g*., the complementary strand, which does not have the NGG PAM on it). In other embodiments, a Cas9 molecule having an H840, *e.g.,* an H840A, mutation can be used as a nickase. H840A inactivates HNH; therefore, the Cas9 nickase has (only) RuvC activity and cuts on the non-complementary strand *(e.g.,* the strand that has the NGG PAM and whose sequence is identical to the gRNA). In other embodiments, a Cas9 molecule having an N863 mutation, *e.g*., the N863A mutation, mutation can be used as a nickase. N863A inactivates HNH therefore the Cas9 nickase has (only) RuvC activity and cuts on the non-complementary strand (the strand that has the NGG PAM and whose sequence is identical to the gRNA).

In certain embodiments, in which a nickase and two gRNAs are used to position two single-strand nicks, one nick is on the + strand and one nick is on the - strand of the target nucleic acid. The PAMs can be outwardly facing or inwardly facing. The gRNAs can be selected such that the gRNAs are separated by, from about 0-50, 0-100, or 0-200 nucleotides. In an embodiment, there is no overlap between the target sequences that are complementary to the targeting domains of the two gRNAs. In an embodiment, the gRNAs do not overlap and are separated by as much as 50, 100, or 200 nucleotides. In an embodiment, the use of two gRNAs can increase specificity, *e.g*., by decreasing off-target binding (Ran 2013).

In certain embodiments, a single nick can be used to induce HDR, *e.g.,* alt-HDR. It is contemplated herein that a single nick can be used to increase the ratio of HR to NHEJ at a given cleavage site. In certain embodiments, a single-strand break is formed in the strand of the target nucleic acid to which the targeting domain of said gRNA is complementary. In certain embodiments, a single-strand break is formed in the strand of the target nucleic acid other than the strand to which the targeting domain of said gRNA is complementary.

### Placement of double-strand or single-strand breaks relative to the target position

A double-strand break or single-strand break in one of the strands should be sufficiently close to target position that an alteration is produced in the desired region, *e.g.,* correction of a mutation occurs. In certain embodiments, the distance is not more than 50, 100, 200, 300, 350 or 400 nucleotides. While not wishing to be bound by theory, in certain embodiments, it is believed that the break should be sufficiently close to target position such that the target position is within the region that is subject to exonuclease-mediated removal during end resection. If the distance between the target position and a break is too great, the sequence desired to be altered may not be included in the end resection and, therefore, may not be altered, as donor sequence, either exogenously provided donor sequence or endogenous genomic donor sequence, in some embodiments is only used to alter sequence within the end resection region.

In certain embodiments, the gRNA targeting domain is configured such that a cleavage event, *e.g.,* a double-strand or single-strand break, is positioned within 1, 2, 3, 4, 5, 10, 15, 20, 25, 30, 35, 40, 45, 50, 60, 70, 80, 90, 100, 150 or 200 nucleotides of the region desired to be altered, *e.g.,* a mutation. The break, *e.g.,* a double-strand or single-strand break, can be positioned upstream or downstream of the region desired to be altered, *e.g.,* a mutation. In some embodiments, a break is positioned within the region desired to be altered, *e.g*., within a region defined by at least two mutant nucleotides. In some embodiments, a break is positioned immediately adjacent to the region desired to be altered, *e.g*., immediately upstream or downstream of a mutation.

In certain embodiments, a single-strand break is accompanied by an additional single-strand break, positioned by a second gRNA molecule, as discussed below. For example, the targeting domains bind configured such that a cleavage event, *e.g.,* the two single-strand breaks, are positioned within 1, 2, 3, 4, 5, 10, 15, 20, 25, 30, 35, 40, 45, 50, 60, 70, 80, 90, 100, 150 or 200 nucleotides of a target position. In an embodiment, the first and second gRNA molecules are configured such that when guiding a Cas9 nickase, a single-strand break is accompanied by an additional single-strand break, positioned by a second gRNA, sufficiently close to one another to result in alteration of the desired region. In an embodiment, the first and second gRNA molecules are configured such that a single-strand break positioned by said second gRNA is within 10, 20, 30, 40, or 50 nucleotides of the break positioned by said first gRNA molecule, *e.g*., when the Cas9 is a nickase. In an embodiment, the two gRNA molecules are configured to position cuts at the same position, or within a few nucleotides of one another, on different strands, *e.g*., essentially mimicking a double-strand break.

In certain embodiments, in which a gRNA (unimolecular (or chimeric) or modular gRNA) and Cas9 nuclease induce a double-strand break for the purpose of inducing HDR-mediated alteration, the cleavage site is between 0-200 bp *(e.g.,* 0-175, 0 to 150, 0 to 125, 0 to 100, 0 to 75, 0 to 50, 0 to 25, 25 to 200, 25 to 175, 25 to 150, 25 to 125, 25 to 100, 25 to 75, 25 to 50, 50 to 200, 50 to 175, 50 to 150, 50 to 125, 50 to 100, 50 to 75, 75 to 200, 75 to 175, 75 to 150, 75 to 125, 75 to 100 bp) away from the target position. In certain embodiments, the cleavage site is between 0-100 bp (*e.g.,* 0 to 75, 0 to 50, 0 to 25, 25 to 100, 25 to 75, 25 to 50, 50 to 100, 50 to 75 or 75 to 100 bp) away from the target position.

In certain embodiments, one can promote HDR by using nickases to generate a break with overhangs. While not wishing to be bound by theory, the single stranded nature of the overhangs can enhance the cell's likelihood of repairing the break by HDR as opposed to, *e.g.,* NHEJ. Specifically, in certain embodiments, HDR is promoted by selecting a first gRNA that targets a first nickase to a first target sequence, and a second gRNA that targets a second nickase to a second target sequence which is on the opposite DNA strand from the first target sequence and offset from the first nick.

In certain embodiment, the targeting domain of a gRNA molecule is configured to position a cleavage event sufficiently far from a preselected nucleotide, *e.g.,* the nucleotide of a coding region, such that the nucleotide is not altered. In certain embodiments, the targeting domain of a gRNA molecule is configured to position an intronic cleavage event sufficiently far from an intron/exon border, or naturally occurring splice signal, to avoid alteration of the exonic sequence or unwanted splicing events. The gRNA molecule may be a first, second, third and/or fourth gRNA molecule, as described herein.

### Placement of a first break and a second break relative to each other

In certain embodiments, a double-strand break can be accompanied by an additional double-strand break, positioned by a second gRNA molecule, as is discussed below.

In certain embodiments, a double-strand break can be accompanied by two additional single-strand breaks, positioned by a second gRNA molecule and a third gRNA molecule.

In certain embodiments, a first and second single-strand breaks can be accompanied by two additional single-strand breaks positioned by a third gRNA molecule and a fourth gRNA molecule.

When two or more gRNAs are used to position two or more cleavage events, *e.g*., double-strand or single-strand breaks, in a target nucleic acid, it is contemplated that the two or more cleavage events may be made by the same or different Cas9 proteins. For example, when two gRNAs are used to position two double stranded breaks, a single Cas9 nuclease may be used to create both double stranded breaks. When two or more gRNAs are used to position two or more single stranded breaks (nicks), a single Cas9 nickase may be used to create the two or more nicks. When two or more gRNAs are used to position at least one double stranded break and at least one single stranded break, two Cas9 proteins may be used, *e.g.,* one Cas9 nuclease and one Cas9 nickase. It is contemplated that when two or more Cas9 proteins are used that the two or more Cas9 proteins may be delivered sequentially to control specificity of a double stranded versus a single stranded break at the desired position in the target nucleic acid.

In some embodiments, the targeting domain of the first gRNA molecule and the targeting domain of the second gRNA molecules are complementary to opposite strands of the target nucleic acid molecule. In some embodiments, the gRNA molecule and the second gRNA molecule are configured such that the PAMs are oriented outward. In some embodiments, the gRNA molecule and the second gRNA molecule are configured such that the PAMs are oriented inward.

In certain embodiments, two gRNA are selected to direct Cas9-mediated cleavage at two positions that are a preselected distance from each other. In certain embodiments, the two points of cleavage are on opposite strands of the target nucleic acid. In some embodiments, the two cleavage points form a blunt ended break, and in other embodiments, they are offset so that the DNA ends comprise one or two overhangs (*e.g*., one or more 5' overhangs and/or one or more 3' overhangs). In some embodiments, each cleavage event is a nick. In some embodiments, the nicks are close enough together that they form a break that is recognized by the double stranded break machinery (as opposed to being recognized by, *e.g.,* the SSBr machinery). In certain embodiments, the nicks are far enough apart that they create an overhang that is a substrate for HDR, *i.e.,* the placement of the breaks mimics a DNA substrate that has experienced some resection. For instance, in some embodiments the nicks are spaced to create an overhang that is a substrate for processive resection. In some embodiments, the two breaks are spaced within 25-65 nucleotides of each other. The two breaks may be, *e.g.,* about 25, 30, 35, 40, 45, 50, 55, 60 or 65 nucleotides of each other. The two breaks may be, *e.g.,* at least about 25, 30, 35, 40, 45, 50, 55, 60 or 65 nucleotides of each other. The two breaks may be, *e.g.,* at most about 30, 35, 40, 45, 50, 55, 60 or 65 nucleotides of each other. In embodiments, the two breaks are about 25-30, 30-35, 35-40, 40-45, 45-50, 50-55, 55-60, or 60-65 nucleotides of each other.

In some embodiments, the break that mimics a resected break comprises a 3' overhang (*e.g*., generated by a DSB and a nick, where the nick leaves a 3' overhang), a 5' overhang (*e.g*., generated by a DSB and a nick, where the nick leaves a 5' overhang), a 3' and a 5' overhang (*e.g.,* generated by three cuts), two 3' overhangs (*e.g.,* generated by two nicks that are offset from each other), or two 5' overhangs (*e.g*., generated by two nicks that are offset from each other).

In certain embodiments, in which two gRNAs (independently, unimolecular (or chimeric) or modular gRNA) complexing with Cas9 nickases induce two single-strand breaks for the purpose of inducing HDR-mediated alteration (*e.g*., correction), the closer nick is between 0-200 bp (*e.g.,* 0-175, 0 to 150, 0 to 125, 0 to 100, 0 to 75, 0 to 50, 0 to 25, 25 to 200, 25 to 175, 25 to 150, 25 to 125, 25 to 100, 25 to 75, 25 to 50, 50 to 200, 50 to 175, 50 to 150, 50 to 125, 50 to 100, 50 to 75, 75 to 200, 75 to 175, 75 to 150, 75 to 125, or 75 to 100 bp) away from the target position and the two nicks will ideally be within 25-65 bp of each other *(e.g.,* 25 to 50, 25 to 45, 25 to 40, 25 to 35, 25 to 30, 30 to 55, 30 to 50, 30 to 45, 30 to 40, 30 to 35, 35 to 55, 35 to 50, 35 to 45, 35 to 40, 40 to 55, 40 to 50, 40 to 45 bp, 45 to 50 bp, 50 to 55 bp, 55 to 60 bp, or 60 to 65 bp) and no more than 100 bp away from each other *(e.g.,* no more than 90, 80, 70, 60, 50, 40, 30, 20, 10, or 5 bp away from each other). In certain embodiments, the cleavage site is between 0-100 bp *(e.g.,* 0 to 75, 0 to 50, 0 to 25, 25 to 100, 25 to 75, 25 to 50, 50 to 100, 50 to 75, or 75 to 100 bp) away from the target position.

In some embodiments, two gRNAs, *e.g*., independently, unimolecular (or chimeric) or modular gRNA, are configured to position a double-strand break on both sides of a target position. In other embodiments, three gRNAs, *e.g*., independently, unimolecular (or chimeric) or modular gRNA, are configured to position a double-strand break *(i.e.,* one gRNA complexes with a cas9 nuclease) and two single-strand breaks or paired single stranded breaks *(i.e.,* two gRNAs complex with Cas9 nickases) on either side of the target position. In other embodiments, four gRNAs, *e.g*., independently, unimolecular (or chimeric) or modular gRNA, are configured to generate two pairs of single stranded breaks (*i.e*., two pairs of two gRNA molecules complex with Cas9 nickases) on either side of the target position. The double-strand break(s) or the closer of the two single-strand nicks in a pair will ideally be within 0-500 bp of the target position (*e.g*., no more than 450, 400, 350, 300, 250, 200, 150, 100, 50 or 25 bp from the target position). When nickases are used, the two nicks in a pair are, in certain embodiments, within 25-65 bp of each other (*e.g*., between 25 to 55, 25 to 50, 25 to 45, 25 to 40, 25 to 35, 25 to 30, 50 to 55, 45 to 55, 40 to 55, 35 to 55, 30 to 55, 30 to 50, 35 to 50, 40 to 50, 45 to 50, 35 to 45, 40 to 45 bp, 45 to 50 bp, 50 to 55 bp, 55 to 60 bp, or 60 to 65 bp) and no more than 100 bp away from each other *(e.g.,* no more than 90, 80, 70, 60, 50, 40, 30, 20, or 10 bp).

When two gRNAs are used to target Cas9 molecules to breaks, different combinations of Cas9 molecules are envisioned. In some embodiments, a first gRNA is used to target a first Cas9 molecule to a first target position, and a second gRNA is used to target a second Cas9 molecule to a second target position. In some embodiments, the first Cas9 molecule creates a nick on the first strand of the target nucleic acid, and the second Cas9 molecule creates a nick on the opposite strand, resulting in a double stranded break *(e.g.,* a blunt ended cut or a cut with overhangs).

Different combinations of nickases can be chosen to target one single stranded break to one strand and a second single stranded break to the opposite strand. When choosing a combination, one can take into account that there are nickases having one active RuvC-like domain, and nickases having one active HNH domain. In certain embodiments, a RuvC-like domain cleaves the non-complementary strand of the target nucleic acid molecule. In certain embodiments, an HNH-like domain cleaves a single stranded complementary domain, *e.g.,* a complementary strand of a double stranded nucleic acid molecule. Generally, if both Cas9 molecules have the same active domain (*e.g*., both have an active RuvC domain or both have an active HNH domain), one will choose two gRNAs that bind to opposite strands of the target. In more detail, in some embodiments, a first gRNA is complementary with a first strand of the target nucleic acid and binds a nickase having an active RuvC-like domain and causes that nickase to cleave the strand that is non-complementary to that first gRNA, *i.e.,* a second strand of the target nucleic acid; and a second gRNA is complementary with a second strand of the target nucleic acid and binds a nickase having an active RuvC-like domain and causes that nickase to cleave the strand that is non-complementary to that second gRNA, *i.e.,* the first strand of the target nucleic acid. Conversely, In some embodiments, a first gRNA is complementary with a first strand of the target nucleic acid and binds a nickase having an active HNH domain and causes that nickase to cleave the strand that is complementary to that first gRNA, *i.e.,* a first strand of the target nucleic acid; and a second gRNA is complementary with a second strand of the target nucleic acid and binds a nickase having an active HNH domain and causes that nickase to cleave the strand that is complementary to that second gRNA, *i.e.,* the second strand of the target nucleic acid. In another arrangement, if one Cas9 molecule has an active RuvC-like domain and the other Cas9 molecule has an active HNH domain, the gRNAs for both Cas9 molecules can be complementary to the same strand of the target nucleic acid, so that the Cas9 molecule with the active RuvC-like domain will cleave the non-complementary strand and the Cas9 molecule with the HNH domain will cleave the complementary strand, resulting in a double stranded break.

In an embodiment, the cleavage event comprises one or more breaks, *e.g*., one or more single-strand breaks, one or more double-strand breaks, or a combination thereof.

In an embodiment, the cleavage event comprises any one of the following: (a) one single-strand break; (b) two single-strand breaks; (c) three single-strand breaks; (d) four single-strand breaks; (e) one double-strand break; (f) two double-strand breaks; (g) one single-strand break and one double-strand break; (h) two single-strand breaks and one double-strand break; or (i) any combination thereof.

In an embodiment, the gRNA molecule and the second gRNA molecule position a cleavage event on each strand of a target nucleic acid.

In an embodiment, the cleavage event flanks the target position, and wherein the terminus (created by the cleavage event) closest to the target position, for each cleavage event, is a 5' terminus, *e.g*., resulting in a 5' overhang.

While not wishing to be bound by theory, it believed that, in an embodiment, the sequence exposed by a cleavage event *(e.g.,* a single-strand cleavage event) mediated by a gRNA molecule and a Cas9 molecule *(e.g.,* a Cas9 nickase, *e.g.,* a Cas9 molecule containing D10A or N863A mutation) may affect (*e.g*., increase or decrease) gene correction efficiency. For example, the sequence exposed by the cleavage event can include a 5' overhang, a 3' overhang, a product of the nucleolytic processing of a 5' overhang, a product of the nucleolytic processing of a 3' overhang, or any combination thereof. In an embodiment, the exposed sequence comprises or consists of a 5' overhang. In another embodiment, the exposed sequence comprises or consists of a 3' overhang. In an embodiment, the exposed sequence comprises or consists of a product of the nucleolytic processing of a 5' overhang. In another embodiment, the exposed sequence comprises or consists of a product of the nucleolytic processing of a 3' overhang.

In an embodiment, the 5' overhang is between 1 and 20000 nucleotides, 5 and 20000 nucleotides, 10 and 20000 nucleotides, 20 and 20000 nucleotides, 30 and 20000 nucleotides, between 35 and 20000 nucleotides, between 40 and 20000 nucleotides, between 50 and 20000 nucleotides, between 1000 and 10000 nucleotides, or between 500 and 5000 nucleotides in length, *e.g*., between 1 and 100 nucleotides, between 1 and 50 nucleotides, between 1 and 25 nucleotides, between 40 and 60 nucleotides, between 40 and 55 nucleotides, or between 45 and 50 nucleotides in length, *e.g.,* at least about 1, 5, 10, 20, 30, 35, 40, 45, 50, 75, 100, 200, 300, 400, 500, 750, 1000, 2000, 3000, 4000, 5000, 6000, 7000, 8000, 9000, 10000, or 15000 nucleotides in length.

In an embodiment, the exposed sequence differs by 1, 2, 3, 4, 5, 10, 25, 50, 100 or fewer, nucleotides with an endogenous homologous sequence. In an embodiment, the exposed sequence has at least 50%, 60%, 70%, 80%, 81%, 82%, 83%, 84%, 85%, 86%, 87%, 88%, 89%, 90%, 91%, 92%, 93%, 94%, 95%, 96%, 97%, 98%, or 99% homology with an endogenous homologous sequence over at least 10, 20, 30, 40, 50, 100, 200, 300, 400, 500, 750, 1000, 2500, 5000, or 10000 nucleotides. In an embodiment, the exposed sequence differs by 1, 2, 3, 4, 5, 10, 25, 50, 100 or fewer, nucleotides with an endogenous homologous sequence over at least 10, 20, 30, 40, 50, 100, 200, 300, 400, 500, 750, 1000, 2500, 5000, or 10000 nucleotides.

In an embodiment, the cleavage event flanks the target position, and the terminus (created by a cleavage event) closest to the target position, for each cleavage event, is a 3' terminus, *e.g*., resulting a 3' overhang.

In an embodiment, the 3' overhang is between 1 and 20000 nucleotides, 5 and 20000 nucleotides, 10 and 20000 nucleotides, 20 and 20000 nucleotides, between 30 and 20000 nucleotides, between 35 and 20000 nucleotides, between 40 and 20000 nucleotides, between 50 and 20000 nucleotides, between 1000 and 10000 nucleotides, or between 500 and 5000 nucleotides in length, *e.g*., between 1 and 100 nucleotides, between 1 and 50 nucleotides, between 1 and 25 nucleotides, between 40 and 60 nucleotides, between 40 and 55 nucleotides, or between 45 and 50 nucleotides in length, *e.g.,* at least about 30, 35, 40, 45, 50, 75, 100, 200, 300, 400, 500, 750, 1000, 2000, 3000, 4000, 5000, 6000, 7000, 8000, 9000, 10000, or 15000 nucleotides in length.

In an embodiment, the distance between the cleavage event and the target position is between 10 and 10000 nucleotides in length, *e.g*., between 50 and 5000 nucleotides, between 100 and 1000 nucleotides, between 200 and 800 nucleotides, between 400 and 600 nucleotides, between 100 and 500 nucleotides, or between 500 and 1000 nucleotides in length, *e.g.,* at least about 20, 30, 40, 50, 75, 100, 200, 300, 400, 500, 750, 1000, 2000, 3000, 4000, 5000, 6000, 7000, 8000, 9000, or 10000 nucleotides in length.

In an embodiment, the cleavage event comprises a single-strand break, and wherein the distance between the single-strand break and the target position is between 10 and 10000 nucleotides in length, *e.g*., between 50 and 5000 nucleotides, between 100 and 1000 nucleotides, between 200 and 800 nucleotides, between 400 and 600 nucleotides, between 100 and 500 nucleotides, or between 500 and 1000 nucleotides in length, *e.g.,* at least about 20, 30, 40, 50, 75, 100, 200, 300, 400, 500, 750, 1000, 2000, 3000, 4000, 5000, 6000, 7000, 8000, 9000, or 10000 nucleotides in length.

In an embodiment, the cleavage event comprises two, three or four single-strand breaks, and wherein the distance between each of the single-strand breaks and the target position is between 10 and 10000 nucleotides in length, *e.g*., between 50 and 5000 nucleotides, between 100 and 1000 nucleotides, between 200 and 800 nucleotides, between 400 and 600 nucleotides, between 100 and 500 nucleotides, or between 500 and 1000 nucleotides in length, *e.g.,* at least about 20, 30, 40, 50, 75, 100, 200, 300, 400, 500, 750, 1000, 2000, 3000, 4000, 5000, 6000, 7000, 8000, 9000, or 10000 nucleotides in length.

In an embodiment, the cleavage event comprises a double-strand break, and wherein the distance between the double-strand break and the target position is between 10 and 10000 nucleotides in length, *e.g*., between 50 and 5000 nucleotides, between 100 and 1000 nucleotides, between 200 and 800 nucleotides, between 400 and 600 nucleotides, between 100 and 500 nucleotides, or between 500 and 1000 nucleotides in length, *e.g.,* at least about 20, 30, 40, 50, 75, 100, 200, 300, 400, 500, 750, 1000, 2000, 3000, 4000, 5000, 6000, 7000, 8000, 9000, or 10000 nucleotides in length.

In an embodiment, the cleavage event comprises two double-strand breaks, and wherein the distance between each of the double-strand breaks and the target position is between 10 and 10000 nucleotides in length, *e.g*., between 50 and 5000 nucleotides, between 100 and 1000 nucleotides, between 200 and 800 nucleotides, between 400 and 600 nucleotides, between 100 and 500 nucleotides, or between 500 and 1000 nucleotides in length, *e.g.,* at least about 20, 30, 40, 50, 75, 100, 200, 300, 400, 500, 750, 1000, 2000, 3000, 4000, 5000, 6000, 7000, 8000, 9000, or 10000 nucleotides in length.

In an embodiment, the cleavage event comprises a single-strand break and a double-strand break, wherein the distance between the single-strand break and the target position is between 10 and 10000 nucleotides in length, *e.g*., between 50 and 5000 nucleotides or between 100 and 1000 nucleotides in length, *e.g.,* about 20, 30, 40, 50, 75, 100, 200, 300, 400, 500, 750, 1000, 2000, 3000, 4000, 5000, 6000, 7000, 8000, 9000, or 10000 nucleotides in length, and
wherein the distance between the double-strand break and the target position is between 10 and 10000 nucleotides in length, *e.g*., between 50 and 5000 nucleotides or between 100 and 1000 nucleotides in length, *e.g.,* at least about 20, 30, 40, 50, 75, 100, 200, 300, 400, 500, 750, 1000, 2000, 3000, 4000, 5000, 6000, 7000, 8000, 9000, or 10000 nucleotides in length.

In an embodiment, the cleavage event comprises two single-strand breaks and a double-strand break,
wherein the distance between each of the single-strand breaks and the target position is between 10 and 10000 nucleotides in length, *e.g.*, between 50 and 5000 nucleotides or between 100 and 1000 nucleotides in length, *e.g.,* about 20, 30, 40, 50, 75, 100, 200, 300, 400, 500, 750, 1000, 2000, 3000, 4000, 5000, 6000, 7000, 8000, 9000, or 10000 nucleotides in length, and
wherein the distance between the double-strand break and the target position is between 10 and 10000 nucleotides in length, *e.g*., between 50 and 5000 nucleotides or between 100 and 1000 nucleotides in length, *e.g.,* at least about 20, 30, 40, 50, 75, 100, 200, 300, 400, 500, 750, 1000, 2000, 3000, 4000, 5000, 6000, 7000, 8000, 9000, or 10000 nucleotides in length.

In an embodiment, the cleavage event comprises two or more single-strand breaks, two or more double-strand breaks, or two single-strand breaks and one double-strand breaks,
wherein the distance between any of the two breaks that are present on the same strand is between 30 and 20000 nucleotides, 40 and 20000 nucleotides, or 50 and 20000 nucleotides in length, *e.g*., between 1000 and 10000 nucleotides or between 500 and 5000 nucleotides in length, *e.g*., between 40 and 60 nucleotides, between 40 and 55 nucleotides, or between 45 and 50 nucleotides in length, *e.g.,* at least about 30, 35, 40, 45, 50, 75, 100, 200, 300, 400, 500, 750, 1000, 2000, 3000, 4000, 5000, 6000, 7000, 8000, 9000, 10000, or 15000 nucleotides in length.

In an embodiment, the cleavage event comprises two or more single-strand breaks, two or more double-strand breaks, or two single-strand breaks and one double-strand breaks,
wherein the distance between at least two breaks that are present on different strands is between 30 and 20000 nucleotides, 40 and 20000 nucleotides, or 50 and 20000 nucleotides in length, *e.g*., between 1000 and 10000 nucleotides or between 500 and 5000 nucleotides in length, *e.g*., between 40 and 60 nucleotides, between 40 and 55 nucleotides, or between 45 and 50 nucleotides in length, *e.g.,* at least about 30, 35, 40, 45, 50, 75, 100, 200, 300, 400, 500, 750, 1000, 2000, 3000, 4000, 5000, 6000, 7000, 8000, 9000, 10000, or 15000 nucleotides in length.

In an embodiment, the cleavage event comprises two single-strand breaks, wherein the distance between the two single breaks is between 30 and 20000 nucleotides, 40 and 20000 nucleotides, or 50 and 20000 nucleotides in length, *e.g*., between 1000 and 10000 nucleotides or between 500 and 5000 nucleotides in length, *e.g*., between 40 and 60 nucleotides, between 40 and 55 nucleotides, or between 45 and 50 nucleotides in length, *e.g.,* at least about 30, 35, 40, 45, 50, 75, 100, 200, 300, 400, 500, 750, 1000, 2000, 3000, 4000, 5000, 6000, 7000, 8000, 9000, 10000, or 15000 nucleotides in length. In an embodiment, the single-strand breaks are present on different strands. In another embodiment, the single-strand breaks are present on the same strand. In an embodiment, the cleavage event further comprises one or more (*e.g*., two) of single-strand break, double-strand break, or both.

In an embodiment, the Cas9 molecule comprises HNH-like domain cleavage activity but has no, or no significant, N-terminal RuvC-like domain cleavage activity. In an embodiment, the eaCas9 molecule is an HNH-like domain nickase, *e.g.,* the Cas9 molecule comprises a mutation at D10, *e.g.,* D10A. In another embodiment, the eaCas9 molecule comprises N-terminal RuvC-like domain cleavage activity but has no, or no significant, HNH-like domain cleavage activity. In an embodiment, the Cas9 molecule is an N-terminal RuvC-like domain nickase, *e.g.,* the eaCas9 molecule comprises a mutation at N863, *e.g.,* N863A.

In an embodiment, the first gRNA molecule positions a cleavage event on a strand that does not bind to the first gRNA molecule.

In an embodiment, the second gRNA molecule positions a cleavage event on a strand that does not bind to the second gRNA molecule.

In an embodiment, the first gRNA molecule positions a cleavage event on a strand that does not bind to the first gRNA and the second gRNA molecule positions a cleavage event on a strand that does not bind to the second gRNA molecule, and wherein the gRNA molecule and the second gRNA molecule bind to different strands, *e.g*., resulting in a 3' overhang on each strand.

In an embodiment, the first gRNA molecule positions a cleavage event 5' to the target position on the first strand. In an embodiment, the first gRNA molecule positions a cleavage event 5' to the target position on the first strand, as shown in the diagram below: 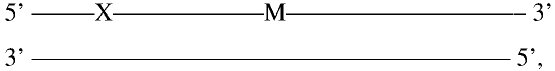 wherein X is the cleavage event and M is the target position.

In an embodiment, the second gRNA molecule positions a cleavage event 3' to the target position (relative to the target position on the first strand) on the second strand. In an embodiment, the second gRNA molecule positions a cleavage event 3' to the target position (relative to the target position on the first strand) on the second strand, as shown in the diagram below: 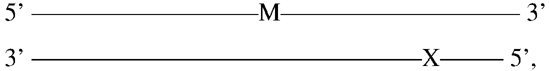 wherein X is the cleavage event and M is the target position.

In an embodiment, the second gRNA molecule positions a cleavage event 5' to the target position on the second strand. In an embodiment, the second gRNA molecule positions a cleavage event 5' to the target position on the second strand, as shown in the diagram below: 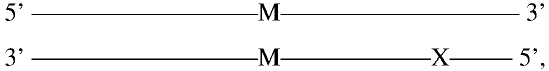 wherein X is the cleavage event and M is the target position.

In an embodiment, the first gRNA molecule positions a cleavage event 5' to the target position on the first strand, and wherein the second gRNA molecule positions a cleavage event 3' to the target position (relative to the target position on the first strand) on the second strand. In an embodiment, the first gRNA molecule positions a cleavage event 5' to the target position on the first strand, and wherein the second gRNA molecule positions a cleavage event 3' to the target position (relative to the target position on the first strand) on the second strand, as shown in the diagram below: 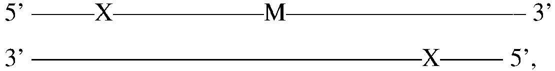 wherein X is the cleavage event and M is the target position.

In an embodiment, the first gRNA molecule positions a cleavage event 3' to the target position on the first strand. In an embodiment, the first gRNA molecule positions a cleavage event 3' to the target position on the first strand, as shown in the diagram below: 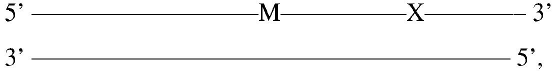 wherein X is the cleavage event and M is the target position.

In an embodiment, the second gRNA molecule positions a cleavage event 5' to the target position (relative to the target position on the first strand) on the second strand. In an embodiment, the second gRNA molecule positions a cleavage event 5' to the target position (relative to the target position on the first strand) on the second strand, as shown in the diagram below: 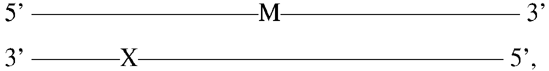 wherein X is the cleavage event and M is the target position.

In an embodiment, the first gRNA molecule positions a cleavage event 3' to the target position on the first strand, and wherein the second gRNA molecule positions a cleavage event 5' to the target position (relative to the target position on the first strand) on the second strand. In an embodiment, the first gRNA molecule positions a cleavage event 3' to the target position on the first strand, and wherein the second gRNA molecule positions a cleavage event 5' to the target position (relative to the target position on the first strand) on the second strand, as shown in the diagram below: 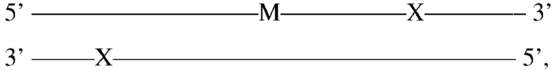 wherein X is the cleavage event and M is the target position.

In an embodiment, the first gRNA molecule positions a cleavage event 5' to the target position on the first strand, and wherein the second gRNA molecule positions a cleavage event 5' to the target position (relative to the target position on the first strand) on the second strand, *e.g.,* to produce a 5' overhang. In an embodiment, the first gRNA molecule positions a cleavage event 5' to the target position on the first strand, and wherein the second gRNA molecule positions a cleavage event 5' to the target position (relative to the target position on the first strand) on the second strand, *e.g.,* to produce a 5' overhang, as shown in the diagram below: 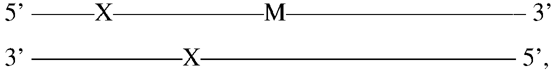 wherein X is the cleavage event and M is the target position.

In an embodiment, the first gRNA molecule positions a cleavage event 3' to the target position on the first strand, and wherein the second gRNA molecule positions a cleavage event 3' to the target position (relative to the target position on the first strand) on the second strand, *e.g.,* to produce a 5' overhang. In an embodiment, the first gRNA molecule positions a cleavage event 3' to the target position on the first strand, and wherein the second gRNA molecule positions a cleavage event 3' to the target position (relative to the target position on the first strand) on the second strand, *e.g.,* to produce a 5' overhang, as shown in the diagram below: 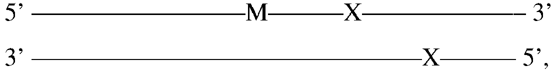 wherein X is the cleavage event and M is the target position.

In an embodiment, the first gRNA molecule positions a cleavage event 5' to the target position on the first strand, and wherein the second gRNA molecule positions a cleavage event 5' to the target position (relative to the target position on the first strand) on the second strand, *e.g.,* to produce a 3' overhang. In an embodiment, the first gRNA molecule positions a cleavage event 5' to the target position on the first strand, and wherein the second gRNA molecule positions a cleavage event 5' to the target position (relative to the target position on the first strand) on the second strand, *e.g.,* to produce a 3' overhang, as shown in the diagram below: 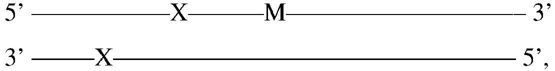 wherein X is the cleavage event and M is the target position.

In an embodiment, the first gRNA molecule positions a cleavage event 3' to the target position on the first strand, and wherein the second gRNA molecule positions a cleavage event 3' to the target position (relative to the target position on the first strand) on the second strand, *e.g.,* to produce a 3' overhang. In an embodiment, the first gRNA molecule positions a cleavage event 3' to the target position on the first strand, and wherein the second gRNA molecule positions a cleavage event 3' to the target position (relative to the target position on the first strand) on the second strand, *e.g.,* to produce a 3' overhang, as shown in the diagram below: 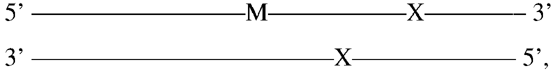 wherein X is the cleavage event and M is the target position.

In one embodiment, the target position comprises a mutation. In one embodiment, the mutation is associated with a disease phenotype.

In an embodiment, the first gRNA molecule positions a cleavage event on a strand that binds to the gRNA molecule.

In an embodiment, the second gRNA molecule positions a cleavage event on a strand that binds to the second gRNA molecule.

In an embodiment, the first gRNA molecule positions a cleavage event on a strand that binds to the gRNA and the second gRNA molecule positions a cleavage event on a strand that binds to the second gRNA molecule, and wherein the first gRNA molecule and the second gRNA molecule bind to different strands, *e.g*., resulting in a 5' overhang on each strand.

In an embodiment, the gRNA molecule, together with the Cas9 molecule *(e.g.,* a nickase), positions a cleavage event on a strand *(e.g.,* a first strand or a second strand),

In an embodiment, the gRNA molecule positions a cleavage event 5' to the target position on the first strand. This embodiment allows the use of a single Cas9 molecule, *e.g.,* a single Cas9 molecule that is a nickase *(e.g.,* a Cas9 molecule with a D10A mutation), *e.g.,* to place a single-strand cleavage event sufficiently close to the target position (*e.g*., within 10000, 9000, 8000, 7000, 6000, 5000, 4000, 3000, 2000, 1000, 800, 600, 500, 400, 300, 200, 100, 75, 50, 40, 30, 20, 10, 5, or 1 bp to the target position). For example, this embodiment can be illustrated as shown in the diagram below: 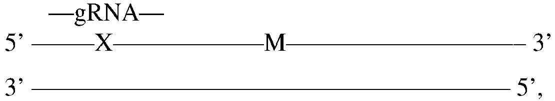 wherein X is the cleavage event, M is the target position.

In an embodiment, the gRNA molecule positions a cleavage event 3' to the target position (relative to the target position on the first strand) on the second strand. This embodiment allows the use of a single Cas9 molecule, *e.g.,* a single Cas9 molecule that is a nickase *(e.g.,* a Cas9 molecule with a D10A mutation), *e.g.,* to place a single-strand cleavage event sufficiently close to the target position (*e.g*., within 10000, 9000, 8000, 7000, 6000, 5000, 4000, 3000, 2000, 1000, 800, 600, 500, 400, 300, 200, 100, 75, 50, 40, 30, 20, 10, 5, or 1 bp to the target position). For example, this embodiment can be illustrated as shown in the diagram below: 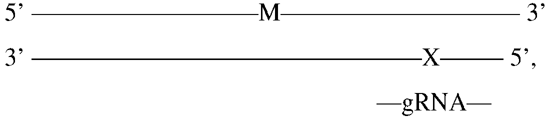 wherein X is the cleavage event, M is the target position.

In an embodiment, the gRNA molecule positions a cleavage event 3' to the target position on the first strand. This embodiment allows the use of a single Cas9 molecule, *e.g.,* a single Cas9 molecule that is a nickase *(e.g.,* a Cas9 molecule with a D10A mutation), *e.g.,* to place a single-strand cleavage event sufficiently close to the target position (*e.g*., within 10000, 9000, 8000, 7000, 6000, 5000, 4000, 3000, 2000, 1000, 800, 600, 500, 400, 300, 200, 100, 75, 50, 40, 30, 20, 10, 5, or 1 bp to the target position). For example, this embodiment can be illustrated as shown in the diagram below: 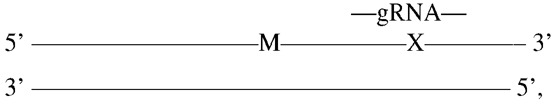 wherein X is the cleavage event, M is the target position.

In an embodiment, the gRNA molecule positions a cleavage event 5' to the target position (relative to the target position on the first strand) on the second strand. This embodiment allows the use of a single Cas9 molecule, *e.g.,* a single Cas9 molecule that is a nickase (*e.g.,* a Cas9 molecule with a D10A mutation), *e.g.,* to place a single-strand cleavage event sufficiently close to the target position (*e.g*., within 10000, 9000, 8000, 7000, 6000, 5000, 4000, 3000, 2000, 1000, 800, 600, 500, 400, 300, 200, 100, 75, 50, 40, 30, 20, 10, 5, or 1 bp to the target position). For example, this embodiment can be illustrated as shown in the diagram below: 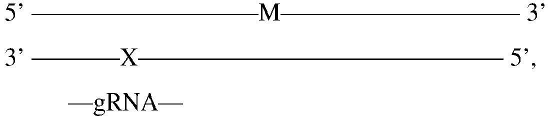 wherein X is the cleavage event, M is the target position.

In an embodiment, the gRNA molecule positions a cleavage event 5' to the target position on the first strand. This embodiment allows the use of a single Cas9 molecule, *e.g.,* a single Cas9 molecule that is a nickase (*e.g.,* a Cas9 molecule with an N863A mutation), *e.g.,* to place a single-strand cleavage event sufficiently close to the target position (*e.g.,* within 10000, 9000, 8000, 7000, 6000, 5000, 4000, 3000, 2000, 1000, 800, 600, 500, 400, 300, 200, 100, 75, 50, 40, 30, 20, 10, 5, or 1 bp to the target position). For example, this embodiment can be illustrated as shown in the diagram below: 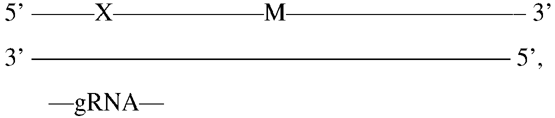 wherein X is the cleavage event, M is the target position.

In an embodiment, the gRNA molecule positions a cleavage event 3' to the target position (relative to the target position on the first strand) on the second strand. This embodiment allows the use of a single Cas9 molecule, *e.g.,* a single Cas9 molecule that is a nickase (*e.g.,* a Cas9 molecule with an N863A mutation), *e.g.,* to place a single-strand cleavage event sufficiently close to the target position (*e.g*., within 10000, 9000, 8000, 7000, 6000, 5000, 4000, 3000, 2000, 1000, 800, 600, 500, 400, 300, 200, 100, 75, 50, 40, 30, 20, 10, 5, or 1 bp to the target position). For example, this embodiment can be illustrated as shown in the diagram below: 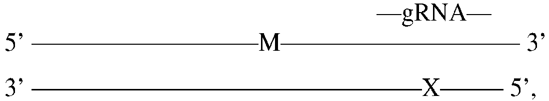 wherein X is the cleavage event, M is the target position.

In an embodiment, the gRNA molecule positions a cleavage event 3' to the target position on the first strand. This embodiment allows the use of a single Cas9 molecule, *e.g.,* a single Cas9 molecule that is a nickase *(e.g.,* a Cas9 molecule with an N863A mutation), *e.g.,* to place a single-strand cleavage event sufficiently close to the target position (*e.g.,* within 10000, 9000, 8000, 7000, 6000, 5000, 4000, 3000, 2000, 1000, 800, 600, 500, 400, 300, 200, 100, 75, 50, 40, 30, 20, 10, 5, or 1 bp to the target position). For example, this embodiment can be illustrated as shown in the diagram below: 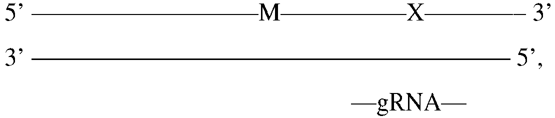 wherein X is the cleavage event, M is the target position.

In an embodiment, the gRNA molecule positions a cleavage event 5' to the target position (relative to the target position on the first strand) on the second strand. This embodiment allows the use of a single Cas9 molecule, *e.g.,* a single Cas9 molecule that is a nickase (*e.g.,* a Cas9 molecule with an N863A mutation), *e.g.,* to place a single-strand cleavage event sufficiently close to the target position (*e.g*., within 10000, 9000, 8000, 7000, 6000, 5000, 4000, 3000, 2000, 1000, 800, 600, 500, 400, 300, 200, 100, 75, 50, 40, 30, 20, 10, 5, or 1 bp to the target position). For example, this embodiment can be illustrated as shown in the diagram below: 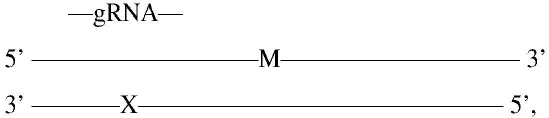 wherein X is the cleavage event, M is the target position.

In an embodiment the gRNA molecule, together with the Cas9 molecule *(e.g.,* a nickase), positions a cleavage event on the strand that binds to the gRNA molecule; and the second gRNA molecule, together with the Cas9 molecule, positions a cleavage event on the strand that binds to the second gRNA molecule, wherein the gRNA molecule and the second gRNA molecule bind to different strands, the gRNA molecule positions a cleavage event 5' to the target position on the first strand, and the second gRNA molecule positions a cleavage event 3' to the target position (relative to the target position on the first strand) on the second strand. This embodiment allows the use of a single Cas9 molecule, *e.g.,* a single Cas9 molecule that is a nickase *(e.g.,* a Cas9 molecule with a D10A mutation), *e.g.,* to place single-strand cleavage events on each side of the target position.

For example, this embodiment can be illustrated as shown in the diagram below: 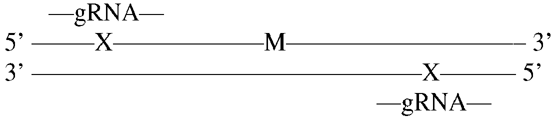 wherein X is the cleavage event, M is the target position.

In an embodiment, the cleavage event positioned by the gRNA molecule and the cleavage event positioned by the second gRNA molecule are separated by 10 to 10000, 10 to 5000, 10 to 2500, 10 to 1000, 10 to 750, 10 to 500, 10 to 400, 10 to 300, 10 to 200, 10 to 100, 10 to 75, 10 to 50, or 10 to 25 base pairs.

In an embodiment:
the gRNA molecule, together with the Cas9 molecule (a nickase), positions a cleavage event on the strand other than the strand that binds to the gRNA molecule; and
the second gRNA molecule, together with the Cas9 molecule, positions a cleavage event on the strand other than the strand that binds to the second gRNA molecule,
wherein:
   the gRNA molecule and the second gRNA molecule bind to different strands,
   the gRNA molecule positions a cleavage event 5' to the target position on the first strand, and
   the second gRNA molecule positions a cleavage event 3' to the target position (relative to the target position on the first strand) on the second strand. This embodiment allows the use of a single Cas9 molecule, *e.g.,* a single Cas9 molecule that is a nickase *(e.g.,* a Cas9 molecule with an N863A mutation), *e.g*., to place single-strand cleavage events on each side of the target position.

For example, this embodiment can be illustrated as shown in the diagram below: 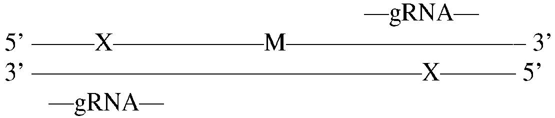 wherein X is the cleavage event and M is the target position.

In an embodiment, the cleavage event positioned by the gRNA molecule and the cleavage event positioned by the second gRNA molecule are separated by 10 to 10000, 10 to 5000, 10 to 2500, 10 to 1000, 10 to 750, 10 to 500, 10 to 400, 10 to 300, 10 to 200, 10 to 100, 10 to 75, 10 to 50, or 10 to 25 base pairs.

In an embodiment:
the gRNA molecule, together with the Cas9 molecule (a nickase), positions a cleavage event on the strand that binds to the gRNA molecule; and
the second gRNA molecule, together with the Cas9 molecule, positions a cleavage event on the strand that binds to the second gRNA molecule,
wherein:
   the gRNA molecule and the second gRNA molecule bind to different strands,
   the gRNA molecule positions a cleavage event 3' to the target position on the first strand, and
   the second gRNA molecule positions a cleavage event 5' to the target position (relative to the target position on the first strand) on the second strand. This embodiment allows the use of a single Cas9 molecule, *e.g.,* a single Cas9 molecule that is a nickase *(e.g.,* a Cas9 molecule with a D10A mutation), *e.g*., to place single-strand cleavage events on each side of the target position.

For example, this embodiment can be illustrated as shown in the diagram below: 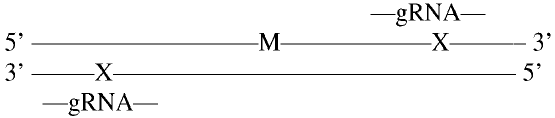 wherein X is the cleavage event and M is the target position.

In an embodiment, the cleavage event positioned by the gRNA molecule and the cleavage event positioned by the second gRNA molecule are separated by 10 to 10000, 10 to 5000, 10 to 2500, 10 to 1000, 10 to 750, 10 to 500, 10 to 400, 10 to 300, 10 to 200, 10 to 100, 10 to 75, 10 to 50, or 10 to 25 base pairs.

In an embodiment:
the gRNA molecule, together with the Cas9 molecule (a nickase), positions a cleavage event on the strand other than the strand that binds to the gRNA molecule; and
the second gRNA molecule, together with the Cas9 molecule, positions a cleavage event on the strand other than the strand that binds to the second gRNA molecule,
wherein:
   the gRNA molecule and the second gRNA molecule bind to different strands,
   the gRNA molecule positions a cleavage event 3' to the target position on the first strand, and
   the second gRNA molecule positions a cleavage event 5' to the target position (relative to the target position on the first strand) on the second strand. This embodiment allows the use of a single Cas9 molecule, *e.g.,* a single Cas9 molecule that is a nickase (*e.g.,* a Cas9 molecule with a N863A mutation), *e.g.*, to place single-strand cleavage events on each side of the target position.

For example, this embodiment can be illustrated as shown in the diagram below: 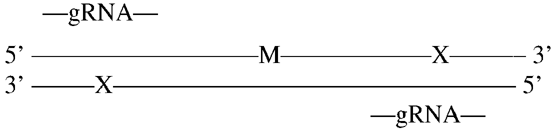 wherein X is the cleavage event and M is the target position.

In an embodiment, the cleavage event positioned by the gRNA molecule and the cleavage event positioned by the second gRNA molecule are separated by 10 to 10000, 10 to 5000, 10 to 2500, 10 to 1000, 10 to 750, 10 to 500, 10 to 400, 10 to 300, 10 to 200, 10 to 100, 10 to 75, 10 to 50, or 10 to 25 base pairs.

In an embodiment:
the gRNA molecule, together with the Cas9 molecule (*e.g.,* a nickase), positions a cleavage event on the strand that binds to the gRNA molecule; and
the second gRNA molecule, together with the Cas9 molecule, positions a cleavage event on the strand that binds to the second gRNA molecule,
wherein:
   the gRNA molecule and the second gRNA molecule bind to different strands,
   the gRNA molecule positions a cleavage event 5' to the target position on the first strand, and
   the second gRNA molecule positions a cleavage event 5' to the target position (relative to the target position on the first strand) on the second strand, *e.g.,* to produce a 5' overhang. This embodiment allows the use of a single Cas9 molecule, *e.g.,* a single Cas9 molecule that is a nickase *(e.g.,* a Cas9 molecule with a D10A mutation), *e.g.,* to place single-strand cleavage events on one side of the target position, *e.g*., to produce a 5' overhang.

For example, this embodiment can be illustrated as shown in the diagram below: 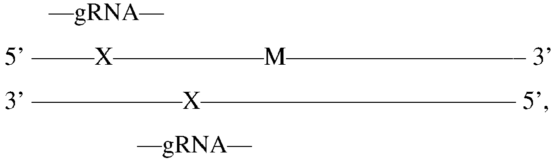 wherein X is the cleavage event and M is the target position.

In an embodiment, the cleavage event positioned by the gRNA molecule and the cleavage event positioned by the second gRNA molecule are separated by 10 to 10000, 10 to 5000, 10 to 2500, 10 to 1000, 10 to 750, 10 to 500, 10 to 400, 10 to 300, 10 to 200, 10 to 100, 10 to 75, 10 to 50, or 10 to 25 base pairs.

In an embodiment:
the gRNA molecule, together with the Cas9 molecule (a nickase), positions a cleavage event on the strand other than the strand that binds to the gRNA molecule; and
the second gRNA molecule, together with the Cas9 molecule, positions a cleavage event on the strand other than the strand that binds to the second gRNA molecule,
wherein:
   the gRNA molecule and the second gRNA molecule bind to different strands,
   the gRNA molecule positions a cleavage event 5' to the target position on the first strand, and
   the second gRNA molecule positions a cleavage event 5' to the target position (relative to the target position on the first strand) on the second strand, *e.g.,* to produce a 5' overhang. This embodiment allows the use of a single Cas9 molecule, *e.g.,* a single Cas9 molecule that is a nickase (*e.g.,* a Cas9 molecule with an N863A mutation), *e.g.,* to place single-strand cleavage events on each side of the target position, *e.g*., to produce a 5' overhang.

For example, this embodiment can be illustrated as shown in the diagram below: 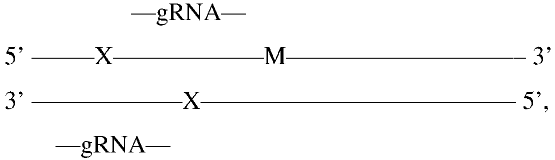 wherein X is the cleavage event and M is the target position.

In an embodiment, the cleavage event positioned by the gRNA molecule and the cleavage event positioned by the second gRNA molecule are separated by 10 to 10000, 10 to 5000, 10 to 2500, 10 to 1000, 10 to 750, 10 to 500, 10 to 400, 10 to 300, 10 to 200, 10 to 100, 10 to 75, 10 to 50, or 10 to 25 base pairs.

In an embodiment:
the gRNA molecule, together with the Cas9 molecule *(e.g.,* a nickase), positions a cleavage event on the strand that binds to the gRNA molecule; and
the second gRNA molecule, together with the Cas9 molecule, positions a cleavage event on the strand that binds to the second gRNA molecule,
wherein:
   the gRNA molecule and the second gRNA molecule bind to different strands,
   the gRNA molecule positions a cleavage event 3' to the target position on the first strand, and
   the second gRNA molecule positions a cleavage event 3' to the target position (relative to the target position on the first strand) on the second strand, *e.g.,* to produce a 5' overhang. This embodiment allows the use of a single Cas9 molecule, *e.g.,* a single Cas9 molecule that is a nickase (*e.g.,* a Cas9 molecule with a D10A mutation), *e.g.,* to place single-strand cleavage events on one side of the target position, *e.g*., to produce a 5' overhang.

For example, this embodiment can be illustrated as shown in the diagram below: 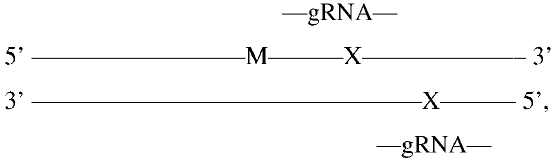 wherein X is the cleavage event and M is the target position.

In an embodiment, the cleavage event positioned by the gRNA molecule and the cleavage event positioned by the second gRNA molecule are separated by 10 to 10000, 10 to 5000, 10 to 2500, 10 to 1000, 10 to 750, 10 to 500, 10 to 400, 10 to 300, 10 to 200, 10 to 100, 10 to 75, 10 to 50, or 10 to 25 base pairs.

In an embodiment:
the gRNA molecule, together with the Cas9 molecule (a nickase), positions a cleavage event on the strand other than the strand that binds to the gRNA molecule; and
the second gRNA molecule, together with the Cas9 molecule, positions a cleavage event on the strand other than the strand that binds to the second gRNA molecule,
wherein:
   the gRNA molecule and the second gRNA molecule bind to different strands,
   the gRNA molecule positions a cleavage event 3' to the target position on the first strand, and
   the second gRNA molecule positions a cleavage event 3' to the target position (relative to the target position on the first strand) on the second strand, *e.g.,* to produce a 5' overhang. This embodiment allows the use of a single Cas9 molecule, *e.g.,* a single Cas9 molecule that is a nickase (*e.g.,* a Cas9 molecule with an N863A mutation), *e.g.,* to place single-strand cleavage events on each side of the target position, *e.g*., to produce a 5' overhang.

For example, this embodiment can be illustrated as shown in the diagram below: 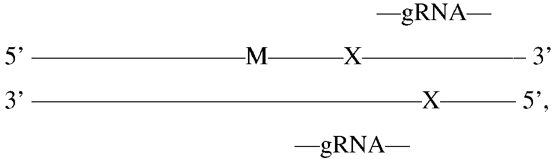 wherein X is the cleavage event and M is the target position.

In an embodiment, the cleavage event positioned by the gRNA molecule and the cleavage event positioned by the second gRNA molecule are separated by 10 to 10000, 10 to 5000, 10 to 2500, 10 to 1000, 10 to 750, 10 to 500, 10 to 400, 10 to 300, 10 to 200, 10 to 100, 10 to 75, 10 to 50, or 10 to 25 base pairs.

In an embodiment:
the gRNA molecule, together with the Cas9 molecule *(e.g.,* a nickase), positions a cleavage event on the strand that binds to the gRNA molecule; and
the second gRNA molecule, together with the Cas9 molecule, positions a cleavage event on the strand that binds to the second gRNA molecule,
wherein:
   the gRNA molecule and the second gRNA molecule bind to different strands,
   the gRNA molecule positions a cleavage event 5' to the target position on the first strand, and
   the second gRNA molecule positions a cleavage event 5' to the target position (relative to the target position on the first strand) on the second strand, *e.g.,* to produce a 3' overhang. This embodiment allows the use of a single Cas9 molecule, *e.g.,* a single Cas9 molecule that is a nickase (*e.g.,* a Cas9 molecule with a D10A mutation), *e.g.,* to place single-strand cleavage events on one side of the target position, *e.g*., to produce a 3' overhang.

For example, this embodiment can be illustrated as shown in the diagram below: 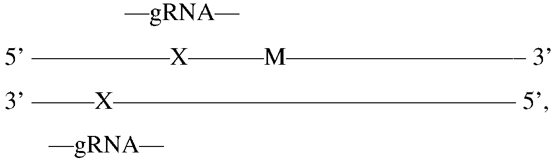 wherein X is the cleavage event and M is the target position.

In an embodiment, the cleavage event positioned by the gRNA molecule and the cleavage event positioned by the second gRNA molecule are separated by 10 to 10000, 10 to 5000, 10 to 2500, 10 to 1000, 10 to 750, 10 to 500, 10 to 400, 10 to 300, 10 to 200, 10 to 100, 10 to 75, 10 to 50, or 10 to 25 base pairs.

In an embodiment:
the gRNA molecule, together with the Cas9 molecule (a nickase), positions a cleavage event on the strand other than the strand that binds to the gRNA molecule; and
the second gRNA molecule, together with the Cas9 molecule, positions a cleavage event on the strand other than the strand that binds to the second gRNA molecule,
wherein:
   the gRNA molecule and the second gRNA molecule bind to different strands,
   the gRNA molecule positions a cleavage event 5' to the target position on the first strand, and
   the second gRNA molecule positions a cleavage event 5' to the target position (relative to the target position on the first strand) on the second strand, *e.g.,* to produce a 3' overhang. This embodiment allows the use of a single Cas9 molecule, *e.g.,* a single Cas9 molecule that is a nickase (*e.g.,* a Cas9 molecule with an N863A mutation), *e.g.,* to place single-strand cleavage events on each side of the target position, *e.g*., to produce a 3' overhang.

For example, this embodiment can be illustrated as shown in the diagram below: 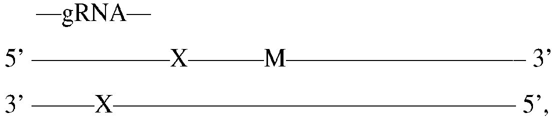 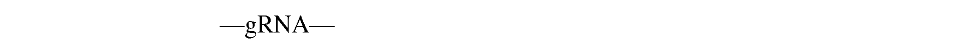 wherein X is the cleavage event and M is the target position.

In an embodiment, the cleavage event positioned by the gRNA molecule and the cleavage event positioned by the second gRNA molecule are separated by 10 to 10000, 10 to 5000, 10 to 2500, 10 to 1000, 10 to 750, 10 to 500, 10 to 400, 10 to 300, 10 to 200, 10 to 100, 10 to 75, 10 to 50, or 10 to 25 base pairs.

In an embodiment:
the gRNA molecule, together with the Cas9 molecule *(e.g.,* a nickase), positions a cleavage event on the strand that binds to the gRNA molecule; and
the second gRNA molecule, together with the Cas9 molecule, positions a cleavage event on the strand that binds to the second gRNA molecule,
wherein:
   the gRNA molecule and the second gRNA molecule bind to different strands,
   the gRNA molecule positions a cleavage event 3' to the target position on the first strand, and
   the second gRNA molecule positions a cleavage event 3' to the target position (relative to the target position on the first strand) on the second strand, *e.g.,* to produce a 3' overhang. This embodiment allows the use of a single Cas9 molecule, *e.g.,* a single Cas9 molecule that is a nickase (*e.g.,* a Cas9 molecule with a D10A mutation), *e.g.,* to place single-strand cleavage events on one side of the target position, *e.g*., to produce a 3' overhang.

For example, this embodiment can be illustrated as shown in the diagram below: 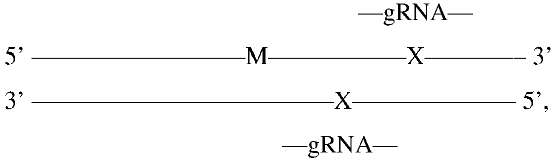 wherein X is the cleavage event and M is the target position.

In an embodiment, the cleavage event positioned by the gRNA molecule and the cleavage event positioned by the second gRNA molecule are separated by 10 to 10000, 10 to 5000, 10 to 2500, 10 to 1000, 10 to 750, 10 to 500, 10 to 400, 10 to 300, 10 to 200, 10 to 100, 10 to 75, 10 to 50, or 10 to 25 base pairs.

In an embodiment:
the gRNA molecule, together with the Cas9 molecule (a nickase), positions a cleavage event on the strand other than the strand that binds to the gRNA molecule; and
the second gRNA molecule, together with the Cas9 molecule, positions a cleavage event on the strand other than the strand that binds to the second gRNA molecule,
wherein:
   the gRNA molecule and the second gRNA molecule bind to different strands,
   the gRNA molecule positions a cleavage event 3' to the target position on the first strand, and
   the second gRNA molecule positions a cleavage event 3' to the target position (relative to the target position on the first strand) on the second strand, *e.g.,* to produce a 3' overhang. This embodiment allows the use of a single Cas9 molecule, *e.g.,* a single Cas9 molecule that is a nickase (*e.g.,* a Cas9 molecule with an N863A mutation), *e.g.,* to place single-strand cleavage events on each side of the target position, *e.g.*, to produce a 3' overhang.

For example, this embodiment can be illustrated as shown in the diagram below: 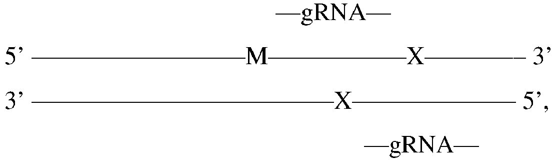 wherein X is the cleavage event and M is the target position.

In an embodiment, the cleavage event positioned by the gRNA molecule and the cleavage event positioned by the second gRNA molecule are separated by 10 to 10000, 10 to 5000, 10 to 2500, 10 to 1000, 10 to 750, 10 to 500, 10 to 400, 10 to 300, 10 to 200, 10 to 100, 10 to 75, 10 to 50, or 10 to 25 base pairs.

### Homology arms of the donor template

A homology arm should extend at least as far as the region in which end resection may occur, *e.g.,* in order to allow the resected single stranded overhang to find a complementary region within the donor template. The overall length could be limited by parameters such as plasmid size or viral packaging limits. In an embodiment, a homology arm does not extend into repeated elements, *e.g*., Alu repeats or LINE repeats.

Exemplary homology arm lengths include at least 50, 100, 250, 500, 750, 1000, 2000, 3000, 4000, or 5000 nucleotides. In some embodiments, the homology arm length is 50-100, 100-250, 250-500, 500-750, 750-1000, 1000-2000, 2000-3000, 3000-4000, or 4000-5000 nucleotides.

Target position, as used herein, refers to a site on a target nucleic acid (*e.g.,* the chromosome) that is modified by a Cas9 molecule-dependent process. For example, the target position can be a modified Cas9 molecule cleavage of the target nucleic acid and template nucleic acid directed modification, *e.g*., correction, of the target position. In an embodiment, a target position can be a site between two nucleotides, *e.g*., adjacent nucleotides, on the target nucleic acid into which one or more nucleotides is added. The target position may comprise one or more nucleotides that are altered, *e.g.,* corrected, by a template nucleic acid. In an embodiment, the target position is within a target sequence (*e.g*., the sequence to which the gRNA binds). In an embodiment, a target position is upstream or downstream of a target sequence (*e.g.,* the sequence to which the gRNA binds).

A template nucleic acid, as that term is used herein, refers to a nucleic acid sequence which can be used in conjunction with a Cas9 molecule and a gRNA molecule to alter the structure of a target position. In certain embodiments, the target nucleic acid is modified to have the some or all of the sequence of the template nucleic acid, typically at or near cleavage site(s). In an embodiment, the template nucleic acid is single stranded. In certain embodiments, the template nucleic acid is double stranded. In certain embodiments, the template nucleic acid is DNA, *e.g.,* double stranded DNA. In other embodiments, the template nucleic acid is single stranded DNA. In certain embodiments, the template nucleic acid is encoded on the same vector backbone, *e.g.*, AAV genome, plasmid DNA, as the Cas9 and gRNA. In an embodiment, the template nucleic acid is excised from a vector backbone *in vivo, e.g.,* it is flanked by gRNA recognition sequences. In certain embodiments, the template nucleic acid comprises endogenous genomic sequence.

In certain embodiments, the template nucleic acid alters the structure of the target position by participating in an HDR event. In certain embodiments, the template nucleic acid alters the sequence of the target position. In certain embodiments, the template nucleic acid results in the incorporation of a modified, or non-naturally occurring base into the target nucleic acid.

Typically, the template sequence undergoes a breakage mediated or catalyzed recombination with the target sequence. In certain embodiments, the template nucleic acid includes sequence that corresponds to a site on the target sequence that is cleaved by an eaCas9 mediated cleavage event. In certain embodiments, the template nucleic acid includes sequence that corresponds to both, a first site on the target sequence that is cleaved in a first Cas9 mediated event, and a second site on the target sequence that is cleaved in a second Cas9 mediated event.

In an embodiment, the template nucleic acid can include sequence which results in an alteration in the coding sequence of a translated sequence, *e.g*., one which results in the substitution of one amino acid for another in a protein product, *e.g*., transforming a mutant allele into a wild type allele, transforming a wild type allele into a mutant allele, and/or introducing a stop codon, insertion of an amino acid residue, deletion of an amino acid residue, or a nonsense mutation.

In other embodiments, the template nucleic acid can include sequence which results in an alteration in a non-coding sequence, *e.g.,* an alteration in an exon or in a 5' or 3' non-translated or non-transcribed region. Such alterations include an alteration in a control element, *e.g.,* a promoter, enhancer, and an alteration in a cis-acting or trans-acting control element.

A template nucleic acid typically comprises the following components:
[5' homology arm]-[replacement sequence]-[3' homology arm].

The homology arms provide for recombination into the chromosome, thus replacing the undesired element, *e.g.,* a mutation or signature, with a replacement sequence. In certain embodiments, the homology arms flank the most distal cleavage sites.

In certain embodiments, the 3' end of the 5' homology arm is the position next to the 5' end of the replacement sequence. In an embodiment, the 5' homology arm can extend at least 10, 20, 30, 40, 50, 100, 200, 300, 400, 500, 600, 700, 800, 900, 1000, 1500, 2000, 3000, 4000, or 5000 nucleotides 5' from the 5' end of the replacement sequence.

In certain embodiments, the 5' end of the 3' homology arm is the position next to the 3' end of the replacement sequence. In certain embodiments, the 3' homology arm can extend at least 10, 20, 30, 40, 50, 100, 200, 300, 400, 500, 600, 700, 800, 900, 1000, 1500, 2000, 3000, 4000, or 5000 nucleotides 3' from the 3' end of the replacement sequence.

In certain embodiments, to alter one or more nucleotides at a target position (*e.g*., to correct a mutation), the homology arms, *e.g.,* the 5' and 3' homology arms, may each comprise about 1000 bp of sequence flanking the most distal gRNAs (*e.g*., 1000 bp of sequence on either side of the target position (*e.g*., the mutation).

It is contemplated herein that one or both homology arms may be shortened to avoid including certain sequence repeat elements, *e.g*., Alu repeats or LINE elements. For example, a 5' homology arm may be shortened to avoid a sequence repeat element. In other embodiments, a 3' homology arm may be shortened to avoid a sequence repeat element. In some embodiments, both the 5' and the 3' homology arms may be shortened to avoid including certain sequence repeat elements.

It is contemplated herein that template nucleic acids for altering the sequence (*e.g*., correcting a mutation) of a target position may be designed for use as a single-stranded oligonucleotide, *e.g.*, a single-stranded oligodeoxynucleotide (ssODN). When using a ssODN, 5' and 3' homology arms may range up to about 200 bp in length, *e.g.,* at least 25, 50, 75, 100, 125, 150, 175, or 200 bp in length. Longer homology arms are also contemplated for ssODNs as improvements in oligonucleotide synthesis continue to be made. In some embodiments, a longer homology arm is made by a method other than chemical synthesis, *e.g*., by denaturing a long double stranded nucleic acid and purifying one of the strands, *e.g*., by affinity for a strand-specific sequence anchored to a solid substrate.

While not wishing to be bound by theory, in certain embodiments alt-HDR proceeds more efficiently when the template nucleic acid has extended homology 5' to the nick (*i.e*., in the 5' direction of the nicked strand). Accordingly, in some embodiments, the template nucleic acid has a longer homology arm and a shorter homology arm, wherein the longer homology arm can anneal 5' of the nick. In some embodiments, the arm that can anneal 5' to the nick is at least 25, 50, 75, 100, 125, 150, 175, or 200, 300, 400, 500, 600, 700, 800, 900, 1000, 1500, 2000, 3000, 4000, or 5000 nucleotides from the nick or the 5' or 3' end of the replacement sequence. In some embodiments, the arm that can anneal 5' to the nick is at least 10%, 20%, 30%, 40%, or 50% longer than the arm that can anneal 3' to the nick. In some embodiments, the arm that can anneal 5' to the nick is at least 2x, 3x, 4x, or 5x longer than the arm that can anneal 3' to the nick. Depending on whether a ssDNA template can anneal to the intact strand or the nicked strand, the homology arm that anneals 5' to the nick may be at the 5' end of the ssDNA template or the 3' end of the ssDNA template, respectively.

Similarly, in some embodiments, the template nucleic acid has a 5' homology arm, a replacement sequence, and a 3' homology arm, such that the template nucleic acid has extended homology to the 5' of the nick. For example, the 5' homology arm and 3' homology arm may be substantially the same length, but the replacement sequence may extend farther 5' of the nick than 3' of the nick. In some embodiments, the replacement sequence extends at least 10%, 20%, 30%, 40%, 50%, 2x, 3x, 4x, or 5x further to the 5' end of the nick than the 3' end of the nick.

While not wishing to be bound by theory, In some embodiments, alt-HDR proceeds more efficiently when the template nucleic acid is centered on the nick. Accordingly, in some embodiments, the template nucleic acid has two homology arms that are essentially the same size. For instance, the first homology arm of a template nucleic acid may have a length that is within 10%, 9%, 8%, 7%, 6%, 5%, 4%, 3%, 2%, or 1% of the second homology arm of the template nucleic acid.

Similarly, in some embodiments, the template nucleic acid has a 5' homology arm, a replacement sequence, and a 3' homology arm, such that the template nucleic acid extends substantially the same distance on either side of the nick. For example, the homology arms may have different lengths, but the replacement sequence may be selected to compensate for this. For example, the replacement sequence may extend further 5' from the nick than it does 3' of the nick, but the homology arm 5' of the nick is shorter than the homology arm 3' of the nick, to compensate. The converse is also possible, *e.g*., that the replacement sequence may extend further 3' from the nick than it does 5' of the nick, but the homology arm 3' of the nick is shorter than the homology arm 5' of the nick, to compensate.

### Exemplary template nucleic acids

In a preferred embodiment, and in order to increase DNA repair via gene correction, the template nucleic acid linked to a gRNA molecule, as described herein. In certain embodiments, the template nucleic acid is double stranded. In other embodiments, the template nucleic acid is single stranded. In certain embodiments, the template nucleic acid comprises a single stranded portion and a double stranded portion. In certain embodiments, the template nucleic acid comprises about 50 to 100, *e.g.,* 55 to 95, 60 to 90, 65 to 85, or 70 to 80 bp, homology on either side of the nick and/or replacement sequence. In certain embodiments, the template nucleic acid comprises about 50, 55, 60, 65, 70, 75, 80, 85, 90, 95, or 100 bp homology 5' of the nick or replacement sequence, 3' of the nick or replacement sequence, or both 5' and 3' of the nick or replacement sequences.

In certain embodiments, the template nucleic acid comprises about 150 to 200 bp, *e.g.,* 155 to 195, 160 to 190, 165 to 185, or 170 to 180 bp, homology 3' of the nick and/or replacement sequence. In certain embodiments, the template nucleic acid comprises about 150, 155, 160, 165, 170, 175, 180, 185, 190, 195, or 200 bp homology 3' of the nick or replacement sequence. In some embodiments, the template nucleic acid comprises less than about 100, 90, 80, 70, 60, 50, 40, 30, 20, 15, or 10 bp homology 5' of the nick or replacement sequence.

In certain embodiments, the template nucleic acid comprises about 150 to 200 bp, *e.g.,* 155 to 195, 160 to 190, 165 to 185, or 170 to 180 bp, homology 5' of the nick and/or replacement sequence. In certain embodiments, the template nucleic acid comprises about 150, 155, 160, 165, 170, 175, 180, 185, 190, 195, or 200 bp homology 5' of the nick or replacement sequence. In certain embodiments, the template nucleic acid comprises less than about 100, 90, 80, 70, 60, 50, 40, 30, 20, 15, or 10 bp homology 3' of the nick or replacement sequence.

In certain embodiments, the template nucleic acid comprises a nucleotide sequence, *e.g.,* of one or more nucleotides, that will be added to or will template a change in the target nucleic acid. In other embodiments, the template nucleic acid comprises a nucleotide sequence that may be used to modify the target position. In other embodiments, the template nucleic acid comprises a nucleotide sequence, *e.g.,* of one or more nucleotides, that corresponds to wild type sequence of the target nucleic acid, *e.g.,* of the target position.

The template nucleic acid may comprise a replacement sequence. In some embodiments, the template nucleic acid comprises a 5' homology arm. In some embodiments, the template nucleic acid comprises a 3' homology arm.

In certain embodiments, the template nucleic acid is linear double stranded DNA. The length may be, *e.g.,* about 150-200 bp, *e.g.,* about 150, 160, 170, 180, 190, or 200 bp. The length may be, *e.g.,* at least 150, 160, 170, 180, 190, or 200 bp. In some embodiments, the length is no greater than 150, 160, 170, 180, 190, or 200 bp. In some embodiments, a double stranded template nucleic acid has a length of about 160 bp, *e.g.,* about 155-165, 150-170, 140-180, 130-190, 120-200, 110-210, 100-220, 90-230, or 80-240 bp.

The template nucleic acid can be linear single stranded DNA. In certain embodiments, the template nucleic acid is (i) linear single stranded DNA that can anneal to the nicked strand of the target nucleic acid, (ii) linear single stranded DNA that can anneal to the intact strand of the target nucleic acid, (iii) linear single stranded DNA that can anneal to the plus strand of the target nucleic acid, (iv) linear single stranded DNA that can anneal to the minus strand of the target nucleic acid, or more than one of the preceding. The length may be, *e.g.,* about 150-200 nucleotides, *e.g.,* about 150, 160, 170, 180, 190, or 200 nucleotides. The length may be, *e.g.,* at least 150, 160, 170, 180, 190, or 200 nucleotides. In some embodiments, the length is no greater than 150, 160, 170, 180, 190, or 200 nucleotides. In some embodiments, a single stranded template nucleic acid has a length of about 160 nucleotides, *e.g*., about 155-165, 150-170, 140-180, 130-190, 120-200, 110-210, 100-220, 90-230, or 80-240 nucleotides.

In some embodiments, the template nucleic acid is circular double stranded DNA, *e.g.,* a plasmid. In some embodiments, the template nucleic acid comprises about 500 to 1000 bp of homology on either side of the replacement sequence and/or the nick. In some embodiments, the template nucleic acid comprises about 300, 400, 500, 600, 700, 800, 900, 1000, 1500, or 2000 bp of homology 5' of the nick or replacement sequence, 3' of the nick or replacement sequence, or both 5' and 3' of the nick or replacement sequence. In some embodiments, the template nucleic acid comprises at least 300, 400, 500, 600, 700, 800, 900, 1000, 1500, or 2000 bp of homology 5' of the nick or replacement sequence, 3' of the nick or replacement sequence, or both 5' and 3' of the nick or replacement sequence. In some embodiments, the template nucleic acid comprises no more than 300, 400, 500, 600, 700, 800, 900, 1000, 1500, or 2000 bp of homology 5' of the nick or replacement sequence, 3' of the nick or replacement sequence, or both 5' and 3' of the nick or replacement sequence.

In certain embodiments, one or both homology arms may be shortened to avoid including certain sequence repeat elements, *e.g*., Alu repeats, LINE elements. For example, a 5' homology arm may be shortened to avoid a sequence repeat element, while a 3' homology arm may be shortened to avoid a sequence repeat element. In some embodiments, both the 5' and the 3' homology arms may be shortened to avoid including certain sequence repeat elements.

In some embodiments, the gRNA fusion molecule comprising the template nucleic acid is an adenovirus vector, *e.g.,* an AAV vector, *e.g.,* a ssDNA molecule of a length and sequence that allows it to be packaged in an AAV capsid. The vector may be, *e.g.,* less than 5 kb and may contain an ITR sequence that promotes packaging into the capsid. The vector may be integration-deficient. In some embodiments, the template nucleic acid comprises about 150 to 1000 nucleotides of homology on either side of the replacement sequence and/or the nick. In some embodiments, the template nucleic acid comprises about 100, 150, 200, 300, 400, 500, 600, 700, 800, 900, 1000, 1500, or 2000 nucleotides 5' of the nick or replacement sequence, 3' of the nick or replacement sequence, or both 5' and 3' of the nick or replacement sequence. In some embodiments, the template nucleic acid comprises at least 100, 150, 200, 300, 400, 500, 600, 700, 800, 900, 1000, 1500, or 2000 nucleotides 5' of the nick or replacement sequence, 3' of the nick or replacement sequence, or both 5' and 3' of the nick or replacement sequence. In some embodiments, the template nucleic acid comprises at most 100, 150, 200, 300, 400, 500, 600, 700, 800, 900, 1000, 1500, or 2000 nucleotides 5' of the nick or replacement sequence, 3' of the nick or replacement sequence, or both 5' and 3' of the nick or replacement sequence.

In some embodiments, the gRNA fusion molecule comprising the template nucleic acid is a lentiviral vector, *e.g.,* an IDLV (integration deficiency lentivirus). In some embodiments, the template nucleic acid comprises about 500 to 1000 base pairs of homology on either side of the replacement sequence and/or the nick. In some embodiments, the template nucleic acid comprises about 300, 400, 500, 600, 700, 800, 900, 1000, 1500, or 2000 bp of homology 5' of the nick or replacement sequence, 3' of the nick or replacement sequence, or both 5' and 3' of the nick or replacement sequence. In some embodiments, the template nucleic acid comprises at least 300, 400, 500, 600, 700, 800, 900, 1000, 1500, or 2000 bp of homology 5' of the nick or replacement sequence, 3' of the nick or replacement sequence, or both 5' and 3' of the nick or replacement sequence. In some embodiments, the template nucleic acid comprises no more than 300, 400, 500, 600, 700, 800, 900, 1000, 1500, or 2000 bp of homology 5' of the nick or replacement sequence, 3' of the nick or replacement sequence, or both 5' and 3' of the nick or replacement sequence.

In an embodiment, the template nucleic acid comprises one or more mutations, *e.g*., silent mutations, that prevent Cas9 from recognizing and cleaving the template nucleic acid. The template nucleic acid may comprise, *e.g*., at least 1, 2, 3, 4, 5, 10, 20, 30, 40, or 50 silent mutations relative to the corresponding sequence in the genome of the cell to be altered. In certain embodiments, the template nucleic acid comprises at most 2, 3, 4, 5, 10, 20, 30, 40, or 50 silent mutations relative to the corresponding sequence in the genome of the cell to be altered. In an embodiment, the template nucleic acid comprises one or more mutations, *e.g*., silent mutations that prevent Cas9 from recognizing and cleaving the template nucleic acid. The template nucleic acid may comprise, *e.g*., at least 1, 2, 3, 4, 5, 10, 20, 30, 40, or 50 silent mutations relative to the corresponding sequence in the genome of the cell to be altered. In certain embodiments, the template nucleic acid comprises at most 2, 3, 4, 5, 10, 20, 30, 40, or 50 silent mutations relative to the corresponding sequence in the genome of the cell to be altered.

In certain embodiments, the template nucleic acid alters the structure of the target position by participating in an HDR event, *e.g.,* gene correction. In some embodiments, the template nucleic acid alters the sequence of the target position. In some embodiments, the template nucleic acid results in the incorporation of a modified, or non-naturally occurring nucleotide base into the target nucleic acid.

Typically, the template sequence undergoes a breakage mediated or catalyzed recombination with the target sequence. In some embodiments, the template nucleic acid includes sequence that corresponds to a site on the target sequence that is cleaved by an eaCas9 mediated cleavage event. In some embodiments, the template nucleic acid includes sequence that corresponds to both, a first site on the target sequence that is cleaved in a first Cas9 mediated event, and a second site on the target sequence that is cleaved in a second Cas9 mediated event.

In some embodiments, the template nucleic acid can include sequence which results in an alteration in the coding sequence of a translated sequence, *e.g*., one which results in the substitution of one amino acid for another in a protein product, *e.g*., transforming a mutant allele into a wild type allele, transforming a wild type allele into a mutant allele, and/or introduction of a stop codon, insertion of an amino acid residue, deletion of an amino acid residue, or a nonsense mutation.

In some embodiments, the template nucleic acid can include sequence which results in an alteration in a non-coding sequence, *e.g*., an alteration in an exon or in a 5' or 3' non-translated or non-transcribed region. Such alterations include an alteration in a control element, *e.g.,* a promoter or enhancer, or an alteration in a cis-acting or trans-acting control element.

In some embodiments, a template nucleic acid having homology with a target position can be used to alter the structure of a target sequence. The template nucleic acid sequence can be used to alter an unwanted structure, *e.g*., an unwanted or mutant nucleotide.

In some embodiments, shorter homology arms, *e.g.,* 5' and/or 3' homology arms may be used. In certain embodiments, the length of the 5' homology arm is about 5 to about 100 nucleotides. In some embodiments, the length of the 5' homology arm is about 10 to about 150 nucleotides. In some embodiments, the length of the 5' homology arm is about 20 to about 150 nucleotides. In certain embodiments, the length of the 5' homology arm is about 10, 20, 50, 100, 150, 200, 250, 300, 350, 400, 450, 500, 550, 600, 650, 700, 750, 800, 850, 900, 950, 1000, 1100, 1200, or more nucleotides in length.

In certain embodiments, the length of the 3' homology arm is about 5 to about 100 nucleotides. In some embodiments, the length of the 3' homology arm is about 10 to about 150 nucleotides. In some embodiments, the length of the 3' homology arm is about 20 to about 150 nucleotides. In certain embodiments, the length of the 3' homology arm is about 10, 20, 50, 100, 150, 200, 250, 300, 350, 400, 450, 500, 550, 600, 650, 700, 750, 800, 850, 900, 950, 1000, 1100, 1200, or more nucleotides in length.

It is contemplated herein that one or both homology arms may be shortened to avoid including certain sequence repeat elements, *e.g*., Alu repeats, LINE elements. For example, a 5' homology arm may be shortened to avoid a sequence repeat element. In one embodiment, a 3' homology arm may be shortened to avoid a sequence repeat element. In one embodiment, both the 5' and the 3' homology arms may be shortened to avoid including certain sequence repeat elements. In some embodiments, the length of the 5' homology arm is at least 50 nucleotides in length, but not long enough to include a repeated element. In some embodiments, the length of the 5' homology arm is at least 100 nucleotides in length, but not long enough to include a repeated element. In some embodiments, the length of the 5' homology arm is at least 150 nucleotides in length, but not long enough to include a repeated element. In some embodiments, the length of the 3' homology arm is at least 50 nucleotides in length, but not long enough to include a repeated element. In some embodiments, the length of the 3' homology arm is at least 100 nucleotides in length, but not long enough to include a repeated element. In some embodiments, the length of the 3' homology arm is at least 150 nucleotides in length, but not long enough to include a repeated element.

It is contemplated herein that template nucleic acids for correcting a mutation may be designed for use as a single-stranded oligonucleotide (ssODN), *e.g.,* a single-stranded oligodeoxynucleotide. When using a ssODN, 5' and 3' homology arms may range up to about 200 bp in length, *e.g.,* at least 25, 50, 75, 100, 125, 150, 175, or 200 bp in length. Longer homology arms are also contemplated for ssODNs as improvements in oligonucleotide synthesis continue to be made.

In one embodiment, an ssODN may be used to correct a mutation.

### Silent mutations in the template nucleic acid

It is contemplated herein that Cas9 could potentially cleave donor constructs either prior to or following homology directed repair (*e.g*., homologous recombination), resulting in a possible non-homologous-end-joining event and further DNA sequence mutation at the chromosomal locus of interest. Therefore, to avoid cleavage of the donor sequence before and/or after Cas9-mediated homology directed repair, in some embodiments, alternate versions of the donor sequence may be used where silent mutations are introduced. These silent mutations may disrupt Cas9 binding and cleavage, but not disrupt the amino acid sequence of the repaired gene.

### Increasing Gene Correction

In certain embodiments of the methods provided herein, the frequency of preferred repair outcomes generated using a Cas9 fusion molecule described herein may be increased as compared to the frequency of preferred repair outcomes with a Cas9 fusion molecule and a template nucleic acid which are not fused. In some embodiments, the frequency of gene correction resulting from a Cas9 fusion molecule induced-lesion in a target position of a target cell overexpressing a gene correction pathway component is increased at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, or more, as compared to the frequency of gene correction resulting from a Cas9 molecule and a target nucleic acid which are not fused in a target position.

In some embodiments, the frequency of gene correction resulting from a Cas9 fusion molecule induced-lesion in a target position of a target cell overexpressing a gene correction pathway component is increased at least 5% (*e.g.,* at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 100%, at least about 150%, at least about 200%, at least about 300%, at least about 400%, at least about 500%, at least about 600%, at least about 700%, at least about 800%, at least about 900%, or more.

### VIII.2 NHEJ Approaches for Gene Targeting

In certain embodiments of the methods provided herein, NHEJ-mediated deletion is used to delete all or part of a target gene. As described herein, nuclease-induced NHEJ can also be used to remove (*e.g*., delete) sequences in a gene of interest.

While not wishing to be bound by theory, it is believed that, in certain embodiments, the genomic alterations associated with the methods described herein rely on nuclease-induced NHEJ and the error-prone nature of the NHEJ repair pathway. NHEJ repairs a double-strand break in the DNA by joining together the two ends; however, generally, the original sequence is restored only if two compatible ends, exactly as they were formed by the double-strand break, are perfectly ligated. The DNA ends of the double-strand break are frequently the subject of enzymatic processing, resulting in the addition or removal of nucleotides, *e.g*., resection, at one or both strands, prior to rejoining of the ends. This results in the presence of insertion and/or deletion (indel) mutations in the DNA sequence at the site of the NHEJ repair. Two-thirds of these mutations typically alter the reading frame and, therefore, produce a non-functional protein. Additionally, mutations that maintain the reading frame, but which insert or delete a significant amount of sequence, can destroy functionality of the protein. This is locus dependent as mutations in critical functional domains are likely less tolerable than mutations in non-critical regions of the protein.

The indel mutations generated by NHEJ are unpredictable in nature; however, at a given break site certain indel sequences are favored and are over represented in the population, likely due to small regions of microhomology. The lengths of deletions can vary widely; most commonly in the 1-50 bp range, but they can easily reach greater than 100-200 bp. In some embodiments, the deletion is at least about 1, 2, 3, 4, 5, 6, 7, 8, 9, 10, 11, 12, 13, 14, 15, 16, 17, 18, 19, 20, 30, 40, 47, 50, 75, 100, 200, 300, 400, 500, 750, 1000, 2000, 3000, 4000, 5000, 6000, 7000, 8000, 9000, 10000, 15000, 20000, 25000, 30000, 40000, 50000, 60000, 70000, 80000, 90000, 100000, 200000, 300000, 400000, 500000, 600000, 700000, 800000, 900000, 1000000 or more nucleotides in length. Insertions tend to be shorter and often include short duplications of the sequence immediately surrounding the break site. However, it is possible to obtain large insertions, and in these cases, the inserted sequence has often been traced to other regions of the genome or to plasmid DNA present in the cells.

Because NHEJ is a mutagenic process, it can also be used to delete small sequence motifs as long as the generation of a specific final sequence is not required. If a double-strand break is targeted near to a short target sequence, the deletion mutations caused by the NHEJ repair often span, and therefore remove, the unwanted nucleotides. For the deletion of larger DNA segments, introducing two double-strand breaks, one on each side of the sequence, can result in NHEJ between the ends with removal of the entire intervening sequence. Both of these approaches can be used to delete specific DNA sequences; however, the error-prone nature of NHEJ may still produce indel mutations at the site of repair.

Both double-strand cleaving eaCas9 molecules and single strand, or nickase, eaCas9 molecules can be used in the methods and compositions described herein to generate NHEJ-mediated indels. NHEJ-mediated indels targeted to the gene, *e.g.,* a coding region, *e.g.,* an early coding region of a gene of interest can be used to knockout (*i.e*., eliminate expression of) a gene of interest. For example, early coding region of a gene of interest includes sequence immediately following a transcription start site, within a first exon of the coding sequence, or within 500 bp of the transcription start site (*e.g*., less than 500, 450, 400, 350, 300, 250, 200, 150, 100 or 50 bp).

### Placement of double-strand or single-strand breaks relative to the target position

In certain embodiments, in which a gRNA and Cas9 nuclease generate a double-strand break for the purpose of inducing NHEJ-mediated indels, a gRNA, *e.g.,* a unimolecular (or chimeric) or modular gRNA molecule, is configured to position one double-strand break in close proximity to a nucleotide of the target position. In one embodiment, the cleavage site is between 0-30 bp away from the target position (*e.g*., less than 30, 25, 20, 15, 10, 9, 8, 7, 6, 5, 4, 3, 2 or 1 bp from the target position).

In certain embodiments, in which two gRNAs complexing with Cas9 nickases induce two single-strand breaks for the purpose of inducing NHEJ-mediated indels, two gRNAs, *e.g*., independently, unimolecular (or chimeric) or modular gRNA, are configured to position two single-strand breaks to provide for NHEJ repair a nucleotide of the target position. In certain embodiments, the gRNAs are configured to position cuts at the same position, or within a few nucleotides of one another, on different strands, essentially mimicking a double-strand break. In certain embodiments, the closer nick is between 0-30 bp away from the target position (*e.g*., less than 30, 25, 20, 15, 10, 9, 8, 7, 6, 5, 4, 3, 2, or 1 bp from the target position), and the two nicks are within 25-55 bp of each other (*e.g*., between 25 to 50, 25 to 45, 25 to 40, 25 to 35, 25 to 30, 50 to 55, 45 to 55, 40 to 55, 35 to 55, 30 to 55, 30 to 50, 35 to 50, 40 to 50, 45 to 50, 35 to 45, or 40 to 45 bp) and no more than 100 bp away from each other (*e.g*., no more than 90, 80, 70, 60, 50, 40, 30, 20, or 10 bp). In certain embodiments, the gRNAs are configured to place a single-strand break on either side of a nucleotide of the target position.

Both double-strand cleaving eaCas9 molecules and single strand, or nickase, eaCas9 molecules can be used in the methods and compositions described herein to generate breaks both sides of a target position. Double-strand or paired single-strand breaks may be generated on both sides of a target position to remove the nucleic acid sequence between the two cuts (*e.g*., the region between the two breaks in deleted). In certain embodiments, two gRNAs, *e.g*., independently, unimolecular (or chimeric) or modular gRNA, are configured to position a double-strand break on both sides of a target position. In other embodiments, three gRNAs, *e.g*., independently, unimolecular (or chimeric) or modular gRNA, are configured to position a double-strand break (*i.e*., one gRNA complexes with a Cas9 nuclease) and two single-strand breaks or paired single-strand breaks (*i.e*., two gRNAs complex with Cas9 nickases) on either side of the target position. In certain embodiments, four gRNAs, *e.g*., independently, unimolecular (or chimeric) or modular gRNA, are configured to generate two pairs of single-strand breaks (*i.e*., two pairs of two gRNAs complex with Cas9 nickases) on either side of the target position. The double-strand break(s) or the closer of the two single-strand nicks in a pair will ideally be within 0-500 bp of the target position (*e.g*., no more than 450, 400, 350, 300, 250, 200, 150, 100, 50, or 25 bp from the target position). When nickases are used, the two nicks in a pair are within 25-55 bp of each other (*e.g*., between 25 to 50, 25 to 45, 25 to 40, 25 to 35, 25 to 30, 50 to 55, 45 to 55, 40 to 55, 35 to 55, 30 to 55, 30 to 50, 35 to 50, 40 to 50, 45 to 50, 35 to 45, or 40 to 45 bp) and no more than 100 bp away from each other (*e.g*., no more than 90, 80, 70, 60, 50, 40, 30, 20, or 10 bp).

### VIII.3 Targeted Knockdown

Unlike CRISPR/Cas-mediated gene knockout, which permanently eliminates expression by mutating the gene at the DNA level, CRISPR/Cas knockdown allows for temporary reduction of gene expression through the use of artificial transcription factors. Mutating key residues in both DNA cleavage domains of the Cas9 molecule *(e.g.,* the D10A and H840A mutations) results in the generation of a catalytically inactive Cas9 (referred to herein as "eiCas9", which is also known as dead Cas9 or dCas9) molecule. An eiCas9 complexes with a gRNA and localizes to the DNA sequence specified by that gRNA's targeting domain, however, it does not cleave the target DNA. Fusion of the eiCas9 to an effector domain, *e.g.,* a transcription repression domain, enables recruitment of the effector to any DNA site specified by the gRNA. Although an eiCas9 itself can block transcription when recruited to early regions in the coding sequence, more robust repression can be achieved by fusing a transcriptional repression domain (for example KRAB, SID or ERD) to the eiCas9, referred to herein as a "Cas9-repressor", and recruiting the transcriptional repression domain to the target knockdown position, *e.g*., within 1000 bp of sequence 3' of the start codon or within 500 bp of a promoter region 5' of the start codon of a gene. It is likely that targeting DNAse I hypersensitive sites (DHSs) of the promoter may yield more efficient gene repression or activation because these regions are more likely to be accessible to the eiCas9 and are also more likely to harbor sites for endogenous transcription factors. Especially for gene repression, it is contemplated herein that blocking the binding site of an endogenous transcription factor would aid in downregulating gene expression. In certain embodiments, one or more eiCas9 molecules may be used to block binding of one or more endogenous transcription factors. In some embodiments, an eiCas9 molecule can be fused to a chromatin modifying protein. Altering chromatin status can result in decreased expression of the target gene. One or more eiCas9 molecules fused to one or more chromatin modifying proteins may be used to alter chromatin status.

In an embodiment, a gRNA molecule can be targeted to a known transcription response elements (*e.g*., promoters, enhancers, etc.), a known upstream activating sequences (UAS), and/or sequences of unknown or known function that are suspected of being able to control expression of the target DNA.

CRISPR/Cas-mediated gene knockdown can be used to reduce expression of an unwanted allele or transcript. Contemplated herein are scenarios wherein permanent destruction of the gene is not ideal. In these scenarios, site-specific repression may be used to temporarily reduce or eliminate expression. It is also contemplated herein that the off-target effects of a Cas9-repressor may be less severe than those of a Cas9-nuclease as a nuclease can cleave any DNA sequence and cause mutations whereas a Cas9-repressor may only have an effect if it targets the promoter region of an actively transcribed gene. However, while nuclease-mediated knockout is permanent, repression may only persist as long as the Cas9-repressor is present in the cells. Once the repressor is no longer present, it is likely that endogenous transcription factors and gene regulatory elements would restore expression to its natural state.

### VIII.4 Single-Strand Annealing

Single-strand annealing (SSA) is another DNA repair process that repairs a double-strand break between two repeat sequences present in a target nucleic acid. Repeat sequences utilized by the SSA pathway are generally greater than 30 nucleotides in length. Resection at the break ends occurs to reveal repeat sequences on both strands of the target nucleic acid. After resection, single-strand overhangs containing the repeat sequences are coated with RPA protein to prevent the repeats sequences from inappropriate annealing, *e.g*., to themselves. RAD52 binds to and each of the repeat sequences on the overhangs and aligns the sequences to enable the annealing of the complementary repeat sequences. After annealing, the single-strand flaps of the overhangs are cleaved. New DNA synthesis fills in any gaps, and ligation restores the DNA duplex. As a result of the processing, the DNA sequence between the two repeats is deleted. The length of the deletion can depend on many factors including the location of the two repeats utilized, and the pathway or processivity of the resection.

In contrast to HDR pathways, SSA does not require a template nucleic acid to alter or correct a target nucleic acid sequence. Instead, the complementary repeat sequence is utilized.

### VIII. 5 Other DNA Repair Pathways

### SSBR (single-strand break repair)

Single-stranded breaks (SSB) in the genome are repaired by the SSBR pathway, which is a distinct mechanism from the DSB repair mechanisms discussed above. The SSBR pathway has four major stages: SSB detection, DNA end processing, DNA gap filling, and DNA ligation. A more detailed explanation is given in Caldecott 2008, and a summary is given here.

In the first stage, when a SSB forms, PARP1 and/or PARP2 recognize the break and recruit repair machinery. The binding and activity of PARP1 at DNA breaks is transient and it seems to accelerate SSBr by promoting the focal accumulation or stability of SSBr protein complexes at the lesion. Arguably the most important of these SSBr proteins is XRCC1, which functions as a molecular scaffold that interacts with, stabilizes, and stimulates multiple enzymatic components of the SSBr process including the protein responsible for cleaning the DNA 3' and 5' ends. For instance, XRCC1 interacts with several proteins (DNA polymerase beta, PNK, and three nucleases, APE1, APTX, and APLF) that promote end processing. APE1 has endonuclease activity. APLF exhibits endonuclease and 3' to 5' exonuclease activities. APTX has endonuclease and 3' to 5' exonuclease activity.

This end processing is an important stage of SSBR since the 3'- and/or 5'-termini of most, if not all, SSBs are damaged. End processing generally involves restoring a damaged 3'-end to a hydroxylated state and and/or a damaged 5' end to a phosphate moiety, so that the ends become ligation-competent. Enzymes that can process damaged 3' termini include PNKP, APE1, and TDP1. Enzymes that can process damaged 5' termini include PNKP, DNA polymerase beta, and APTX. LIG3 (DNA ligase III) can also participate in end processing. Once the ends are cleaned, gap filling can occur.

At the DNA gap filling stage, the proteins typically present are PARP1, DNA polymerase beta, XRCC1, FEN1 (flap endonuclease 1), DNA polymerase delta/epsilon, PCNA, and LIG1. There are two ways of gap filling, the short patch repair and the long patch repair. Short patch repair involves the insertion of a single nucleotide that is missing. At some SSBs, "gap filling" might continue displacing two or more nucleotides (displacement of up to 12 bases have been reported). FEN1 is an endonuclease that removes the displaced 5'-residues. Multiple DNA polymerases, including Pοlβ, are involved in the repair of SSBs, with the choice of DNA polymerase influenced by the source and type of SSB.

In the fourth stage, a DNA ligase such as LIG1 (Ligase I) or LIG3 (Ligase III) catalyzes joining of the ends. Short patch repair uses Ligase III and long patch repair uses Ligase I.

Sometimes, SSBR is replication-coupled. This pathway can involve one or more of CtIP, MRN, ERCC1, and FEN1. Additional factors that may promote SSBR include: aPARP, PARP1, PARP2, PARG, XRCC1, DNA polymerase β, DNA polymerase delta, DNA polymerase epsilon, PCNA, LIG1, PNK, PNKP, APE1, APTX, APLF, TDP1, LIG3, FEN1, CtIP, MRN, and ERCC1.

### MMR (mismatch repair)

Cells contain three excision repair pathways: MMR, BER, and NER. The excision repair pathways have a common feature in that they typically recognize a lesion on one strand of the DNA, then exo/endonucleases remove the lesion and leave a 1-30 nucleotide gap that is sub-sequentially filled in by DNA polymerase and finally sealed with ligase. A more complete picture is given in Li 2008, and a summary is provided here.

Mismatch repair (MMR) operates on mispaired DNA bases.

The MSH2/6 or MSH2/3 complexes both have ATPase activity that plays an important role in mismatch recognition and the initiation of repair. MSH2/6 preferentially recognizes base-base mismatches and identifies mispairs of 1 or 2 nucleotides, while MSH2/3 preferentially recognizes larger ID mispairs.

hMLH1 heterodimerizes with hPMS2 to form hMutLα which possesses an ATPase activity and is important for multiple steps of MMR. It possesses a PCNA/replication factor C (RFC)-dependent endonuclease activity which plays an important role in 3' nick-directed MMR involving EXO1 (EXO1 is a participant in both HR and MMR). It regulates termination of mismatch-provoked excision. Ligase I is the relevant ligase for this pathway. Additional factors that may promote MMR include: EXO1, MSH2, MSH3, MSH6, MLH1, PMS2, MLH3, DNA Pol delta, RPA, HMGB 1, RFC, and DNA ligase I.

### Base excision repair (BER)

The base excision repair (BER) pathway is active throughout the cell cycle; it is responsible primarily for removing small, non-helix-distorting base lesions from the genome. In contrast, the related Nucleotide Excision Repair pathway (discussed in the next section) repairs bulky helix-distorting lesions. A more detailed explanation is given in Caldecott 2008, and a summary is given here.

Upon DNA base damage, base excision repair (BER) is initiated and the process can be simplified into five major steps: (a) removal of the damaged DNA base; (b) incision of the subsequent a basic site; (c) clean-up of the DNA ends; (d) insertion of the desired nucleotide into the repair gap; and (e) ligation of the remaining nick in the DNA backbone. These last steps are similar to the SSBR.

In the first step, a damage-specific DNA glycosylase excises the damaged base through cleavage of the N-glycosidic bond linking the base to the sugar phosphate backbone. Then AP endonuclease-1 (APE1) or bifunctional DNA glycosylases with an associated lyase activity incises the phosphodiester backbone to create a DNA single-strand break (SSB). The third step of BER involves cleaning-up of the DNA ends. The fourth step in BER is conducted by Pol β that adds a new complementary nucleotide into the repair gap and in the final step XRCC1/Ligase III seals the remaining nick in the DNA backbone. This completes the short-patch BER pathway in which the majority (-80%) of damaged DNA bases are repaired. However, if the 5'-ends in step 3 are resistant to end processing activity, following one nucleotide insertion by Pol β there is then a polymerase switch to the replicative DNA polymerases, Pol δ/ε, which then add ~2-8 more nucleotides into the DNA repair gap. This creates a 5'-flap structure, which is recognized and excised by flap endonuclease-1 (FEN-1) in association with the processivity factor proliferating cell nuclear antigen (PCNA). DNA ligase I then seals the remaining nick in the DNA backbone and completes long-patch BER. Additional factors that may promote the BER pathway include: DNA glycosylase, APE1, Pοlβ, Pol delta, Pol epsilon, XRCC1, Ligase III, FEN-1, PCNA, RECQL4, WRN, MYH, PNKP, and APTX.

### Nucleotide excision repair (NER)

Nucleotide excision repair (NER) is an important excision mechanism that removes bulky helix-distorting lesions from DNA. Additional details about NER are given in Marteijn *et al.* 2014, and a summary is given here. NER a broad pathway encompassing two smaller pathways: global genomic NER (GG-NER) and transcription coupled repair NER (TC-NER). GG-NER and TC-NER use different factors for recognizing DNA damage. However, they utilize the same machinery for lesion incision, repair, and ligation.

Once damage is recognized, the cell removes a short single-stranded DNA segment that contains the lesion. Endonucleases XPF/ERCC1 and XPG (encoded by ERCC5) remove the lesion by cutting the damaged strand on either side of the lesion, resulting in a single-strand gap of 22-30 nucleotides. Next, the cell performs DNA gap filling synthesis and ligation. Involved in this process are: PCNA, RFC, DNA Pol δ, DNA Pol ε or DNA Pol κ, and DNA ligase I or XRCC1/Ligase III. Replicating cells tend to use DNA pol ε and DNA ligase I, while non-replicating cells tend to use DNA Pol δ, DNA Pol κ, and the XRCC1/ Ligase III complex to perform the ligation step.

NER can involve the following factors: XPA-G, POLH, XPF, ERCC1, XPA-G, and LIG1. Transcription-coupled NER (TC-NER) can involve the following factors: CSA, CSB, XPB, XPD, XPG, ERCC1, and TTDA. Additional factors that may promote the NER repair pathway include XPA-G, POLH, XPF, ERCC1, XPA-G, LIG1, CSA, CSB, XPA, XPB, XPC, XPD, XPF, XPG, TTDA, UVSSA, USP7, CETN2, RAD23B, UV-DDB, CAK subcomplex, RPA, and PCNA.

### Interstrand Crosslink (ICL)

A dedicated pathway called the ICL repair pathway repairs interstrand crosslinks. Interstrand crosslinks, or covalent crosslinks between bases in different DNA strand, can occur during replication or transcription. ICL repair involves the coordination of multiple repair processes, in particular, nucleolytic activity, translesion synthesis (TLS), and HDR. Nucleases are recruited to excise the ICL on either side of the crosslinked bases, while TLS and HDR are coordinated to repair the cut strands. ICL repair can involve the following factors: endonucleases, *e.g*., XPF and RAD51C, endonucleases such as RAD51, translesion polymerases, *e.g*., DNA polymerase zeta and Rev1, and the Fanconi anemia (FA) proteins, *e.g.,* FancJ.

### Other pathways

Several other DNA repair pathways exist in mammals.

Translesion synthesis (TLS) is a pathway for repairing a single stranded break left after a defective replication event and involves translesion polymerases, *e.g*., DNA pol ζ and Rev 1.

Error-free postreplication repair (PRR) is another pathway for repairing a single stranded break left after a defective replication event.

### V.6 Examples of gRNA Fusion Molecules in Genome Editing Methods

gRNA fusion molecules as described herein can be used with Cas9 molecules that generate a double-strand break or a single-strand break to alter the sequence of a target nucleic acid, *e.g.,* a target position or target genetic signature. gRNA molecules useful in these methods are described below.

In certain embodiments, the gRNA portion of the gRNA fusion molecule can be, *e.g.,* a chimeric gRNA, and can be configured such that it comprises one or more of the following properties;
a) it can position, *e.g*., when targeting a Cas9 molecule that makes double-strand breaks, a double-strand break (i) within 50, 100, 150, 200, 250, 300, 350, 400, 450, or 500 nucleotides of a target position, or (ii) sufficiently close that the target position is within the region of end resection;
b) it has a targeting domain of at least 16 nucleotides, *e.g*., a targeting domain of (i) 16, (ii), 17, (iii) 18, (iv) 19, (v) 20, (vi) 21, (vii) 22, (viii) 23, (ix) 24, (x) 25, or (xi) 26 nucleotides; and
(c)(i) the proximal and tail domain, when taken together, comprise at least 15, 18, 20, 25, 30, 31, 35, 40, 45, 49, 50, or 53 nucleotides, *e.g*., at least 15, 18, 20, 25, 30, 31, 35, 40, 45, 49, 50, or 53 nucleotides from a naturally occurring *S. pyogenes* or *S*. *aureus,* tail and proximal domain, or a sequence that differs by no more than 1, 2, 3, 4, 5, 6, 7, 8, 9, or 10 nucleotides therefrom;
(c)(ii) there are at least 15, 18, 20, 25, 30, 31, 35, 40, 45, 49, 50, or 53 nucleotides 3' to the last nucleotide of the second complementarity domain, *e.g.,* at least 15, 18, 20, 25, 30, 31, 35, 40, 45, 49, 50, or 53 nucleotides from the corresponding sequence of a naturally occurring *S. pyogenes* or *S*. *aureus* gRNA, or a sequence that differs by no more than 1, 2, 3, 4, 5, 6, 7, 8, 9, or 10 nucleotides therefrom;
(c)(iii) there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain, *e.g.,* at least 16, 19, 21, 26, 31, 32, 36, 41, 46, 50, 51, or 54 nucleotides from the corresponding sequence of a naturally occurring *S. pyogenes* or *S*. *aureus* gRNA, or a sequence that differs by no more than 1, 2, 3, 4, 5, 6, 7, 8, 9, or 10 nucleotides therefrom;
(c)(iv) the tail domain is at least 10, 15, 20, 25, 30, 35 or 40 nucleotides in length, *e.g.,* it comprises at least 10, 15, 20, 25, 30, 35 or 40 nucleotides from a naturally occurring S. *pyogenes* or *S*. *aureus* tail domain, or a sequence that differs by no more than 1, 2, 3, 4, 5, 6, 7, 8, 9, or 10 nucleotides therefrom; or
(c)(v) the tail domain comprises 15, 20, 25, 30, 35, 40 nucleotides or all of the corresponding portions of a naturally occurring tail domain, *e.g.,* a naturally occurring *S*. *pyogenes* or *S*. *aureus* tail domain.

In certain embodiments, the gRNA is configured such that it comprises properties a and b(i); a and b(ii); a and b(iii); a and b(iv); a and b(v); a and b(vi); a and b(vii); a and b(viii); a and b(ix); a and b(x); a and b(xi); a and c; a, b, and c; a(i), b(i), and c(i); a(i), b(i), and c(ii); a(i), b(ii), and c(i); a(i), b(ii), and c(ii); a(i), b(iii), and c(i); a(i), b(iii), and c(ii); a(i), b(iv), and c(i); a(i), b(iv), and c(ii); a(i), b(v), and c(i); a(i), b(v), and c(ii); a(i), b(vi), and c(i); a(i), b(vi), and c(ii); a(i), b(vii), and c(i); a(i), b(vii), and c(ii); a(i), b(viii), and c(i); a(i), b(viii), and c(ii); a(i), b(ix), and c(i); a(i), b(ix), and c(ii); a(i), b(x), and c(i); a(i), b(x), and c(ii); a(i), b(xi), or c(i); a(i), b(xi), and c(ii).

In certain embodiments, the gRNA, *e.g.,* a chimeric gRNA, is configured such that it comprises one or more of the following properties:
(a) one or both of the gRNAs can position, *e.g*., when targeting a Cas9 molecule that makes single-strand breaks, a single-strand break within (i) 50, 100, 150, 200, 250, 300, 350, 400, 450, or 500 nucleotides of a target position, or (ii) sufficiently close that the target position is within the region of end resection;
(b) one or both have a targeting domain of at least 16 nucleotides, *e.g*., a targeting domain of (i) 16, (ii), 17, (iii) 18, (iv) 19, (v) 20, (vi) 21, (vii) 22, (viii) 23, (ix) 24, (x) 25, or (xi) 26 nucleotides; and
(c)(i) the proximal and tail domain, when taken together, comprise at least 15, 18, 20, 25, 30, 31, 35, 40, 45, 49, 50, or 53 nucleotides, *e.g*., at least 15, 18, 20, 25, 30, 31, 35, 40, 45, 49, 50, or 53 nucleotides from a naturally occurring *S. pyogenes* or *S*. *aureus* tail and proximal domain, or a sequence that differs by no more than 1, 2, 3, 4, 5, 6, 7, 8, 9, or 10 nucleotides therefrom;
(c)(ii) there are at least 15, 18, 20, 25, 30, 31, 35, 40, 45, 49, 50, or 53 nucleotides 3' to the last nucleotide of the second complementarity domain, *e.g*., at least 15, 18, 20, 25, 30, 31, 35, 40, 45, 49, 50, or 53 nucleotides from the corresponding sequence of a naturally occurring *S. pyogenes,* or *S*. *aureus* gRNA, or a sequence that differs by no more than 1, 2, 3, 4, 5, 6, 7, 8, 9, or 10 nucleotides therefrom;
(c)(iii) there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain, *e.g.,* at least 16, 19, 21, 26, 31, 32, 36, 41, 46, 50, 51, or 54 nucleotides from the corresponding sequence of a naturally occurring *S. pyogenes* or *S*. *aureus* gRNA, or a sequence that differs by no more than 1, 2, 3, 4, 5, 6, 7, 8, 9, or 10 nucleotides therefrom;
(c)(iv) the tail domain is at least 10, 15, 20, 25, 30, 35 or 40 nucleotides in length, *e.g.,* it comprises at least 10, 15, 20, 25, 30, 35 or 40 nucleotides from a naturally occurring S. *pyogenes,* or S. *aureus* tail domain, or a sequence that differs by no more than 1, 2, 3, 4, 5, 6, 7, 8, 9, or 10 nucleotides therefrom; or
(c)(v) the tail domain comprises 15, 20, 25, 30, 35, 40 nucleotides or all of the corresponding portions of a naturally occurring tail domain, *e.g.,* a naturally occurring *S*. *pyogenes* or *S*. *aureus* tail domain.

In certain embodiments, the gRNA is configured such that it comprises properties: a and b(i); a and b(ii); a and b(iii); a and b(iv); a and b(v); a and b(vi); a and b(vii); a and b(viii); a and b(ix); a and b(x); a and b(xi); a and c; a, b, and c; a(i), b(i), and c(i); a(i), b(i), and c(ii); a(i), b(ii), and c(i); a(i), b(ii), and c(ii); a(i), b(iii), and c(i); a(i), b(iii), and c(ii); a(i), b(iv), and c(i); a(i), b(iv), and c(ii); a(i), b(v), and c(i); a(i), b(v), and c(ii); a(i), b(vi), and c(i); a(i), b(vi), and c(ii); a(i), b(vii), and c(i); a(i), b(vii), and c(ii); a(i), b(viii), and c(i); a(i), b(viii), and c(ii); a(i), b(ix), and c(i); a(i), b(ix), and c(ii); a(i), b(x), and c(i); a(i), b(x), and c(ii); a(i), b(xi), and c(i); or a(i), b(xi), and c(ii).

In certain embodiments, the gRNA is used with a Cas9 nickase molecule having HNH activity, *e.g.,* a Cas9 molecule having the RuvC activity inactivated, *e.g.,* a Cas9 molecule having a mutation at D10, *e.g.,* the D10A mutation.

In an embodiment, the gRNA is used with a Cas9 nickase molecule having RuvC activity, *e.g.,* a Cas9 molecule having the HNH activity inactivated, *e.g.,* a Cas9 molecule having a mutation at 840, *e.g.,* the H840A.

In an embodiment, the gRNAs are used with a Cas9 nickase molecule having RuvC activity, *e.g.,* a Cas9 molecule having the HNH activity inactivated, *e.g.,* a Cas9 molecule having a mutation at N863, *e.g.,* the N863A mutation.

In embodiment, a pair of gRNAs, *e.g.,* a pair of chimeric gRNAs, comprising a first and a second gRNA, is configured such that they comprises one or more of the following properties:
a) one or both of the gRNA molecules can position, *e.g*., when targeting a Cas9 molecule that makes single-strand breaks, a single-strand break within (i) 50, 100, 150, 200, 250, 300, 350, 400, 450, or 500 nucleotides of a target position, or (ii) sufficiently close that the target position is within the region of end resection;
b) one or both have a targeting domain of at least 16 nucleotides, *e.g.,* a targeting domain of (i) 16, (ii), 17, (iii) 18, (iv) 19, (v) 20, (vi) 21, (vii) 22, (viii) 23, (ix) 24, (x) 25, or (xi) 26 nucleotides;
(c)(i) the proximal and tail domain, when taken together, comprise at least 15, 18, 20, 25, 30, 31, 35, 40, 45, 49, 50, or 53 nucleotides, *e.g*., at least 15, 18, 20, 25, 30, 31, 35, 40, 45, 49, 50, or 53 nucleotides from a naturally occurring *S. pyogenes* or *S*. *aureus* tail and proximal domain, or a sequence that differs by no more than 1, 2, 3, 4, 5, 6, 7, 8, 9, or 10 nucleotides therefrom;
(c)(ii) there are at least 15, 18, 20, 25, 30, 31, 35, 40, 45, 49, 50, or 53 nucleotides 3' to the last nucleotide of the second complementarity domain, *e.g.,* at least 15, 18, 20, 25, 30, 31, 35, 40, 45, 49, 50, or 53 nucleotides from the corresponding sequence of a naturally occurring *S. pyogenes* or *S*. *aureus* gRNA, or a sequence that differs by no more than 1, 2, 3, 4, 5, 6, 7, 8, 9, or 10 nucleotides therefrom;
(c)(iii) there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain, *e.g.,* at least 16, 19, 21, 26, 31, 32, 36, 41, 46, 50, 51, or 54 nucleotides from the corresponding sequence of a naturally occurring *S. pyogenes* or *S*. *aureus* gRNA, or a sequence that differs by no more than 1, 2, 3, 4, 5, 6, 7, 8, 9, or 10 nucleotides therefrom;
(c)(iv) the tail domain is at least 10, 15, 20, 25, 30, 35 or 40 nucleotides in length, *e.g.,* it comprises at least 10, 15, 20, 25, 30, 35 or 40 nucleotides from a naturally occurring S. *pyogenes* or *S*. *aureus* tail domain; or, or a sequence that differs by no more than 1, 2, 3, 4, 5, 6, 7, 8, 9, or 10 nucleotides therefrom; or
(c)(v) the tail domain comprises 15, 20, 25, 30, 35, or 40 nucleotides or all of the corresponding portions of a naturally occurring tail domain, *e.g.,* a naturally occurring *S*. *pyogenes* or *S*. *aureus* tail domain;
(d) the gRNAs are configured such that, when hybridized to target nucleic acid, they are separated by 0-50, 0-100, 0-200, at least 10, at least 20, at least 30 or at least 50 nucleotides;
(e) the breaks made by the first gRNA and second gRNA are on different strands; and
(f) the PAMs are facing outwards.

In certain embodiments, one or both of the gRNAs is configured such that it comprises properties a and b(i); a and b(ii); a and b(iii); a and b(iv); a and b(v); a and b(vi); a and b(vii); a and b(viii); a and b(ix); a and b(x); a and b(xi); a and c; a, b, and c; a(i), b(i), and c(i); a(i), b(i), and c(ii); a(i), b(i), c, and d; a(i), b(i), c, and e; a(i), b(i), c, d, and e; a(i), b(ii), and c(i); a(i), b(ii), and c(ii); a(i), b(ii), c, and d; a(i), b(ii), c, and e; a(i), b(ii), c, d, and e; a(i), b(iii), and c(i); a(i), b(iii), and c(ii); a(i), b(iii), c, and d; a(i), b(iii), c, and e; a(i), b(iii), c, d, and e; a(i), b(iv), and c(i); a(i), b(iv), and c(ii); a(i), b(iv), c, and d; a(i), b(iv), c, and e; a(i), b(iv), c, d, and e; a(i), b(v), and c(i); a(i), b(v), and c(ii); a(i), b(v), c, and d; a(i), b(v), c, and e; a(i), b(v), c, d, and e; a(i), b(vi), and c(i); a(i), b(vi), and c(ii); a(i), b(vi), c, and d; a(i), b(vi), c, and e; a(i), b(vi), c, d, and e; a(i), b(vii), and c(i); a(i), b(vii), and c(ii); a(i), b(vii), c, and d; a(i), b(vii), c, and e; a(i), b(vii), c, d, and e; a(i), b(viii), and c(i); a(i), b(viii), and c(ii); a(i), b(viii), c, and d; a(i), b(viii), c, and e; a(i), b(viii), c, d, and e; a(i), b(ix), and c(i); a(i), b(ix), and c(ii); a(i), b(ix), c, and d; a(i), b(ix), c, and e; a(i), b(ix), c, d, and e; a(i), b(x), and c(i); a(i), b(x), and c(ii); a(i), b(x), c, and d; a(i), b(x), c, and e; a(i), b(x), c, d, and e; a(i), b(xi), and c(i); a(i), b(xi), and c(ii); a(i), b(xi), c, and d; a(i), b(xi), c, and e; or a(i), b(xi), c, d, and e.

In certain embodiments, the gRNAs are used with a Cas9 nickase molecule having HNH activity, *e.g.,* a Cas9 molecule having the RuvC activity inactivated, *e.g.,* a Cas9 molecule having a mutation at D10, *e.g.,* the D10A mutation.

In certain embodiments, the gRNAs are used with a Cas9 nickase molecule having RuvC activity, *e.g.,* a Cas9 molecule having the HNH activity inactivated, *e.g.,* a Cas9 molecule having a mutation at H840, *e.g.,* the H840A mutation.

In certain embodiments, the gRNAs are used with a Cas9 nickase molecule having RuvC activity, *e.g.,* a Cas9 molecule having the HNH activity inactivated, *e.g.,* a Cas9 molecule having a mutation at N863, *e.g.,* the N863A mutation.

### IX. Target Cells

Cas9 molecules and gRNA fusion molecules, *e.g.,* a Cas9 molecule/gRNA fusion molecule complex, can be used to manipulate a cell, *e.g.,* to edit a target nucleic acid, in a wide variety of cells. Additional details on types of cells that can be manipulated may be found in the section entitled "VIIA. TARGETS: CELLS" of PCT Application WO 2015/048577.

In certain embodiments, a cell is manipulated by editing (*e.g*., introducing a mutation in) a target gene as described herein. In one embodiment, a cell, or a population of cells, is manipulated by editing one or more non-coding sequences, *e.g*., an alteration in an intron or in a 5' or 3' non-translated or non-transcribed region. In one embodiment, a cell, or a population of cells, is manipulated by editing the sequence of a control element, *e.g.,* a promoter, enhancer, or a cis-acting or trans-acting control element. In one embodiment, a cell, or a population of cells, is manipulated by editing one or more coding sequences, *e.g*., an alteration in an exon. In some embodiments, a cell, or a population of cells, is manipulated *in vitro.* In other embodiments, a cell, or a population of cells, is manipulated *ex vivo.* In some embodiments, a cell, or a population of cells, is manipulated *in vivo.* In some embodiments, the expression of one or more target genes *(e.g.,* one or more target genes described herein) is modulated, *e.g., in vivo.* In other embodiments, the expression of one or more target genes *(e.g.,* one or more target genes described herein) is modulated, *e.g., ex vivo.* In other embodiments, the expression of one or more target genes *(e.g.,* one or more target genes described herein) is modulated, *e.g., in vitro.*

In one embodiment, a cell, or a population of cells, is manipulated by editing (*e.g.,* inducing a mutation in) the target gene, *e.g.,* as described herein. In one embodiment, the expression of the target gene is modulated, *e.g., in vivo.* In another embodiment, the expression of the target gene is modulated, *e.g., ex vivo.*
The Cas9 and gRNA molecules described herein can be delivered to a target cell. In certain embodiments, the target cell is an erythroid cell, *e.g.,* an erythroblast. In certain embodiments, erythroid cells are preferentially targeted, *e.g.,* at least about 90%, 95%, 96%, 97%, 98%, 99%, or 100% of the targeted cells are erythroid cells. For example, in the case of *in vivo* delivery, erythroid cells are preferentially targeted, and if cells are treated *ex vivo* and returned to the subject, erythroid cells are preferentially modified. In certain embodiments, the target cell is a circulating blood cell, *e.g.,* a reticulocyte, megakaryocyte erythroid progenitor (MEP) cell, myeloid progenitor cell (CMP/GMP), lymphoid progenitor (LP) cell, hematopoietic stem/progenitor cell (HSC), or endothelial cell (EC). In certain embodiments, the target cell is a bone marrow cell *(e.g.,* a reticulocyte, an erythroid cell *(e.g.,* erythroblast), an MEP cell, myeloid progenitor cell (CMP/GMP), LP cell, erythroid progenitor (EP) cell, HSC, multipotent progenitor (MPP) cell, endothelial cell (EC), hemogenic endothelial (HE) cell, or mesenchymal stem cell). In certain embodiments, the target cell is a myeloid progenitor cell *(e.g.,* a common myeloid progenitor (CMP) cell or granulocyte macrophage progenitor (GMP) cell). In certain embodiments, the target cell is a lymphoid progenitor cell, *e.g.,* a common lymphoid progenitor (CLP) cell. In certain embodiments, the target cell is an erythroid progenitor cell *(e.g.,* an MEP cell). In certain embodiments, the target cell is a hematopoietic stem/progenitor cell *(e.g.,* a long term HSC (LT-HSC), short term HSC (ST-HSC), MPP cell, or lineage restricted progenitor (LRP) cell). In certain embodiments, the target cell is a CD34⁺ cell, CD34⁺CD90⁺ cell, CD34⁺CD38⁻ cell, CD34⁺CD90⁺CD49f⁺CD38⁻ CD45RA⁻ cell, CD105⁺ cell, CD31⁺, or CD133⁺ cell, or a CD34⁺CD90⁺ CD133⁺ cell. In certain embodiments, the target cell is an umbilical cord blood CD34⁺ HSPC, umbilical cord venous endothelial cell, umbilical cord arterial endothelial cell, amniotic fluid CD34⁺ cell, amniotic fluid endothelial cell, placental endothelial cell, or placental hematopoietic CD34⁺ cell. In certain embodiments, the target cell is a mobilized peripheral blood hematopoietic CD34⁺cell (after the patient is treated with a mobilization agent, *e.g.,* G-CSF or Plerixafor). In certain embodiments, the target cell is a peripheral blood endothelial cell.

In certain embodiments, a target cell is manipulated *ex vivo* by editing *(e.g.,* inducing a mutation in) the gene and/or modulating the expression of the target gene, then the target cell is administered to the subject. Sources of target cells for *ex vivo* manipulation may include, for example, the subject's blood, cord blood, or marrow. Other sources of target cells for *ex vivo* manipulation may include, for example, heterologous donor blood, cord blood, or bone marrow.

In certain embodiments, an erythrocyte is removed from a subject, manipulated *ex vivo* as described above, and the erythrocyte is returned to the subject. In other embodiments, a hematopoietic stem cell is removed from a subject, manipulated *ex vivo* as described above, and the hematopoietic stem cell is returned to the subject. In certain embodiments, an erythroid progenitor cell is removed from a subject, manipulated *ex vivo* as described above, and the erythroid progenitor cell is returned to the subject. In certain embodiments, an myeloid progenitor cell is removed from a subject, manipulated *ex vivo* as described above, and the myeloid progenitor cell is returned to the subject. In certain embodiments, a hematopoietic stem/progenitor cell (HSC) is removed from a subject, manipulated *ex vivo* as described above, and returned to the subject. In certain embodiments, a CD34⁺ HSC is removed from a subject, manipulated *ex vivo* as described above, and returned to the subject.

In certain embodiments wherein modified HSCs generated *ex vivo* are administered to a subject without myeloablative pre-conditioning. In other embodiments, the modified HSCs are administered after mild myeloblative conditioning such that, followed engraftment, some of the hematopoietic cells are derived from the modified HSCs. In still other embodiments, the modified HSCs are administered after full myeloblation such that, following engraftment, 100% of the hematopoietic cells are derived from the modified HSCs.

A suitable cell can also include a stem cell such as, by way of example, an embryonic stem cell, induced pluripotent stem cell, hematopoietic stem cell, or hemogenic endothelial (HE) cell (precursor to both hematopoietic stem cells and endothelial cells). In certain embodiments, the cell is an induced pluripotent stem (iPS) cell or a cell derived from an iPS cell, *e.g.,* an iPS cell generated from the subject, modified using methods disclosed herein and differentiated into a clinically relevant cell such as *e.g.,* an erythrocyte. In an embodiment, AAV is used to transduce the target cells, *e.g.,* the target cells described herein.

Cells produced by the methods described herein may be used immediately. Alternatively, the cells may be frozen *(e.g.,* in liquid nitrogen) and stored for later use. The cells will usually be frozen in 10% dimethylsulfoxide (DMSO), 50% serum, 40% buffered medium, or some other such solution as is commonly used in the art to preserve cells at such freezing temperature and thawed in such a manner as commonly known in the art for thawing frozen cultured cells. Cells may also be thermostabilized for prolonged storage at 4°C.

### X. Delivery, Formulations and Routes of Administration

The components, *e.g.,* a Cas9 molecule and a gRNA fusion molecule *(e.g.,* a Cas9 molecule/gRNA fusion molecule complex), can be delivered, formulated, or administered, in a variety of forms, see, *e.g.*, **Tables 6-7**. In certain embodiments, one Cas9 molecule and two or more (*e.g.,* 2, 3, 4, or more) different gRNA fusion molecules are delivered, *e.g.,* by an AAV vector. In certain embodiments, the sequence encoding the Cas9 molecule and the sequence(s) encoding the two or more (*e.g.,* 2, 3, 4, or more) different gRNA fusion molecules are present on the same nucleic acid molecule, *e.g.,* an AAV vector. When a Cas9 or gRNA component is delivered encoded in DNA, the DNA will typically include a control region, *e.g.*, comprising a promoter, to effect expression. Useful promoters for Cas9 molecule sequences include CMV, SFFV, EFS, EF-1a, PGK, CAG, and CBH promoters, or a blood cell specific promoter. In an embodiment, the promoter is a constitutive promoter. In another embodiment, the promoter is a tissue specific promoter. Useful promoters for gRNA fusion molecules include T7, H1, EF-1a, U6, U1, and tRNA promoters. Promoters with similar or dissimilar strengths can be selected to tune the expression of components. Sequences encoding a Cas9 molecule can comprise a nuclear localization signal (NLS), *e.g.,* an SV40 NLS. In an embodiment, the sequence encoding a Cas9 molecule comprises at least two nuclear localization signals. In an embodiment a promoter for a Cas9 molecule or a gRNA molecule can be, independently, inducible, tissue specific, or cell specific.

**Table 6** provides examples of how the components can be formulated, delivered, or administered.

**Table 6**

| Elements | | | |
|---|---|---|---|
| Cas9 Molecule(s) | gRNA Molecule(s) | Donor Template Nucleic Acid | Comments |
| DNA | DNA | | In this embodiment, a Cas9 molecule, typically an eaCas9 molecule, a gRNA molecule, and the template nucleic acid are transcribed from DNA. In this embodiment, the donor template is provided on the same DNA molecule that encodes the gRNA molecule. In this embodiment, the Cas9 molecule and the gRNA fusion molecule are encoded on separate molecules. |
| DNA | | | In this embodiment, a Cas9 molecule, typically an eaCas9 molecule, a gRNA molecule, and a template nucleic acid are transcribed from DNA. In this embodiment, the Cas9 molecule and the gRNA fusion molecule are encoded on the same DNA molecule. |
| mRNA | DNA | | In this embodiment, a Cas9 molecule, typically an eaCas9 molecule, is translated from *in vitro* transcribed mRNA, and a gRNA fusion molecule is transcribed from DNA. |
| Protein | DNA | In this embodiment, a Cas9 molecule, typically an eaCas9 molecule, is provided as a protein, and a gRNA fusion molecule is transcribed from DNA. | |
| Protein | RNA | In this embodiment, an eaCas9 molecule is provided as a protein, and a gRNA fusion molecule is provided as transcribed or synthesized RNA. | |

**Table 7** summarizes various delivery methods for the components of a Cas system, *e.g.,* the Cas9 molecule component and the gRNA molecule component, as described herein.

**Table 7**

| **Delivery Vector/Mode** | | **Delivery into Non-Dividing Cells** | **Duration of Expression** | **Genome Integration** | **Type of Molecule Delivered** |
|---|---|---|---|---|---|
| **Physical (*e.g.*, electroporation, particle gun, calcium phosphate transfection, cell compression or squeezing)** | | YES | Transient | NO | Nucleic Acids and Proteins |
| ***Viral*** | **Retrovirus** | NO | Stable | YES | RNA |
| | **Lentivirus** | YES | Stable | YES/NO with modifications | RNA |
| | **Adenovirus** | YES | Transient | NO | DNA |
| | **Adeno-Associated Virus (AAV)** | YES | Stable | NO | DNA |
| | **Vaccinia Virus** | YES | Very Transient | NO | DNA |
| | **Herpes Simplex Virus** | YES | Stable | NO | DNA |
| ***Non-Viral*** | **Cationic Liposomes** | YES | Transient | Depends on what is delivered | Nucleic Acids and Proteins |
| | **Polymeric Nanoparticles** | YES | Transient | Depends on what is delivered | Nucleic Acids and Proteins |
| ***Biological Non-Viral Delivery Vehicles*** | **Attenuated Bacteria** | YES | Transient | NO | Nucleic Acids |
| | **Engineered Bacteriophages** | YES | Transient | NO | Nucleic Acids |
| | **Mammalian Virus-like** | YES | Transient | NO | Nucleic Acids |
| | **Particles** | | | | |
| | **Biological liposomes: Erythrocyte Ghosts and Exosomes** | YES | Transient | NO | Nucleic Acids |

### DNA-based Delivery of a Cas9 molecule and/or one or more gRNA fusion molecule and/or a donor template

Nucleic acids encoding Cas9 molecules (*e.g.*, eaCas9 molecules), gRNA fusion molecules, or any combination thereof, can be administered to subjects or delivered into cells by art-known methods or as described herein. For example, Cas9-encoding and/or gRNA-encoding DNA, as well as donor template nucleic acids, can be delivered by, *e.g.*, vectors (*e.g.,* viral or non-viral vectors), non-vector based methods (*e.g.,* using naked DNA or DNA complexes), or a combination thereof.

Nucleic acids encoding Cas9 molecules (*e.g.*, eaCas9 molecules) and/or gRNA fusion molecules can be conjugated to molecules (*e.g.*, N-acetylgalactosamine) promoting uptake by the target cells (*e.g.*, erythrocytes, HSCs).

In some embodiments, the Cas9- and/or gRNA-encoding DNA is delivered by a vector (*e.g.*, viral vector/virus or plasmid).

Vectors can comprise a sequence that encodes a Cas9 molecule and/or a gRNA molecule and/or a donor template with high homology to the region (*e.g.,* target sequence) being targeted. In certain embodiments, the donor template comprises all or part of a target sequence. Exemplary donor templates are a repair template, *e.g.,* a gene correction template, or a gene mutation template, *e.g.,* point mutation (*e.g.,* single nucleotide (nt) substitution) template. A vector can also comprise a sequence encoding a signal peptide (*e.g.,* for nuclear localization, nucleolar localization, or mitochondrial localization), fused, *e.g.,* to a Cas9 molecule sequence. For example, the vectors can comprise a nuclear localization sequence (*e.g.*, from SV40) fused to the sequence encoding the Cas9 molecule.

One or more regulatory/control elements, *e.g.*, promoters, enhancers, introns, polyadenylation signals, Kozak consensus sequences, internal ribosome entry sites (IRES), can be included in the vectors. In some embodiments, the promoter is recognized by RNA polymerase II (*e.g.,* a CMV promoter). In other embodiments, the promoter is recognized by RNA polymerase III (*e.g.,* a U6 promoter). In some embodiments, the promoter is a regulated promoter (*e.g.*, inducible promoter). In other embodiment, the promoter is a constitutive promoter. In some embodiments, the promoter is a tissue specific promoter. In other embodiments, the promoter is a viral promoter. In some embodiments, the promoter is a non-viral promoter.

In some embodiments, the vector is a viral vector (*e.g.,* for generation of recombinant viruses). In some embodiments, the virus is a DNA virus (*e.g.*, dsDNA or ssDNA virus). In other embodiments, the virus is an RNA virus (*e.g.*, an ssRNA virus). In some embodiments, the virus infects dividing cells. In other embodiments, the virus infects non-dividing cells. Exemplary viral vectors/viruses include, *e.g.*, retroviruses, lentiviruses, adenovirus, adeno-associated virus (AAV), vaccinia viruses, poxviruses, and herpes simplex viruses.

In some embodiments, the virus infects both dividing and non-dividing cells. In some embodiments, the virus can integrate into the host genome. In some embodiments, the virus is engineered to have reduced immunity, *e.g.*, in human. In some embodiments, the virus is replication-competent. In other embodiments, the virus is replication-defective, *e.g.*, having one or more coding regions for the genes necessary for additional rounds of virion replication and/or packaging replaced with other genes or deleted. In some embodiments, the virus causes transient expression of the Cas9 molecule and/or the gRNA molecule. In other embodiments, the virus causes long-lasting, *e.g.*, at least 1 week, 2 weeks, 1 month, 2 months, 3 months, 6 months, 9 months, 1 year, 2 years, or permanent expression, of the Cas9 molecule and/or the gRNA molecule. The packaging capacity of the viruses may vary, *e.g.,* from at least about 4 kb to at least about 30 kb, *e.g.,* at least about 5 kb, 10 kb, 15 kb, 20 kb, 25 kb, 30 kb, 35 kb, 40 kb, 45 kb, or 50 kb.

In an embodiment, the viral vector recognizes a specific cell type or tissue. For example, the viral vector can be pseudotyped with a different/alternative viral envelope glycoprotein; engineered with a cell type-specific receptor (*e.g.,* genetic modification(s) of one or more viral envelope glycoproteins to incorporate a targeting ligand such as a peptide ligand, a single chain antibody, or a growth factor); and/or engineered to have a molecular bridge with dual specificities with one end recognizing a viral glycoprotein and the other end recognizing a moiety of the target cell surface (*e.g.,* a ligand-receptor, monoclonal antibody, avidin-biotin and chemical conjugation).

In some embodiments, the Cas9- and/or gRNA-encoding nucleic acid sequence is delivered by a recombinant retrovirus. In some embodiments, the retrovirus (*e.g.*, Moloney murine leukemia virus) comprises a reverse transcriptase, *e.g.*, that allows integration into the host genome. In some embodiments, the retrovirus is replication-competent. In other embodiments, the retrovirus is replication-defective, *e.g.*, having one of more coding regions for the genes necessary for additional rounds of virion replication and packaging replaced with other genes, or deleted.

In an embodiment, the Cas9- and/or gRNA-encoding nucleic acid sequence is delivered by a recombinant lentivirus. In an embodiment, the donor template nucleic acid is delivered by a recombinant lentivirus. For example, the lentivirus is replication-defective, *e.g.,* does not comprise one or more genes required for viral replication.

In some embodiments, the Cas9- and/or gRNA-encoding nucleic acid sequence is delivered by a recombinant adenovirus. In an embodiment, the donor template nucleic acid is delivered by a recombinant adenovirus. In some embodiments, the adenovirus is engineered to have reduced immunity in human.

In some embodiments, the Cas9- and/or gRNA-encoding nucleic acid sequence is delivered by a recombinant AAV. In an embodiment, the donor template nucleic acid is delivered by a recombinant AAV. In some embodiments, the AAV does not incorporate its genome into that of a host cell, *e.g.,* a target cell as describe herein. In some embodiments, the AAV can incorporate its genome into that of a host cell. In some embodiments, the AAV is a self-complementary adeno-associated virus (scAAV), *e.g.,* a scAAV that packages both strands which anneal together to form double stranded DNA.

In an embodiment, an AAV capsid that can be used in the methods described herein is a capsid sequence from serotype AAV1, AAV2, AAV3, AAV4, AAV5, AAV6, AAV7, AAV8, AAV9, AAV.rh8, AAV.rh10, AAV.rh32/33, AAV.rh43, AAV.rh64R1, or AAV7m8.

In an embodiment, the Cas9- and/or gRNA-encoding DNA is delivered in a re-engineered AAV capsid, *e.g.,* with 50% or greater, *e.g.,* 60% or greater, 70% or greater, 80% or greater, 90% or greater, or 95% or greater, sequence homology with a capsid sequence from serotypes AAV1, AAV2, AAV3, AAV4, AAV5, AAV6, AAV7, AAV8, AAV9, AAV.rh8, AAV.rh10, AAV.rh32/33, AAV.rh43, or AAV.rh64R1.

In an embodiment, the Cas9- and/or gRNA-encoding DNA is delivered by a chimeric AAV capsid. In an embodiment, the donor template nucleic acid is delivered by a chimeric AAV capsid. Exemplary chimeric AAV capsids include, but are not limited to, AAV9i1, AAV2i8, AAV-DJ, AAV2G9, AAV2i8G9, or AAV8G9.

In an embodiment, the AAV is a self-complementary adeno-associated virus (scAAV), *e.g.,* a scAAV that packages both strands which anneal together to form double stranded DNA.

In some embodiments, the Cas9- and/or gRNA-encoding DNA is delivered by a hybrid virus, *e.g.,* a hybrid of one or more of the viruses described herein. In an embodiment, the hybrid virus is hybrid of an AAV (*e.g.,* of any AAV serotype), with a Bocavirus, B19 virus, porcine AAV, goose AAV, feline AAV, canine AAV, or MVM.

A packaging cell is used to form a virus particle that is capable of infecting a target cell. Exemplary packaging cells include 293 cells, which can package adenovirus, and ψ2 or PA317 cells, which can package retrovirus. A viral vector used in gene therapy is usually generated by a producer cell line that packages a nucleic acid vector into a viral particle. The vector typically contains the minimal viral sequences required for packaging and subsequent integration into a host or target cell (if applicable), with other viral sequences being replaced by an expression cassette encoding the protein to be expressed, e.g. Cas9. For example, an AAV vector used in gene therapy typically only possesses inverted terminal repeat (ITR) sequences from the AAV genome which are required for packaging and gene expression in the host or target cell. The missing viral functions can be supplied in *trans* by the packaging cell line and/or plasmid containing E2A, E4, and VA genes from adenovirus, and plasmid encoding *Rep* and *Cap* genes from AAV, as described in "Triple Transfection Protocol." Henceforth, the viral DNA is packaged in a cell line, which contains a helper plasmid encoding the other AAV genes, namely rep and cap, but lacking ITR sequences. In certain embodiments, the viral DNA is packaged in a producer cell line, which contains E1A and/or E1B genes from adenovirus. The cell line is also infected with adenovirus as a helper. The helper virus (*e.g.*, adenovirus or HSV) or helper plasmid promotes replication of the AAV vector and expression of AAV genes from the helper plasmid with ITRs. The helper plasmid is not packaged in significant amounts due to a lack of ITR sequences. Contamination with adenovirus can be reduced by, *e.g.,* heat treatment to which adenovirus is more sensitive than AAV.

In certain embodiments, the viral vector is capable of cell type and/or tissue type recognition. For example, the viral vector can be pseudotyped with a different/alternative viral envelope glycoprotein; engineered with a cell type-specific receptor *(e.g.,* genetic modification of the viral envelope glycoproteins to incorporate targeting ligands such as a peptide ligand, single chain antibody, or growth factor); and/or engineered to have a molecular bridge with dual specificities with one end recognizing a viral glycoprotein and the other end recognizing a moiety of the target cell surface (*e.g.*, ligand-receptor, monoclonal antibody, avidin-biotin and chemical conjugation).

In certain embodiments, the viral vector achieves cell type specific expression. For example, a tissue-specific promoter can be constructed to restrict expression of the transgene (Cas9 and gRNA) to only the target cell. The specificity of the vector can also be mediated by microRNA-dependent control of transgene expression. In an embodiment, the viral vector has increased efficiency of fusion of the viral vector and a target cell membrane. For example, a fusion protein such as fusion-competent hemagglutin (HA) can be incorporated to increase viral uptake into cells. In an embodiment, the viral vector has the ability of nuclear localization. For example, a virus that requires the breakdown of the nuclear envelope (during cell division) and therefore will not infect a non-diving cell can be altered to incorporate a nuclear localization peptide in the matrix protein of the virus thereby enabling the transduction of non-proliferating cells.

In some embodiments, the Cas9- and/or gRNA-encoding DNA is delivered by a non-vector based method (*e.g.*, using naked DNA or DNA complexes). For example, the DNA can be delivered, *e.g.,* by organically modified silica or silicate (Ormosil), electroporation, transient cell compression or squeezing (see, *e.g.,* Lee 2012), gene gun, sonoporation, magnetofection, lipid-mediated transfection, dendrimers, inorganic nanoparticles, calcium phosphates, or a combination thereof.

In an embodiment, delivery via electroporation comprises mixing the cells with the Cas9-and/or gRNA-encoding DNA in a cartridge, chamber or cuvette and applying one or more electrical impulses of defined duration and amplitude. In an embodiment, delivery via electroporation is performed using a system in which cells are mixed with the Cas9-and/or gRNA-encoding DNA in a vessel connected to a device (*e.g.,* a pump) which feeds the mixture into a cartridge, chamber or cuvette wherein one or more electrical impulses of defined duration and amplitude are applied, after which the cells are delivered to a second vessel.

In some embodiments, the Cas9- and/or gRNA-encoding DNA is delivered by a combination of a vector and a non-vector based method. In an embodiment, the donor template nucleic acid is delivered by a combination of a vector and a non-vector based method. For example, virosomes combine liposomes with an inactivated virus (*e.g.*, HIV or influenza virus), which can result in more efficient gene transfer, *e.g.*, in respiratory epithelial cells than either viral or liposomal methods alone.

As described above, a nucleic acid may comprise (a) a sequence encoding a gRNA molecule comprising a targeting domain that is complementary with a target domain in the gene, and (b) a sequence encoding a Cas9 molecule. In an embodiment, (a) and (b) are present on the same nucleic acid molecule, *e.g.,* the same vector, *e.g.,* the same viral vector, *e.g.,* the same adeno-associated virus (AAV) vector. In an embodiment, the nucleic acid molecule is an AAV vector. Exemplary AAV vectors that may be used in any of the described compositions and methods include an AAV2 vector, a modified AAV2 vector, an AAV3 vector, a modified AAV3 vector, an AAV6 vector, a modified AAV6 vector, an AAV8 vector and an AAV9 vector. In another embodiment, (a) is present on a first nucleic acid molecule, *e.g.,* a first vector, *e.g.,* a first viral vector, *e.g.,* a first AAV vector; and (b) is present on a second nucleic acid molecule, *e.g.,* a second vector, *e.g.,* a second vector, *e.g.,* a second AAV vector. The first and second nucleic acid molecules may be AAV vectors. In yet another embodiment, the nucleic acid may further comprise (c) a sequence that encodes a second, third and/or fourth gRNA molecule as described herein. In an embodiment, the nucleic acid comprises (a), (b) and (c). Each of (a) and (c) may be present on the same nucleic acid molecule, *e.g.,* the same vector, *e.g.,* the same viral vector, *e.g.,* the same adeno-associated virus (AAV) vector. In an embodiment, the nucleic acid molecule is an AAV vector.

In another embodiment, (a) and (c) are on different vectors. For example, (a) may be present on a first nucleic acid molecule, *e.g.,* a first vector, *e.g.,* a first viral vector, *e.g.,* a first AAV vector; and (c) may be present on a second nucleic acid molecule, *e.g.,* a second vector, *e.g.,* a second vector, *e.g.,* a second AAV vector. In an embodiment, the first and second nucleic acid molecules are AAV vectors. In yet another embodiment, each of (a), (b), and (c) are present on the same nucleic acid molecule, *e.g.,* the same vector, *e.g.,* the same viral vector, *e.g.,* an AAV vector. In an embodiment, the nucleic acid molecule is an AAV vector. In an alternate embodiment, one of (a), (b), and (c) is encoded on a first nucleic acid molecule, *e.g.,* a first vector, *e.g.,* a first viral vector, *e.g.,* a first AAV vector; and a second and third of (a), (b), and (c) is encoded on a second nucleic acid molecule, *e.g.,* a second vector, *e.g.,* a second vector, *e.g.,* a second AAV vector. The first and second nucleic acid molecule may be AAV vectors.

In an embodiment, (a) is present on a first nucleic acid molecule, *e.g.,* a first vector, *e.g.,* a first viral vector, a first AAV vector; and (b) and (c) are present on a second nucleic acid molecule, *e.g.,* a second vector, *e.g.,* a second vector, *e.g.,* a second AAV vector. The first and second nucleic acid molecule may be AAV vectors. In another embodiment, (b) is present on a first nucleic acid molecule, *e.g.,* a first vector, *e.g.,* a first viral vector, *e.g.,* a first AAV vector; and (a) and (c) are present on a second nucleic acid molecule, *e.g.,* a second vector, *e.g.,* a second vector, *e.g.,* a second AAV vector. The first and second nucleic acid molecule may be AAV vectors.

In another embodiment, (c) is present on a first nucleic acid molecule, *e.g.,* a first vector, *e.g.,* a first viral vector, *e.g.,* a first AAV vector; and (b) and (a) are present on a second nucleic acid molecule, *e.g.,* a second vector, *e.g.,* a second vector, *e.g.,* a second AAV vector. The first and second nucleic acid molecule may be AAV vectors.

In another embodiment, each of (a), (b) and (c) are present on different nucleic acid molecules, *e.g.,* different vectors, *e.g.,* different viral vectors, *e.g.,* different AAV vector. For example, (a) may be on a first nucleic acid molecule, (b) on a second nucleic acid molecule, and (c) on a third nucleic acid molecule. The first, second and third nucleic acid molecule may be AAV vectors.

In another embodiment, when a third and/or fourth gRNA molecule are present, each of (a), (b), (c)(i), (c)(ii) and (c)(iii) may be present on the same nucleic acid molecule, *e.g.,* the same vector, *e.g.,* the same viral vector, *e.g.,* an AAV vector. In an embodiment, the nucleic acid molecule is an AAV vector. In an alternate embodiment, each of (a), (b), (c)(i), (c)(ii) and (c)(iii) may be present on the different nucleic acid molecules, *e.g.*, different vectors, *e.g.,* the different viral vectors, *e.g.,* different AAV vectors. In further embodiments, each of (a), (b), (c)(i), (c)(ii) and (c)(iii) may be present on more than one nucleic acid molecule, but fewer than five nucleic acid molecules, *e.g.*, AAV vectors.

In another embodiment, when (d) a template nucleic acid is present, each of (a), (b), and (d) may be present on the same nucleic acid molecule, *e.g.,* the same vector, *e.g.,* the same viral vector, *e.g.,* an AAV vector. In an embodiment, the nucleic acid molecule is an AAV vector. In an alternate embodiment, each of (a), (b), and (d) may be present on the different nucleic acid molecules, *e.g.,* different vectors, *e.g.,* the different viral vectors, *e.g.,* different AAV vectors. In further embodiments, each of (a), (b), and (d) may be present on more than one nucleic acid molecule, but fewer than three nucleic acid molecules, *e.g.*, AAV vectors.

In another embodiment, when (d) a template nucleic acid is present, each of (a), (b), (c)(i) and (d) may be present on the same nucleic acid molecule, *e.g.,* the same vector, *e.g.,* the same viral vector, *e.g.,* an AAV vector. In an embodiment, the nucleic acid molecule is an AAV vector. In an alternate embodiment, each of (a), (b), (c)(i) and (d) may be present on the different nucleic acid molecules, *e.g.,* different vectors, *e.g.,* the different viral vectors, *e.g.,* different AAV vectors. In further embodiments, each of (a), (b), (c)(i) and (d) may be present on more than one nucleic acid molecule, but fewer than four nucleic acid molecules, *e.g.,* AAV vectors.

In another embodiment, when (d) a template nucleic acid is present, each of (a), (b), (c)(i), (c)(ii) and (d) may be present on the same nucleic acid molecule, *e.g.,* the same vector, *e.g.,* the same viral vector, *e.g.,* an AAV vector. In an embodiment, the nucleic acid molecule is an AAV vector. In an alternate embodiment, each of (a), (b), (c)(i), (c)(ii) and (d) may be present on the different nucleic acid molecules, *e.g.,* different vectors, *e.g.,* the different viral vectors, *e.g.*, different AAV vectors. In further embodiments, each of (a), (b), (c)(i), (c)(ii) and (d) may be present on more than one nucleic acid molecule, but fewer than five nucleic acid molecules, *e.g.*, AAV vectors.

In another embodiment, when (d) a template nucleic acid is present, each of (a), (b), (c)(i), (c)(ii), (c)(iii) and (d) may be present on the same nucleic acid molecule, *e.g.,* the same vector, *e.g.,* the same viral vector, *e.g.,* an AAV vector. In an embodiment, the nucleic acid molecule is an AAV vector. In an alternate embodiment, each of (a), (b), (c)(i), (c)(ii), (c)(iii) and (d) may be present on the different nucleic acid molecules, *e.g.,* different vectors, *e.g.,* the different viral vectors, *e.g.*, different AAV vectors. In further embodiments, each of (a), (b), (c)(i), (c)(ii), (c)(iii) and (d) may be present on more than one nucleic acid molecule, but fewer than six nucleic acid molecules, *e.g.*, AAV vectors.

The nucleic acids described herein may comprise a promoter operably linked to the sequence that encodes the gRNA molecule of (a), *e.g.,* a promoter described herein. The nucleic acid may further comprise a second promoter operably linked to the sequence that encodes the second, third and/or fourth gRNA molecule of (c), *e.g.,* a promoter described herein. The promoter and second promoter differ from one another. In an embodiment, the promoter and second promoter are the same.

The nucleic acids described herein may further comprise a promoter operably linked to the sequence that encodes the Cas9 molecule of (b), *e.g.,* a promoter described herein.

In certain embodiments, the delivery vehicle is a non-viral vector, and in certain of these embodiments the non-viral vector is an inorganic nanoparticle. Exemplary inorganic nanoparticles include, *e.g.,* magnetic nanoparticles (*e.g.,* Fe₃MnO₂) or silica. The outer surface of the nanoparticle can be conjugated with a positively charged polymer *(e.g.,* polyethylenimine, polylysine, polyserine) which allows for attachment (*e.g.*, conjugation or entrapment) of payload. In an embodiment, the non-viral vector is an organic nanoparticle (*e.g.*, entrapment of the payload inside the nanoparticle). Exemplary organic nanoparticles include, *e.g.*, SNALP liposomes that contain cationic lipids together with neutral helper lipids which are coated with polyethylene glycol (PEG) and protamine and nucleic acid complex coated with lipid coating.

Exemplary lipids for gene transfer are shown below in **Table 8.**

**Table 8. Lipids Used for Gene Transfer**

| **Lipid** | **Abbreviation** | **Feature** |
|---|---|---|
| 1,2-Dioleoyl-sn-glycero-3-phosphatidylcholine | DOPC | Helper |
| 1,2-Dioleoyl-sn-glycero-3-phosphatidylethanolamine | DOPE | Helper |
| Cholesterol | | Helper |
| N-[1-(2,3-Dioleyloxy)propyl]N,N,N-trimethylammonium chloride | DOTMA | Cationic |
| 1,2-Dioleoyloxy-3-trimethylammonium-propane | DOTAP | Cationic |
| Dioctadecylamidoglycylspermine | DOGS | Cationic |
| N-(3-Aminopropyl)-N,N-dimethyl-2,3-bis(dodecyloxy)-1-propanaminium bromide | GAP-DLRIE | Cationic |
| Cetyltrimethylammonium bromide | CTAB | Cationic |
| 6-Lauroxvhexvl ornithinate | LHON | Cationic |
| 1-(2,3-Dioleoyloxypropyl)-2,4,6-trimethylpyridinium | 2Oc | Cationic |
| 2,3-Dioleyloxy-N-[2(sperminecarboxamido-ethyl]-N,N-dimethyl-1-propanaminium trifluoroacetate | DOSPA | Cationic |
| 1,2-Dioleyl-3-trimethylammonium-propane | DOPA | Cationic |
| N-(2-Hydroxyethyl)-N,N-dimethyl-2,3-bis(tetradecyloxy)-1-propanaminium bromide | MDRIE | Cationic |
| Dimyristooxypropyl dimethyl hydroxyethyl ammonium bromide | DMRI | Cationic |
| 3β-[N-(N',N'-Dimethylaminoethane)-carbamoyl]cholesterol | DC-Chol | Cationic |
| Bis-guanidium-tren-cholesterol | BGTC | Cationic |
| 1,3-Diodeoxy-2-(6-carboxy-spermyl)-propylamide | DOSPER | Cationic |
| Dimethyloctadecylammonium bromide | DDAB | Cationic |
| Dioctadecylamidoglicylspermidin | DSL | Cationic |
| rac-[(2,3-Dioctadecyloxypropyl)(2-hydroxyethyl)]-dimethylammonium chloride | CLIP-1 | Cationic |
| rac-[2(2,3-Dihexadecyloxypropyl-oxymethyloxy)ethyl]trimethylammonium bromide | CLIP-6 | Cationic |
| Ethyldimyristoylphosphatidylcholine | EDMPC | Cationic |
| 1,2-Distearyloxy-N,N-dimethyl-3-aminopropane | DSDMA | Cationic |
| 1,2-Dimyristoyl-trimethylammonium propane | DMTAP | Cationic |
| O,O'-Dimyristyl-N-lysyl aspartate | DMKE | Cationic |
| 1,2-Distearoyl-sn-glycero-3-ethylphosphocholine | DSEPC | Cationic |
| N-Palmitoyl D-erythro-sphingosyl carbamoyl-spermine | CCS | Cationic |
| N-t-Butyl-N0-tetradecyl-3-tetradecylaminopropionamidine | diC 14-amidine | Cationic |
| Octadecenolyoxy[ethyl-2-heptadecenyl-3 hydroxyethyl] imidazolinium chloride | DOTIM | Cationic |
| N1-Cholesteryloxycarbonyl-3,7-diazanonane-1,9-diamine | CDAN | Cationic |
| 2-(3-[Bis(3-amino-propyl)-amino]propylamino)-N-ditetradecylcarbamoylme-ethyl-acetamide | RPR209120 | Cationic |
| 1,2-dilinolevloxy-3- dimethylaminopropane | DLinDMA | Cationic |
| 2,2-dilinoleyl-4-dimethylaminoethyl-[1,3]- dioxolane | DLin-KC2-DMA | Cationic |
| dilinoleyl- methyl-4-dimethylaminobutyrate | DLin-MC3-DMA | Cationic |

Exemplary polymers for gene transfer are shown below in **Table 9.**

**Table 9. Polymers Used for Gene Transfer**

| **Polymer** | **Abbreviation** |
|---|---|
| Poly(ethylene)glycol | PEG |
| Polyethylenimine | PEI |
| Dithiobis(succinimidylpropionate) | DSP |
| Dimethyl-3,3'-dithiobispropionimidate | DTBP |
| Poly(ethylene imine) biscarbamate | PEIC |
| Poly(L-lysine) | PLL |
| Histidine modified PLL | |
| Poly(*N*-vinylpyrrolidone) | PVP |
| Poly(propylenimine) | PPI |
| Poly(amidoamine) | PAMAM |
| Poly(amido ethylenimine) | SS-PAEI |
| Triethylenetetramine | TETA |
| Poly(β-aminoester) | |
| Poly(4-hydroxy-L-proline ester) | PHP |
| Poly(allylamine) | |
| Poly(α-[4-aminobutyl]-L-glycolic acid) | PAGA |
| Poly(D,L-lactic-co-glycolic acid) | PLGA |
| Poly(*N*-ethyl-4-vinylpyridinium bromide) | |
| Poly(phosphazene)s | PPZ |
| Poly(phosphoester)s | PPE |
| Poly(phosphoramidate)s | PPA |
| Poly(*N*-2-hydroxypropylmethacrylamide) | pHPMA |
| Poly (2-(dimethylamino)ethyl methacrylate) | pDMAEMA |
| Poly(2-aminoethyl propylene phosphate) | PPE-EA |
| Chitosan | |
| Galactosylated chitosan | |
| *N*-Dodacylated chitosan | |
| Histone | |
| Collagen | |
| Dextran-spermine | D-SPM |

In an embodiment, the vehicle has targeting modifications to increase target cell update of nanoparticles and liposomes, *e.g.*, cell specific antigens, monoclonal antibodies, single chain antibodies, aptamers, polymers, sugars (*e.g.*, N-acetylgalactosamine (GalNAc)), and cell penetrating peptides. In an embodiment, the vehicle uses fusogenic and endosome-destabilizing peptides/polymers. In an embodiment, the vehicle undergoes acid-triggered conformational changes (*e.g.,* to accelerate endosomal escape of the cargo). In an embodiment, a stimuli-cleavable polymer is used, *e.g.,* for release in a cellular compartment. For example, disulfide-based cationic polymers that are cleaved in the reducing cellular environment can be used.

In an embodiment, the delivery vehicle is a biological non-viral delivery vehicle. In an embodiment, the vehicle is an attenuated bacterium (*e.g.*, naturally or artificially engineered to be invasive but attenuated to prevent pathogenesis and expressing the transgene (*e.g., Listeria monocytogenes,* certain *Salmonella strains, Bifidobacterium longum,* and modified *Escherichia coli*), bacteria having nutritional and tissue-specific tropism to target specific tissues, bacteria having modified surface proteins to alter target tissue specificity). In an embodiment, the vehicle is a genetically modified bacteriophage (*e.g.*, engineered phages having large packaging capacity, less immunogenic, containing mammalian plasmid maintenance sequences and having incorporated targeting ligands). In an embodiment, the vehicle is a mammalian virus-like particle. For example, modified viral particles can be generated (*e.g.,* by purification of the "empty" particles followed by *ex vivo* assembly of the virus with the desired cargo). The vehicle can also be engineered to incorporate targeting ligands to alter target tissue specificity. In an embodiment, the vehicle is a biological liposome. For example, the biological liposome is a phospholipid-based particle derived from human cells (*e.g.*, erythrocyte ghosts, which are red blood cells broken down into spherical structures derived from the subject (*e.g.*, tissue targeting can be achieved by attachment of various tissue or cell-specific ligands), or secretory exosomes -subject (*i.e.,* patient) derived membrane-bound nano vesicle (30 -100 nm) of endocytic origin (*e.g.,* can be produced from various cell types and can therefore be taken up by cells without the need of for targeting ligands).

In an embodiment, one or more nucleic acid molecules (*e.g.*, DNA molecules) other than the components of a Cas system, *e.g.*, the Cas9 molecule component and/or the gRNA molecule component described herein, are delivered. In an embodiment, the nucleic acid molecule is delivered at the same time as one or more of the components of the Cas system are delivered. In an embodiment, the nucleic acid molecule is delivered before or after (*e.g.,* less than about 30 minutes, 1 hour, 2 hours, 3 hours, 6 hours, 9 hours, 12 hours, 1 day, 2 days, 3 days, 1 week, 2 weeks, or 4 weeks) one or more of the components of the Cas system are delivered. In an embodiment, the nucleic acid molecule is delivered by a different means than one or more of the components of the Cas system, *e.g.*, the Cas9 molecule component and/or the gRNA molecule component, are delivered. The nucleic acid molecule can be delivered by any of the delivery methods described herein. For example, the nucleic acid molecule can be delivered by a viral vector, *e.g.,* an integration-deficient lentivirus, and the Cas9 molecule component and/or the gRNA molecule component can be delivered by electroporation, *e.g.,* such that the toxicity caused by nucleic acids (*e.g.,* DNAs) can be reduced. In an embodiment, the nucleic acid molecule encodes a therapeutic protein, *e.g.,* a protein described herein. In an embodiment, the nucleic acid molecule encodes an RNA molecule, *e.g.,* an RNA molecule described herein.

### Delivery of RNA encoding a Cas9 molecule

RNA encoding Cas9 molecules and/or gRNA molecules, can be delivered into cells, *e.g.,* target cells described herein, by art-known methods or as described herein. For example, Cas9-encoding and/or gRNA-encoding RNA can be delivered, *e.g.,* by microinjection, electroporation, transient cell compression or squeezing (see, *e.g.,* Lee 2012), lipid-mediated transfection, peptide-mediated delivery, or a combination thereof. Cas9-encoding and/or gRNA-encoding RNA can be conjugated to molecules) promoting uptake by the target cells (*e.g.,* target cells described herein).

In an embodiment, delivery via electroporation comprises mixing the cells with the RNA encoding Cas9 molecules and/or gRNA molecules, with or without donor template nucleic acid molecules, in a cartridge, chamber or cuvette and applying one or more electrical impulses of defined duration and amplitude. In an embodiment, delivery via electroporation is performed using a system in which cells are mixed with the RNA encoding Cas9 molecules and/or gRNA molecules, with or without donor template nucleic acid molecules in a vessel connected to a device (*e.g.,* a pump) which feeds the mixture into a cartridge, chamber or cuvette wherein one or more electrical impulses of defined duration and amplitude are applied, after which the cells are delivered to a second vessel. Cas9-encoding and/or gRNA-encoding RNA can be conjugated to molecules to promote uptake by the target cells (*e.g.,* target cells described herein).

### Delivery of Cas9

Cas9 molecules can be delivered into cells by art-known methods or as described herein. For example, Cas9 protein molecules can be delivered, *e.g.,* by microinjection, electroporation, transient cell compression or squeezing (see, *e.g.,* Lee 2012), lipid-mediated transfection, peptide-mediated delivery, or a combination thereof. Delivery can be accompanied by DNA encoding a gRNA or by a gRNA. Cas9 protein can be conjugated to molecules promoting uptake by the target cells (*e.g.,* target cells described herein).

In an embodiment, delivery via electroporation comprises mixing the cells with the Cas9 molecules and/or gRNA molecules, with or without donor nucleic acid, in a cartridge, chamber or cuvette and applying one or more electrical impulses of defined duration and amplitude. In an embodiment, delivery via electroporation is performed using a system in which cells are mixed with the Cas9 molecules and/or gRNA molecules, with or without donor nucleic acid in a vessel connected to a device (*e.g.,* a pump) which feeds the mixture into a cartridge, chamber or cuvette wherein one or more electrical impulses of defined duration and amplitude are applied, after which the cells are delivered to a second vessel. Cas9-encoding and/or gRNA-encoding RNA can be conjugated to molecules to promote uptake by the target cells (*e.g.,* target cells described herein).

### Route of Administration

Systemic modes of administration include oral and parenteral routes. Parenteral routes include, by way of example, intravenous, intramarrow, intrarterial, intramuscular, intradermal, subcutaneous, intranasal and intraperitoneal routes. Components administered systemically may be modified or formulated to target, *e.g.,* HSCs, hematopoetic stem/progenitor cells, or erythroid progenitors or precursor cells.

Local modes of administration include, by way of example, intramarrow injection into the trabecular bone or intrafemoral injection into the marrow space, and infusion into the portal vein. In an embodiment, significantly smaller amounts of the components (compared with systemic approaches) may exert an effect when administered locally (for example, directly into the bone marrow) compared to when administered systemically (for example, intravenously). Local modes of administration can reduce or eliminate the incidence of potentially toxic side effects that may occur when therapeutically effective amounts of a component are administered systemically.

Administration may be provided as a periodic bolus (*e.g.*, intravenously) or as continuous infusion from an internal reservoir or from an external reservoir (for example, from an intravenous bag or implantable pump). Components may be administered locally, for example, by continuous release from a sustained release drug delivery device.

In addition, components may be formulated to permit release over a prolonged period of time. A release system can include a matrix of a biodegradable material or a material which releases the incorporated components by diffusion. The components can be homogeneously or heterogeneously distributed within the release system. A variety of release systems may be useful, however, the choice of the appropriate system will depend upon rate of release required by a particular application. Both non-degradable and degradable release systems can be used. Suitable release systems include polymers and polymeric matrices, non-polymeric matrices, or inorganic and organic excipients and diluents such as, but not limited to, calcium carbonate and sugar (for example, trehalose). Release systems may be natural or synthetic. However, synthetic release systems are preferred because generally they are more reliable, more reproducible and produce more defined release profiles. The release system material can be selected so that components having different molecular weights are released by diffusion through or degradation of the material.

Representative synthetic, biodegradable polymers include, for example: polyamides such as poly(amino acids) and poly(peptides); polyesters such as poly(lactic acid), poly(glycolic acid), poly(lactic-co-glycolic acid), and poly(caprolactone); poly(anhydrides); polyorthoesters; polycarbonates; and chemical derivatives thereof (substitutions, additions of chemical groups, for example, alkyl, alkylene, hydroxylations, oxidations, and other modifications routinely made by those skilled in the art), copolymers and mixtures thereof. Representative synthetic, non-degradable polymers include, for example: polyethers such as poly(ethylene oxide), poly(ethylene glycol), and poly(tetramethylene oxide); vinyl polymers-polyacrylates and polymethacrylates such as methyl, ethyl, other alkyl, hydroxyethyl methacrylate, acrylic and methacrylic acids, and others such as poly(vinyl alcohol), poly(vinyl pyrolidone), and poly(vinyl acetate); poly(urethanes); cellulose and its derivatives such as alkyl, hydroxyalkyl, ethers, esters, nitrocellulose, and various cellulose acetates; polysiloxanes; and any chemical derivatives thereof (substitutions, additions of chemical groups, for example, alkyl, alkylene, hydroxylations, oxidations, and other modifications routinely made by those skilled in the art), copolymers and mixtures thereof.

Poly(lactide-co-glycolide) microsphere can also be used for injection. Typically the microspheres are composed of a polymer of lactic acid and glycolic acid, which are structured to form hollow spheres. The spheres can be approximately 15-30 microns in diameter and can be loaded with components described herein.

### Bi-Modal or Differential Delivery of Components

Separate delivery of the components of a Cas system, *e.g.*, the Cas9 molecule component and the gRNA molecule component, and more particularly, delivery of the components by differing modes, can enhance performance, *e.g.*, by improving tissue specificity and safety.

In an embodiment, the Cas9 molecule and the gRNA molecule are delivered by different modes, or as sometimes referred to herein as differential modes. Different or differential modes, as used herein, refer modes of delivery that confer different pharmacodynamic or pharmacokinetic properties on the subject component molecule, *e.g.,* a Cas9 molecule, gRNA molecule, template nucleic acid, or payload. For example, the modes of delivery can result in different tissue distribution, different half-life, or different temporal distribution, *e.g.*, in a selected compartment, tissue, or organ.

Some modes of delivery, *e.g.*, delivery by a nucleic acid vector that persists in a cell, or in progeny of a cell, *e.g.,* by autonomous replication or insertion into cellular nucleic acid, result in more persistent expression of and presence of a component. Examples include viral, *e.g.,* AAV or lentivirus, delivery.

By way of example, the components, *e.g.*, a Cas9 molecule and a gRNA molecule, can be delivered by modes that differ in terms of resulting half-life or persistent of the delivered component the body, or in a particular compartment, tissue or organ. In an embodiment, a gRNA molecule can be delivered by such modes. The Cas9 molecule component can be delivered by a mode which results in less persistence or less exposure to the body or a particular compartment or tissue or organ.

More generally, in an embodiment, a first mode of delivery is used to deliver a first component and a second mode of delivery is used to deliver a second component. The first mode of delivery confers a first pharmacodynamic or pharmacokinetic property. The first pharmacodynamic property can be, *e.g.*, distribution, persistence, or exposure, of the component, or of a nucleic acid that encodes the component, in the body, a compartment, tissue or organ. The second mode of delivery confers a second pharmacodynamic or pharmacokinetic property. The second pharmacodynamic property can be, *e.g.*, distribution, persistence, or exposure, of the component, or of a nucleic acid that encodes the component, in the body, a compartment, tissue or organ.

In certain embodiments, the first pharmacodynamic or pharmacokinetic property, *e.g.,* distribution, persistence or exposure, is more limited than the second pharmacodynamic or pharmacokinetic property.

In certain embodiments, the first mode of delivery is selected to optimize, *e.g.,* minimize, a pharmacodynamic or pharmacokinetic property, *e.g.*, distribution, persistence or exposure.

In certain embodiments, the second mode of delivery is selected to optimize, *e.g.,* maximize, a pharmacodynamic or pharmacokinetic property, *e.g.*, distribution, persistence or exposure.

In an embodiments, the first mode of delivery comprises the use of a relatively persistent element, *e.g.,* a nucleic acid, *e.g.,* a plasmid or viral vector, *e.g.,* an AAV or lentivirus. As such vectors are relatively persistent product transcribed from them would be relatively persistent.

In certain embodiments, the second mode of delivery comprises a relatively transient element, *e.g.,* an RNA or protein.

In certain embodiments, the first component comprises gRNA molecule, and the delivery mode is relatively persistent, *e.g.*, the gRNA is transcribed from a plasmid or viral vector, *e.g.,* an AAV or lentivirus. Transcription of these genes would be of little physiological consequence because the genes do not encode for a protein product, and the gRNAs are incapable of acting in isolation. The second component, a Cas9 molecule, is delivered in a transient manner, for example as mRNA or as protein, ensuring that the full Cas9 molecule/gRNA molecule complex is only present and active for a short period of time.

Furthermore, the components can be delivered in different molecular form or with different delivery vectors that complement one another to enhance safety and tissue specificity.

Use of differential delivery modes can enhance performance, safety and/or efficacy, *e.g.,* the likelihood of an eventual off-target modification can be reduced. Delivery of immunogenic components, *e.g.*, Cas9 molecules, by less persistent modes can reduce immunogenicity, as peptides from the bacterially-derived Cas enzyme are displayed on the surface of the cell by MHC molecules. A two-part delivery system can alleviate these drawbacks.

Differential delivery modes can be used to deliver components to different, but overlapping target regions. The formation active complex is minimized outside the overlap of the target regions. Thus, in an embodiment, a first component, *e.g.*, a gRNA molecule is delivered by a first delivery mode that results in a first spatial, *e.g.*, tissue, distribution. A second component, *e.g.,* a Cas9 molecule is delivered by a second delivery mode that results in a second spatial, *e.g*., tissue, distribution. In an embodiment, the first mode comprises a first element selected from a liposome, nanoparticle, *e.g.*, polymeric nanoparticle, and a nucleic acid, *e.g.,* viral vector. The second mode comprises a second element selected from the group. In an embodiment, the first mode of delivery comprises a first targeting element, *e.g.,* a cell specific receptor or an antibody, and the second mode of delivery does not include that element. In certain embodiments, the second mode of delivery comprises a second targeting element, *e.g.,* a second cell specific receptor or second antibody.

When the Cas9 molecule is delivered in a virus delivery vector, a liposome, or polymeric nanoparticle, there is the potential for delivery to and therapeutic activity in multiple tissues, when it may be desirable to only target a single tissue. A two-part delivery system can resolve this challenge and enhance tissue specificity. If the gRNA molecule and the Cas9 molecule are packaged in separated delivery vehicles with distinct but overlapping tissue tropism, the fully functional complex is only be formed in the tissue that is targeted by both vectors.

Disclosed herein are methods of altering a cell, *e.g.,* altering the structure, *e.g.,* altering the sequence, of a target nucleic acid of a cell, comprising contacting said cell with: (a) a gRNA molecule that targets a gene, *e.g.,* a gRNA molecule as described herein; (b) a Cas9 molecule, *e.g.,* a Cas9 molecule as described herein; and optionally, (c) a second, third and/or fourth gRNA that targets the gene, *e.g.,* a gRNA molecule; and optionally, (d) a template nucleic acid, as described herein. The method may comprise contacting said cell with (a) and (b); (a), (b), and (c); or (a), (b), (c) and (d). The gRNA may target the gene and no exogenous template nucleic acid is contacted with the cell.

The contacting step of the method may comprise contacting the cell with a nucleic acid, *e.g.,* a vector, *e.g.,* an AAV vector, that expresses at least one of (a), (b), (c) and (d) or each of (a), (b), and (c). The contacting step of the method may comprise delivering to the cell a Cas9 molecule of (b), a nucleic acid which encodes a gRNA molecule of (a) and a template nucleic acid of (d), and optionally, a second gRNA molecule (c)(i) (and further optionally, a third gRNA molecule (c)(iv) and/or fourth gRNA molecule (c)(iii).

Contacting may comprise contacting the cell with a nucleic acid, *e.g.,* a vector, *e.g.,* an AAV vector, *e.g.,* an AAV2 vector, a modified AAV2 vector, an AAV3 vector, a modified AAV3 vector, an AAV6 vector, a modified AAV6 vector, an AAV8 vector or an AAV9 vector.

Contacting may comprise delivering to the cell a Cas9 molecule of (b), as a protein or an mRNA, and a nucleic acid which encodes (a) and optionally a second, third and/or fourth gRNA molecule of (c).

Contacting may comprises delivering to the cell a Cas9 molecule of (b), as a protein or an mRNA, said gRNA molecule of (a), as an RNA, and optionally said second, third and/or fourth gRNA molecule of (c), as an RNA.

Contacting may comprise delivering to the cell a gRNA of (a) as an RNA, optionally said second, third and/or fourth gRNA molecule of (c) as an RNA, and a nucleic acid that encodes the Cas9 molecule of (b).

Contacting may comprise contacting the subject with a nucleic acid, *e.g.,* a vector, *e.g.,* an AAV vector, described herein, *e.g.,* a nucleic acid that encodes at least one of (a), (b), (d) and optionally (c)(i), further optionally (c)(ii), and still further optionally (c)(iii).

Contacting may comprise delivering to said subject said Cas9 molecule of (b), as a protein or mRNA, and a nucleic acid which encodes (a), a nucleic acid of (d) and optionally (c)(i), further optionally (c)(ii), and still further optionally (c)(iii).

Contacting may comprise delivering to the subject the Cas9 molecule of (b), as a protein or mRNA, the gRNA molecule of (a), as an RNA, a nucleic acid of (d) and optionally the second, third and/or fourth gRNA molecule of (c), as an RNA.

Contacting may comprise delivering to the subject the gRNA molecule of (a), as an RNA, optionally said second, third and/or fourth gRNA molecule of (c), as an RNA, a nucleic acid that encodes the Cas9 molecule of (b), and a nucleic acid of (d).

A cell of the subject may be contacted *ex vivo* with (a), (b) and optionally (c)(i), further optionally (c)(ii), and still further optionally (c)(iii). Said cell may be returned to the subject's body.

A cell of the subject may be contacted *in vivo* with (a), (b) and optionally (c)(i), further optionally (c)(ii), and still further optionally (c)(iii). The cell of the subject may be contacted *in vivo* by (a) intravenous delivery, (b) intramuscular delivery, (c) subcutaneous delivery or (d) intra-bone marrow (IBM) delivery, of (a), (b) and optionally (c)(i), further optionally (c)(ii), and still further optionally (c)(iii).

Contacting may comprise contacting the subject with a nucleic acid, *e.g.,* a vector, *e.g.,* an AAV vector, described herein, *e.g.,* a nucleic acid that encodes at least one of (a), (b), and optionally (c)(i), further optionally (c)(ii), and still further optionally (c)(iii).

Contacting may comprise delivering to said subject said Cas9 molecule of (b), as a protein or mRNA, and a nucleic acid which encodes (a) and optionally (c)(i), further optionally (c)(ii), and still further optionally (c)(iii).

Contacting may comprise delivering to the subject the Cas9 molecule of (b), as a protein or mRNA, the gRNA molecule of (a), as an RNA, and optionally the second, third and/or fourth gRNA molecule of (c), as an RNA.

Contacting may comprise delivering to the subject the gRNA molecule of (a), as an RNA, optionally said second, third and/or fourth gRNA molecule of (c), as an RNA, and a nucleic acid that encodes the Cas9 molecule of (b).

Further disclosed herein are kits comprising compositions of the invention and instructions for use.

### Ex vivo delivery

In some embodiments, components described in **Table 6** are introduced into cells which are then introduced into the subject. Methods of introducing the components can include, *e.g.*, any of the delivery methods described in **Table 7.**

### XI. Modified Nucleosides, Nucleotides, and Nucleic Acids

Modified nucleosides and modified nucleotides can be present in nucleic acids, *e.g.,* particularly gRNA molecule, but also other forms of RNA, *e.g.,* mRNA, RNAi, or siRNA. As described herein, "nucleoside" is defined as a compound containing a five-carbon sugar molecule (a pentose or ribose) or derivative thereof, and an organic base, purine or pyrimidine, or a derivative thereof. As described herein, "nucleotide" is defined as a nucleoside further comprising a phosphate group.

Modified nucleosides and nucleotides can include one or more of:
(i) alteration, *e.g.*, replacement, of one or both of the non-linking phosphate oxygens and/or of one or more of the linking phosphate oxygens in the phosphodiester backbone linkage;
(ii) alteration, *e.g.,* replacement, of a constituent of the ribose sugar, *e.g.,* of the 2' hydroxyl on the ribose sugar;
(iii) wholesale replacement of the phosphate moiety with "dephospho" linkers;
(iv) modification or replacement of a naturally occurring nucleobase;
(v) replacement or modification of the ribose-phosphate backbone;
(vi) modification of the 3' end or 5' end of the oligonucleotide, *e.g.*, removal, modification or replacement of a terminal phosphate group or conjugation of a moiety; and
(vii) modification of the sugar.

The modifications listed above can be combined to provide modified nucleosides and nucleotides that can have two, three, four, or more modifications. For example, a modified nucleoside or nucleotide can have a modified sugar and a modified nucleobase. In an embodiment, every base of a gRNA is modified, *e.g.,* all bases have a modified phosphate group, *e.g.,* all are phosphorothioate groups. In an embodiment, all, or substantially all, of the phosphate groups of a unimolecular (or chimeric) or modular gRNA molecule are replaced with phosphorothioate groups.

In an embodiment, modified nucleotides, *e.g*., nucleotides having modifications as described herein, can be incorporated into a nucleic acid, *e.g.,* a "modified nucleic acid." In an embodiment, the modified nucleic acids comprise one, two, three or more modified nucleotides. In an embodiment, at least 5% (*e.g.,* at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or about 100%) of the positions in a modified nucleic acid are a modified nucleotides.

Unmodified nucleic acids can be prone to degradation by, *e.g.,* cellular nucleases. For example, nucleases can hydrolyze nucleic acid phosphodiester bonds. Accordingly, in one aspect the modified nucleic acids described herein can contain one or more modified nucleosides or nucleotides, *e.g.*, to introduce stability toward nucleases.

In an embodiment, the modified nucleosides, modified nucleotides, and modified nucleic acids described herein can exhibit a reduced innate immune response when introduced into a population of cells, both *in vivo* and *ex vivo.* The term "innate immune response" includes a cellular response to exogenous nucleic acids, including single stranded nucleic acids, generally of viral or bacterial origin, which involves the induction of cytokine expression and release, particularly the interferons, and cell death. In an embodiment, the modified nucleosides, modified nucleotides, and modified nucleic acids described herein can disrupt binding of a major groove interacting partner with the nucleic acid. In an embodiment, the modified nucleosides, modified nucleotides, and modified nucleic acids described herein can exhibit a reduced innate immune response when introduced into a population of cells, both *in vivo* and *ex vivo,* and also disrupt binding of a major groove interacting partner with the nucleic acid.

### Definitions of Chemical Groups

As used herein, "alkyl" is meant to refer to a saturated hydrocarbon group which is straight-chained or branched. Example alkyl groups include methyl (Me), ethyl (Et), propyl (*e.g.,* n-propyl and isopropyl), butyl (*e.g.,* n-butyl, isobutyl, t-butyl), pentyl (*e.g.,* n-pentyl, isopentyl, neopentyl), and the like. An alkyl group can contain from 1 to about 20, from 2 to about 20, from 1 to about 12, from 1 to about 8, from 1 to about 6, from 1 to about 4, or from 1 to about 3 carbon atoms.

As used herein, "aryl" refers to monocyclic or polycyclic (*e.g.*, having 2, 3 or 4 fused rings) aromatic hydrocarbons such as, for example, phenyl, naphthyl, anthracenyl, phenanthrenyl, indanyl, indenyl, and the like. In an embodiment, aryl groups have from 6 to about 20 carbon atoms.

As used herein, "alkenyl" refers to an aliphatic group containing at least one double bond.

As used herein, "alkynyl" refers to a straight or branched hydrocarbon chain containing 2-12 carbon atoms and characterized in having one or more triple bonds. Examples of alkynyl groups include, but are not limited to, ethynyl, propargyl, and 3-hexynyl.

As used herein, "arylalkyl" or "aralkyl" refers to an alkyl moiety in which an alkyl hydrogen atom is replaced by an aryl group. Aralkyl includes groups in which more than one hydrogen atom has been replaced by an aryl group. Examples of "arylalkyl" or "aralkyl" include benzyl, 2-phenylethyl, 3-phenylpropyl, 9-fluorenyl, benzhydryl, and trityl groups.

As used herein, "cycloalkyl" refers to a cyclic, bicyclic, tricyclic, or polycyclic nonaromatic hydrocarbon groups having 3 to 12 carbons. Examples of cycloalkyl moieties include, but are not limited to, cyclopropyl, cyclopentyl, and cyclohexyl.

As used herein, "heterocyclyl" refers to a monovalent radical of a heterocyclic ring system. Representative heterocyclyls include, without limitation, tetrahydrofuranyl, tetrahydrothienyl, pyrrolidinyl, pyrrolidonyl, piperidinyl, pyrrolinyl, piperazinyl, dioxanyl, dioxolanyl, diazepinyl, oxazepinyl, thiazepinyl, and morpholinyl.

As used herein, "heteroaryl" refers to a monovalent radical of a heteroaromatic ring system. Examples of heteroaryl moieties include, but are not limited to, imidazolyl, oxazolyl, thiazolyl, triazolyl, pyrrolyl, furanyl, indolyl, thiophenyl pyrazolyl, pyridinyl, pyrazinyl, pyridazinyl, pyrimidinyl, indolizinyl, purinyl, naphthyridinyl, quinolyl, and pteridinyl.

### Phosphate Backbone Modifications

### Phosphate Group

In an embodiment, the phosphate group of a modified nucleotide can be modified by replacing one or more of the oxygens with a different substituent. Further, the modified nucleotide, *e.g.,* modified nucleotide present in a modified nucleic acid, can include the wholesale replacement of an unmodified phosphate moiety with a modified phosphate as described herein. In an embodiment, the modification of the phosphate backbone can include alterations that result in either an uncharged linker or a charged linker with unsymmetrical charge distribution.

Examples of modified phosphate groups include, phosphorothioate, phosphoroselenates, borano phosphates, borano phosphate esters, hydrogen phosphonates, phosphoroamidates, alkyl or aryl phosphonates and phosphotriesters. In an embodiment, one of the non-bridging phosphate oxygen atoms in the phosphate backbone moiety can be replaced by any of the following groups: sulfur (S), selenium (Se), BR₃ (wherein R can be, *e.g.,* hydrogen, alkyl, or aryl), C (*e.g.,* an alkyl group, an aryl group, and the like), H, NR₂ (wherein R can be, *e.g.,* hydrogen, alkyl, or aryl), or OR (wherein R can be, *e.g.,* alkyl or aryl). The phosphorous atom in an unmodified phosphate group is achiral. However, replacement of one of the non-bridging oxygens with one of the above atoms or groups of atoms can render the phosphorous atom chiral; that is to say that a phosphorous atom in a phosphate group modified in this way is a stereogenic center. The stereogenic phosphorous atom can possess either the "R" configuration (herein Rp) or the "S" configuration (herein Sp).

Phosphorodithioates have both non-bridging oxygens replaced by sulfur. The phosphorus center in the phosphorodithioates is achiral which precludes the formation of oligoribonucleotide diastereomers. In an embodiment, modifications to one or both non-bridging oxygens can also include the replacement of the non-bridging oxygens with a group independently selected from S, Se, B, C, H, N, and OR (R can be, *e.g.,* alkyl or aryl).

The phosphate linker can also be modified by replacement of a bridging oxygen, (*i.e.,* the oxygen that links the phosphate to the nucleoside), with nitrogen (bridged phosphoroamidates), sulfur (bridged phosphorothioates) and carbon (bridged methylenephosphonates). The replacement can occur at either linking oxygen or at both of the linking oxygens.

### Replacement of the Phosphate Group

The phosphate group can be replaced by non-phosphorus containing connectors. In an embodiment, the charge phosphate group can be replaced by a neutral moiety.

Examples of moieties which can replace the phosphate group can include, without limitation, *e.g.*, methyl phosphonate, hydroxylamino, siloxane, carbonate, carboxymethyl, carbamate, amide, thioether, ethylene oxide linker, sulfonate, sulfonamide, thioformacetal, formacetal, oxime, methyleneimino, methylenemethylimino, methylenehydrazo, methylenedimethylhydrazo and methyleneoxymethylimino.

### Replacement of the Ribophosphate Backbone

Scaffolds that can mimic nucleic acids can also be constructed wherein the phosphate linker and ribose sugar are replaced by nuclease resistant nucleoside or nucleotide surrogates. In an embodiment, the nucleobases can be tethered by a surrogate backbone. Examples can include, without limitation, the morpholino, cyclobutyl, pyrrolidine and peptide nucleic acid (PNA) nucleoside surrogates.

### Sugar Modifications

The modified nucleosides and modified nucleotides can include one or more modifications to the sugar group. For example, the 2' hydroxyl group (OH) can be modified or replaced with a number of different "oxy" or "deoxy" substituents. In an embodiment, modifications to the 2' hydroxyl group can enhance the stability of the nucleic acid since the hydroxyl can no longer be deprotonated to form a 2'-alkoxide ion. The 2'-alkoxide can catalyze degradation by intramolecular nucleophilic attack on the linker phosphorus atom.

Examples of "oxy"-2' hydroxyl group modifications can include alkoxy or aryloxy (OR, wherein "R" can be, *e.g.,* alkyl, cycloalkyl, aryl, aralkyl, heteroaryl or a sugar); polyethyleneglycols (PEG), O(CH₂CH₂O)ₙCH₂CH₂OR wherein R can be, *e.g.,* H or optionally substituted alkyl, and n can be an integer from 0 to 20 (*e.g.,* from 0 to 4, from 0 to 8, from 0 to 10, from 0 to 16, from 1 to 4, from 1 to 8, from 1 to 10, from 1 to 16, from 1 to 20, from 2 to 4, from 2 to 8, from 2 to 10, from 2 to 16, from 2 to 20, from 4 to 8, from 4 to 10, from 4 to 16, and from 4 to 20). In an embodiment, the "oxy"-2' hydroxyl group modification can include "locked" nucleic acids (LNA) in which the 2' hydroxyl can be connected, *e.g.,* by a C₁₋₆ alkylene or C₁₋₆ heteroalkylene bridge, to the 4' carbon of the same ribose sugar, where exemplary bridges can include methylene, propylene, ether, or amino bridges; O-amino (wherein amino can be, *e.g.,* NH₂; alkylamino, dialkylamino, heterocyclyl, arylamino, diarylamino, heteroarylamino, or diheteroarylamino, ethylenediamine, or polyamino) and aminoalkoxy, O(CH₂)ₙ-amino, (wherein amino can be, *e.g.,* NH₂; alkylamino, dialkylamino, heterocyclyl, arylamino, diarylamino, heteroarylamino, or diheteroarylamino, ethylenediamine, or polyamino). In an embodiment, the "oxy"-2' hydroxyl group modification can include the methoxyethyl group (MOE), (OCH₂CH₂OCH₃, *e.g.,* a PEG derivative).

"Deoxy" modifications can include hydrogen (i.e. deoxyribose sugars, *e.g.,* at the overhang portions of partially ds RNA); halo (*e.g.,* bromo, chloro, fluoro, or iodo); amino (wherein amino can be, *e.g.,* NH₂; alkylamino, dialkylamino, heterocyclyl, arylamino, diarylamino, heteroarylamino, diheteroarylamino, or amino acid); NH(CH₂CH₂NH)ₙCH₂CH₂-amino (wherein amino can be, *e.g.,* as described herein), - NHC(O)R (wherein R can be, *e.g.,* alkyl, cycloalkyl, aryl, aralkyl, heteroaryl or sugar), cyano; mercapto; alkyl-thio-alkyl; thioalkoxy; and alkyl, cycloalkyl, aryl, alkenyl and alkynyl, which may be optionally substituted with *e.g.,* an amino as described herein.

The sugar group can also contain one or more carbons that possess the opposite stereochemical configuration than that of the corresponding carbon in ribose. Thus, a modified nucleic acid can include nucleotides containing *e.g.*, arabinose, as the sugar. The nucleotide "monomer" can have an alpha linkage at the 1' position on the sugar, *e.g.,* alpha-nucleosides. The modified nucleic acids can also include "abasic" sugars, which lack a nucleobase at C-1'. These abasic sugars can also be further modified at one or more of the constituent sugar atoms. The modified nucleic acids can also include one or more sugars that are in the L form, *e.g.*, L-nucleosides.

Generally, RNA includes the sugar group ribose, which is a 5-membered ring having an oxygen. Exemplary modified nucleosides and modified nucleotides can include, without limitation, replacement of the oxygen in ribose (*e.g.*, with sulfur (S), selenium (Se), or alkylene, such as, *e.g.,* methylene or ethylene); addition of a double bond (*e.g.,* to replace ribose with cyclopentenyl or cyclohexenyl); ring contraction of ribose (*e.g.,* to form a 4-membered ring of cyclobutane or oxetane); ring expansion of ribose (*e.g.,* to form a 6- or 7-membered ring having an additional carbon or heteroatom, such as for example, anhydrohexitol, altritol, mannitol, cyclohexanyl, cyclohexenyl, and morpholino that also has a phosphoramidate backbone). In an embodiment, the modified nucleotides can include multicyclic forms (*e.g.*, tricyclo; and "unlocked" forms, such as glycol nucleic acid (GNA) (*e.g.,* R-GNA or S-GNA, where ribose is replaced by glycol units attached to phosphodiester bonds), threose nucleic acid (TNA, where ribose is replaced with α-L-threofuranosyl-(3'→2')).

### Modifications on the Nucleobase

The modified nucleosides and modified nucleotides described herein, which can be incorporated into a modified nucleic acid, can include a modified nucleobase. Examples of nucleobases include, but are not limited to, adenine (A), guanine (G), cytosine (C), and uracil (U). These nucleobases can be modified or wholly replaced to provide modified nucleosides and modified nucleotides that can be incorporated into modified nucleic acids. The nucleobase of the nucleotide can be independently selected from a purine, a pyrimidine, a purine or pyrimidine analog. In an embodiment, the nucleobase can include, for example, naturally-occurring and synthetic derivatives of a base.

### Uracil

In an embodiment, the modified nucleobase is a modified uracil. Exemplary nucleobases and nucleosides having a modified uracil include without limitation pseudouridine (ψ), pyridin-4-one ribonucleoside, 5-aza-uridine, 6-aza-uridine, 2-thio-5-aza-uridine, 2-thio-uridine (s2U), 4-thio-uridine (s4U), 4-thio-pseudouridine, 2-thio-pseudouridine, 5-hydroxy-uridine (ho⁵U), 5-aminoallyl-uridine, 5-halo-uridine (*e.g.*, 5-iodo-uridine or 5-bromo-uridine), 3-methyl-uridine (m³U), 5-methoxy-uridine (mo⁵U), uridine 5-oxyacetic acid (cmo⁵U), uridine 5-oxyacetic acid methyl ester (mcmo⁵U), 5-carboxymethyl-uridine (cm⁵U), 1-carboxymethyl-pseudouridine, 5-carboxyhydroxymethyl-uridine (chm⁵U), 5-carboxyhydroxymethyl-uridine methyl ester (mchm⁵U), 5-methoxycarbonylmethyl-uridine (mcm⁵U), 5-methoxycarbonylmethyl-2-thio-uridine (mcm⁵s2U), 5-aminomethyl-2-thio-uridine (nm⁵s2U), 5-methylaminomethyl-uridine (mnm⁵U), 5-methylaminomethyl-2-thio-uridine (mnm⁵s2U), 5-methylaminomethyl-2-seleno-uridine (mnm⁵se²U), 5-carbamoylmethyl-uridine (ncm⁵U), 5-carboxymethylaminomethyl-uridine (cmnm⁵U), 5-carboxymethylaminomethyl-2-thio-uridine (cmnm ⁵s2U), 5-propynyl-uridine, 1-propynyl-pseudouridine, 5-taurinomethyl-uridine (τcm⁵U), 1-taurinomethyl-pseudouridine, 5-taurinomethyl-2-thio-uridine(τm⁵s2U), 1-taurinomethyl-4-thio-pseudouridine, 5-methyl-uridine (m⁵U, *i.e.,* having the nucleobase deoxythymine), 1-methyl-pseudouridine (m¹ψ), 5-methyl-2-thio-uridine (m⁵s2U), 1-methyl-4-thio-pseudouridine (m¹s⁴ψ), 4-thio-1-methyl-pseudouridine, 3-methyl-pseudouridine (m³ψ), 2-thio-1-methyl-pseudouridine, 1-methyl-1-deaza-pseudouridine, 2-thio-1-methyl-1-deaza-pseudouridine, dihydrouridine (D), dihydropseudouridine, 5,6-dihydrouridine, 5-methyl-dihydrouridine (m⁵D), 2-thio-dihydrouridine, 2-thio-dihydropseudouridine, 2-methoxy-uridine, 2-methoxy-4-thio-uridine, 4-methoxy-pseudouridine, 4-methoxy-2-thio-pseudouridine, N1-methyl-pseudouridine, 3-(3-amino-3-carboxypropyl)uridine (acp³U), 1-methyl-3-(3-amino-3-carboxypropyl)pseudouridine (acp³ψ), 5-(isopentenylaminomethyl)uridine (inm⁵U), 5-(isopentenylaminomethyl)-2-thio-uridine (inm⁵s2U), α-thio-uridine, 2'-O-methyl-uridine (Um), 5,2'-O-dimethyl-uridine (m⁵Um), 2'-O-methyl-pseudouridine (ψm), 2-thio-2'-O-methyl-uridine (s2Um), 5-methoxycarbonylmethyl-2'-O-methyl-uridine (mcm ⁵Um), 5-carbamoylmethyl-2'-O-methyl-uridine (ncm ⁵Um), 5-carboxymethylaminomethyl-2'-O-methyl-uridine (cmnm ⁵Um), 3,2'-O-dimethyl-uridine (m Um), 5-(isopentenylaminomethyl)-2'-O-methyl-uridine (inm ⁵Um), 1-thio-uridine, deoxythymidine, 2'-F-ara-uridine, 2'-F-uridine, 2'-OH-ara-uridine, 5-(2-carbomethoxyvinyl) uridine, 5-[3-(1-E-propenylamino)uridine, pyrazolo[3,4-d]pyrimidines, xanthine, and hypoxanthine.

### Cytosine

In an embodiment, the modified nucleobase is a modified cytosine. Exemplary nucleobases and nucleosides having a modified cytosine include without limitation 5-aza-cytidine, 6-aza-cytidine, pseudoisocytidine, 3-methyl-cytidine (m³C), N4-acetyl-cytidine (act), 5-formyl-cytidine (f⁵C), N4-methyl-cytidine (m⁴C), 5-methyl-cytidine (m⁵C), 5-halo-cytidine (*e.g.*, 5-iodo-cytidine), 5-hydroxymethyl-cytidine (hm⁵C), 1-methyl-pseudoisocytidine, pyrrolo-cytidine, pyrrolo-pseudoisocytidine, 2-thio-cytidine (s2C), 2-thio-5-methyl-cytidine, 4-thio-pseudoisocytidine, 4-thio-1-methyl-pseudoisocytidine, 4-thio-1-methyl-1-deaza-pseudoisocytidine, 1-methyl-1-deaza-pseudoisocytidine, zebularine, 5-azazebularine, 5-methyl-zebularine, 5-aza-2-thio-zebularine, 2-thio-zebularine, 2-methoxy-cytidine, 2-methoxy-5-methyl-cytidine, 4-methoxy-pseudoisocytidine, 4-methoxy-1-methyl-pseudoisocytidine, lysidine (k²C), α-thio-cytidine, 2'-O-methyl-cytidine (Cm), 5,2'-O-dimethyl-cytidine (m⁵Cm), N4-acetyl-2'-O-methyl-cytidine (ac⁴Cm), N4,2'-O-dimethyl-cytidine (m⁴Cm), 5-formyl-2'-O-methyl-cytidine (f⁵Cm), N4,N4,2'-O-trimethyl-cytidine (m⁴₂Cm), 1-thio-cytidine, 2'-F-ara-cytidine, 2'-F-cytidine, and 2'-OH-ara-cytidine.

### Adenine

In an embodiment, the modified nucleobase is a modified adenine. Exemplary nucleobases and nucleosides having a modified adenine include without limitation 2-amino-purine, 2,6-diaminopurine, 2-amino-6-halo-purine (*e.g.*, 2-amino-6-chloro-purine), 6-halo-purine (*e.g.*, 6-chloro-purine), 2-amino-6-methyl-purine, 8-azido-adenosine, 7-deaza-adenosine, 7-deaza-8-aza-adenosine, 7-deaza-2-amino-purine, 7-deaza-8-aza-2-amino-purine, 7-deaza-2,6-diaminopurine, 7-deaza-8-aza-2,6-diaminopurine, 1-methyl-adenosine (m¹A), 2-methyl-adenosine (m²A), N6-methyl-adenosine (m⁶A), 2-methylthio-N6-methyl-adenosine (ms2m⁶A), N6-isopentenyl-adenosine (i⁶A), 2-methylthio-N6-isopentenyl-adenosine (ms²i⁶A), N6-(cis-hydroxyisopentenyl)adenosine (io⁶A), 2-methylthio-N6-(cis-hydroxyisopentenyl)adenosine (ms2io⁶A), N6-glycinylcarbamoyl-adenosine (g⁶A), N6-threonylcarbamoyl-adenosine (t⁶A), N6-methyl-N6-threonylcarbamoyl-adenosine (m⁶t⁶A), 2-methylthio-N6-threonylcarbamoyl-adenosine (ms²g⁶A), N6,N6-dimethyl-adenosine (m⁶₂A), N6-hydroxynorvalylcarbamoyl-adenosine (hn⁶A), 2-methylthio-N6-hydroxynorvalylcarbamoyl-adenosine (ms2hn⁶A), N6-acetyl-adenosine (ac⁶A), 7-methyl-adenosine, 2-methylthio-adenosine, 2-methoxy-adenosine, α-thio-adenosine, 2'-O-methyl-adenosine (Am), N⁶,2'-O-dimethyl-adenosine (m⁶Am), N⁶-Methyl-2'-deoxyadenosine, N6,N6,2'-O-trimethyl-adenosine (m⁶₂Am), 1,2'-O-dimethyl-adenosine (m¹Am), 2'-O-ribosyladenosine (phosphate) (Ar(p)), 2-amino-N6-methyl-purine, 1-thio-adenosine, 8-azido-adenosine, 2'-F-ara-adenosine, 2'-F-adenosine, 2'-OH-ara-adenosine, and N6-(19-amino-pentaoxanonadecyl)-adenosine.

### Guanine

In an embodiment, the modified nucleobase is a modified guanine. Exemplary nucleobases and nucleosides having a modified guanine include without limitation inosine (I), 1-methyl-inosine (m¹I), wyosine (imG), methylwyosine (mimG), 4-demethyl-wyosine (imG-14), isowyosine (imG2), wybutosine (yW), peroxywybutosine (o₂yW), hydroxywybutosine (OHyW), undermodified hydroxywybutosine (OHyW*), 7-deaza-guanosine, queuosine (Q), epoxyqueuosine (oQ), galactosyl-queuosine (galQ), mannosyl-queuosine (manQ), 7-cyano-7-deaza-guanosine (preQ₀), 7-aminomethyl-7-deaza-guanosine (preQ₁), archaeosine (G⁺), 7-deaza-8-aza-guanosine, 6-thio-guanosine, 6-thio-7-deaza-guanosine, 6-thio-7-deaza-8-aza-guanosine, 7-methyl-guanosine (m⁷G), 6-thio-7-methyl-guanosine, 7-methyl-inosine, 6-methoxy-guanosine, 1-methyl-guanosine (m'G), N2-methyl-guanosine (m²G), N2,N2-dimethyl-guanosine (m²₂G), N2,7-dimethyl-guanosine (m²,7G), N2, N2,7-dimethyl-guanosine (m²,2,7G), 8-oxo-guanosine, 7-methyl-8-oxo-guanosine, 1-methyl-6- thio-guanosine, N2-methyl-6-thio-guanosine, N2,N2-dimethyl-6-thio-guanosine, α-thio-guanosine, 2'-O-methyl-guanosine (Gm), N2-methyl-2'-O-methyl-guanosine (m²Gm), N2,N2-dimethyl-2'-O-methyl-guanosine (m²₂Gm), 1-methyl-2'-O-methyl-guanosine (m'Gm), N2,7-dimethyl-2'-O-methyl-guanosine (m²,7Gm), 2'-O-methyl-inosine (Im), 1,2'-O-dimethyl-inosine (m'Im), O⁶-phenyl-2'-deoxyinosine, 2'-O-ribosylguanosine (phosphate) (Gr(p)), 1-thio-guanosine, O⁶-methyl-guanosine, O⁶-Methyl-2'-deoxyguanosine, 2'-F-ara-guanosine, and 2'-F-guanosine.

### Exemplary Modified gRNAs

In some embodiments, the modified nucleic acids can be modified gRNAs. It is to be understood that any of the gRNAs described herein can be modified in accordance with this section.

As discussed above, transiently expressed or delivered nucleic acids can be prone to degradation by, *e.g.,* cellular nucleases. Accordingly, in one aspect the modified gRNAs described herein can contain one or more modified nucleosides or nucleotides which introduce stability toward nucleases. While not wishing to be bound by theory it is also believed that certain modified gRNAs described herein can exhibit a reduced innate immune response when introduced into a population of cells, particularly the cells of the present disclosure. As noted above, the term "innate immune response" includes a cellular response to exogenous nucleic acids, including single stranded nucleic acids, generally of viral or bacterial origin, which involves the induction of cytokine expression and release, particularly the interferons, and cell death.

While some of the exemplary modification discussed in this section may be included at any position within the gRNA sequence, in some embodiments, a gRNA molecule comprises a modification at or near its 5' end (*e.g.*, within 1-10, 1-5, or 1-2 nucleotides of its 5' end). In some embodiments, a gRNA comprises a modification at or near its 3' end (*e.g.,* within 1-10, 1-5, or 1-2 nucleotides of its 3' end). In some embodiments, a gRNA molecule comprises both a modification at or near its 5' end and a modification at or near its 3' end.

In an embodiment, the 5' end of a gRNA is modified by the inclusion of a eukaryotic mRNA cap structure or cap analog (*e.g.,* a *G(5')ppp(5')G* cap analog, a *m7G(5')ppp(5')G* cap analog, or a *3'-O-Me-m7G(5')ppp(5')G* anti reverse cap analog (ARCA)). The cap or cap analog can be included during either chemical synthesis or *in vitro* transcription of the gRNA.

In an embodiment, an *in vitro* transcribed gRNA is modified by treatment with a phosphatase (*e.g.*, calf intestinal alkaline phosphatase) to remove the 5' triphosphate group.

In an embodiment, the 3' end of a gRNA is modified by the addition of one or more (*e.g.,* 25-200) adenine (A) residues. The polyA tract can be contained in the nucleic acid (*e.g.,* plasmid, PCR product, viral genome) encoding the gRNA, or can be added to the gRNA during chemical synthesis, or following *in vitro* transcription using a polyadenosine polymerase (*e.g., E. coli* Poly(A)Polymerase).

In another aspect, methods and compositions discussed herein provide methods and compositions for gene editing by using a gRNA molecule which comprises a polyA tail. In one embodiment, a polyA tail of undefined length ranging from 1 to 1000 nucleotide is added enzymatically using a polymerase such as *E. coli* polyA polymerase (E-PAP). In one embodiment, the polyA tail of a specified length (*e.g.,* 1, 5, 10, 20, 30, 40, 50, 60, 100, or 150 nucleotides) is encoded on a DNA template and transcribed with the gRNA via an RNA polymerase (*e.g.,* T7 RNA polymerase). In one embodiment, a polyA tail of defined length (*e.g.,* 1, 5, 10, 20, 30, 40, 50, 60, 100, or 150 nucleotides) is synthesized as a synthetic oligonucleotide and ligated on the 3' end of the gRNA with either an RNA ligase or a DNA ligase with our without a splinted DNA oligonucleotide complementary to the guide RNA and the polyA oligonucleotide. In one embodiment, the entire gRNA including a defined length of polyA tail is made synthetically, in one or several pieces, and ligated together by either an RNA ligase or a DNA ligase with or without a splinted DNA oligonucleotide.

In an embodiment, *in vitro* transcribed gRNA molecule contains both a 5' cap structure or cap analog and a 3' polyA tract. In an embodiment, an *in vitro* transcribed gRNA is modified by treatment with a phosphatase (*e.g.*, calf intestinal alkaline phosphatase) to remove the 5' triphosphate group and comprises a 3' polyA tract.

In some embodiments, gRNAs can be modified at a 3' terminal U ribose. For example, the two terminal hydroxyl groups of the U ribose can be oxidized to aldehyde groups and a concomitant opening of the ribose ring to afford a modified nucleoside as shown below: 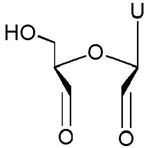 wherein "U" can be an unmodified or modified uridine.

In another embodiment, the 3' terminal U can be modified with a 2'3' cyclic phosphate as shown below: 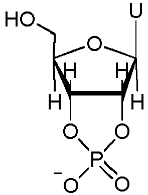 wherein "U" can be an unmodified or modified uridine.

In some embodiments, the gRNA molecules may contain 3' nucleotides which can be stabilized against degradation, *e.g.*, by incorporating one or more of the modified nucleotides described herein. In this embodiment, *e.g.*, uridines can be replaced with modified uridines, *e.g.*, 5-(2-amino)propyl uridine, and 5-bromo uridine, or with any of the modified uridines described herein; adenosines and guanosines can be replaced with modified adenosines and guanosines, *e.g.,* with modifications at the 8-position, *e.g.,* 8-bromo guanosine, or with any of the modified adenosines or guanosines described herein.

In some embodiments, sugar-modified ribonucleotides can be incorporated into the gRNA molecule, *e.g*., wherein the 2' OH-group is replaced by a group selected from H, -OR, -R (wherein R can be, *e.g.,* alkyl, cycloalkyl, aryl, aralkyl, heteroaryl or sugar), halo, -SH, - SR (wherein R can be, *e.g.,* alkyl, cycloalkyl, aryl, aralkyl, heteroaryl or sugar), amino (wherein amino can be, *e.g.,* NH₂; alkylamino, dialkylamino, heterocyclyl, arylamino, diarylamino, heteroarylamino, diheteroarylamino, or amino acid); or cyano (-CN). In some embodiments, the phosphate backbone can be modified as described herein, *e.g.*, with a phosphothioate group. In some embodiments, one or more of the nucleotides of the gRNA can each independently be a modified or unmodified nucleotide including, but not limited to 2'-sugar modified, such as, 2'-O-methyl, 2'-O-methoxyethyl, or 2'-Fluoro modified including, *e.g*., 2'-F or 2'-O-methyl, adenosine (A), 2'-F or 2'-O-methyl, cytidine (C), 2'-F or 2'-O-methyl, uridine (U), 2'-F or 2'-O-methyl, thymidine (T), 2'-F or 2'-O-methyl, guanosine (G), 2'-O-methoxyethyl-5-methyluridine (Teo), 2'-O-methoxyethyladenosine (Aeo), 2'-O-methoxyethyl-5-methylcytidine (m5Ceo), and any combinations thereof.

In some embodiments, a gRNA can include "locked" nucleic acids (LNA) in which the 2' OH-group can be connected, *e.g.,* by a C1-6 alkylene or C1-6 heteroalkylene bridge, to the 4' carbon of the same ribose sugar, where exemplary bridges can include methylene, propylene, ether, or amino bridges; O-amino (wherein amino can be, *e.g.,* NH₂; alkylamino, dialkylamino, heterocyclyl, arylamino, diarylamino, heteroarylamino, or diheteroarylamino, ethylenediamine, or polyamino) and aminoalkoxy or O(CH₂)ₙ-amino (wherein amino can be, *e.g.,* NH₂; alkylamino, dialkylamino, heterocyclyl, arylamino, diarylamino, heteroarylamino, or diheteroarylamino, ethylenediamine, or polyamino).

In some embodiments, a gRNA can include a modified nucleotide which is multicyclic (*e.g.,* tricyclo; and "unlocked" forms, such as glycol nucleic acid (GNA) (*e.g.,* R-GNA or S-GNA, where ribose is replaced by glycol units attached to phosphodiester bonds), or threose nucleic acid (TNA, where ribose is replaced with α-L-threofuranosyl-(3'→2')).

Generally, gRNA molecules include the sugar group ribose, which is a 5-membered ring having an oxygen. Exemplary modified gRNAs can include, without limitation, replacement of the oxygen in ribose (*e.g.*, with sulfur (S), selenium (Se), or alkylene, such as, *e.g.,* methylene or ethylene); addition of a double bond (*e.g.,* to replace ribose with cyclopentenyl or cyclohexenyl); ring contraction of ribose (*e.g.,* to form a 4-membered ring of cyclobutane or oxetane); ring expansion of ribose (*e.g.,* to form a 6- or 7-membered ring having an additional carbon or heteroatom, such as for example, anhydrohexitol, altritol, mannitol, cyclohexanyl, cyclohexenyl, and morpholino that also has a phosphoramidate backbone). Although the majority of sugar analog alterations are localized to the 2' position, other sites are amenable to modification, including the 4' position. In an embodiment, a gRNA comprises a 4'-S, 4'-Se or a 4'-C-aminomethyl-2'-O-Me modification.

In some embodiments, deaza nucleotides, *e.g.*, 7-deaza-adenosine, can be incorporated into the gRNA molecule. In some embodiments, O- and N-alkylated nucleotides, *e.g.*, N6-methyl adenosine, can be incorporated into the gRNA molecule. In some embodiments, one or more or all of the nucleotides in a gRNA molecule are deoxynucleotides.

### miRNA binding sites

microRNAs (or miRNAs) are naturally occurring cellular 19-25 nucleotide long noncoding RNAs. They bind to nucleic acid molecules having an appropriate miRNA binding site, *e.g.,* in the 3' UTR of an mRNA, and down-regulate gene expression. While not wishing to be bound by theory it is believed that this down regulation occurs either by reducing nucleic acid molecule stability or by inhibiting translation. An RNA species disclosed herein, *e.g.,* an mRNA encoding Cas9 can comprise an miRNA binding site, *e.g.,* in its 3'UTR. The miRNA binding site can be selected to promote down regulation of expression is a selected cell type. By way of example, the incorporation of a binding site for miR-122, a microRNA abundant in liver, can inhibit the expression of the gene of interest in the liver.

### EXAMPLES

The following Examples are merely illustrative and are not intended to limit the scope or content of the invention in any way.

### Example 1: Cloning and Initial Screening of gRNA Molecules

The suitability of candidate gRNA molecules can be evaluated as described in this example. Although described for a chimeric gRNA molecule, the approach can also be used to evaluate modular gRNA molecules.

### Cloning gRNAs into Vectors

For each gRNA molecule, a pair of overlapping oligonucleotides is designed and obtained. Oligonucleotides are annealed and ligated into a digested vector backbone containing an upstream U6 promoter and the remaining sequence of a long chimeric gRNA molecule. Plasmid is sequence-verified and prepped to generate sufficient amounts of transfection-quality DNA. Alternate promoters maybe used to drive *in vivo* transcription (*e.g.,* H1 promoter) or for *in vitro* transcription (*e.g*., a T7 promoter).

### Cloning gRNAs in linear dsDNA molecule (STITCHR)

For each gRNA molecule, a single oligonucleotide is designed and obtained. The U6 promoter and the gRNA scaffold (*e.g.*, including everything except the targeting domain, *e.g.,* including sequences derived from the crRNA and tracrRNA, *e.g.*, including a first complementarity domain; a linking domain; a second complementarity domain; a proximal domain; and a tail domain) are separately PCR amplified and purified as dsDNA molecules. The gRNA-specific oligonucleotide is used in a PCR reaction to stitch together the U6 and the gRNA scaffold, linked by the targeting domain specified in the oligonucleotide. Resulting dsDNA molecule (STITCHR product) is purified for transfection. Alternate promoters may be used to drive *in vivo* transcription (*e.g.,* H1 promoter) or for *in vitro* transcription (*e.g.*, T7 promoter). Any gRNA scaffold may be used to create gRNAs compatible with Cas9 molecules from any bacterial species.

### Initial gRNA Screen

Each gRNA to be tested is transfected, along with a plasmid expressing Cas9 and a small amount of a GFP-expressing plasmid into human cells. In preliminary experiments, these cells can be immortalized human cell lines such as 293T, K562 or U2OS. Alternatively, primary human cells may be used. In this case, cells may be relevant to the eventual therapeutic cell target (for example, an erythroid cell). The use of primary cells similar to the potential therapeutic target cell population may provide important information on gene targeting rates in the context of endogenous chromatin and gene expression.

Transfection may be performed using lipid transfection (such as Lipofectamine or Fugene) or by electroporation (such as Lonza Nucleofection). Following transfection, GFP expression can be determined either by fluorescence microscopy or by flow cytometry to confirm consistent and high levels of transfection. These preliminary transfections can comprise different gRNAs and different targeting approaches (17-mers, 20-mers, nuclease, dual-nickase, etc.) to determine which gRNAs/combinations of gRNAs give the greatest activity.

Efficiency of cleavage with each gRNA may be assessed by measuring NHEJ-induced indel formation at the target locus by a T7E1-type assay or by sequencing. Alternatively, other mismatch-sensitive enzymes, such as Cell/Surveyor nuclease, may also be used.

For the T7E1 assay, PCR amplicons are approximately 500-700bp with the intended cut site placed asymmetrically in the amplicon. Following amplification, purification and size-verification of PCR products, DNA is denatured and re-hybridized by heating to 95°C and then slowly cooling. Hybridized PCR products are then digested with T7 Endonuclease I (or other mismatch-sensitive enzyme) which recognizes and cleaves non-perfectly matched DNA. If indels are present in the original template DNA, when the amplicons are denatured and re-annealed, this results in the hybridization of DNA strands harboring different indels and therefore lead to double-stranded DNA that is not perfectly matched. Digestion products may be visualized by gel electrophoresis or by capillary electrophoresis. The fraction of DNA that is cleaved (density of cleavage products divided by the density of cleaved and uncleaved) may be used to estimate a percent NHEJ using the following equation: %NHEJ = (1-(1-fraction cleaved)^{½}). The T7E1 assay is sensitive down to about 2-5% NHEJ.

Sequencing may be used instead of, or in addition to, the T7E1 assay. For Sanger sequencing, purified PCR amplicons are cloned into a plasmid backbone, transformed, miniprepped and sequenced with a single primer. Sanger sequencing may be used for determining the exact nature of indels after determining the NHEJ rate by T7E1.

Sequencing may also be performed using next generation sequencing techniques. When using next generation sequencing, amplicons may be 300-500bp with the intended cut site placed asymmetrically. Following PCR, next generation sequencing adapters and barcodes (for example Illumina multiplex adapters and indexes) may be added to the ends of the amplicon, *e.g.*, for use in high throughput sequencing (for example on an Illumina MiSeq). This method allows for detection of very low NHEJ rates.

### Example 2: Assessment of Gene Targeting by NHEJ

The gRNAs that induce the greatest levels of NHEJ in initial tests can be selected for further evaluation of gene targeting efficiency. In this case, cells are derived from disease subjects and, therefore, harbor the relevant mutation.

Following transfection (usually 2-3 days post-transfection,) genomic DNA may be isolated from a bulk population of transfected cells and PCR may be used to amplify the target region. Following PCR, gene targeting efficiency to generate the desired mutations (either knockout of a target gene or removal of a target sequence motif) may be determined by sequencing. For Sanger sequencing, PCR amplicons may be 500-700 bp long. For next generation sequencing, PCR amplicons may be 300-500 bp long. If the goal is to knockout gene function, sequencing may be used to assess what percent of alleles have undergone NHEJ-induced indels that result in a frameshift or large deletion or insertion that would be expected to destroy gene function. If the goal is to remove a specific sequence motif, sequencing may be used to assess what percent of alleles have undergone NHEJ-induced deletions that span this sequence.

### Example 3: Assessment of Gene Targeting by HDR

The gRNAs that induce the greatest levels of NHEJ in initial tests can be selected for further evaluation of gene targeting efficiency.

Following transfection (usually 2-3 days post-transfection), genomic DNA may be isolated from a bulk population of transfected cells and PCR may be used to amplify the target region. Following PCR, gene targeting efficiency can be determined by several methods.

Determination of gene targeting frequency involves measuring the percentage of alleles that have undergone homologous directed repair (HDR) with the exogenously provided donor template or endogenous genomic donor sequence and which therefore have incorporated the desired correction. If the desired HDR event creates or destroys a restriction enzyme site, the frequency of gene targeting may be determined by a RFLP assay. If no restriction site is created or destroyed, sequencing may be used to determine gene targeting frequency. If a RFLP assay is used, sequencing may still be used to verify the desired HDR event and ensure that no other mutations are present. If an exogenously provided donor template is employed, at least one of the primers is placed in the endogenous gene sequence outside of the region included in the homology arms, which prevents amplification of donor template still present in the cells. Therefore, the length of the homology arms present in the donor template may affect the length of the PCR amplicon. PCR amplicons can either span the entire donor region (both primers placed outside the homology arms) or they can span only part of the donor region and a single junction between donor and endogenous DNA (one internal and one external primer). If the amplicons span less than the entire donor region, two different PCRs should be used to amplify and sequence both the 5' and the 3' junction.

If the PCR amplicon is short (less than 600bp) it is possible to use next generation sequencing. Following PCR, next generation sequencing adapters and barcodes (for example Illumina multiplex adapters and indexes) may be added to the ends of the amplicon, *e.g.,* for use in high throughput sequencing (for example on an Illumina MiSeq). This method allows for detection of very low gene targeting rates.

If the PCR amplicon is too long for next generation sequencing, Sanger sequencing can be performed. For Sanger sequencing, purified PCR amplicons are cloned into a plasmid backbone (for example, TOPO cloned using the LifeTech Zero Blunt^{®} TOPO^{®} cloning kit), transformed, miniprepped and sequenced.

The same or similar assays described above can be used to measure the percentage of alleles that have undergone HDR with endogenous genomic donor sequence and which therefore have incorporated the desired correction.

### Example 4: Repair of Cas9 induced lesions using gRNA

Previous reports have shown that synthetic RNA can be used as a template for HDR in yeast as well as in human cells (see, *e.g.*, Storici 2007, and Shen 2011). More recently, it has been shown that endogenous RNA transcripts can mediate homologous recombination with chromosomal DNA in yeast (Keskin 2014). However, it has not yet been established that sequences associated with gRNAs can participated in HDR-based repair. Therefore, gRNA elongated at the 5' with a stretch of sequence encoding a donor sequence were tested to determine if they could drive gene correction.

As shown in **Fig. 1****,** simply elongating the gRNA with a 179 nucleotide encoding for the donor sequence reduced the efficiency of cutting of the gRNA. Briefly, U2OS cells were nucleofected with gRNA 8 or 15 or with the reciprocal gRNA with an elongation at the 3' with a sequence encoding either the upper strand (+) or the bottom strand (-) of the donor template sequence. In all cases, elongation of the gRNA had a strong effect on the overall cutting efficiency, measured as the total percent of modification using Sanger sequencing after amplifying the locus from Topo clones. Without wishing to be bound by theory, it is believed that the template sequence in the elongated gRNA is able to interact with the targeting domain of the gRNA and/or DNA-binding domains of the Cas9 molecule.

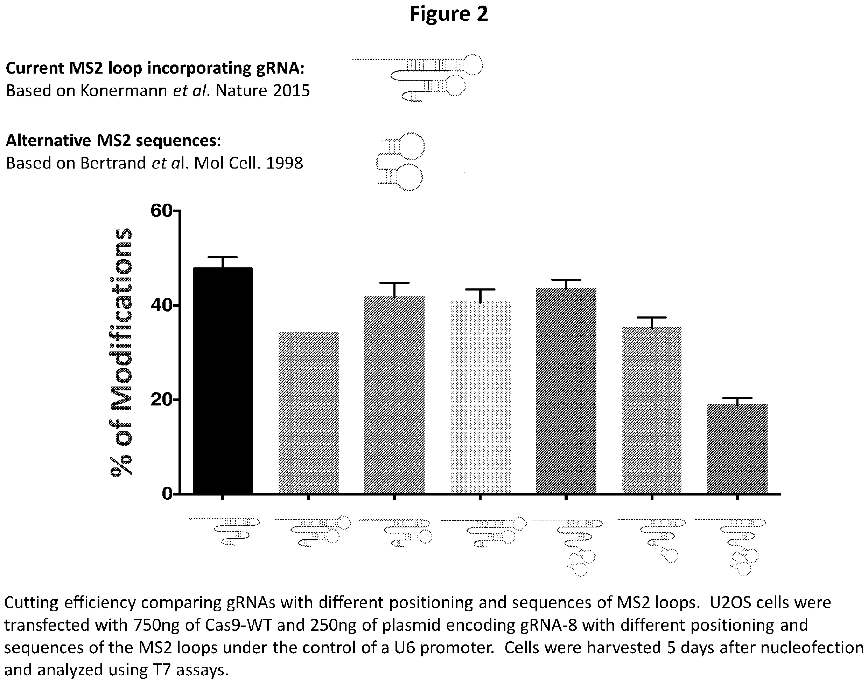

It was hypothesized that the inclusion of a rigid structure between the TRACR portion of the gRNA and the donor sequence would reduce any problematic interactions and improve the cutting efficiency of elongated gRNAs. To test this hypothesis, MS2 hairpin structures were added to the 3' ends of the TRACR portions of the gRNA, as illustrated in **Fig. 2****.** U2OS cells were nucleofected with different gRNA comprising one of two different MS2 loops at different positions and of different composition. MS2 sequences are described, for example, in Konermann 2015, and in Bertrand 1998. Among the structures tested, the MS2 sequence described in Bertrand 1998 resulted in mostly unaltered activity by the gRNA (**Fig. 2**), while other stem loop structures based on Konermann 2015, or based on combinations of stem loop structures described by Bertrand 1998 and Konnerman 2015, exhibited varying levels of activity.

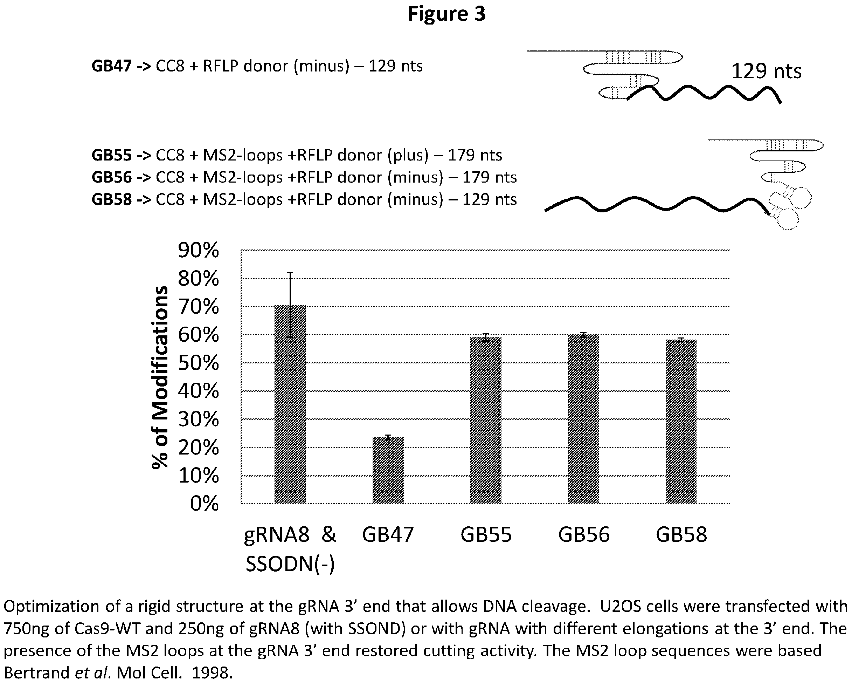

Once it was established that MS2 hairpin structures did not themselves disrupt the cutting activity mediated by the gRNAs, we assessed whether MS2 hairpin structures placed between the 3' end of the TRACR portion of the gRNA and the 5' end of the donor sequence were able to restore cutting activity of elongated gRNAs. As illustrated in **Fig. 3**, we compared edit rates of elongated gRNAs with- and without an MS2 hairpin structure between the TRACR and the donor. The presence of the hairpin between the tracer and the donor (*i.e.,* GB55, GB56, GB58) restored editing to a level comparable to the gRNA with no 3' elongation.

### Effect of gRNA elongation on HDR frequency

To examine the effect of elongated gRNAs on repair of DSBs by HDR, U20S cells were electroporated with 200 ng of gRNA (8) elongated with the donor sequence. Five days after electroporation genomic DNA was extracted, PCR amplification of the *HBB* locus was performed and products were subcloned into a Topo Blunt Vector. For each condition in each experiment colonies were sequenced by Sanger sequencing. **Fig. 4** shows that in cells expressing elongated gRNAs, HDR-mediated repair frequency increased as compared to non-elongated gRNAs.

### Example 5: Covalent Recruitment of a Donor DNA to the gRNA

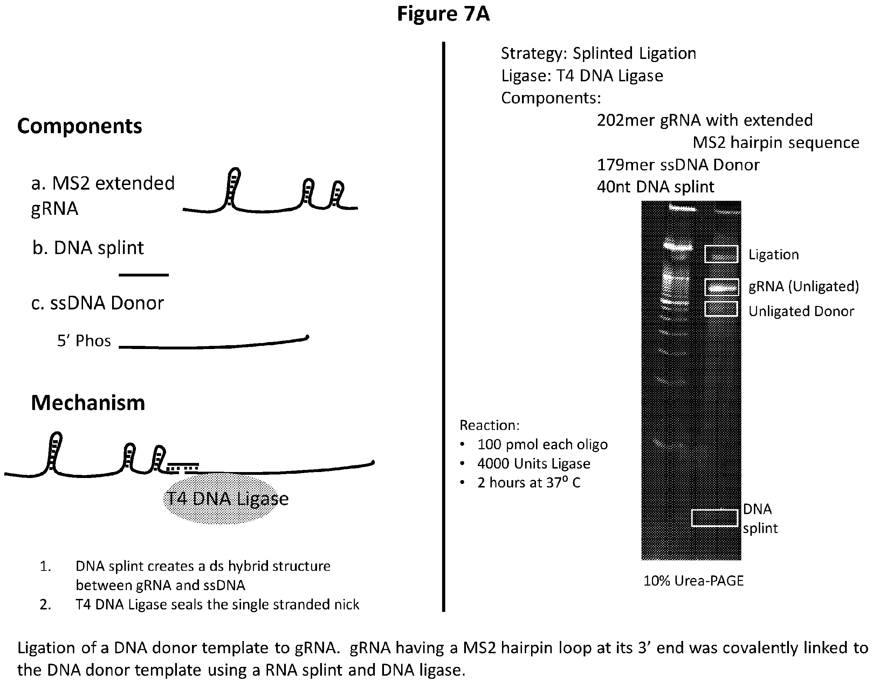

Skilled artisans will readily appreciate that RNA template sequences may not be ideal drivers of HDR, and that it may be preferable, in some instances, to provide a DNA template sequence. In order to provide DNA template sequences linked to gRNAs, single-stranded donor DNA (*i.e.,* template nucleic acid) was ligated to the 3' end of TracrRNA and other gRNA constructs (see, *e.g.,* **Fig. 7A**). Two distinct strategies to acheive this ligation were used: splinted ligation, and adenylated DNA ligation.

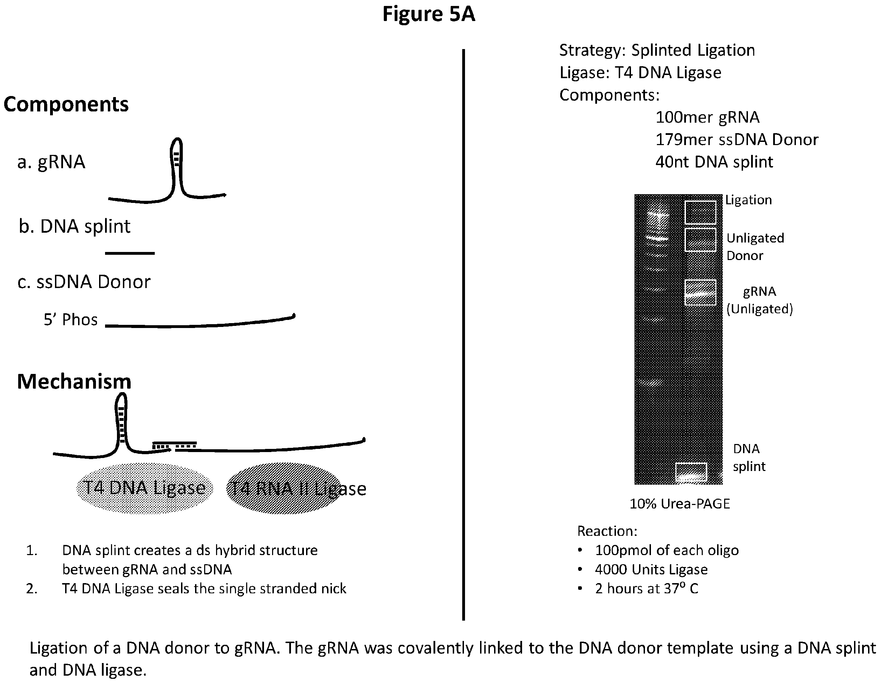

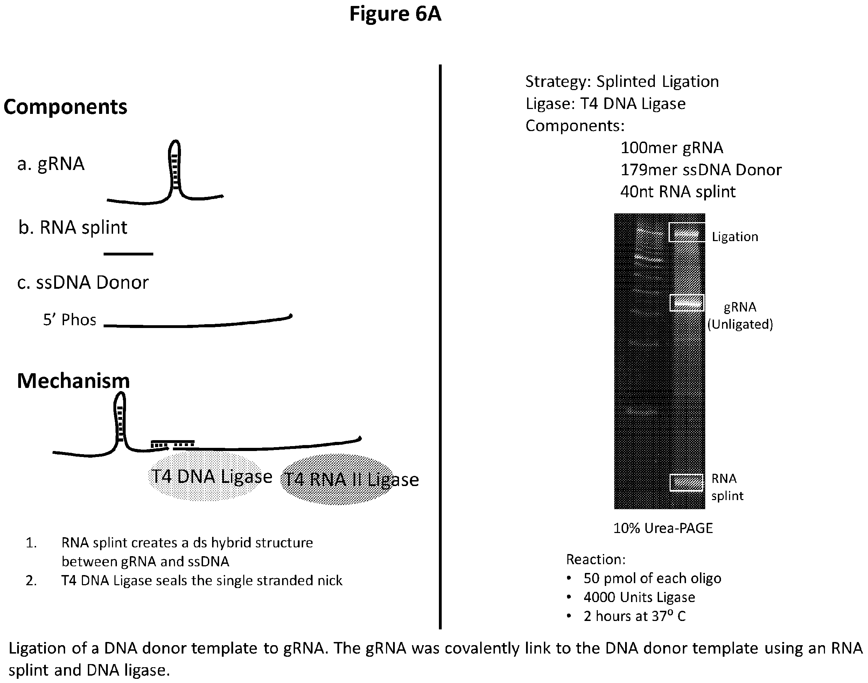

In **Figs. 5A**, **6A** and **7A**, the DNA donor template was ligated to the gRNA using splint ligation. A 40nt DNA (**Fig. 5A**), RNA (**Fig. 6A**), or hybrid (RNA and DNA) oligo splint that is complimentary to the 3' TRACR (20nt base pairing) and the 5' ssDNA donor (20nt base pairing) was used to create a double stranded structure with a single stranded nick between the 3' Tracr and 5' DNA. Once annealed, either T4 DNA Ligase or T4 RNA II Ligase was used to seal the single stranded nick, covalently joining the TRACR RNA to the DNA donor template. Note that 5' phosphorylation of the DNA donor is necessary for ligation to ocurr. The ligation product was purified under denaturing conditions, which removed the splint, leaving a single tranded hybrid species.

Cells can be transfected with these gRNAs modified to be covalently ligated to the donor to target the *HBB* locus. For each condition in each experiment colonies can be sequenced using Sanger sequencing and HDR efficiency measured.

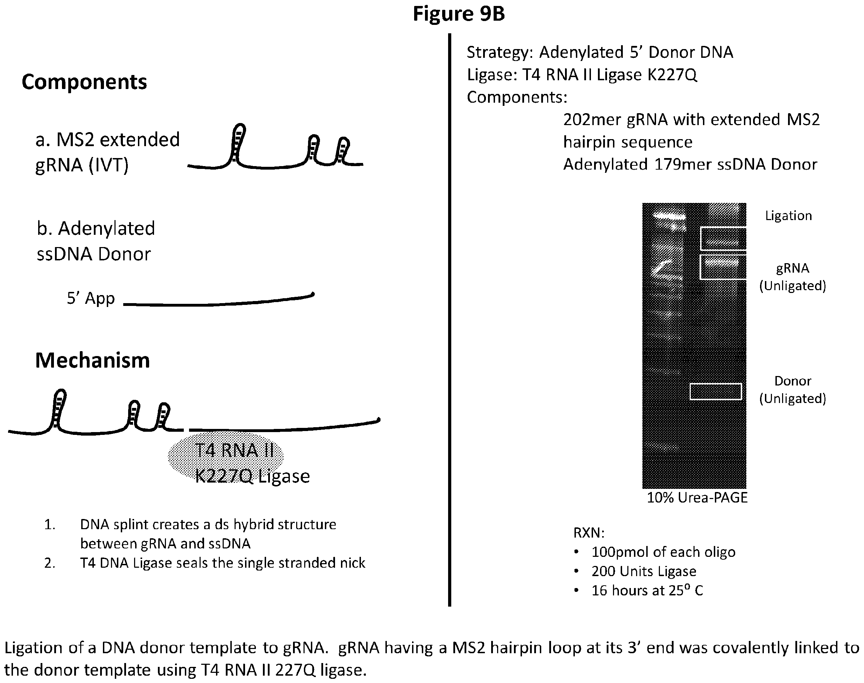

Alternatively, adenylated DNA ligation is used to ligate the donor template to the gRNA, in which the 5' of the donor template is adenylated. In **Fig. 9B****,** the 3' TRACR (with or without an additional linker) was ligated to adenylated 5' DNA donor using T4 RNA II K227Q Truncated Ligase. Although purification is necessary when this method is used, no double stranded RNA or DNA species are formed and hence the purified gRNAs may be more effective.

Cells can be transfected with these gRNAs modified to be covalently ligated to the donor template to target, *e.g.,* the *HBB* locus. For each condition in each experiment colonies can be sequenced using Sanger sequencing and HDR efficiency measured.

The experiments and methods described in Examples 4 and 5 are described in more detail in the Examples below.

### Example 6: Gene Targeting of the HBB Locus by CRISPR/Cas9 Using Elongated gRNAs Comprising Donor Template

To examine whether CRISPR/Cas9 systems comprising an elongated gRNA having a donor template sequence could be used to generate DNA lesions resolved by cellular DNA repair mechanisms, U2OS cells (ATCC #HTB-96) were nucleofected using the Lonza 4D Nucleofector with both 750 ng of a plasmid encoding wild-type *S. pyogenes* Cas9, and 250 ng of either (i) a plasmid encoding gRNA-8, which has the targeting domain sequence GUAACGGCAGACUUCUCCUC (SEQ ID NO: 208), alone, or connected at the 3' end to either a plus strand or minus strand 179 nt donor template, under the control of a U6 promoter, or (ii) a plasmid encoding gRNA-15, which has the targeting domain sequence AAGGUGAACGUGGAUGAAGU (SEQ ID NO: 209), alone, or followed by either a plus strand or minus strand 179 nt donor template, under the control of a U6 promoter. Control cells transfected with plasmids encoding gRNAs without donor template were further nucleofected with 50 pmol of a 179 nt single-stranded deoxynucleotide (ssODN) donor template. Cells were harvested 5 days post-nucleofection and genomic DNA was extracted. PCR amplification of the *HBB* locus was performed and subcloned into a Topo Blunt Vector and sequenced using Sanger sequencing.

As shown in **Fig. 1**, a significant reduction in the overall rate of genetic modification at the HBB locus was observed when using an elongated gRNAs fused at the 3' end to the donor template, as compared the rate of genetic modification observed when using gRNA in combination with a ssODN donor template, not fused to the gRNA.

### Example 7: Design of gRNAs Comprising 3' Hairpin Sequences

To enhance the overall rate of genetic modification resulting from CRISPR/Cas9-generated DNA lesions using elongated gRNAs comprising a 3' donor template, the gRNA portion of the fusion molecule was modified to further comprise a hairpin sequence positioned between the gRNA core sequence and the donor template sequence. Specifically, plasmids were constructed encoding elongated gRNA comprising gRNA-8 followed at the 3' end by two hairpin sequences (which selectively bind dimerized bacteriophage coat protein MS2 in mammalian cells; see Valegard 1997), each comprising a 19-nucleotide RNA stem loop, and either (a) the plus strand of a 179 nt donor template ("GB55"), (b) the minus strand of a 179 nt donor template ("GB56"), or (c) the minus strand of a 129 nt donor template ("GB58"), under the control of a U6 promoter. U2OS cells were nucleofected with both 750 ng of a plasmid encoding wild-type *S. pyogenes* Cas9, and either 250 ng of plasmid encoding one of the above described elongated gRNAs (i.e., GB55, GB56, or GB58), or 250 ng of plasmid encoding an elongated gRNA comprising gRNA-8 directly followed (*i.e.,* without hairpin sequences) by the minus strand of a 129 nt donor template under the control of a U6 promoter, as a control ("GB47"). As a control, U2OS cells were nucleofected with both 750 ng of a plasmid encoding wild-type *S. pyogenes* Cas9, 250 ng of plasmid encoding gRNA-8 and either 50 pmol of a 129 nt minus strand ssODN donor template or no donor template. As shown in **Fig. 3**, the inclusion of the two MS2 hairpin sequences in the elongated gRNAs comprising 3' donor template restored the overall rate of genetic modification resulting from CRISPR/Cas9-generated DNA lesions at the HBB locus, as compared to the rate of genetic modification observed when using gRNA lacking a fused 3' donor template and ssODN donor template. While not wishing to be bound by theory, it is possible that inclusion of 3' hairpin sequences in these elongated gRNAs enhances the cleavage of the target nucleic acid, the stability of the gRNA molecules (*e.g.*, protects them from nuclease degradation), the availability of donor template used by endogenous DNA repair mechanisms, and/or the ability of endogenous DNA repair molecules to resolve the Cas9-generated DNA lesion. The inclusion of hairpin sequences at the 3' end of the gRNA may also prevent the fused template nucleic acid from interfering with gRNA binding to the target sequence.

To investigate the fate of the WT Cas9-generated DNA lesions using elongated gRNAs comprising a hairpin apatmer sequence and a donor template sequence, the frequency of gene conversion and gene correction events resulting after the cleavage event was analyzed. As shown in **Fig. 4**, gene correction events were not observed when DNA lesions were induced using the GB47 elongated gRNA, which lacks the MS2 hairpin sequences but includes a 129 nt donor template. Similarly, gene correction events were not observed when DNA lesions were induced using the GB58 elongated gRNA which comprises MS2 hairpin sequences and a minus strand 129 nt donor template. Surprisingly, CRISPR/Cas9-generated lesions using the GB55 and GB56 elongated gRNAs, which include MS-2 hairpin sequences and the longer 179 nt donor template, resulted in gene correction events (~ 0.8%), irrespective of whether the minus strand donor template (GB56) or plus strand donor template (GB55) was included in the gRNA.

To further examine the effect of 3' hairpin sequence inclusion in gRNAs, the rate of overall genetic modification at the HBB locus was examined using different gRNAs incorporating one or two hairpin sequences at various positions of the gRNA in U2OS cells, as described above.

As shown if **Fig. 2**, the overall rate of genetic modification at the *HBB* locus varied depending on the positioning and sequence of the inserted hairpin sequence.

### Example 8: Methods for Generating gRNAs Covalently Linked to Donor Template Using DNA Splint Ligation with T4 DNA Ligase

To generate gRNAs covalently linked to donor template, DNA splint ligation was performed using T4 DNA ligase, which ligates the 3'-OH end of the gRNA to the 5' end of the ssDNA donor template (see, *e.g.*, Kershaw and O'Keefe 2012). Briefly, a 100mer gRNA was generated by *in vitro* transcription using T7 polymerase. A 40 nt ssDNA splint and a 179 nt ssDNA template were synthesized (Integrated DNA Technologies (IDT)). The DNA splint ligation was performed using 100 pmol of gRNA, 100 pmol of 179 nt ssDNA donor template, 100 pmol of DNA splint, and 4,000 units of T4 DNA ligase, in T4 DNA Ligase Reaction Buffer (50 mM Tris-HCl; 10 mM MgCl₂; 1 mM ATP; 10 mM DTT; pH 7.5). The reaction was incubated at 37 °C for 2 hours, after which the ligation reaction was analyzed by denaturing urea polyacrylamide gel electrophoresis using a 10% polyacrylamide gel. As shown in **Fig. 5A**, the ligation reaction yielded elongated gRNAs wherein the gRNA molecule was successfully ligated to the ssDNA donor template.

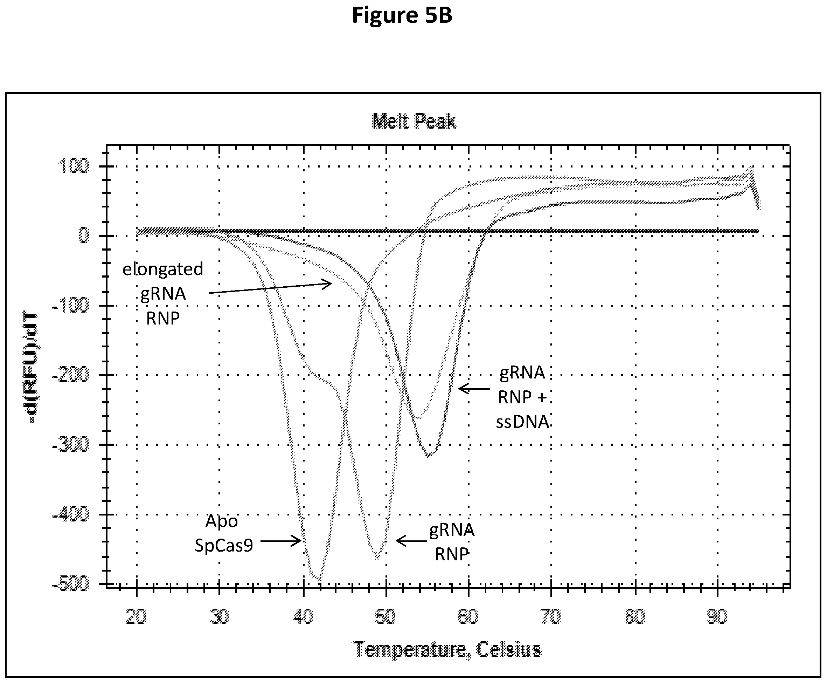

To test the ability of the elongated gRNAs to complex with wild-type *S. pyrogenes* Cas9 (SpCas9), Differential Scanning Fluorimetry (DSF) assay, a thermal shift assay that quantifies the change in the thermal denaturation temperature of Cas9 protein with and without complexing to gRNA, was performed. Briefly, the elongated gRNAs were gel purified, and the quality and quantity of elongated gRNA evaluated with a Bioanalyzer (Nanochip^{®}) to determine RNA concentration. The SpCas9 protein was mixed with either unmodified gRNA, unmodified gRNA and unligated 179 nt ssDNA donor template, or elongated gRNA (*i.e.,* ligated to the 179 nt ssDNA donor template), and allowed to form complexes for 10 minutes. SpCas9 protein and elongated gRNA were mixed at a molar ration of 1:1, and the DSF assay performed as a measure of Cas9 stability and as an indirect measure of gRNA quality, since a 1:1 ratio of gRNA:Cas9 should support a thermal shift if the gRNA is of good quality. As shown in **Fig. 5B****,** apo SpCas9 exhibited a melting temperature of 42 °C, the SpCas9 reaction mixture with unmodified gRNA exhibited a melting temperature of 49 °C, the SpCas9 reaction mixture with unmodified gRNA and unligated 179 nt ssDNA donor template exhibited a melting temperature of 55 °C, and the SpCas9 reaction mixture with elongated gRNA exhibited a melting temperature of 54 °C. The shift in melting temperature of the SpCas9 reaction mixture with elongated gRNA indicates stable RNP complex formation.

### Example 9: Methods for Generating gRNAs Covalently Linked to Donor Template Using RNA Splint Ligation with T4 DNA Ligase

To generate gRNAs covalently linked to donor template, RNA splint ligation was performed using T4 DNA ligase. Briefly, a 100mer gRNA was generated by *in vitro* transcription using T7 polymerase. A 40 nt ssRNA splint and a 179 nt ssDNA template were synthesized (Integrated DNA Technologies (IDT)). The RNA splint ligation was performed using 50 pmol of gRNA, 50 pmol of 179 nt ssDNA donor template, 50 pmol of RNA splint, and 4,000 units of T4 DNA ligase, in T4 DNA Ligase Reaction Buffer (50 mM Tris-HCl; 10 mM MgCl₂; 1 mM ATP; 10 mM DTT; pH 7.5). The reaction was incubated at 37 °C for 2 hours, after which the ligation reaction was analyzed by denaturing urea polyacrylamide gel electrophoresis using a 10% polyacrylamide gel. As shown in **Fig. 6A**, the ligation reaction yielded elongated gRNAs wherein gRNA molecule was succesfully ligated to the ssDNA donor template.

### Example 10: Methods for Generating gRNAs Covalently Linked to Donor Template Using DNA Splint Ligation with T4 DNA Ligase

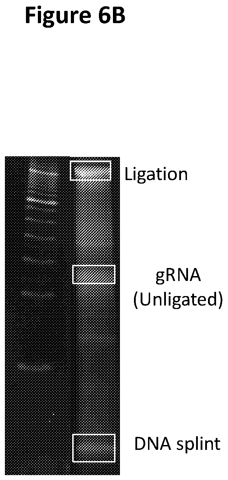

To generate hybrid gRNAs covalently linked to donor template, DNA splint ligation was performed using T4 DNA ligase. Briefly, a hybrid 90mer gRNA comprising a 3' DNA tail, a 40 nt DNA splint, and a 179 nt ssDNA template were synthesized (Integrated DNA Technologies (IDT)). The DNA splint ligation was performed using 50 pmol of hybrid gRNA, 50 pmol of 179 nt ssDNA donor template, 50 pmol of DNA splint, and 4,000 units of T4 DNA ligase, in T4 DNA Ligase Reaction Buffer (50 mM Tris-HCl; 10 mM MgCl₂; 1 mM ATP; 10 mM DTT; pH 7.5). The reaction was incubated at 37 °C for 2 hours, after which the ligation reaction was analyzed by denaturing urea polyacrylamide gel electrophoresis using a 10% polyacrylamide gel. As shown in **Fig. 6B**, the ligation reaction yielded elongated gRNAs wherein the hybrid gRNA molecule was successfully ligated to the ssDNA donor template.

### Example 11: Methods for Generating gRNAs with 3' Hairpins Covalently Linked to Donor Template Using DNA Splint Ligation with T4 DNA Ligase

To generate gRNAs with 3' hairpin sequences covalently linked to donor template, DNA splint ligation was performed using T4 DNA ligase. Briefly, a 202mer gRNA comprising two MS2 hairpin sequences were generated by *in vitro* transcription using T7 polymerase. A 40 nt DNA splint and a 179 nt ssDNA template were synthesized (Integrated DNA Technologies (IDT)). The DNA splint ligation was performed using 100 pmol of gRNA, 100 pmol of 179 nt ssDNA donor template, 100 pmol of DNA splint, and 4,000 units of T4 DNA ligase, in T4 DNA Ligase Reaction Buffer (50 mM Tris-HCl; 10 mM MgCl₂; 1 mM ATP; 10 mM DTT; pH 7.5). The reaction was incubated at 37 °C for 2 hours, after which the ligation reaction was analyzed by denaturing urea polyacrylamide gel electrophoresis using a 10% polyacrylamide gel. As shown in **Fig. 7A**, the ligation reaction yielded elongated gRNAs wherein gRNA molecule with two 3' hairpin sequences was successfully ligated to the ssDNA donor template.

To test the ability of the elongated gRNAs (comprising two 3' hairpins preceding the donor template sequence) to complex with WT *S. pyrogenes* Cas9 (SpCas9), DSF assays were performed. Briefly, the elongated gRNAs were gel purified, and the quality and quantity of elongated gRNA evaluated with a Bioanalyzer (Nanochip^{®}) to determine RNA concentration. SpCas9 protein was mixed with either unmodified gRNA, unmodified gRNA and unligated 179 nt ssDNA donor template, or elongated gRNA (*i.e.,* ligated to a 179 nt ssDNA donor template), and allowed to form complexes for 10 minutes. SpCas9 protein and elongated gRNA were mixed at a molar ration of 1:1, and the DSF assay performed as a measure of Cas9 stability and as an indirect measure of gRNA quality, since a 1:1 ratio of gRNA:Cas9 should support a thermal shift if the gRNA is of good quality. As shown in **Fig. 7B****,** apo SpCas9 exhibited a melting temperature of 39 °C, the SpCas9 reaction mixture with unmodified gRNA exhibited a melting temperature of 47 °C), the SpCas9 reaction mixture with unmodified gRNA and unligated 179 nt ssDNA donor template exhibited a melting temperature of 52 °C, and the SpCas9 reaction mixture with elongated gRNA exhibited a melting temperature of 53 °C. The shift in melting temperature of the SpCas9 reaction mixture with elongated gRNA indicates stable RNP complex formation.

### Example 12: Methods for Generating gRNAs Covalently Linked to Donor Template Using DNA Splint Ligation with T4 RNA Ligase 2

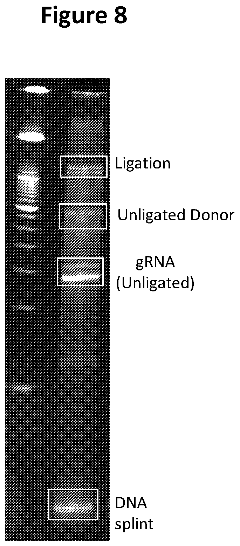

To generate gRNAs covalently linked to donor template, DNA splint ligation was performed using T4 RNA ligase 2 (also known as dsRNA ligase). Briefly, a 100mer gRNA was generated by *in vitro* transcription using T7 polymerase. A 40 nt DNA splint and a 179 nt ssDNA template were synthesized (Integrated DNA Technologies (IDT)). The DNA splint ligation was performed using 100 pmol of gRNA, 100 pmol of 179 nt ssDNA donor template, 100 pmol of DNA splint, and 10 units of T4 RNA ligase 2, in T4 RNA Ligase 2 Reaction Buffer (50 mM Tris-HCl; 2 mM MgCl₂; 400 µM ATP; 1 mM DTT; pH 7.5). The reaction was incubated at 16 °C for 16 hours, after which the ligation reaction was analyzed by denaturing urea polyacrylamide gel electrophoresis using a 10% polyacrylamide gel. As shown in **Fig. 8****,** the ligation reaction yielded elongated gRNAs wherein gRNA molecule was successfully ligated to the ssDNA donor template.

### Example 13: Methods for Generating gRNAs Covalently Linked to Donor Template Using Adenylated Ligation with T4 RNA Ligase 2 - Truncated K227Q

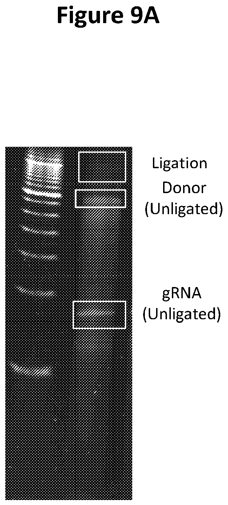

To generate gRNAs covalently linked to donor template, adenylated ligation was performed using T4 RNA ligase 2, truncated K227Q (also known as T4 Rnl2tr K227Q), which specifically ligates the pre-adenylated 5' end of DNA to the 3' end of RNA. Briefly, a 100mer gRNA comprising was generated by *in vitro* transcription using a T7 polymerase. An adenylated 179 nt ssDNA donor template was synthesized (Integrated DNA Technologies (IDT)). The adenylated ligation was performed using 50 pmol of gRNA, 50 pmol of adenylated 179 nt ssDNA donor template, and 200 units of T4 RNA ligase 2, truncated K227Q, in T4 RNA Ligase Reaction Buffer (50mM Tris-HCl; 10mM MgCl₂; 1mM DTT; pH 7.5). The reaction was incubated at 25 °C for 16 hours, after which the ligation reaction was analyzed by denaturing urea polyacrylamide gel electrophoresis using a 10% polyacrylamide gel. As shown in **Fig. 9A**, the ligation reaction yielded elongated gRNAs wherein gRNA molecule was successfully ligated to the adenylated ssDNA donor template.

The same strategy was used to generate gRNAs with 3' hairpin sequences covalently linked to donor template. Briefly, a 202mer gRNA comprising two hairpin sequences encoding MS2-binding sites was generated by *in vitro* transcription using a T7 polymerase. An adenylated 179 nt ssDNA donor template was synthesized (Integrated DNA Technologies (IDT)). The adenylated ligation was performed using 50 pmol of gRNA, 50 pmol of adenylated 179 nt ssDNA donor template, and 200 units of T4 RNA ligase 2, truncated K227Q, in T4 RNA Ligase Reaction Buffer (50mM Tris-HCl; 10mM MgCl₂; 1mM DTT; pH 7.5). The reaction was incubated at 25 °C for 16 hours, after which the ligation reaction was analyzed by denaturing urea polyacrylamide gel electrophoresis using a 10% polyacrylamide gel. As shown in **Fig. 9B**, the ligation reaction yielded elongated gRNAs comprising 3' hairpins wherein gRNA molecule was successfully ligated to the adenylated ssDNA donor template.

To test the ability of the elongated gRNAs (comprising two 3' hairpins preceding the donor template sequence) to complex with WT SpCas9, DSF assays were performed. Briefly, the elongated gRNAs were gel purified, and the quality and quantity of elongated gRNA evaluated with a Bioanalyzer (Nanochip^{®}) to determine RNA concentration. SpCas9 protein was mixed with either unmodified gRNA, unmodified gRNA and unligated 179 nt ssDNA donor template, or elongated gRNA (*i.e.,* ligated to a 179 nt ssDNA donor template), and allowed to form complexes for 10 minutes. SpCas9 protein and elongated gRNA were mixed at a molar ration of 1:1, and the DSF assay performed as a measure of Cas9 stability and as an indirect measure of gRNA quality, since a 1:1 ratio of gRNA:Cas9 should support a thermal shift if the gRNA is of good quality. As shown in **Fig. 9C**, apo SpCas9 exhibited a melting temperature of 42 °C, the SpCas9 reaction mixture with unmodified gRNA exhibited a melting temperature of 48 °C, the SpCas9 reaction mixture with unmodified gRNA and unligated 179 nt ssDNA donor template exhibited a melting temperature of 54 °C, and the SpCas9 reaction mixture with elongated gRNA exhibited a melting temperature of 52 °C. The shift in melting temperature of the SpCas9 reaction mixture with elongated gRNA indicates stable RNP complex formation.

### Example 14: Methods for Generating gRNAs Non-Covalently Linked to Donor Template Using a DNA Splint

To generate gRNAs non-covalently linked to donor template, DNA splints were utilized to hybridize gRNA to ssDNA donor template. Briefly, a hybrid 90mer gRNA comprising a 3' DNA tail, a 40 nt ssDNA splint, and a 179 nt ssDNA template were synthesized (Integrated DNA Technologies (IDT)). DNA splint was annealed to ssDNA template and hybrid gRNA.

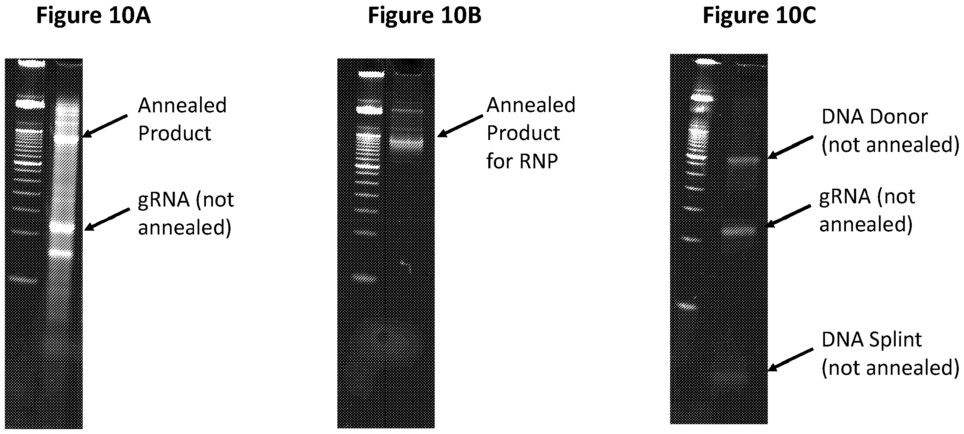

Hybridization of gRNA to the ssDNA donor template via the DNA splint was assessed by polyacrylamide gel electrophoresis using a non-denaturing 10% polyacrylamide gel. As shown in **Fig. 10A**, hybridization of the hybrid gRNA to the ssDNA template using a DNA splint successfully yielded annealed product. Annealed product was isolated and purified from extracted gel slices by electroelution using the Elutrap^{®} electroelution system, according to the manufacturer's directions (see **Fig. 10B**). The composition of purified annealed product was was analyzed by denaturing urea polyacrylamide gel electrophoresis using a 10% polyacrylamide gel, as shown in **Fig. 10C**, which showed that the annealed product was composed of ssDNA donor template, hybrid gRNA and DNA splint.

### References

Anders et al. Nature 513(7519):569-573 (2014)
Bae et al. Bioinformatics 30(10):1473-1475 (2014)
Bertrand et al. Mol. Cell 2: 437-445 (1998)
Caldecott Nat. Rev. Genet. 9(8):619-631 (2008)
Cong et al. Science 399(6121):819-823 (2013)
Chylinski et al. RNA Biol. 10(5):726-737 (2013)
Deveau et al. J. Bacteriol. 190(4): 1390-1400 (2008)
Esvelt et al. Nature 472(7344): 499-503 (2011)
Friedland et al. Genome Biol. 16:257 (2015)
Fu et al. Nat. Biotechnol. 32:279-284 (2014)
Haft et al. PLoS Computational Biology 1(6): e60 (2005)
Heigwer et al. Nat. Methods 11(2): 122-3 (2014)
Horvath et al. Science 3 27(5 962): 167-170 (2010)
Hsu et al. Nat. Biotechnol. 31(9): 827-32 (2013)
Jinek et al. Science 337(6096):816-821 (2012)
Jinek et al. Science 343(6176):1247997 (2014)
Kershaw and O'Keefe Methods Mol. Biol. 941: 257-69 (2012)
Keskin et al. Nature 515(7527): 436-9 (2014).
Kleinstiver et al. Nat. Biotechnol. 33(12):1293-8 (2015)
Konermann et al. Nature 517(7536):583-588 (2015)
Lee et al. Nano Lett. 12(12):6322-6327 (2012)
Li Cell. Res. 18(1):85-98 (2008)
Makarova et al. Nature Review Microbiology 9:467-477 (2011)
Mali et al. Science 399(6121): 823-826 (2013)
Marteijn et al. Nat. Rev. Mol. Cell. Biol. 15(7):465-481 (2014)
Mayle et al. Science 349: 742-47 (2015)
Neelsen and Lopes Nat. Rev. Mol. Cell. Biol. 16: 207-20 (2015)
Nishimasu et al. Cell 156(5):935-949 (2014)
Ran et al. Cell 154(6): 1380-1389 (2013)
Saleh-Gohari et al. Mol. Cell. Biol. 25: 7158-69 (2005)
Schlacher et al. Cancer Cell 22: 106-16 (2012)
Shen et al. Mutat. Res. 717(1-2): 91-8 (2011)
Sternberg et al. Nature 507(7490): 62-67 (2014)
Storici et al. Nature 447(7142): 338-41 (2007)
Wang et al. Cell 153(4):910-918 (2013)
Xiao A. et al.Bioinformatics 30 (8):1180-1182 (2014)
Zellweger et al. J. Cell. Biol. 205: 563-79 (2015)
Zhou et al., Nucleic Acids Res. 42(3):e19 (2014)

## Claims

1. A gRNA fusion molecule, comprising a gRNA molecule and a donor template nucleic acid, wherein the gRNA molecule is covalently linked to the donor template nucleic acid,
wherein the 3' end of the gRNA molecule comprises one or more hairpin loops
and wherein the 3' end of the gRNA molecule is linked to the 5' end of the donor template nucleic acid.

2. The gRNA fusion molecule of claim 1, wherein the donor template nucleic acid comprises single-stranded RNA, single-stranded DNA, or double-stranded DNA.

3. The gRNA fusion molecule of claim 1, wherein the donor template nucleic acid comprises RNA, and wherein the 3' end of the gRNA molecule is linked to the 5' end of the donor template nucleic acid by a phosphodiester bond.

4. The gRNA fusion molecule of claim 1, wherein the gRNA molecule is linked to the donor template nucleic acid by a linker.

5. The gRNA fusion molecule of claim 1, wherein the gRNA fusion molecule exhibits an enhanced efficiency of DNA repair via gene correction pathways, as compared to an unlinked molecule.

6. The gRNA fusion molecule of claim 1, wherein the 3' end of the gRNA molecule comprises 2 hairpin loops, 3 hairpin loops, 4 hairpin loops, or 5 hairpin loops.

7. A gene editing system, comprising the gRNA fusion molecule of claim 1; and at least one Cas9 molecule.

8. A cell comprising the gRNA fusion molecule of claim 1.

9. A cell comprising the gene editing system of claim 7.

10. A nucleic acid molecule that encodes the RNA fusion molecule of claim 1, wherein the gRNA molecule and the donor template nucleic acid are expressed in tandem.

11. A vector comprising the nucleic acid molecule of claim 10.

12. A cell comprising the nucleic acid molecule of claim 10.

13. An *in vitro* method of modifying a target nucleic acid in a cell, the method comprising:
contacting the cell with a Cas9 molecule and the gRNA fusion molecule of claim 1,
wherein the gRNA fusion molecule and the Cas9 molecule associate with the target nucleic acid and generate a double strand break in the target nucleic acid; and
wherein the double strand break in the target nucleic acid is repaired by gene correction using the donor template nucleic acid in the gRNA fusion molecule,
thereby modifying the target nucleic acid in the cell.

## Patentansprüche

1. gRNA-Fusionsmolekül, umfassend ein gRNA-Molekül und eine Donor-Matrizen-Nukleinsäure,
wobei das gRNA-Molekül kovalent mit der Donor-Matrizen-Nukleinsäure verbunden ist,
wobei das 3'-Ende des gRNA-Moleküls eine oder mehrere Haarnadelschleifen umfasst, und
wobei das 3'-Ende des gRNA-Moleküls mit dem 5'-Ende der Donor-Matrizen-Nukleinsäure verbunden ist.

2. gRNA-Fusionsmolekül nach Anspruch 1, wobei die Donor-Matrizen-Nukleinsäure einzelsträngige RNA, einzelsträngige DNA oder doppelsträngige DNA umfasst.

3. gRNA-Fusionsmolekül nach Anspruch 1, wobei die Donor-Matrizen-Nukleinsäure RNA umfasst, und wobei das 3'-Ende des gRNA-Moleküls mit dem 5'-Ende der Donor-Matrizen-Nukleinsäure durch eine Phosphodiester-Bande verbunden ist.

4. gRNA-Fusionsmolekül nach Anspruch 1, wobei das gRNA-Molekül durch einen Linker an die Donor-Matrizen-Nukleinsäure gebunden ist.

5. gRNA-Fusionsmolekül nach Anspruch 1, wobei das gRNA-Fusionsmolekül im Vergleich zu einem ungebundenen Molekül eine erhöhte Effizienz der DNA-Reparatur über Genkorrekturwege aufweist.

6. gRNA-Fusionsmolekül nach Anspruch 1, wobei das 3'-Ende des gRNA-Moleküls 2 Haarnadel schleifen, 3 Haarnadel schleifen, 4 Haarnadelschleifen oder 5 Haarnadelschleifen umfasst.

7. Gen-Editing-System, umfassend das gRNA-Fusionsmolekül nach Anspruch 1 und mindestens ein Cas9-Molekül.

8. Zelle, die das gRNA-Fusionsmolekül nach Anspruch 1 enthält.

9. Zelle, umfassend das Gen-Editiersystem nach Anspruch 7.

10. Nukleinsäuremolekül, das für das RNA-Fusionsmolekül nach Anspruch 1 kodiert, wobei das gRNA-Molekül und die Donor-Template-Nukleinsäure im Tandem exprimiert werden.

11. Vektor, umfassend das Nukleinsäuremolekül nach Anspruch 10.

12. Zelle, umfassend das Nukleinsäuremolekül nach Anspruch 10.

13. In vitro-Verfahren zur Modifizierung einer Zielnukleinsäure in einer Zelle, wobei das Verfahren umfasst:
Inkontaktbringen der Zelle mit einem Cas9-Molekül und dem gRNA-Fusionsmolekül nach Anspruch 1, wobei das gRNA-Fusionsmolekül und das Cas9-Molekül mit der Zielnukleinsäure assoziieren und einen Doppelstrangbruch in der Zielnukleinsäure erzeugen; und
wobei der Doppelstrangbruch in der Zielnukleinsäure durch Genkorrektur unter Verwendung der Donor-Matrizen-Nukleinsäure in dem gRNA-Fusionsmolekül repariert wird, wodurch die Zielnukleinsäure in der Zelle modifiziert wird.

## Revendications

1. Molécule de fusion d'ARNg, comprenant une molécule d'ARNg et un acide nucléique matrice donneur, dans laquelle la molécule d'ARNg est reliée de manière covalente à l'acide nucléique matrice donneur,
dans laquelle l'extrémité 3' de la molécule d'ARNg comprend une ou plusieurs boucles en épingle à cheveux
et dans laquelle l'extrémité 3' de la molécule d'ARNg est reliée à l'extrémité 5' de l'acide nucléique matrice donneur.

2. Molécule de fusion d'ARNg selon la revendication 1, dans laquelle l'acide nucléique matrice donneur comprend de l'ARN simple brin, de l'ADN simple brin ou de l'ADN double brin.

3. Molécule de fusion d'ARNg selon la revendication 1, dans laquelle l'acide nucléique matrice donneur comprend de l'ARN, et dans laquelle l'extrémité 3' de la molécule d'ARNg est reliée à l'extrémité 5' de l'acide nucléique matrice donneur par une liaison phosphodiester.

4. Molécule de fusion d'ARNg selon la revendication 1, dans laquelle la molécule d'ARNg est reliée à l'acide nucléique matrice donneur par un lieur.

5. Molécule de fusion d'ARNg selon la revendication 1, dans laquelle la molécule de fusion d'ARNg présente une efficacité accrue de réparation d'ADN via des voies de correction génique, par rapport à une molécule non reliée.

6. Molécule de fusion d'ARNg selon la revendication 1, dans laquelle l'extrémité 3' de la molécule d'ARNg comprend 2 boucles en épingle à cheveux, 3 boucles en épingle à cheveux, 4 boucles en épingle à cheveux, ou 5 boucles en épingle à cheveux.

7. Système d'édition de gènes, comprenant la molécule de fusion d'ARNg de la revendication 1 ; et au moins une molécule Cas9.

8. Cellule comprenant la molécule de fusion d'ARNg de la revendication 1.

9. Cellule comprenant le système d'édition de gènes de la revendication 7.

10. Molécule d'acide nucléique qui encode la molécule de fusion d'ARN de la revendication 1, dans laquelle la molécule d'ARNg et l'acide nucléique matrice donneur sont exprimés en tandem.

11. Vecteur comprenant la molécule d'acide nucléique de la revendication 10.

12. Cellule comprenant la molécule d'acide nucléique de la revendication 10.

13. Procédé *in vitro* de modification d'un acide nucléique cible dans une cellule, le procédé comprenant :
la mise en contact de la cellule avec une molécule Cas9 et la molécule de fusion d'ARNg de la revendication 1,
dans lequel la molécule de fusion d'ARNg et la molécule Cas9 s'associent à l'acide nucléique cible et génèrent une cassure double brin dans l'acide nucléique cible ; et
dans lequel la cassure double brin dans l'acide nucléique cible est réparée par correction génique à l'aide de l'acide nucléique matrice donneur dans la molécule de fusion d'ARNg,
modifiant de ce fait l'acide nucléique cible dans la cellule.
